# EUROPEAN PATENT APPLICATION

(11) **EP 4 516 321 A1**
(43) Date of publication of application: **05.03.2025**
(21) Application number: 23795143.9
(22) Date of filing: 19.04.2023
(51) Int. Cl.: A61K 47/68, A61P 35/00, C07D 498/14, A61K 31/4353

(54) **ANTIBODY-DRUG CONJUGATE, PHARMACEUTICAL COMPOSITION THEREOF AND USE THEREOF**

(30) Priority: 29.04.2022 CN 202210465896
(71) Applicant: Sichuan Kelun-Biotech Biopharmaceutical Co., Ltd., Chengdu, Sichuan 611138 (CN)
(72) Inventor: LIU, Jinming, Chengdu, Sichuan 611138 (CN); REN, Yun, Chengdu, Sichuan 611138 (CN); HE, Ting, Chengdu, Sichuan 611138 (CN); TIAN, Qiang, Chengdu, Sichuan 611138 (CN); SONG, Hongmei, Chengdu, Sichuan 611138 (CN); GE, Junyou, Chengdu, Sichuan 611138 (CN)
(74) Representative: Meissner Bolte Partnerschaft mbB
(86) International application number: PCT/CN2023/089224
(87) International publication number: WO 2023/207710

(57) **Abstract**

Provided are an antibody-drug conjugate, a pharmaceutical composition thereof and use thereof. Particularly, provided are an antibody-drug conjugate of formula (I), a drug-linker for preparing the antibody-drug conjugate, and use of the antibody-drug conjugate in combating tumors.

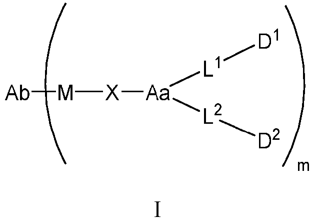

## Description

The present application is based on the application with CN application number of 202210465896.5 and the filing date of April 29, 2022, and claims its priority. The disclosure of the CN application is hereby incorporated into the present application in its entirety.

### Technical Field

The present application belongs to the field of medicine, and specifically relates to an antibody-drug conjugate, pharmaceutical composition thereof and use thereof.

### Background Art

In an antibody-drug conjugate (ADC), an antibody is coupled to a small molecular cytotoxic drug through a specific linker, the antibody serves as a carrier to transport the small molecular cytotoxic drug into a target cell, thereby reducing systemic exposure and improving safety. Thus, it is a hot topic in the research and development of targeted therapy for tumor.

With the development of ADC technology, immune-stimulating antibody conjugate (ISAC) has been developed. ISAC is formed by coupling an immune agonist or immune modulator such as TLR7 and/or TLR8 agonist to an antibody through a cleavable/non-cleavable linker. The immune-stimulatory antibody conjugate combines the precise targeting of the antibody with the immune activation and long-lasting immune memory effects of the immunostimulant, thereby achieving safe systemic administration and inducing anti-tumor immune responses.

In the research of anti-tumor treatment, there is an obvious development trend from single treatment to combination treatment. From the perspective of clinical needs, the ideal combination of drugs should be able to improve clinical efficacy, reduce drug toxicity, and delay or avoid the development of drug resistance. Combination of drugs refers to, on the one hand, the combined use of two existing drugs, such as the combined use of a cytotoxic drug and an immunostimulant, and on the other hand, the combination of two active ingredients to form a new single drug.

With the aid of the targeting properties of antibodies, dural-payload bifunctional immunomodulatory antibody-drug conjugates (iADC) obtained by coupling a cytotoxic drug and an immunostimulant to an antibody can enter into tumor cells and tumor immune microenvironment simultaneously to exert dual effects, i.e., the tumor killing effect of the cytotoxic drug and the local immune activation of the immunostimulant, thereby improving the efficacy of the antibody-drug conjugate in the treatment of cancers.

### Contents of the present invention

In the present invention, a dural-payload bifunctional immunomodulatory antibody-drug conjugate is obtained by coupling a cytotoxic drug and a TLR agonist to an antibody, it can enter into tumor cells and tumor immune microenvironment at the same time to exert dual effects, i.e., the tumor killing effect of the cytotoxic drug and the local immune activation of the TLR agonist, thereby improving cancer treatment effects as compared to an antibody-drug conjugate in which cytotoxic drug alone is coupled.

Specifically, the present invention provides an antibody-drug conjugate of formula (I): 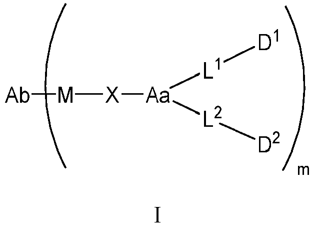 wherein,
Ab represents a targeting moiety;
M represents a linking site connected to the targeting moiety;
X represents a linker connecting M and Aa;
Aa represents an amino acid fragment, or a peptide fragment formed of two or more amino acids;
L¹ represents a covalent bond or a linker connecting Aa and D¹;
L² represents a linker connecting Aa and D²;
D¹ is a cytotoxic drug fragment;
D² is a TLR agonist fragment;
m is selected from 1 to 10, such as 1, 2, 3, 4, 5, 6, 7, 8, 9 or 10.

In some embodiments, Ab is a targeting moiety capable of targeting an antigen of interest, and the targeting moiety targets a cell surface receptor or a tumor surface antigen.

In some embodiments, Ab is an antibody or antigen-binding fragment thereof, wherein the antibody or antigen-binding fragment comprises a monoclonal antibody, a polyclonal antibody, a linear antibody, a bispecific antibody, a multispecific antibody, a chimeric antibody, a murine antibody, a humanized antibody, a fully human antibody, or a fusion protein containing an antigen-binding region of an antibody.

In some embodiments, the Ab is an antibody or antigen-binding fragment thereof, wherein the antigen-binding fragment is selected from the group consisting of Fab, Fab', F(ab')₂, Fv, disulfide bond-linked Fv, and scFv.

In some embodiments, the antibody or antigen-binding fragment thereof is selected from the group consisting of anti-Her-2 antibody, anti-EGFR antibody, anti-VEGFR antibody, anti-PD-L1 antibody, anti-PD-1 antibody, anti-CTLA-4 antibody and anti-Trop-2 antibody.

In some embodiments, the antibody or antigen-binding fragment thereof is Trastuzumab.

In some embodiments, M is a covalent bond or selected from the group consisting of the following structures: 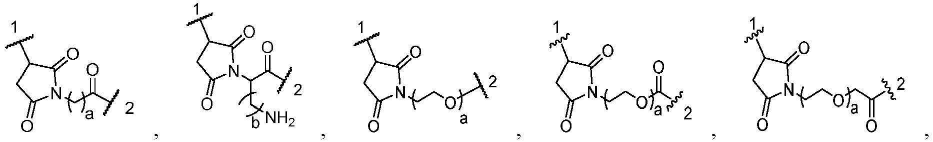 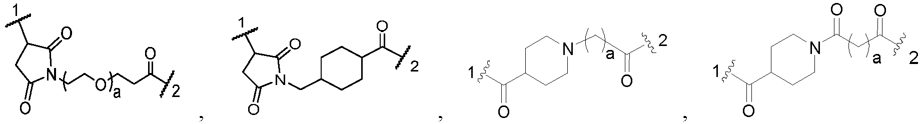 and 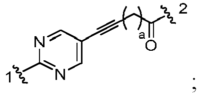 wherein, each a is independently an integer selected from 1 to 5, b is an integer selected from 1 to 3, Position 1 of M is connected to Ab, and Position 2 of M is connected to X.

In some embodiments, M is a covalent bond or selected from the group consisting of the following structures: 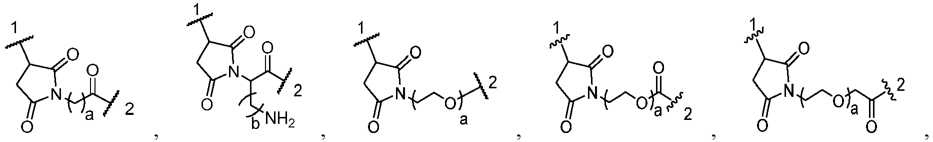 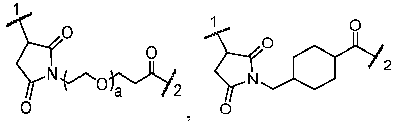 and 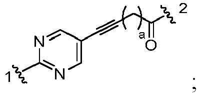 wherein, each a is independently an integer selected from 1 to 5, b is an integer selected from 1 to 3, and Position 1 of M is connected to Ab, and Position 2 of M is connected to X.

In some embodiments, M is a covalent bond or selected from the group consisting of the following structures: 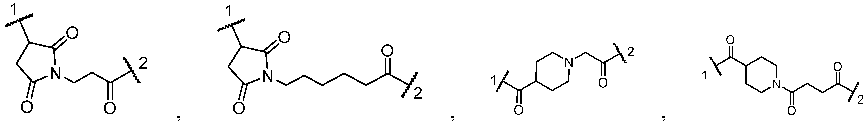 and

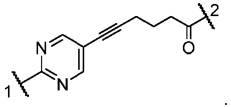

In some embodiments, M is a covalent bond or selected from the group consisting of the following structures: 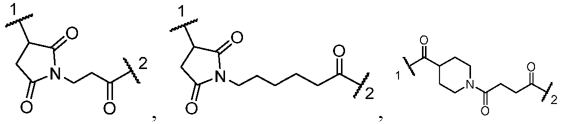 and

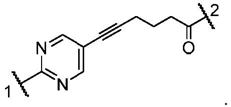

In some embodiments, M is a covalent bond or selected from the group consisting of the following structures: 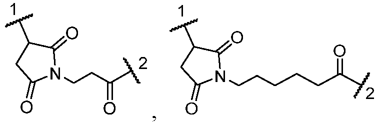 and

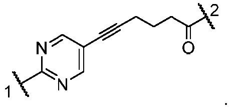

In some embodiments, X is a covalent bond or selected from the group consisting of C₁₋₆ alkylene, -NH-(CH₂)_{c}-C(O)-, 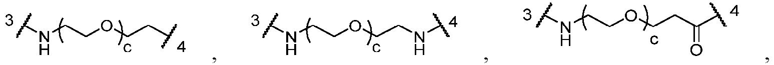 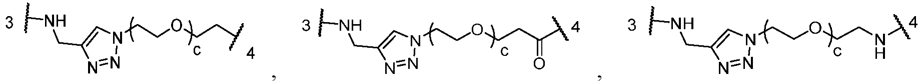 and 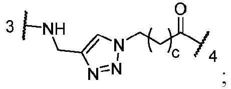 wherein, each c is independently an integer selected from 1 to 10; Position 3 of X is connected to M, and Position 4 of X is connected to Aa.

In some embodiments, X is a covalent bond or 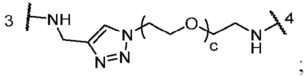 wherein, each c is independently an integer selected from 1 to 10 (e.g., an integer from 5 to 10, e.g., an integer from 8 to 10); Position 3 of X is connected to M, and Position 4 of X is connected to Aa.

In some embodiments, Aa is selected from the group consisting of an amino acid fragment, and a peptide fragment formed of two or more amino acids, wherein the amino acid is selected from the group consisting of Gly, Phe, Ala, Val, Ser, Thr, His, Trp, Cys, Asp, Glu, Lys, Tyr and Arg, optionally, each amino acid is independently modified by C₁₋₆ alkyl or a polyethylene glycol hydrophilic structural unit. In some embodiments, the polyethylene glycol hydrophilic structural unit is selected from the group consisting of 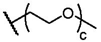 and 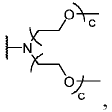 wherein each c is independently an integer selected from 1 to 10 (e.g., an integer from 5 to 10, e.g., an integer from 8 to 10).

In some embodiments, Aa is selected from the group consisting of a fragment of the following amino acids, and a peptide fragment formed of any combination of two or more of them: Gly, Phe, Ala, Val, Cys, Asp, Glu, Lys, and Arg, and each amino acid is independently optionally modified with a C₁₋₆ alkyl group or a polyethylene glycol hydrophilic structural unit. In some embodiments, the polyethylene glycol hydrophilic structural unit is selected from the group consisting of 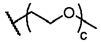 and 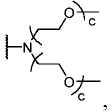 wherein each c is independently an integer selected from 1 to 10 (e.g., an integer from 5 to 10, e.g., an integer from 8 to 10).

In some embodiments, Aa is selected from the group consisting of a fragment of the following amino acids, and a peptide fragment formed of any combination of two or more of them: Val, Cys, Asp, Glu and Lys, wherein the carboxy-terminus of Glu and Lys are optionally modified with 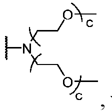 wherein each c is independently an integer selected from 1 to 10 (e.g., an integer from 5 to 10, e.g., an integer from 8 to 10). In some embodiments, Aa is selected from the group consisting of the following structures: 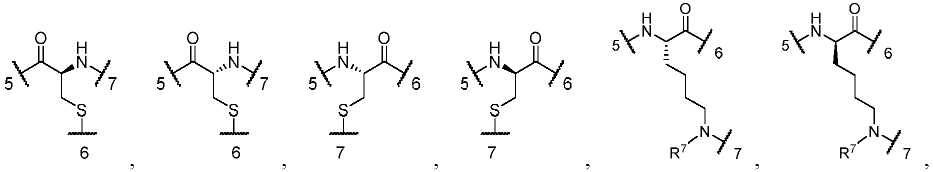 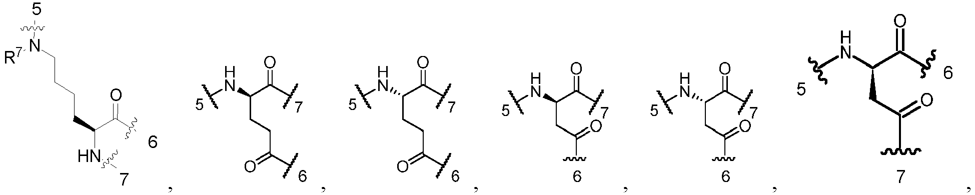 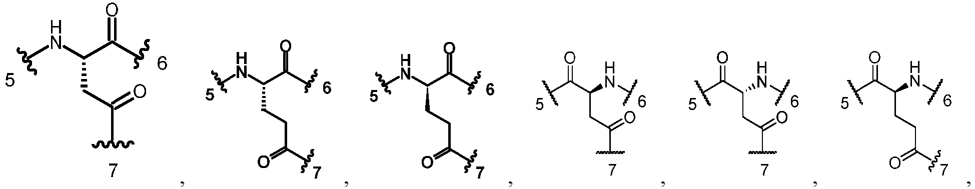 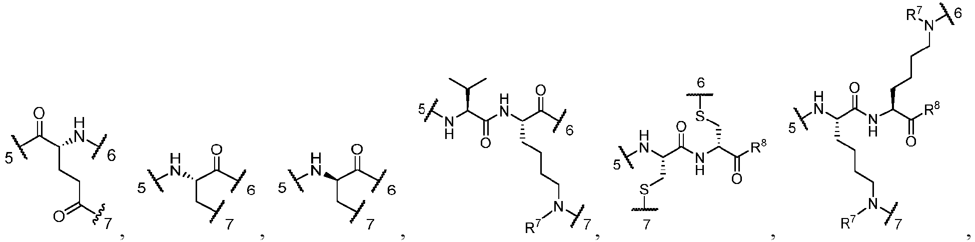 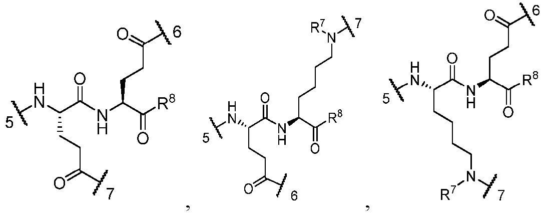 and 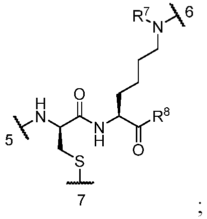 wherein, each R⁷ is independently selected from the group consisting of H, C₁₋₆ alkyl and a polyethylene glycol hydrophilic structural unit; each R⁸ is independently selected from the group consisting of hydroxyl and a polyethylene glycol hydrophilic structural unit; Position 5 is connected to X, Position 6 is connected to one of L¹ and L², and Position 7 is connected to the other of L¹ and L².

In some embodiments, Position 5 is connected to X, Position 6 is connected to L¹, and Position 7 is connected to L².

In some embodiments, Position 5 is connected to X, Position 6 is connected to L², and Position 7 is connected to L¹.

In some embodiments, the polyethylene glycol hydrophilic structural unit is selected from the group consisting of 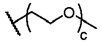 and 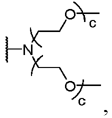 wherein each c is independently an integer selected from 1 to 10 (e.g., an integer from 5 to 10, e.g., an integer from 8 to 10).

In some embodiments, R⁷ is H.

In some embodiments, Aa is selected from the group consisting of the following structures: 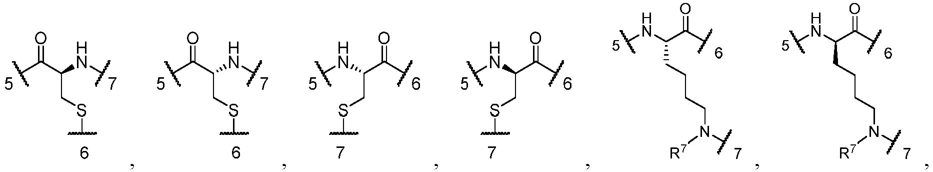 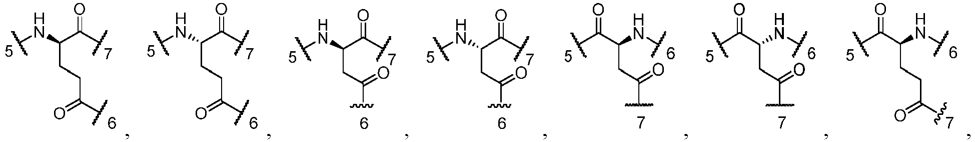 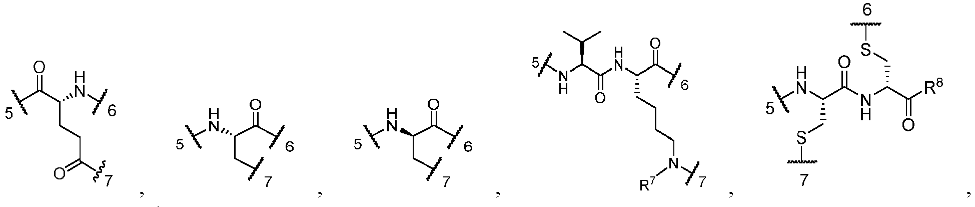 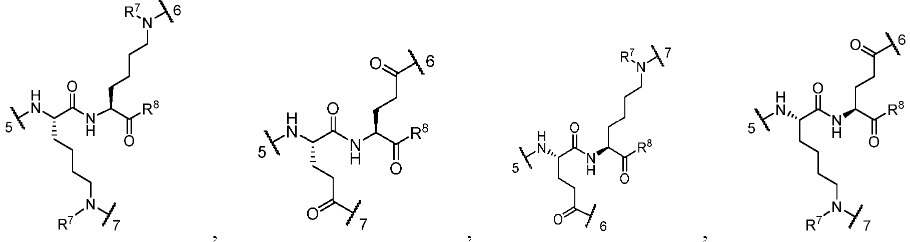 and 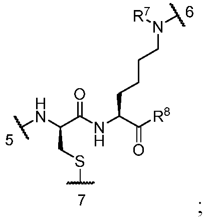 wherein, each R⁷ is independently selected from the group consisting of H, C₁₋₆ alkyl and a polyethylene glycol hydrophilic structural unit; each R⁸ is independently selected from the group consisting of hydroxyl and a polyethylene glycol hydrophilic structural unit; Position 5 is connected to X, Position 6 is connected to one of L¹ and L², and Position 7 is connected to the other of L¹ and L²; preferably, Position 5 is connected to X, Position 6 is connected to L¹, and Position 7 is connected to L².

In some embodiments, the polyethylene glycol hydrophilic structural unit is selected from the group consisting of 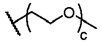 and 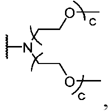 wherein each c is independently an integer selected from 1 to 10 (e.g., an integer from 5 to 10, e.g., an integer from 8 to 10).

In some embodiments, L¹ is a covalent bond or a non-cleavable linker or cleavable linker, wherein the cleavable linker is capable of being cleaved by an enzyme present in a pathological environment, and the enzyme is selected from the group consisting of protease, phosphatase, pyrophosphatase, β-glucuronidase, β-galactosidase and sulfatase.

In some embodiments, L¹ is a covalent bond or -L^{a}-L^{b}-L^{c}-, wherein:
L^{a} is a covalent bond or selected from the group consisting of C₁₋₆ alkylene, -NH-(CH₂)_{c}-C(O)-, 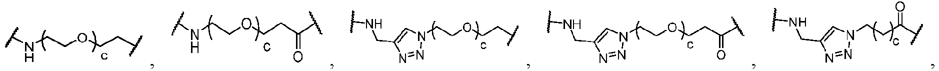 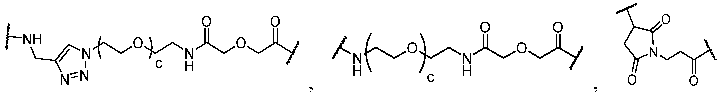 and 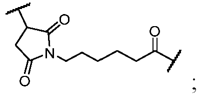 wherein, each c is independently an integer selected from 1 to 10;
L^{b} is a covalent bond or selected from the group consisting of an amino acid fragment, and a peptide fragment formed of two or more amino acids, wherein the amino acid is selected from the group consisting of Val, Cit, Glu, Lys, Arg, Phe, Leu, Gly, Ala and Asn;
L^{c} is a covalent bond, -NH-CH₂- or selected from the group consisting of the following structures: 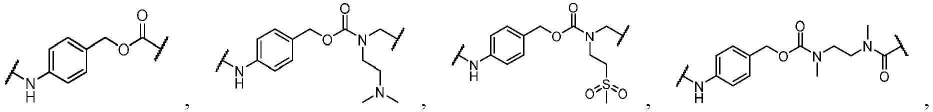 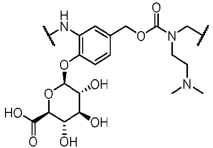 and

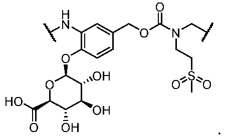

In some embodiments, L¹ is a covalent bond or -L^{a}-L^{b}-L^{c}-, wherein:
L^{a} is a covalent bond or selected from the group consisting of C₁₋₆ alkylene, -NH-(CH₂)_{c}-C(O)-, 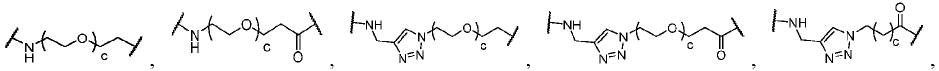 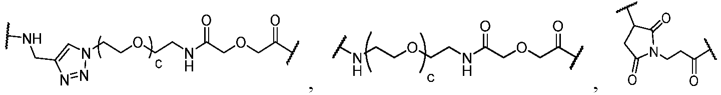 and 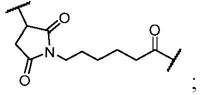 wherein, each c is independently an integer selected from 1 to 10;
L^{b} is a covalent bond or selected from the group consisting of an amino acid fragment, and a peptide fragment formed of two or more amino acids, wherein the amino acid is selected from the group consisting of Val, Cit, Glu, Lys, Arg, Phe, Leu, Gly, Ala and Asn;
L^{c} is a covalent bond, -NH-CH₂- or selected from the group consisting of the following structures: 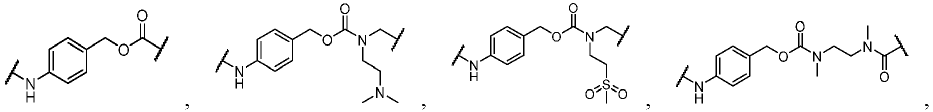 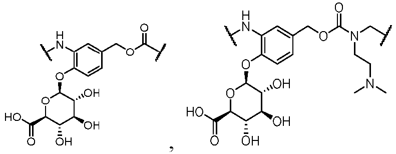 and

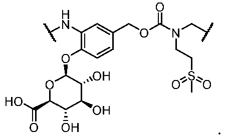

In some embodiments, L^{a} is a covalent bond or is selected from the group consisting of 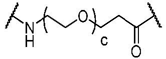 and 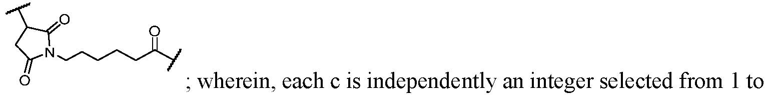 10.

In some embodiments, L^{b} is a covalent bond or is selected from the group consisting of the following structures: 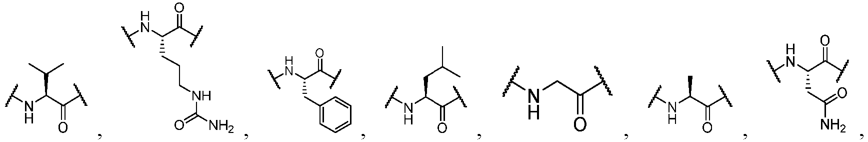 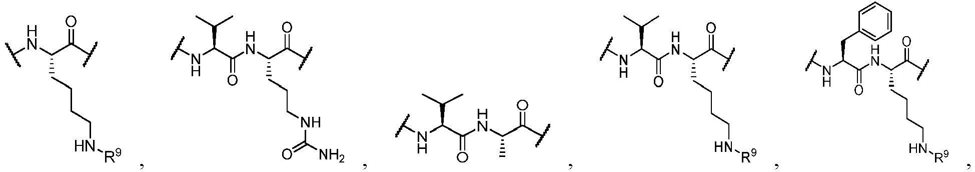 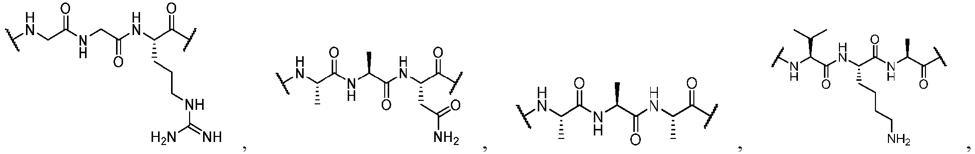 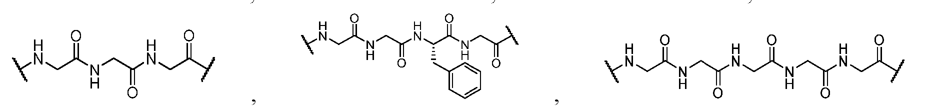 and 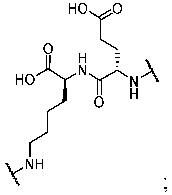 wherein, R⁹ is selected from the group consisting of H, acetyl, fluorenyloxycarbonyl, trityl and a polyethylene glycol hydrophilic structural unit. In some embodiments, the polyethylene glycol hydrophilic structural unit is 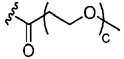 or 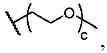 wherein each c is independently an integer selected from 1 to 10.

In some embodiments, L^{c} is -NH-CH₂- or

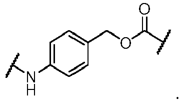

In some embodiments, L¹ is a covalent bond or -L^{a}-L^{b}-L^{c}-, wherein:
L^{a} is a covalent bond or selected from the group consisting of 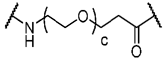 and 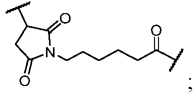 wherein, each c is independently an integer selected from 1 to 10;
L^{b} is a covalent bond or selected from the group consisting of 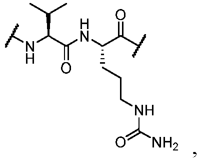 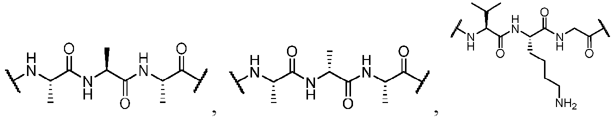 and 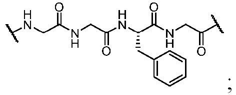
L^{c} is -NH-CH₂- or

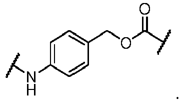

In some embodiments, L¹ is a covalent bond or selected from the group consisting of the following structures: 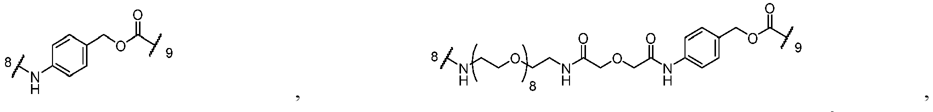 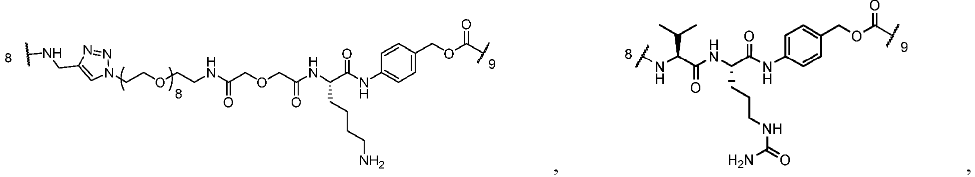 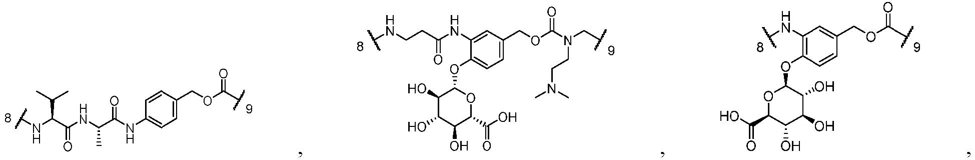 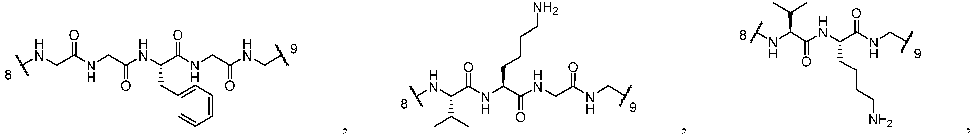 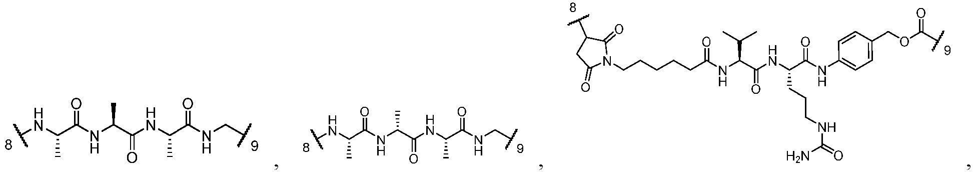 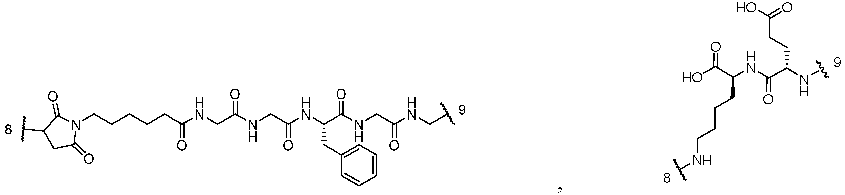 and 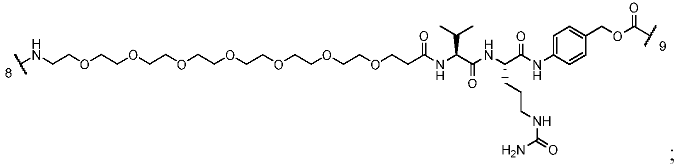 wherein, Position 8 is connected to Aa, and Position 9 is connected to D¹.

In some embodiments, L¹ is a covalent bond or selected from the group consisting of the following structures: 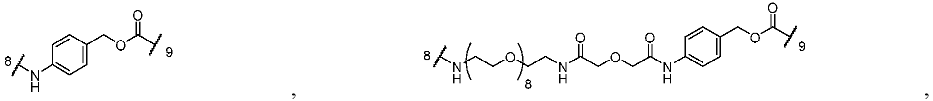 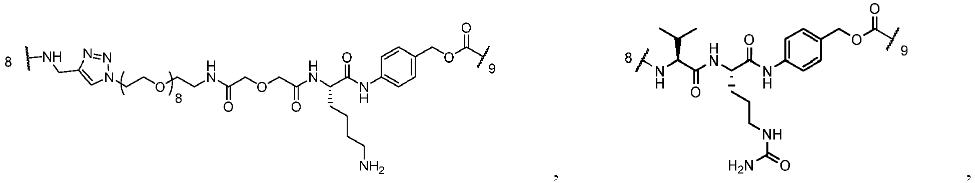 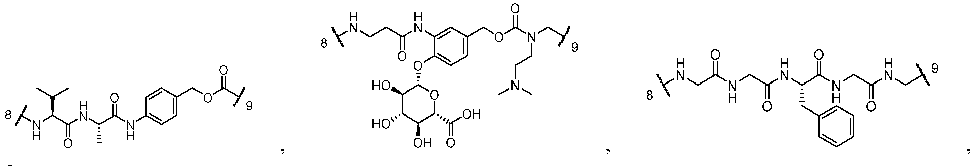 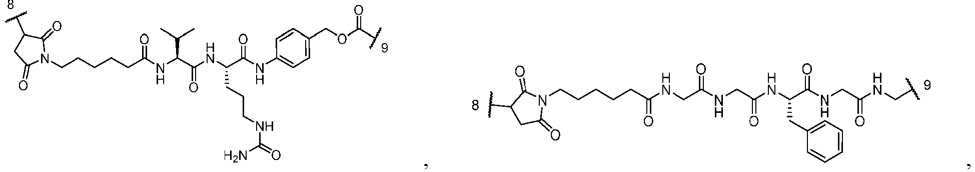 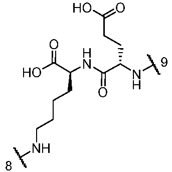 and 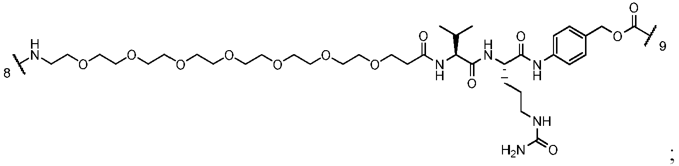 wherein, Position 8 is connected to Aa, and Position 9 is connected to D¹.

In some embodiments, L¹ is selected from the group consisting of: 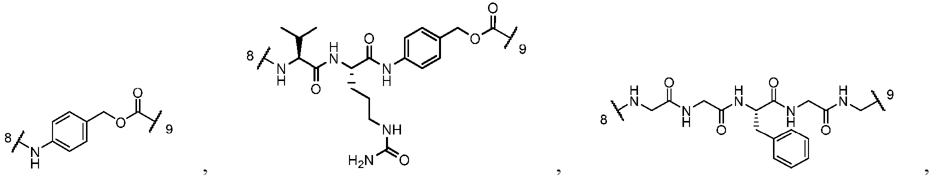 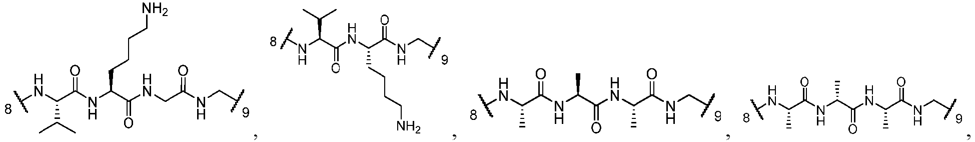 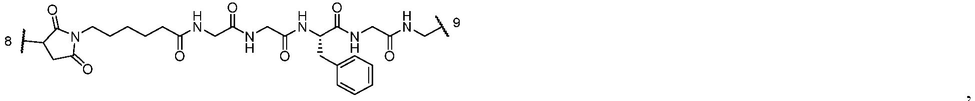 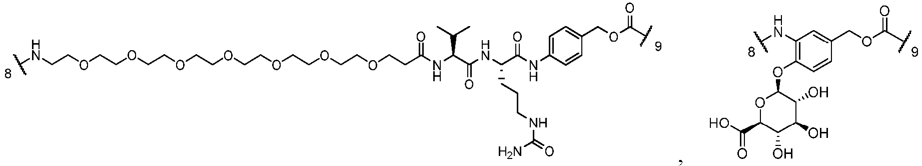 and 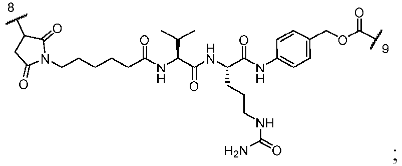 wherein, Position 8 is connected to Aa, and Position 9 is connected to D¹.

In some embodiments, L¹ is selected from the group consisting of: 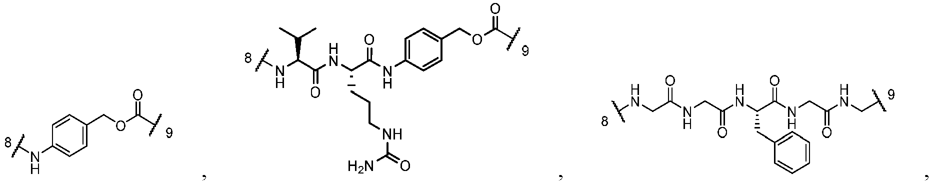 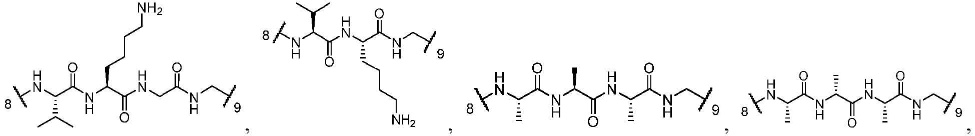 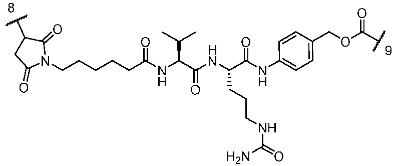 and 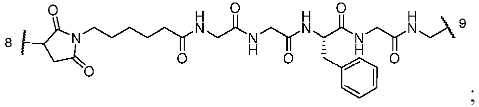 wherein, Position 8 is connected to Aa, and Position 9 is connected to D¹.

In some embodiments, L¹ is: 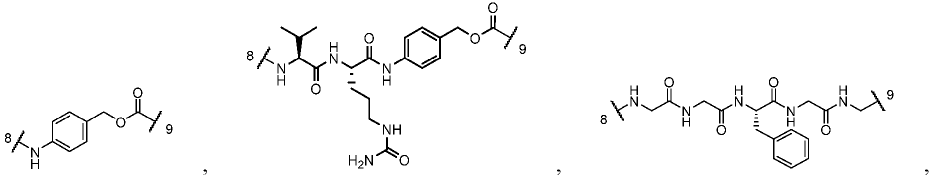 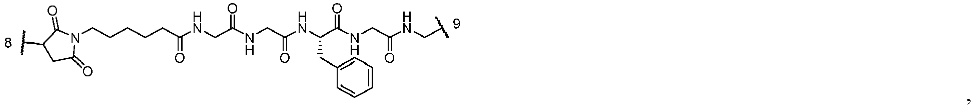 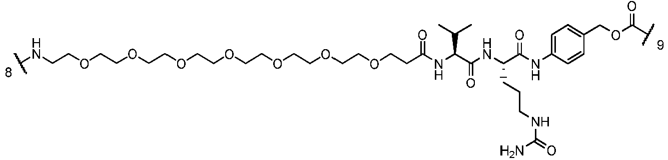 and 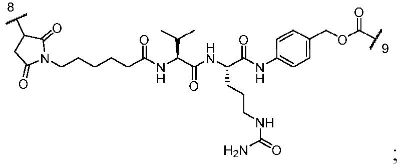 wherein, Position 8 is connected to Aa, and Position 9 is connected to D¹.

In some embodiments, L² is a non-cleavable linker or a cleavable linker, wherein the cleavable linker is capable of being cleaved by an enzyme present in a pathological environment, and the enzyme is selected from the group consisting of protease, phosphatase, pyrophosphatase, *β*-glucuronidase, *β*-galactosidase and sulfatase.

In some embodiments, L² is -L^{d}-L^{e}-L^{f}-, wherein:
L^{d} is a covalent bond or a divalent structure consisting of one or more selected from the following: C₁₋₆ alkylene, -C(O)-(CH₂)_{d}-NH-, 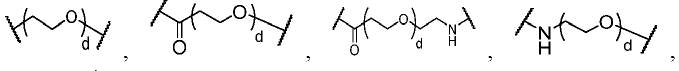 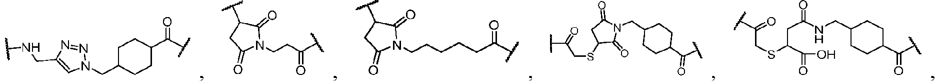 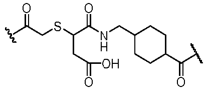 and 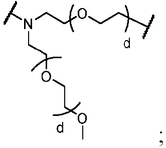 wherein, each d is independently an integer selected from 1 to 12;
L^{e} is a covalent bond or selected from the group consisting of an amino acid fragment, and a peptide fragment formed of two or more amino acids, wherein the amino acid is selected from the group consisting of Val, Cit, Glu, Lys, Arg, Phe, Leu, Gly, Ala and Asn;
L^{f} is a covalent bond, -CH₂-NH- or selected from the group consisting of the following structures: 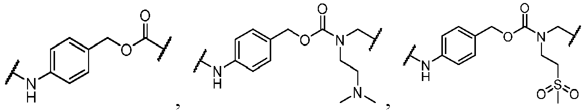 and

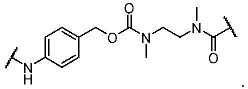

In some embodiments, L² is -L^{d}-L^{e}-L^{f}-, wherein:
L^{d} is a covalent bond or a divalent structure consisting of one or more selected from the following: C₁₋₆ alkylene, -C(O)-(CH₂)_{d}-NH-, 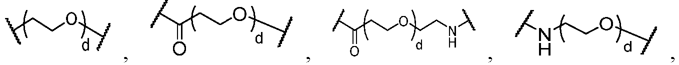 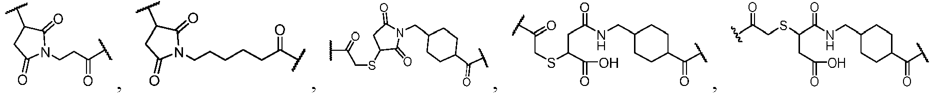 and 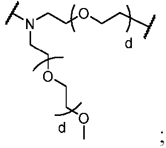 wherein, each d is independently an integer selected from 1 to 12;
L^{e} is a covalent bond or selected from the group consisting of an amino acid fragment, and a peptide fragment formed of two or more amino acids, wherein the amino acid is selected from the group consisting of Val, Cit, Glu, Lys, Arg, Phe, Leu, Gly, Ala and Asn;
L^{f} is a covalent bond, -CH₂-NH- or selected from the group consisting of the following structures:

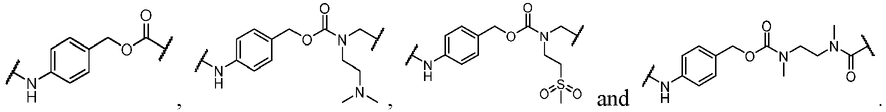

In some embodiments, L^{e} is a covalent bond or selected from the group consisting of the following structures: 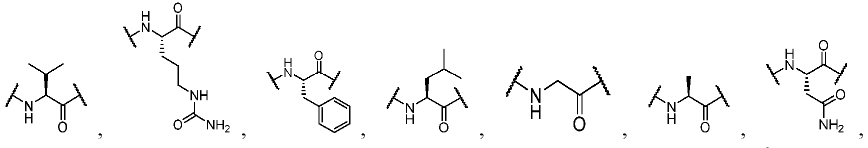 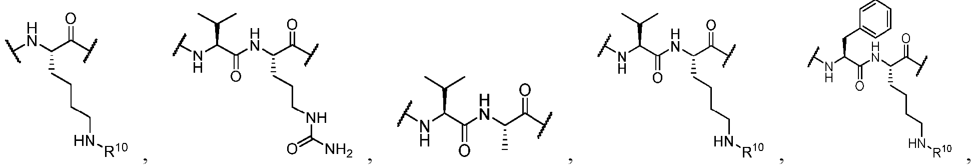 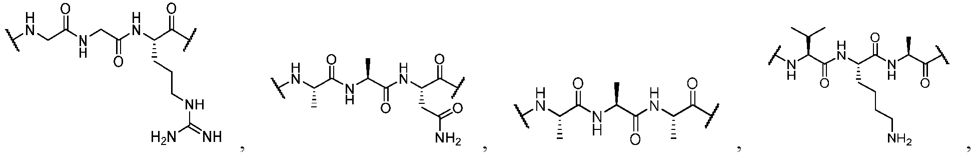 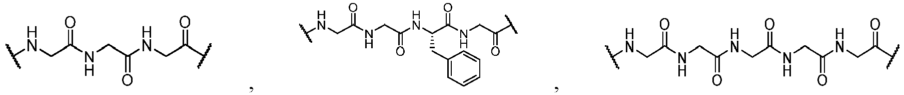 and 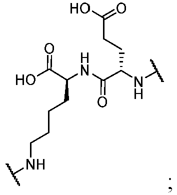 wherein, each R¹⁰ is independently selected from the group consisting of H, acetyl, fluorenyloxycarbonyl, trityl and a polyethylene glycol hydrophilic structural unit; preferably, the polyethylene glycol hydrophilic structural unit is 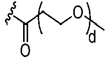 or 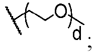 each d is independently an integer selected from 1 to 10.

In some embodiments, L² is selected from the group consisting of the following structures: 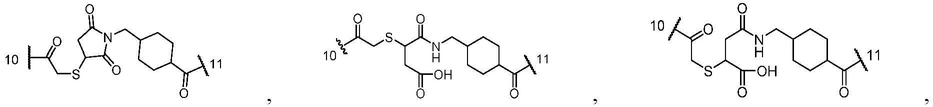 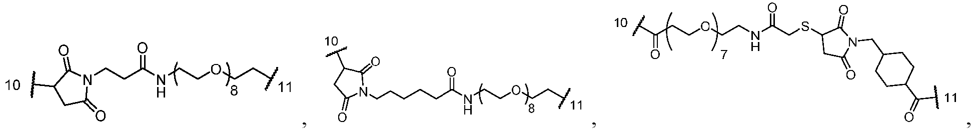 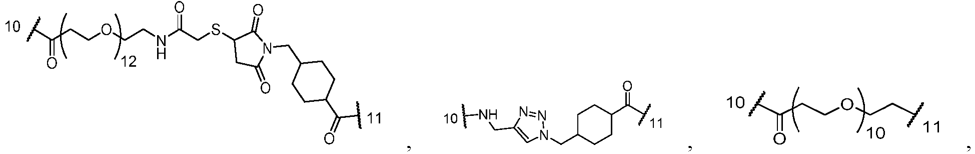 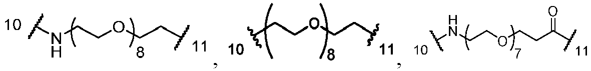 and 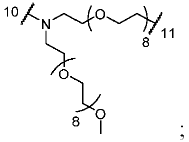 wherein, Position 10 is connected to Aa, and Position 11 is connected to D².

In some embodiments, L² is selected from the group consisting of the following structures: 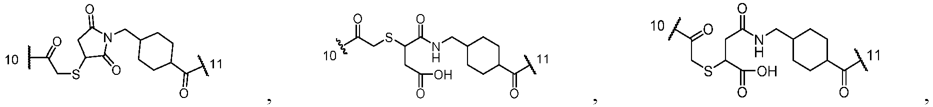 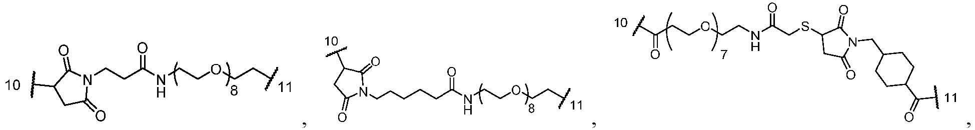 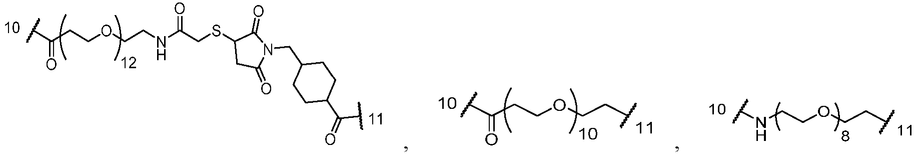 and 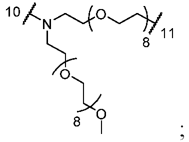 wherein, Position 10 is connected to Aa, and Position 11 is connected to D²;

In some embodiments, L² is selected from the group consisting of the following structures: 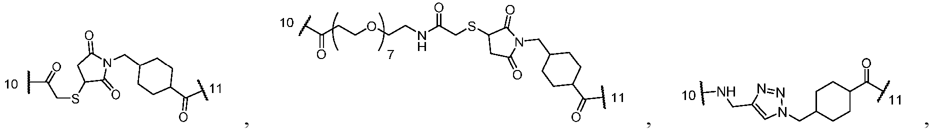 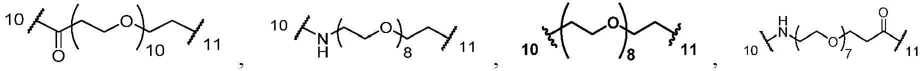 and 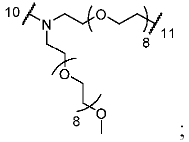 wherein, Position 10 is connected to Aa, and Position 11 is connected to D².

In some embodiments, L² is: 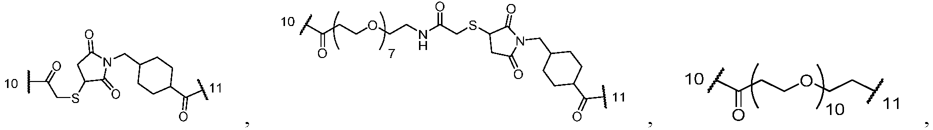 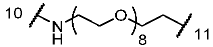 and 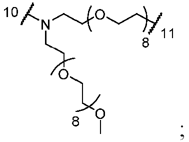 wherein, Position 10 is connected to Aa, and Position 11 is connected to D².

In some embodiments, L² is: 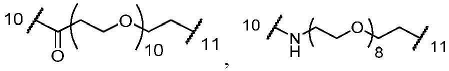 and 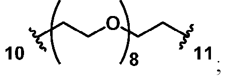 wherein, Position 10 is connected to Aa, and Position 11 is connected to D².

In some embodiments, D¹ is selected from the group consisting of cytotoxic drug fragments, and the cytotoxic drug is selected from the group consisting of tubulin inhibitors, DNA damaging agents and topoisomerase inhibitors, the tubulin inhibitor comprises, but not limited to dolastatin, auristatins, maytansines, tubulysins and cryptomycins, the DNA damaging agent comprises, but not limited to PBDs, duocarmycins and calicheamicins, and the topoisomerase inhibitor comprises, but not limited to camptothecin and derivatives thereof.

In some embodiments, the tubulin inhibitor is selected from the group consisting of dolastatin 10, MMAE, MMAF, maytansine, DM1, DM3 and DM4, and the topoisomerase inhibitor is selected from the group consisting of camptothecin, SN-38, exatecan, topotecan, belotecan, 10-hydroxy-camptothecin, 9-amino-camptothecin, doxorubicin, epirubicin and PNU-159682.

In some embodiments, D¹ is selected from the group consisting of the following structures: 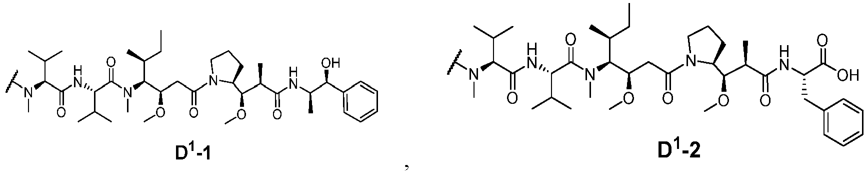 and

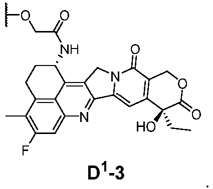

In some embodiments, D² is a TLR agonist fragment.

In some embodiments, the TLR agonist is selected from the group consisting of TLR2 agonists, TLR4 agonists, TLR6 agonists, TLR7 agonists, TLR8 agonists, TLR7/8 agonists, and TLR9 agonists.

In some embodiments, D² is a TLR agonist fragment, and the TLR agonist is selected from the group consisting of TLR7 agonists, TLR8 agonists and TLR7/8 agonists.

In some embodiments, D² is a TLR agonist fragment, and the TLR agonist is a compound represented by Formula (II): 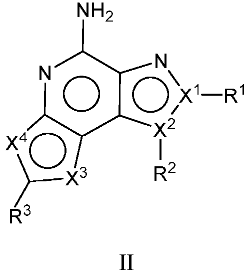 wherein,
X¹ is N or C;
X² is N or C;
and at least one of X¹ and X² is N;
X³ is selected from the group consisting of O, S and N;
X⁴ is selected from the group consisting of O, S, N and CR⁴;
R¹ is selected from the group consisting of C₁₋₆ alkyl and -C₁₋₆ alkylene-O-C₁₋₆ alkyl;
R² is hydrogen or the formula -L³-L⁴-L⁵-L⁶;
L³ is a covalent bond or C₁₋₆ alkylene;
L⁴ is selected from the group consisting of a covalent bond, C₃₋₁₀ cycloalkyl, 3- to 12-membered heterocyclyl, C₆₋₁₀ aryl, and 5- to 10-membered heteroaryl, and the cycloalkyl, heterocyclyl, aryl and heteroaryl is optionally substituted with one or more groups selected from the group consisting of hydrogen, halogen, cyano, C₁₋₆ alkyl, C₁₋₆ alkoxy and -C₁₋₆ alkylene-NH₂;
L⁵ is selected from the group consisting of a covalent bond, -O-, -S-, -C(O)-, -O-C(O)-, -C(O)-O-, -O-C(O)-O-, -NR⁵-, -C(O)-NR⁵-, -NR⁵-C(O)-, -O-C(O)-NR⁵-, -NR⁵-C(O)-O-, -NR⁵-C(O)-NR⁵-, -S(O)ᵣ-NR⁵-, -NR⁵-S(O)ᵣ- and -NR⁵-S(O)ᵣ-NR⁵-;
L⁶ is selected from the group consisting of hydrogen, C₁₋₆ alkyl, -(O-CH₂CH₂)ₙ-O-C₁₋₆ alkyl and -C₁₋₆ alkylene-(O-CH₂CH₂)ₙ-O-C₁₋₆ alkyl, and the alkyl is optionally substituted with one or more groups selected from the group consisting of hydrogen, halogen, hydroxyl, -NH₂, -NH-C(O)-O-C₁₋₆ alkyl, C₁₋₆ alkoxy, carboxyl and -C(O)-O-C₁₋₆ alkyl;
R³ is hydrogen or the formula -L⁷-L⁸-L⁹-L¹⁰;
L⁷ is selected from the group consisting of a covalent bond and C₁₋₆ alkylene;
L⁸ is selected from the group consisting of a covalent bond, C₃₋₁₀ cycloalkyl, 3- to 12-membered heterocyclyl, C₆₋₁₀ aryl, and 5- to 10-membered heteroaryl, and the cycloalkyl, heterocyclyl, aryl and heteroaryl is optionally substituted with one or more groups selected from the group consisting of hydrogen, halogen, cyano, C₁₋₆ alkyl, C₁₋₆ alkoxy and -C₁₋₆ alkylene-NH₂;
L⁹ is selected from the group consisting of a covalent bond, -O-, -S-, -C(O)-, -O-C(O)-, -C(O)-O-, -O-C(O)-O-, -NR⁶-, -C(O)-NR⁶-, -NR⁶-C(O)-, -O-C(O)-NR⁶-, -NR⁶-C(O)-O-, -NR⁶-C(O)-NR⁶-, -S(O)ₚ-NR⁶-, -NR⁶-S(O)ₚ- and -NR⁶-S(O)ₚ-NR⁶-;
L¹⁰ is selected from the group consisting of hydrogen, C₁₋₆ alkyl, -(O-CH₂CH₂)_{q}-O-C₁₋₆ alkyl, and -C₁₋₆ alkylene-(O-CH₂CH₂)_{q}-O-C₁₋₆ alkyl, and the alkyl is optionally substituted with one or more groups selected from the group consisting of hydrogen, halogen, hydroxyl, -NH₂, -NH-C(O)-O-C₁₋₆ alkyl, C₁₋₆ alkoxy, carboxyl and -C(O)-O-C₁₋₆ alkyl;
R⁴ is selected from the group consisting of hydrogen, halogen, cyano, hydroxyl, C₁₋₆ alkyl, C₁₋₆ haloalkyl, C₁₋₆ alkoxy and C₃₋₁₀ cycloalkyl;
each R⁵ is independently hydrogen or C₁₋₆ alkyl;
each R⁶ is independently hydrogen or C₁₋₆ alkyl;
each r is independently 1 or 2;
each n is independently an integer selected from 1 to 25;
each p is independently 1 or 2;
each q is independently an integer selected from 1 to 25;
D² is connected to L² through R² or R³ in Formula (II).

In some embodiments, each n is independently selected from 1 to 15, preferably, n is selected from the group consisting of 1, 2, 3, 4, 5, 6, 7, 8, 9, 10, 11, 12, 13, 14 and 15.

In some embodiments, each q is independently selected from 1 to 15, preferably, q is selected from the group consisting of 1, 2, 3, 4, 5, 6, 7, 8, 9, 10, 11, 12, 13, 14 and 15.

In some embodiments, X³ is selected from the group consisting of S and N.

In some embodiments, L³ is selected from the group consisting of a covalent bond and C₁₋₃ alkylene.

In some embodiments, L⁴ is selected from the group consisting of a covalent bond, 3- to 12-membered heterocyclyl, C₆₋₁₀ aryl, and 5- to 10-membered heteroaryl, and the heterocyclyl, aryl, and heteroaryl are optionally substituted with one or more groups selected from the group consisting of hydrogen, halogen, cyano, C₁₋₃ alkyl, C₁₋₃ alkoxy and -C₁₋₆ alkylene-NH₂.

In some embodiments, L¹ is selected from the group consisting of a covalent bond, -NR⁵-, -C(O)-NR⁵-, -NR⁵-C(O)-, -O-C(O)-NR⁵-, -NR⁵-C(O)-O-, -NR⁵-C(O)-NR⁵-, -S(O)ᵣ-NR⁵-, -NR⁵-S(O)ᵣ-, and -NR⁵-S(O)ᵣ-NR⁵-, each R⁵ is independently selected from the group consisting of hydrogen and C₁₋₆ alkyl; preferably, L⁵ is selected from the group consisting of a covalent bond and -NR⁵-, and each R⁵ is independently selected from the group consisting of hydrogen and C₁₋₆ alkyl.

In some embodiments, L⁶ is selected from the group consisting of hydrogen, C₁₋₆ alkyl, and -C₁₋₆ alkylene-(O-CH₂CH₂)ₙ-O-C₁₋₆ alkyl, and the alkyl is optionally substituted with one or more groups selected from the group consisting of hydrogen, halogen, hydroxyl, -NH₂, -NH-C(O)-O-C₁₋₆ alkyl, C₁₋₆ alkoxy, carboxyl and -C(O)-O-C₁₋₆ alkyl, n is 1 to 10.

In some embodiments, L⁸ is selected from the group consisting of a covalent bond and 3- to 12-membered heterocyclyl, and the heterocyclyl is optionally substituted with one or more groups selected from the group consisting of hydrogen, halogen, cyano and C₁₋₆ alkyl.

In some embodiments, L⁹ is a covalent bond.

In some embodiments, L¹⁰ is selected from the group consisting of hydrogen, C₁₋₆ alkyl, and -C₁₋₆ alkylene-(O-CH₂CH₂)_{q}-O-C₁₋₆ alkyl, q is 1 to 10.

In some embodiments, R³ is selected from the group consisting of hydrogen and the formula -L⁷-L⁸-L⁹⁻L¹⁰;
L⁸ is selected from the group consisting of a covalent bond and 3- to 12-membered heterocyclyl, and the heterocyclyl is optionally substituted with one or more groups selected from the group consisting of hydrogen, halogen, cyano and C₁₋₆ alkyl;
L⁹ is a covalent bond;
L¹⁰ is selected from the group consisting of hydrogen, C₁₋₆ alkyl and -C₁₋₆ alkylene-(O-CH₂CH₂)_{q}-O-C₁₋₆ alkyl;
q is selected from 1 to 10, such as 1, 2, 3, 4, 5, 6, 7, 8, 9 and 10.

In some embodiments, X¹ is C;
X² is N;
X³ is S;
X⁴ is selected from CR⁴;
R¹ is selected from C₁₋₆ alkyl;
R² is selected from the formula -L³-L⁴-L⁵-L⁶;
L³ is selected from the group consisting of a covalent bond and C₁₋₆ alkylene;
L⁴ is selected from the group consisting of a covalent bond, 3- to 12-membered heterocyclyl, C₆₋₁₀ aryl and 5- to 10-membered heteroaryl, and the heterocyclyl, aryl and heteroaryl are optionally substituted with one or more groups selected from the group consisting of hydrogen, halogen, cyano, C₁₋₃ alkyl, C₁₋₃ alkoxy and -C₁₋₆ alkylene-NH₂;
L⁵ is selected from the group consisting of a covalent bond and -NR⁵-, and each R⁵ is independently selected from the group consisting of hydrogen and C₁₋₆ alkyl;
L⁶ is selected from the group consisting of hydrogen, C₁₋₆ alkyl and -C₁₋₆ alkylene-(O-CH₂CH₂)ₙ-O-C₁₋₆ alkyl, and the alkyl group is optionally substituted with one or more groups selected from the group consisting of hydrogen, halogen, hydroxyl, -NH₂, -NH-C(O)-O-C₁₋₆ alkyl, C₁₋₆ alkoxy, carboxyl and -C(O)-O-C₁₋₆ alkyl.

In some embodiments, D² is selected from the group consisting of the following structures:

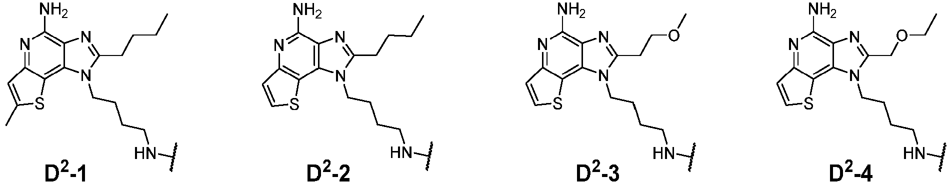

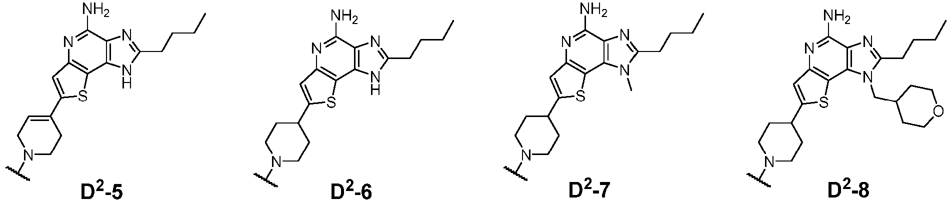

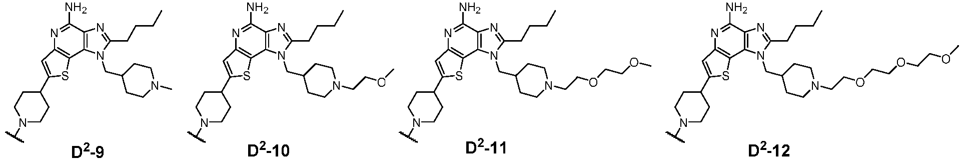

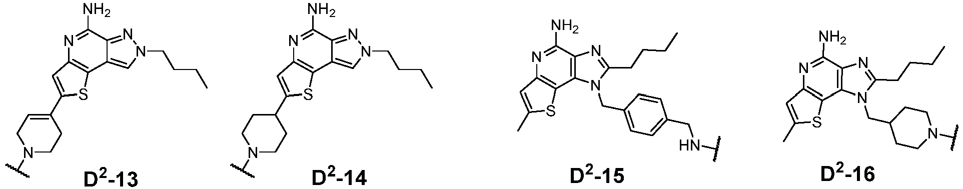

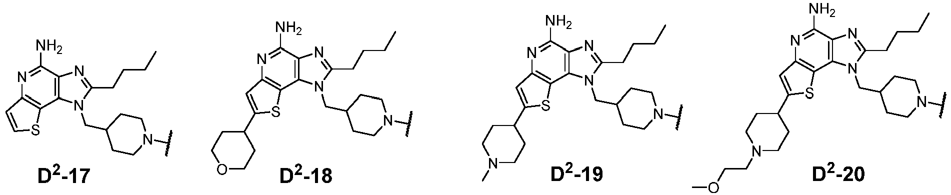

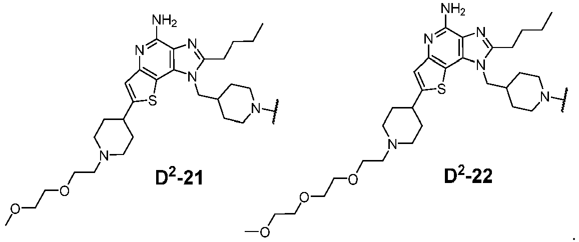

In some embodiments, the antibody-drug conjugate is selected from the group consisting of the following structures: 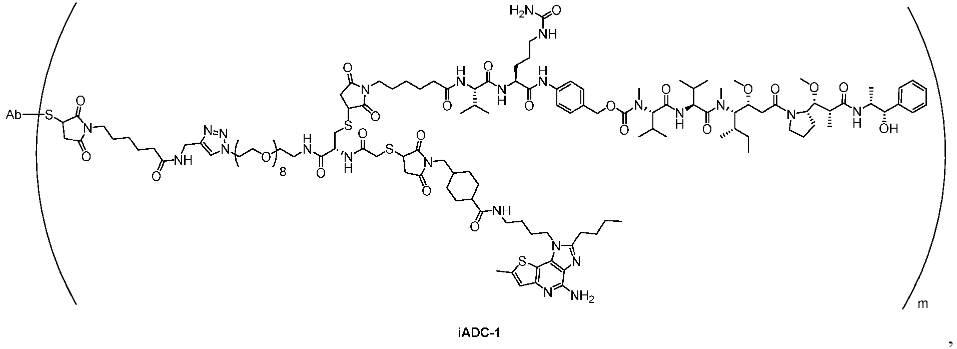 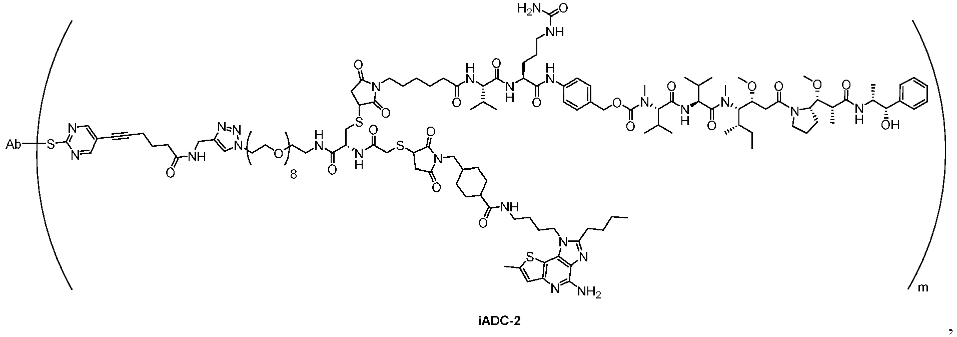 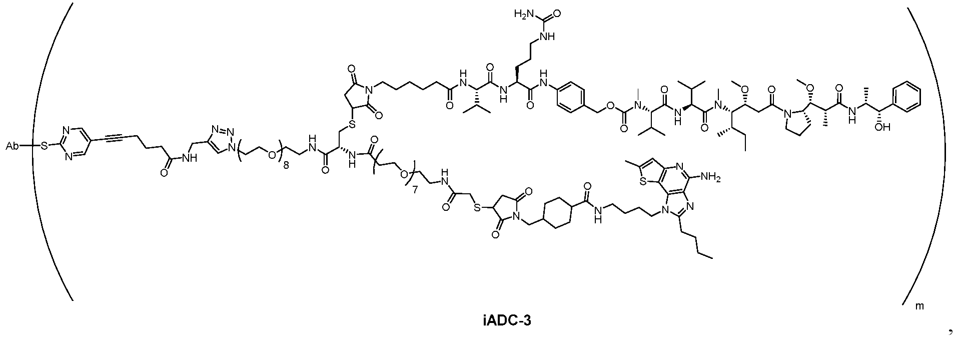 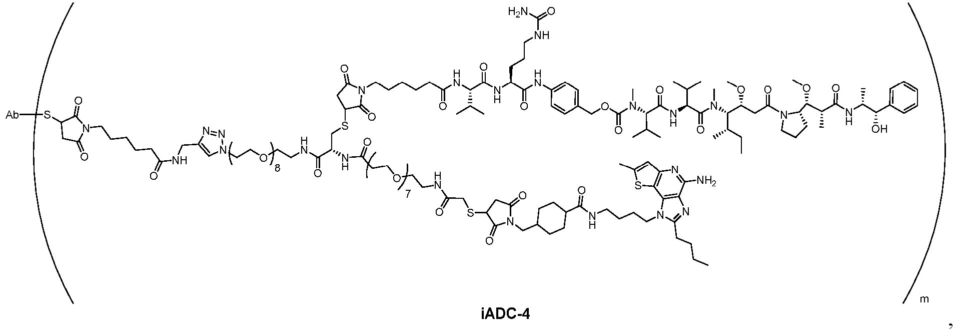 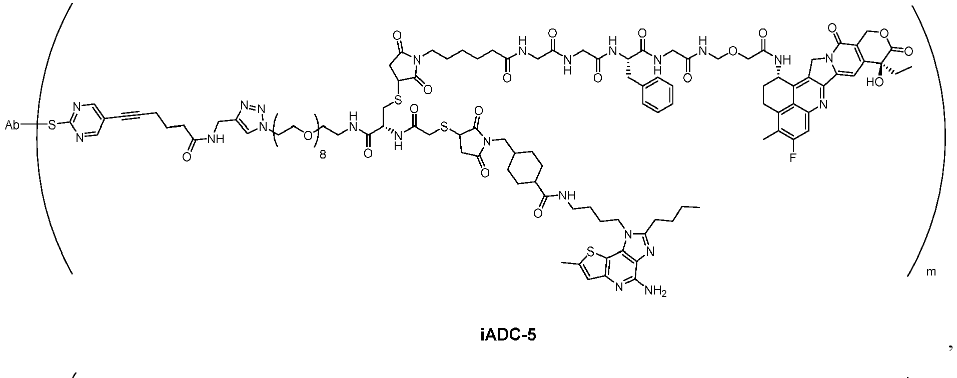 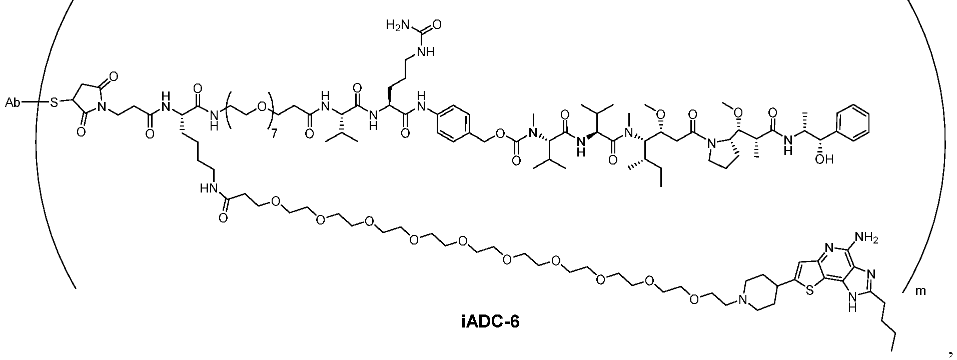 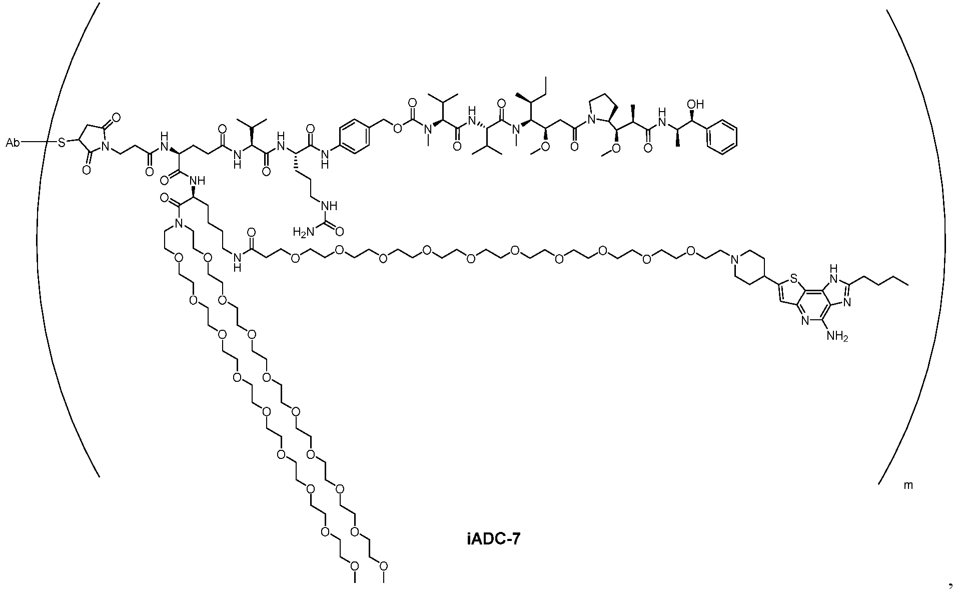 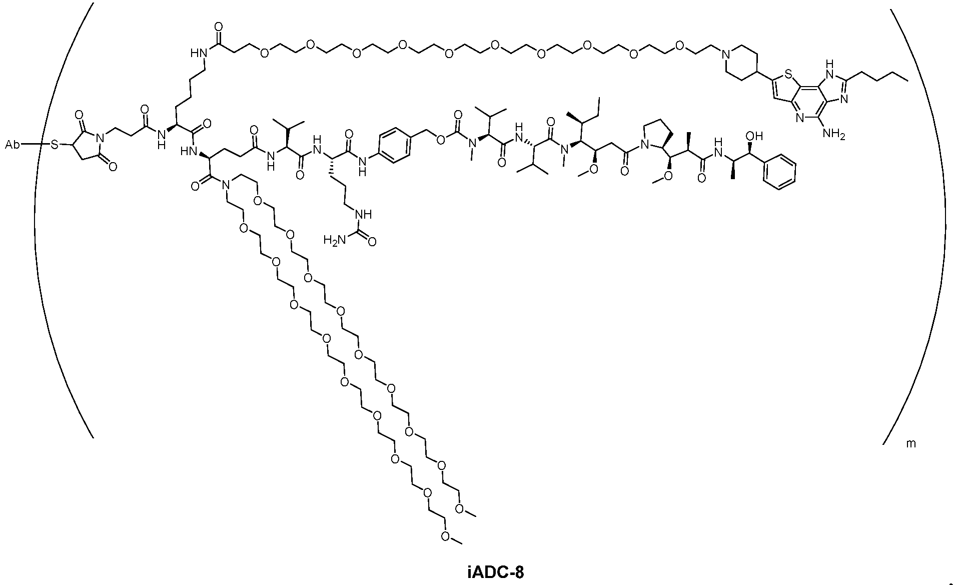 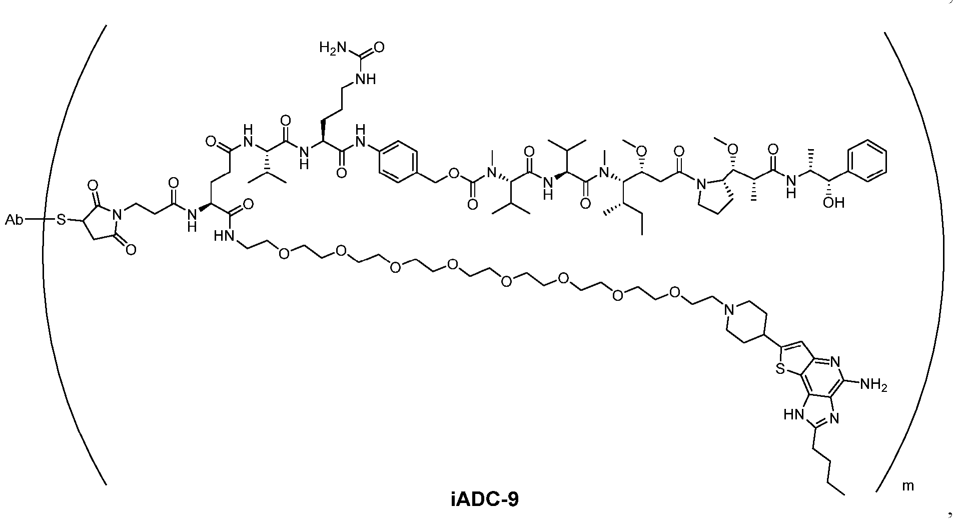 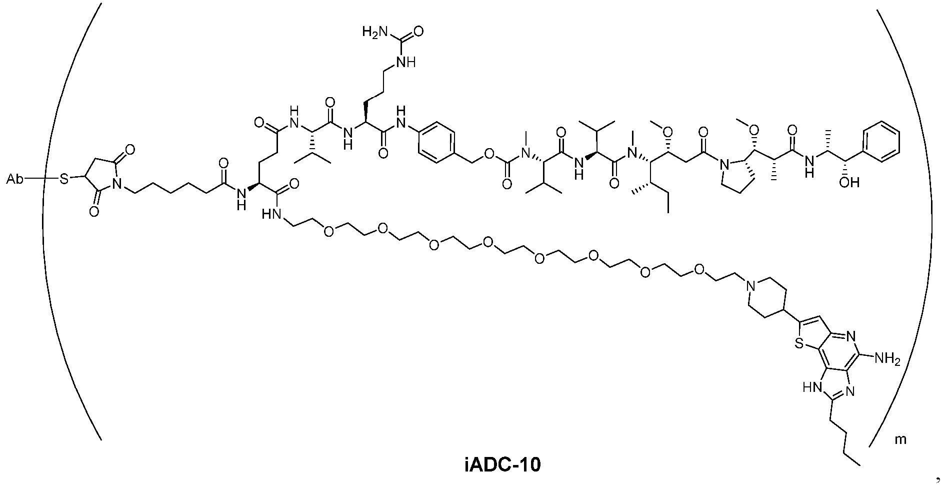 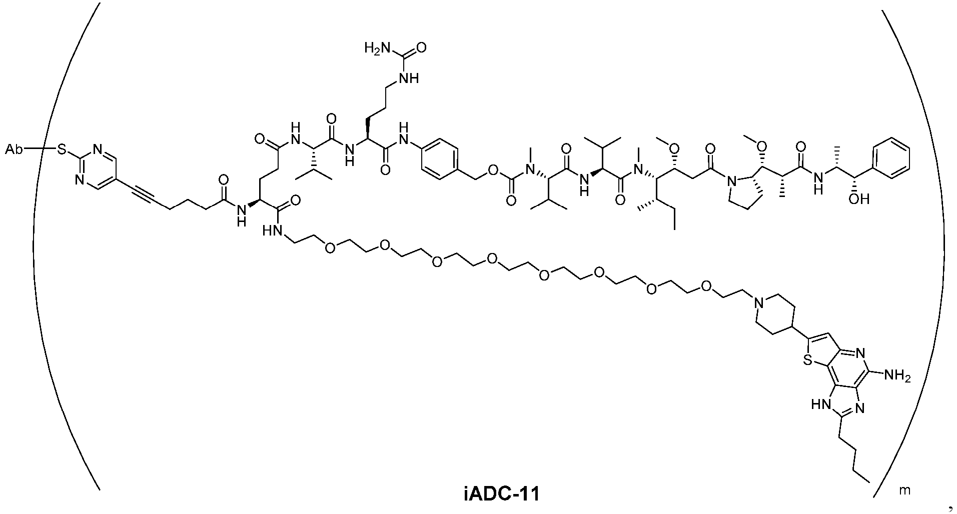 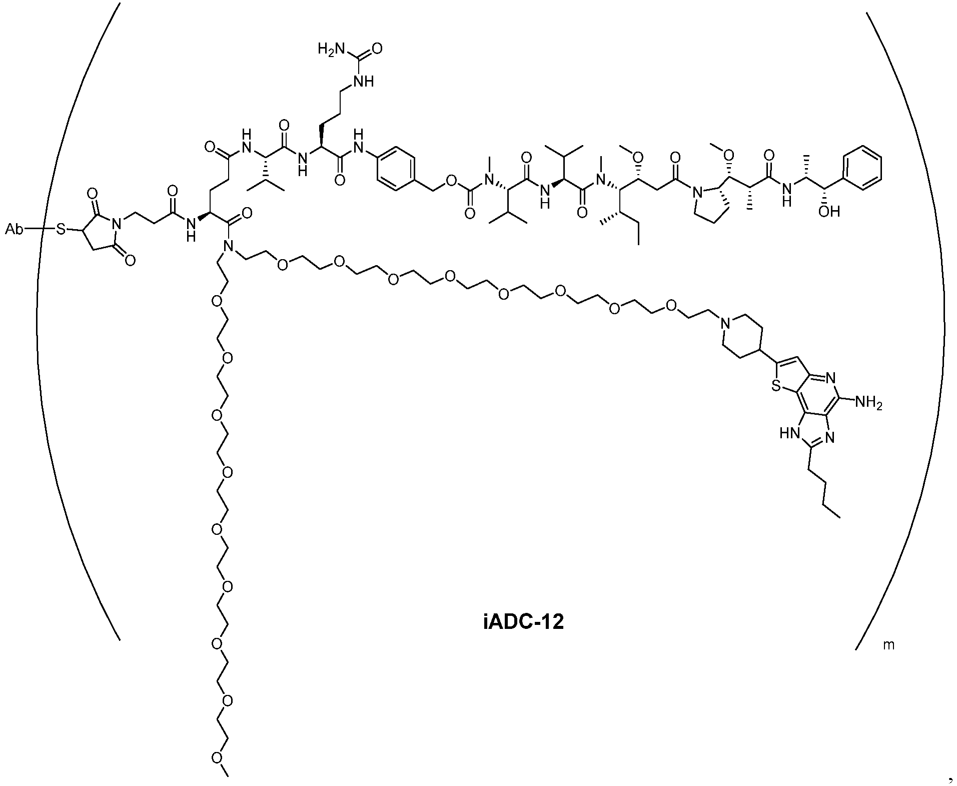 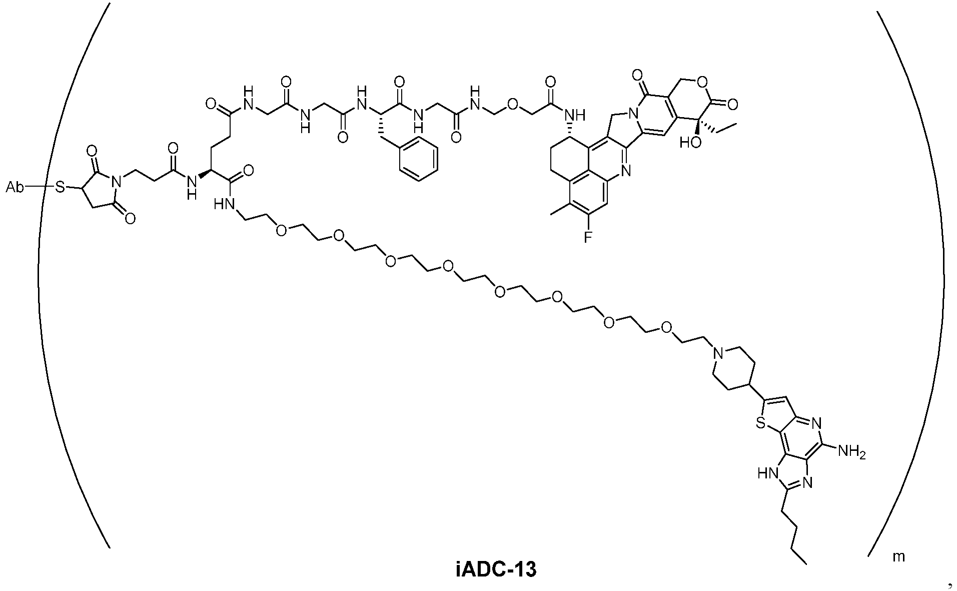 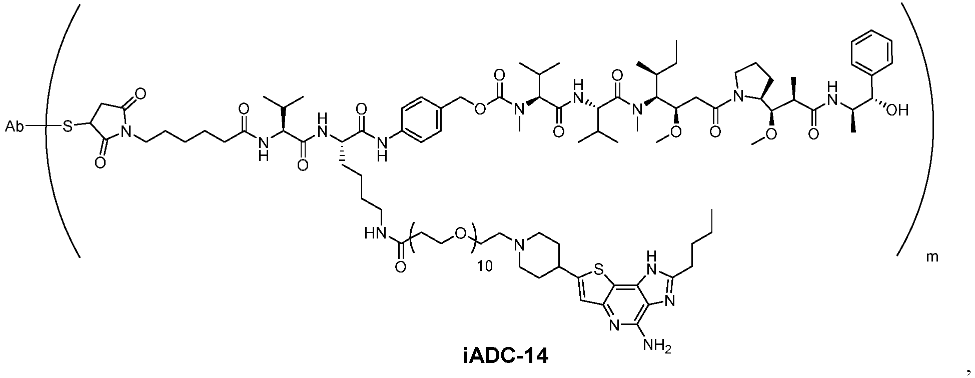 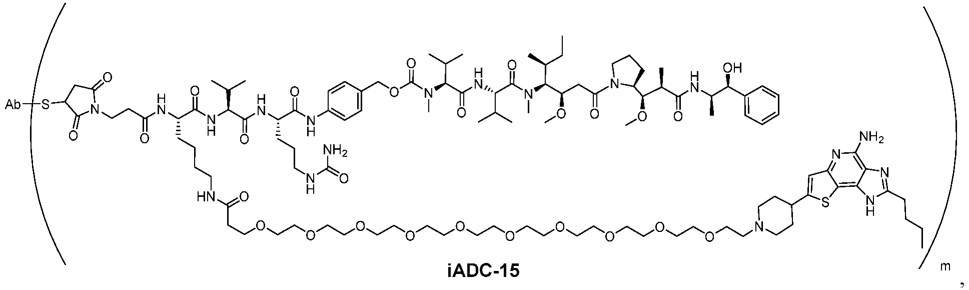 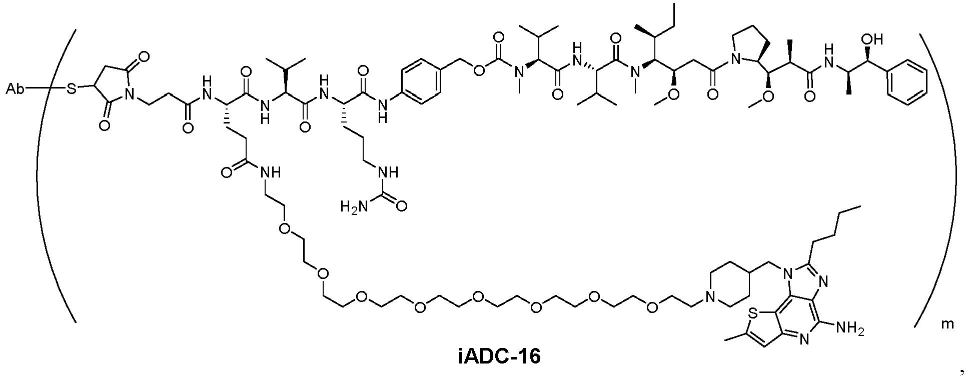 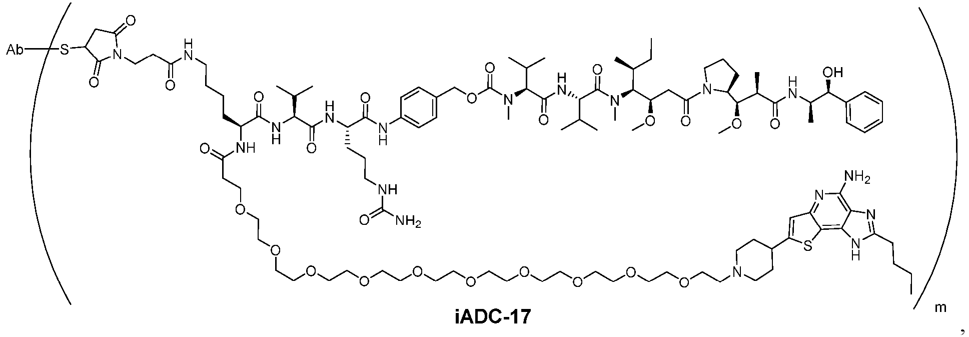 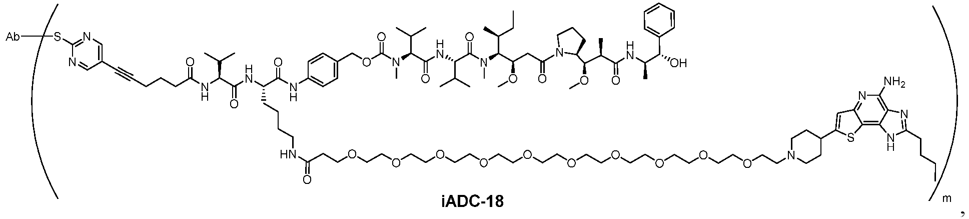 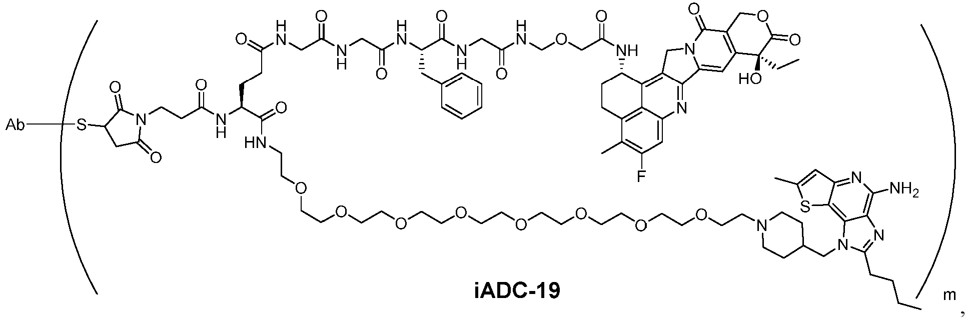 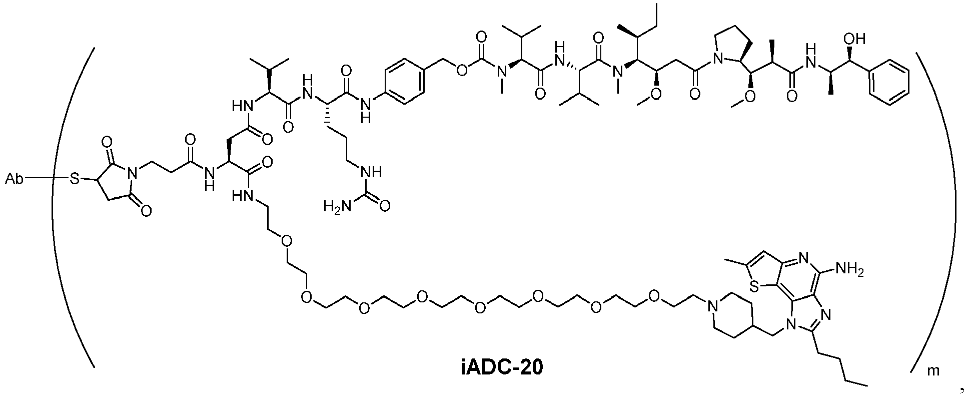 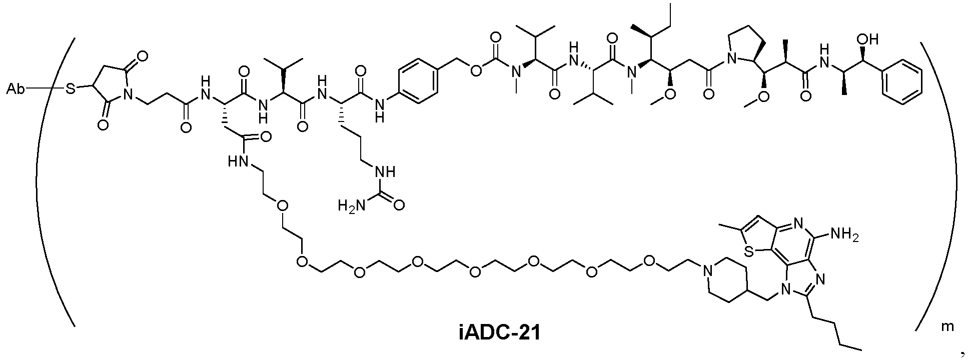 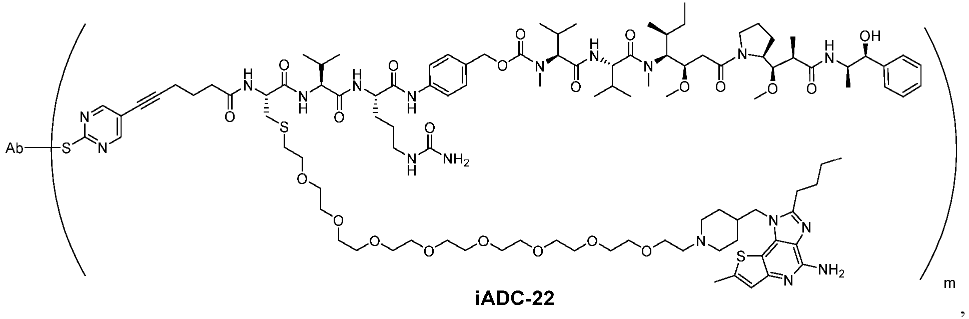 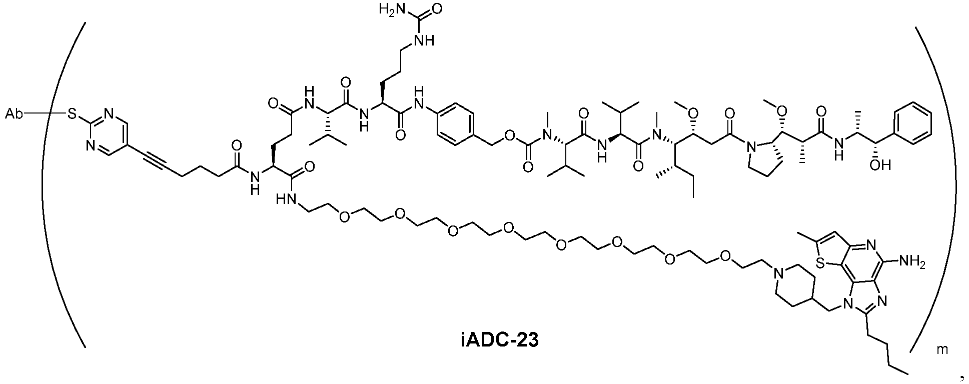 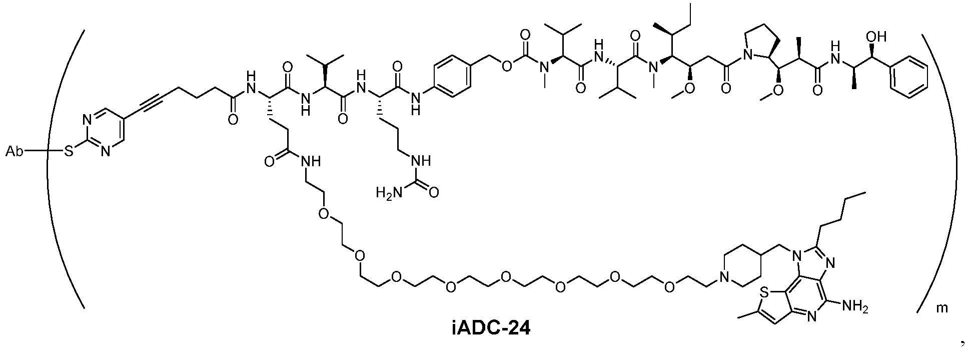 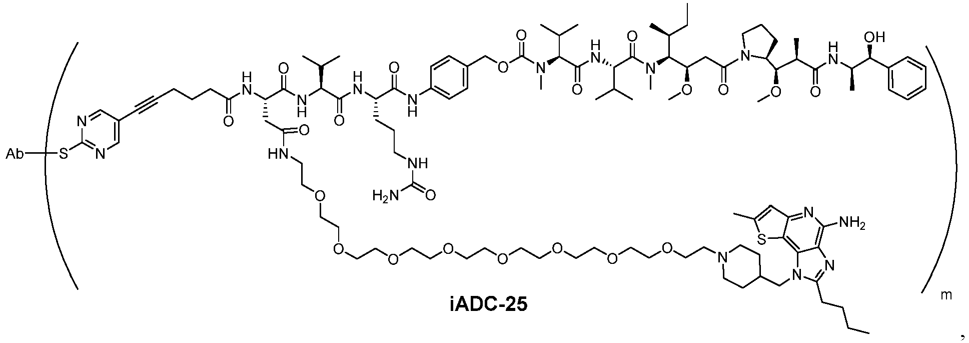 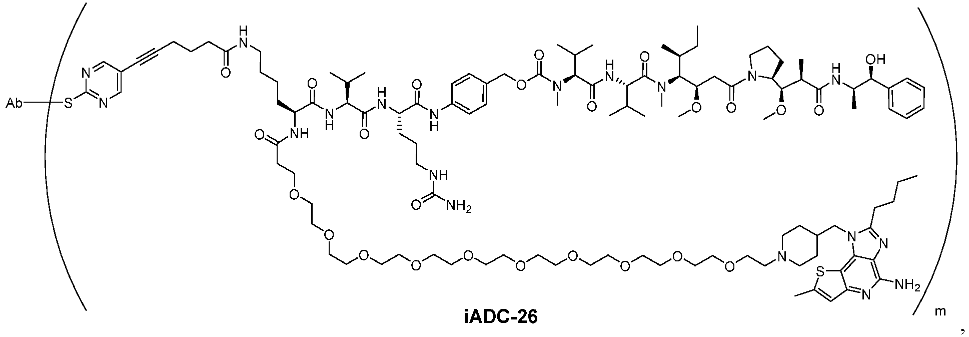 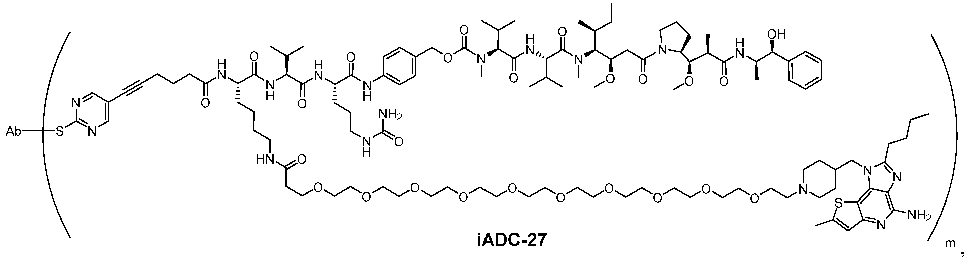 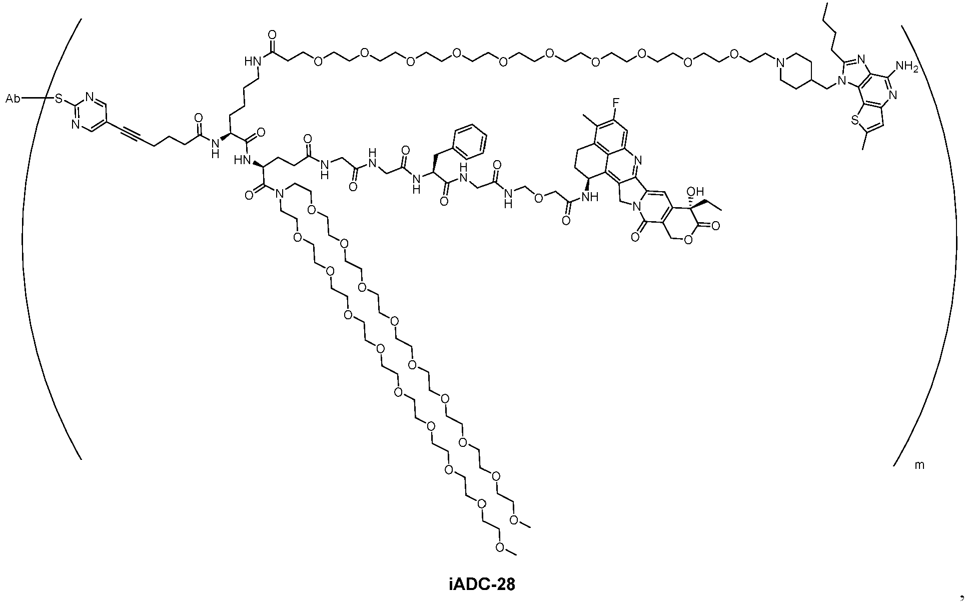 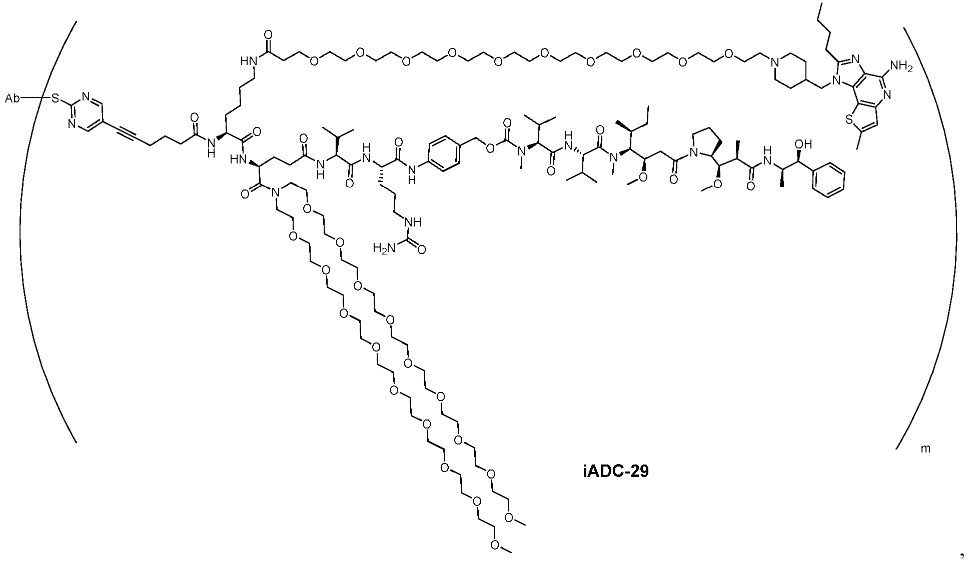 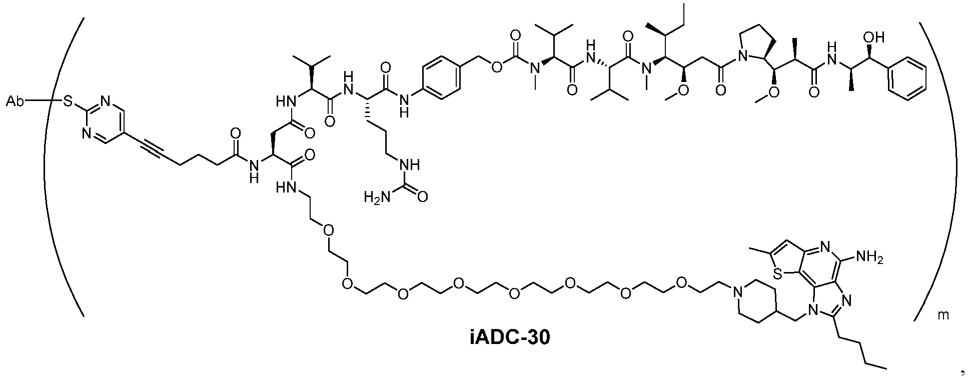 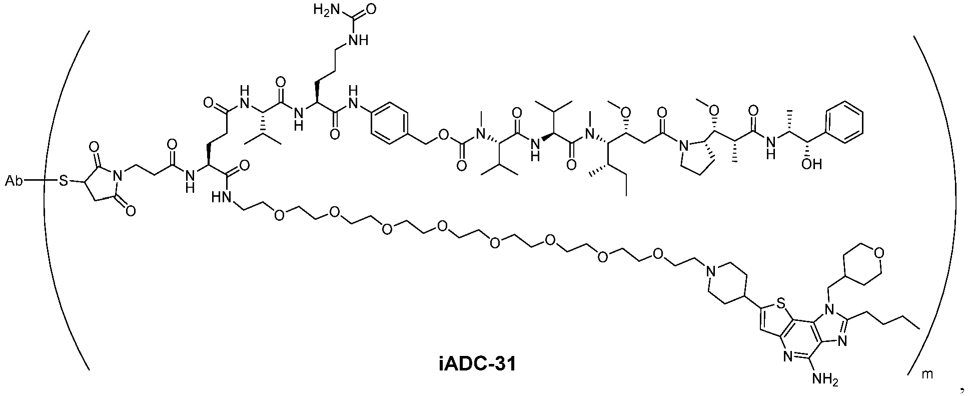 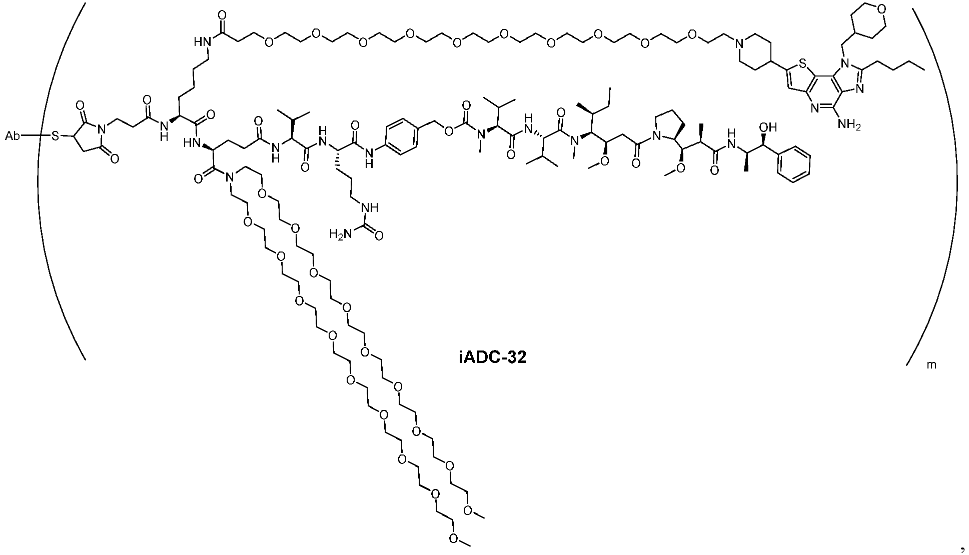 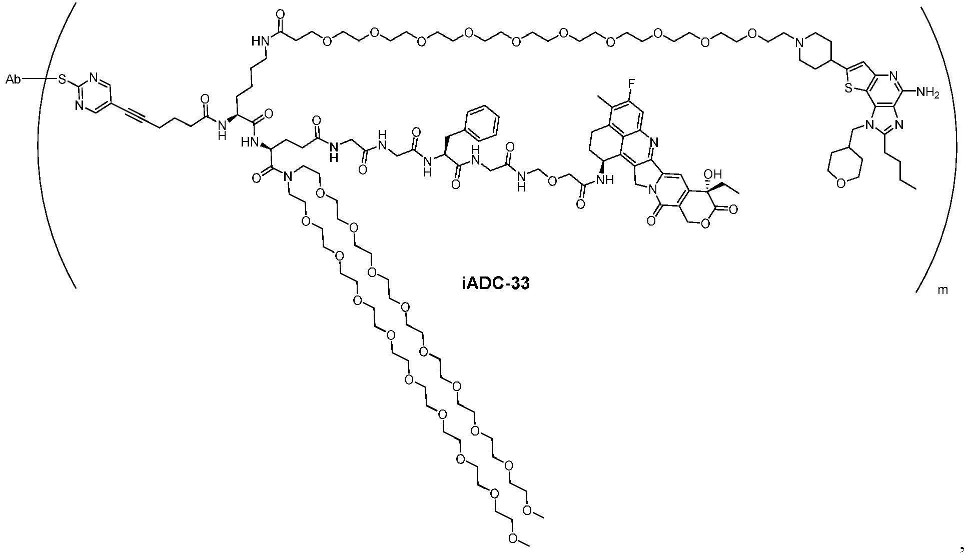 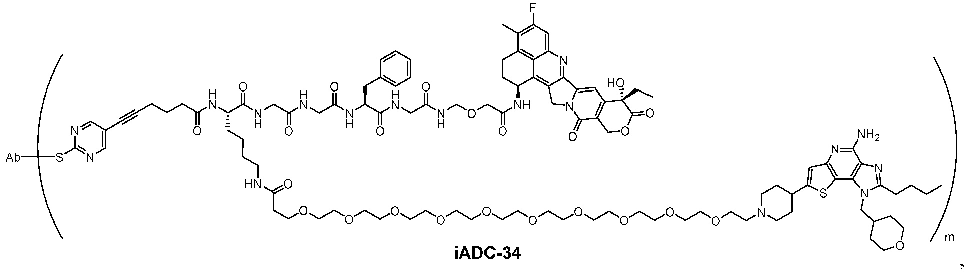 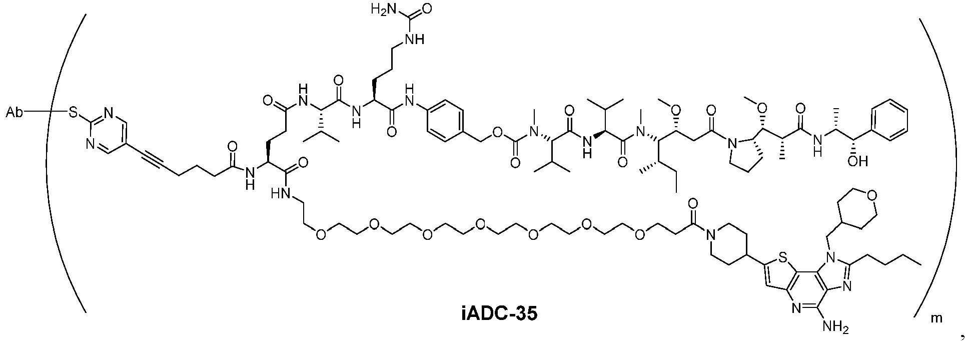 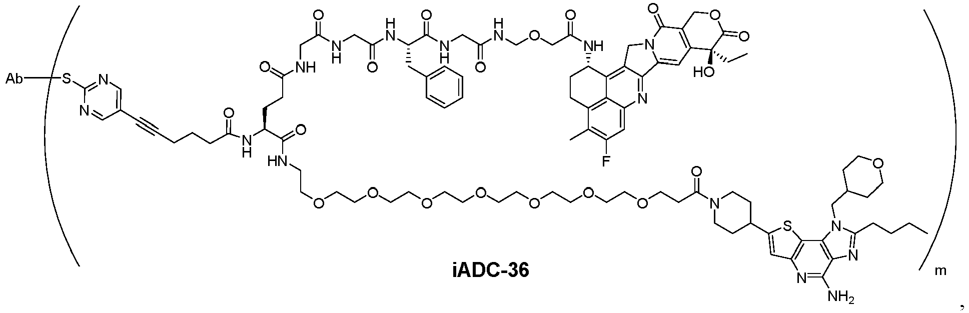 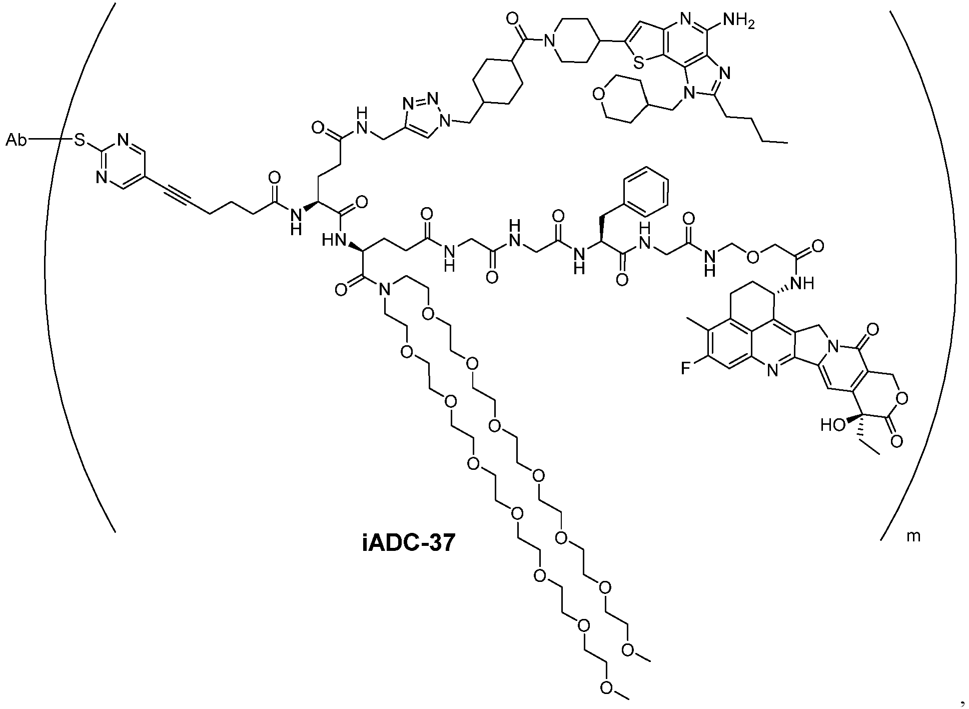 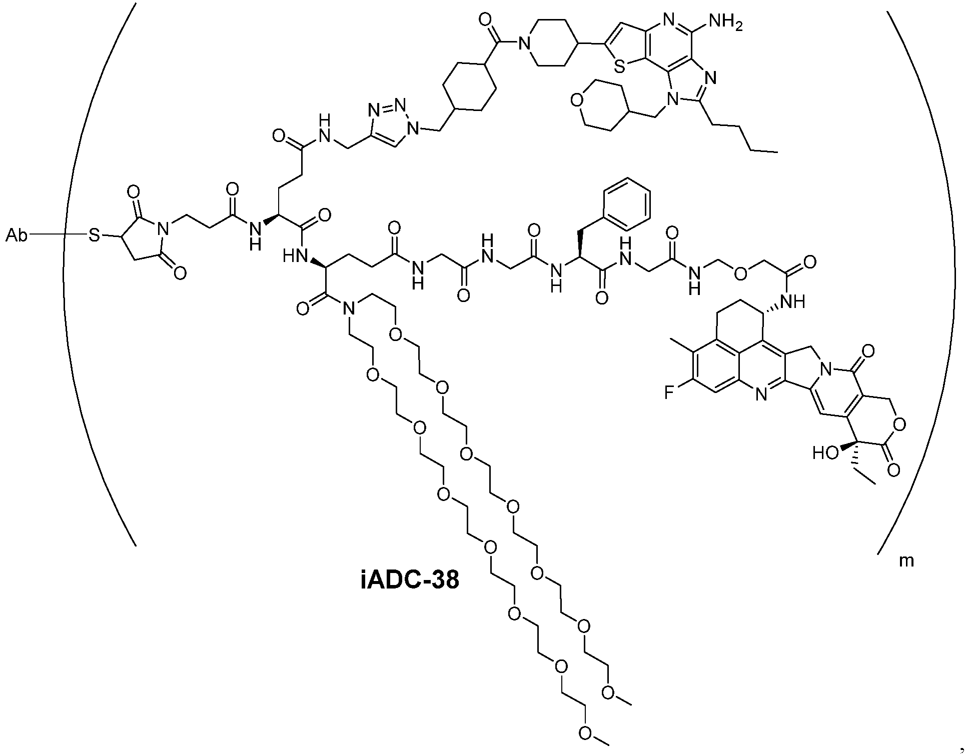 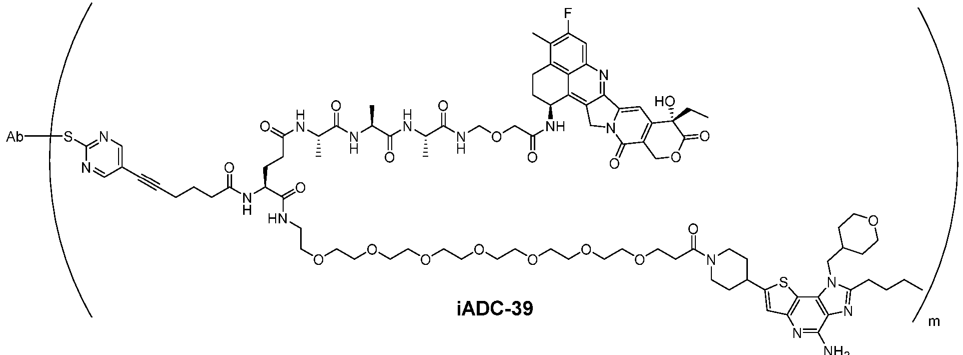 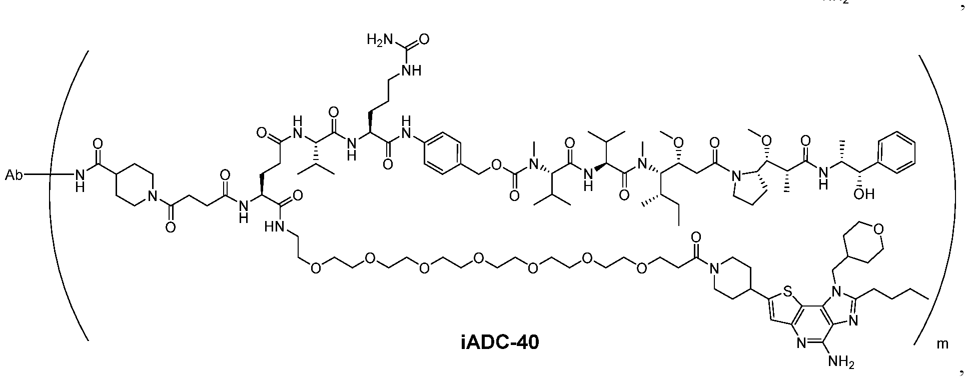 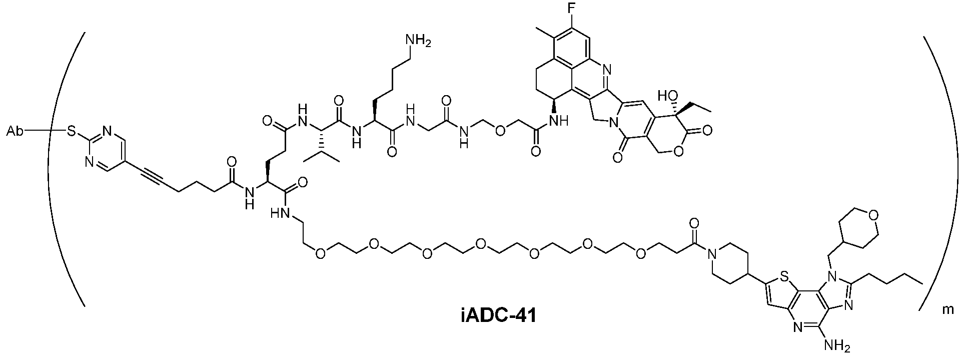 and 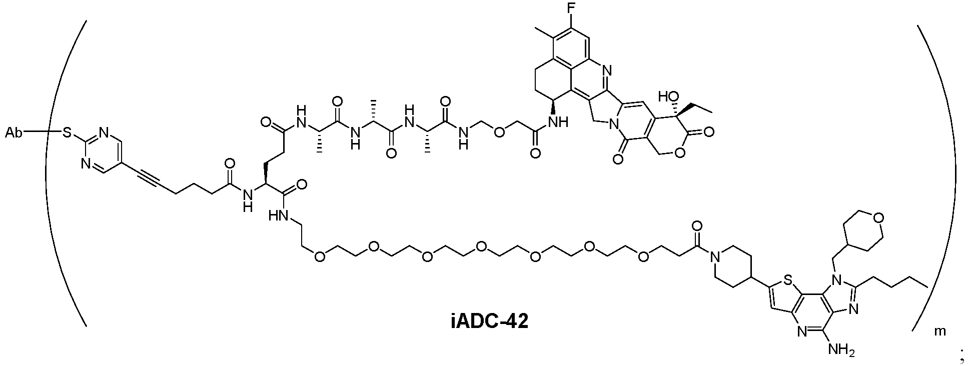 each m is independently selected from 1 to 10, such as 1, 2, 3, 4, 5, 6, 7, 8, 9 or 10.

In some embodiments, Ab is Trastuzumab.

In some embodiments, the antibody-drug conjugate has a DAR value of 2.0 to 5.0, such as 2.0 to 2.5, 2.0 to 3.0, 2.0 to 3.5, 2.0 to 4.0, 2.0 to 4.5, 2.0 to 5.0, 2.5 to 3.0, 2.5 to 3.5, 2.5 to 4.0, 2.5 to 4.5, 2.5 to 5.0, 3.0 to 3.5, 3.0 to 4.0, 3.0 to 4.5, 3.0 to 5.0, 3.5 to 4.0, 3.5 to 4.5, 3.5 to 5.0, 4.0 to 4.5, 4.0 to 5.0, or 4.0 to 5.0.

### Drug-linker

In another aspect, the present invention provides a drug-linker simultaneously coupled with a cytotoxic drug and a TLR agonist, which can be used to prepare the aforementioned antibody-drug conjugate.

Specifically, the present invention provides a compound of Formula (III) or pharmaceutically acceptable salt, ester, stereoisomer, tautomer, polymorph, solvate, N-oxide, isotopically labeled compound, metabolite or prodrug thereof: 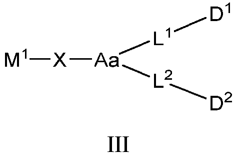 wherein,
M¹ is a precursor of a linking site connected to a targeting moiety, wherein the targeting moiety is as defined in any one of the aforementioned;
X is a linker connecting M and Aa;
Aa is an amino acid fragment, or a peptide fragments formed of two or more amino acids;
L¹ is a covalent bond or a linker connecting Aa and D¹;
L² is a linker connecting Aa and D²;
D¹ is a cytotoxic drug fragment;
D² is a TLR agonist fragment.

In some embodiments, M¹ is selected from the group consisting of the following structures: 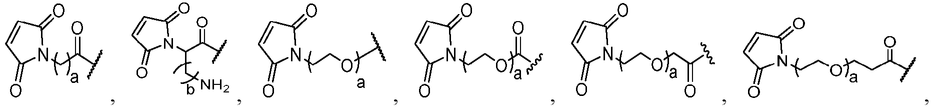 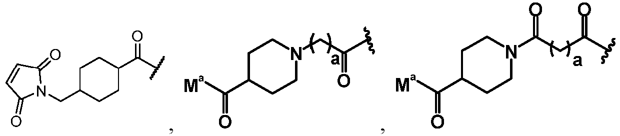 and 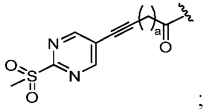 wherein, each a is independently an integer selected from 1 to 5, b is an integer selected from 1 to 3, and M^{a} is a carboxyl-activating group (e.g., a pentafluorophenoxy group).

In some embodiments, M¹ is selected from the group consisting of the following structures: 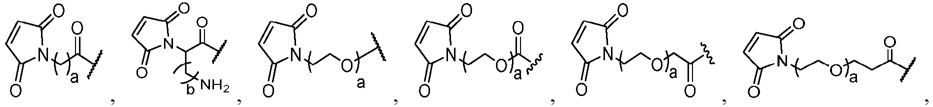 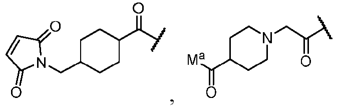 and 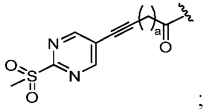 wherein, each a is independently an integer selected from 1 to 5, b is an integer selected from 1 to 3, and M^{a} is a carboxyl-activating group.

In some embodiments, M¹ is a covalent bond or selected from the group consisting of the following structures: 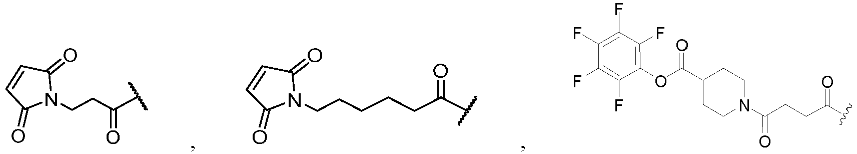 and

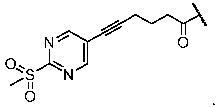

In some embodiments, M¹ is a covalent bond or selected from the group consisting of the following structures:

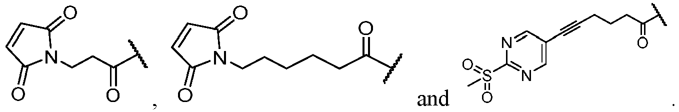

In some embodiments, X is a covalent bond or selected from the group consisting of C₁₋₆ alkylene, -NH-(CH₂)_{c}-C(O)-, 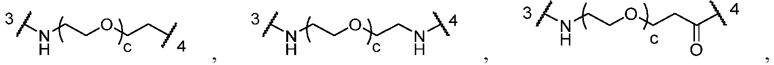 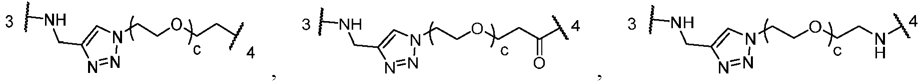 and 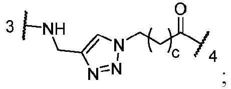 wherein, each c is independently an integer selected from 1 to 10; Position 3 of X is connected to M, and Position 4 of X is connected to Aa.

In some embodiments, X is a covalent bond or 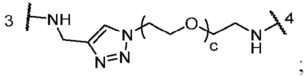 wherein, each c is independently an integer selected from 1 to 10 (e.g., an integer from 5 to 10, e.g., an integer from 8 to 10); Position 3 of X is connected to M, and Position 4 of X is connected to Aa.

In some embodiments, Aa is selected from the group consisting of an amino acid fragment, and a peptide fragment formed of two or more amino acids, wherein the amino acid is selected from the group consisting of Gly, Phe, Ala, Val, Ser, Thr, His, Trp, Cys, Asp, Glu, Lys, Tyr and Arg, optionally, each amino acid is independently modified with C₁₋₆ alkyl or a polyethylene glycol hydrophilic structural unit. In some embodiments, the polyethylene glycol hydrophilic structural unit is selected from the group consisting of 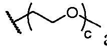 and 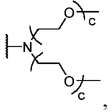 wherein each c is independently an integer selected from 1 to 10 (e.g., an integer from 5 to 10, e.g., an integer from 8 to 10).

In some embodiments, Aa is selected from the group consisting of a fragment of the following amino acids, and a peptide fragment formed of any combination of two or more of them: Gly, Phe, Ala, Val, Cys, Asp, Glu, Lys, and Arg, and each amino acid is independently optionally modified with C₁₋₆ alkyl group or a polyethylene glycol hydrophilic structural unit. In some embodiments, the polyethylene glycol hydrophilic structural unit is selected from the group consisting of 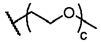 and 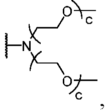 wherein each c is independently an integer selected from 1 to 10 (e.g., an integer from 5 to 10, e.g., an integer from 8 to 10).

In some embodiments, Aa is selected from the group consisting of a fragment of the following amino acids, and a peptide fragment formed of any combination of two or more of them: Val, Cys, Asp, Glu and Lys, wherein the carboxy-terminus of Glu and Lys are optionally modified with 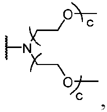 wherein each c is independently an integer selected from 1 to 10 (e.g., an integer from 5 to 10, e.g., an integer from 8 to 10).

In some embodiments, Aa is selected from the group consisting of the following structures: 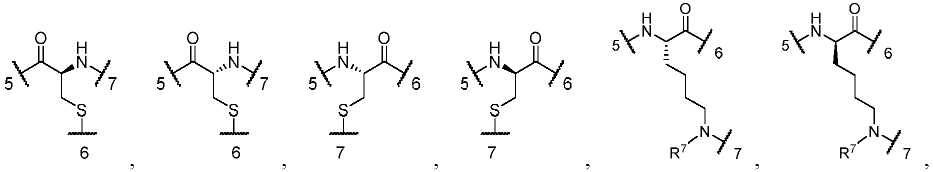 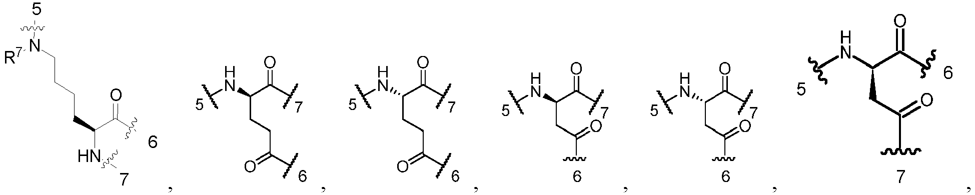 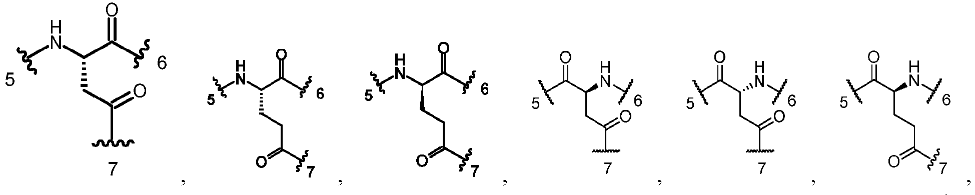 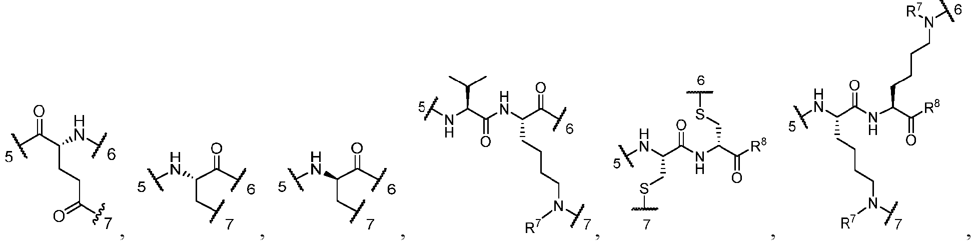 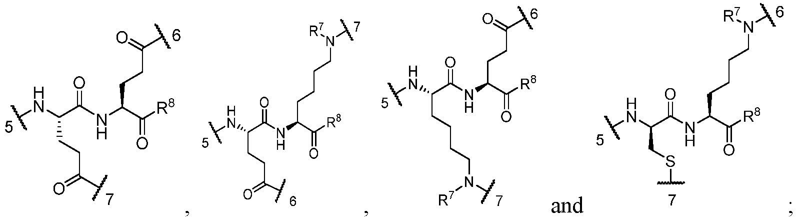 wherein, each R⁷ is independently selected from the group consisting of H, C₁₋₆ alkyl and a polyethylene glycol hydrophilic structural unit; each R⁸ is independently selected from the group consisting of hydroxyl and a polyethylene glycol hydrophilic structural unit; Position 5 is connected to X, Position 6 is connected to one of L¹ and L², and Position 7 is connected to the other of L¹ and L². In some embodiments, Position 5 is connected to X, Position 6 is connected to L¹, and Position 7 is connected to L². In some embodiments, Position 5 is connected to X, Position 6 is connected to L², and Position 7 is connected to L¹. In some embodiments, the polyethylene glycol hydrophilic structural unit is selected from the group consisting of 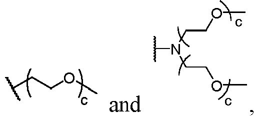 wherein each c is independently an integer selected from 1 to 10 (e.g., an integer from 5 to 10, e.g., an integer from 8 to 10). In some embodiments, R⁷ is H.

In some embodiments, Aa is selected from the group consisting of the following structures: 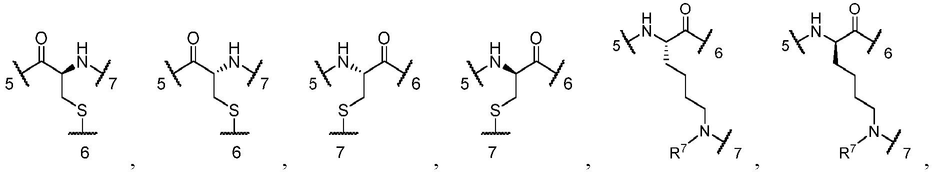 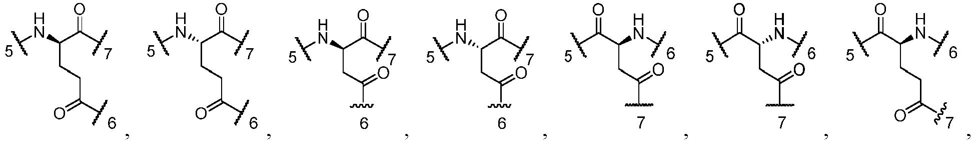 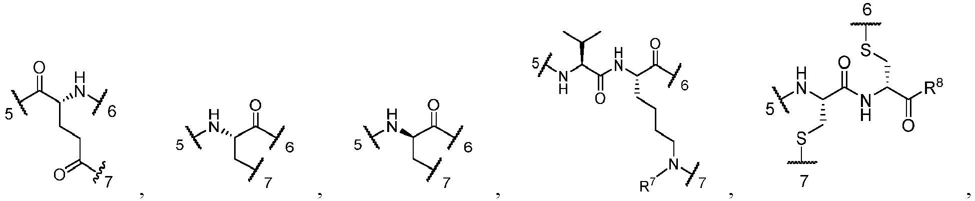 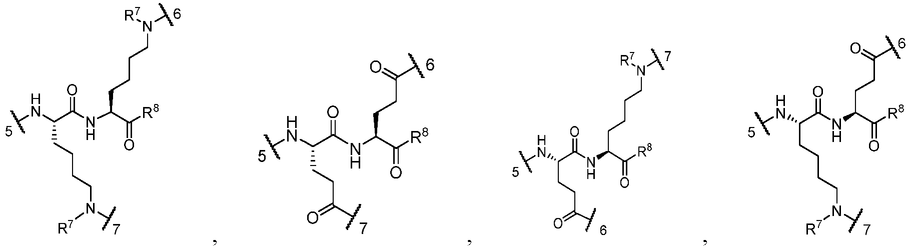 and 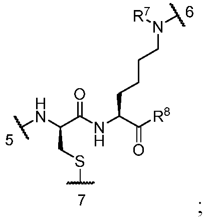 wherein, each R⁷ is independently selected from the group consisting of H, C₁₋₆ alkyl and a polyethylene glycol hydrophilic structural unit; each R⁸ is independently selected from the group consisting of hydroxyl and a polyethylene glycol hydrophilic structural unit; Position 5 is connected to X, Position 6 is connected to one of L¹ and L², and Position 7 is connected to the other of L¹ and L²; preferably, Position 5 is connected to X, Position 6 is connected to L¹, and Position 7 is connected to L². In some embodiments, the polyethylene glycol hydrophilic structural unit is selected from the group consisting of 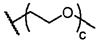 and 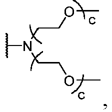 wherein each c is independently an integer selected from 1 to 10 (e.g., an integer from 5 to 10, e.g., an integer from 8 to 10).

In some embodiments, L¹ is a covalent bond or a non-cleavable linker or cleavable linker, wherein the cleavable linker is cleaved by an enzyme present in a pathological environment, and the enzyme is selected from the group consisting of protease, phosphatase, pyrophosphatase, *β*-glucuronidase, *β*-galactosidase, and sulfatase.

In some embodiments, L¹ is a covalent bond or -L^{a}-L^{b}-L^{c}-, wherein:
L^{a} is a covalent bond or selected from the group consisting of C₁₋₆ alkylene, -NH-(CH₂)_{c}-C(O)-, 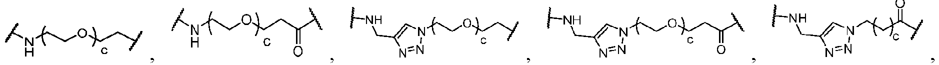 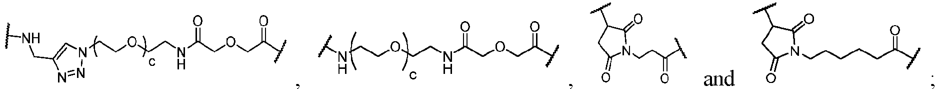 wherein, each c is independently an integer selected from 1 to 10;
L^{b} is a covalent bond or selected from the group consisting of an amino acid fragment, and a peptide fragment formed of two or more amino acids, wherein the amino acid is selected from the group consisting of Val, Cit, Glu, Lys, Arg, Phe, Leu, Gly, Ala and Asn;
L^{c} is a covalent bond, -NH-CH₂- or selected from the group consisting of the following structures:

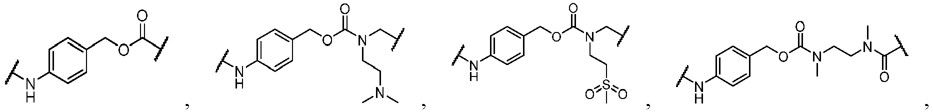

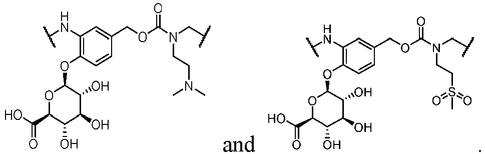

In some embodiments, L¹ is a covalent bond or -L^{a}-L^{b}-L^{c}-, wherein:
L^{a} is a covalent bond or selected from the group consisting of C₁₋₆ alkylene, -NH-(CH₂)_{c}-C(O)-, 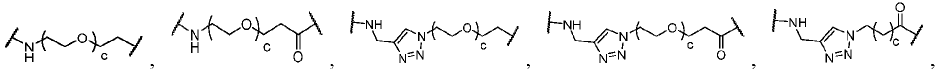 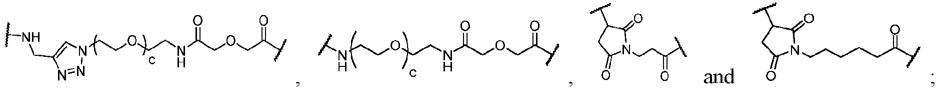 wherein, each c is independently an integer selected from 1 to 10;
L^{b} is a covalent bond or selected from the group consisting of an amino acid fragment, and a peptide fragment formed of two or more amino acids, wherein the amino acid is selected from the group consisting of Val, Cit, Glu, Lys, Arg, Phe, Leu, Gly, Ala and Asn;
L^{c} is a covalent bond, -NH-CH₂- or selected from the group consisting of the following structures:

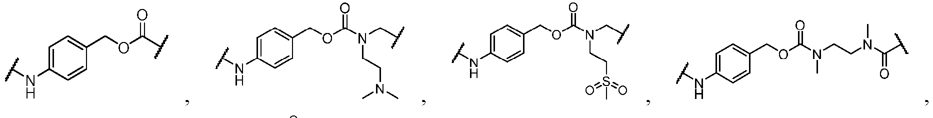

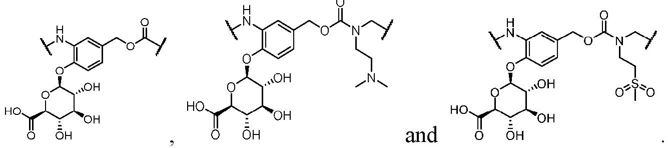

In some embodiments, L^{a} is a covalent bond or is selected from the group consisting of 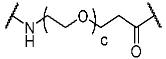 and 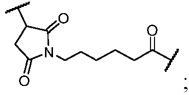 wherein, each c is independently an integer selected from 1 to 10. In some embodiments, L^{b} is a covalent bond or selected from the group consisting of the following structures: 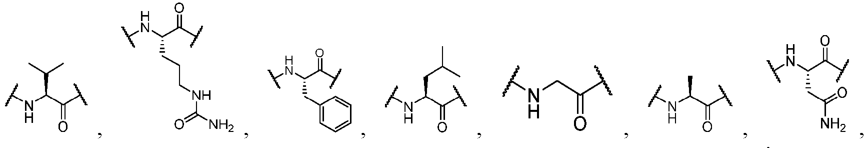 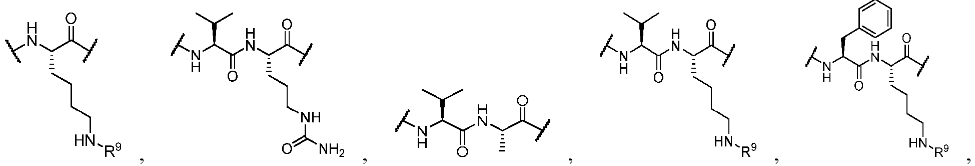 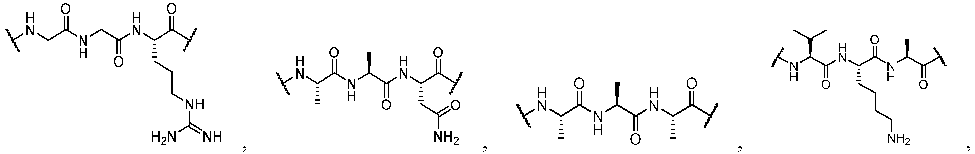 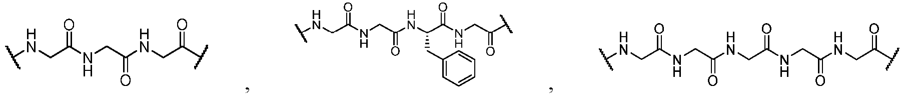 and 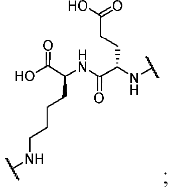 wherein, R⁹ is selected from the group consisting of H, acetyl, fluorenyloxycarbonyl, trityl and a polyethylene glycol hydrophilic structural unit. In some embodiments, the polyethylene glycol hydrophilic structural unit is 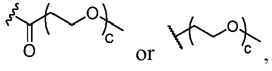 wherein each c is independently an integer selected from 1 to 10.

In some embodiments, L^{c} is -NH-CH₂- or

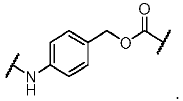

In some embodiments, L¹ is a covalent bond or -L^{a}-L^{b}-L^{c}-, wherein:
L^{a} is a covalent bond or selected from the group consisting of 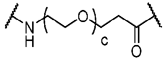 and 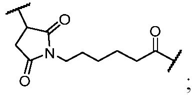 wherein, each c is independently an integer selected from 1 to 10;
L^{b} is a covalent bond or selected from the group consisting of 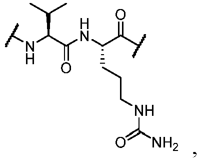 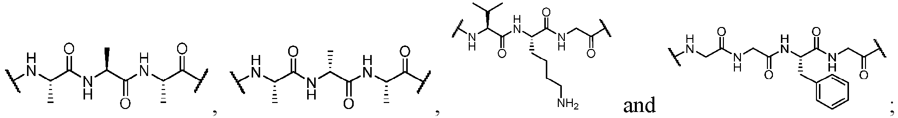
L^{c} is -NH-CH₂- or

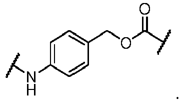

In some embodiments, L¹ is a covalent bond or selected from the group consisting of the following structures: 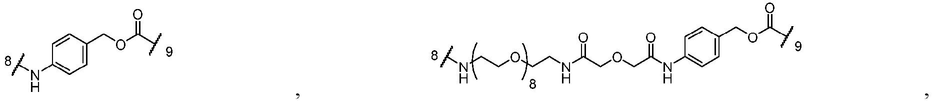 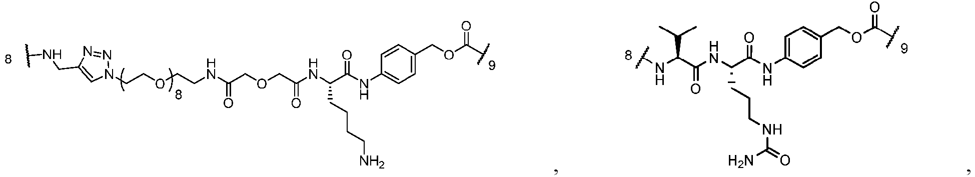 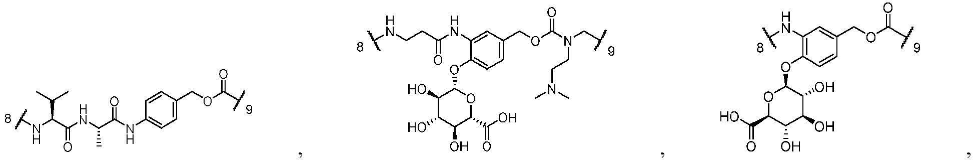 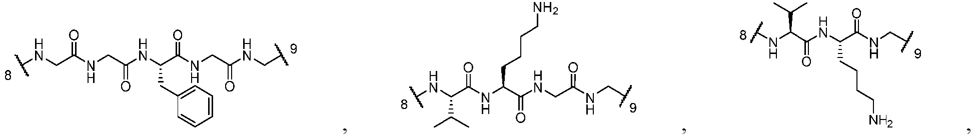 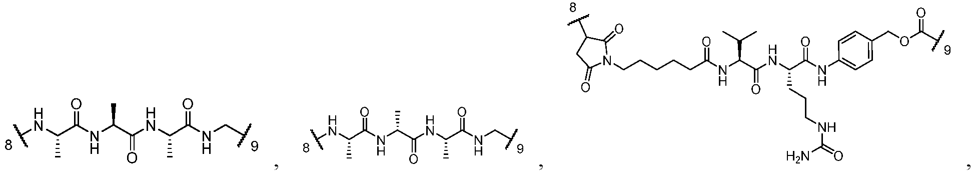 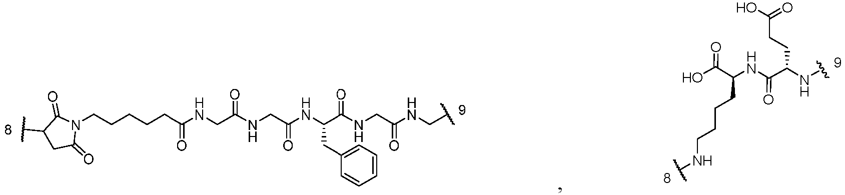 and 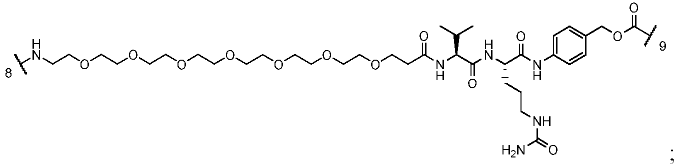 wherein, Position 8 is connected to Aa, and Position 9 is connected to D¹.

In some embodiments, L¹ is a covalent bond or selected from the group consisting of the following structures: 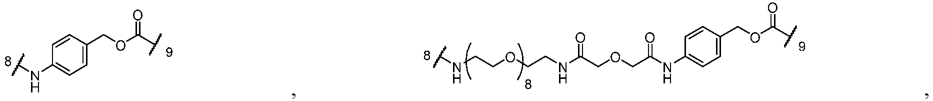 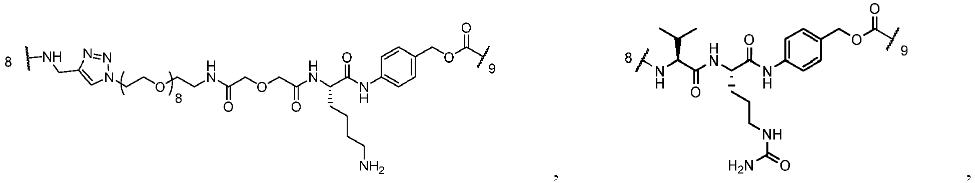 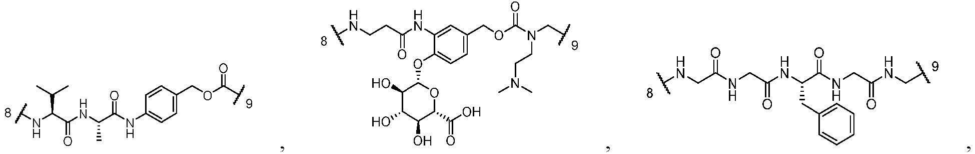 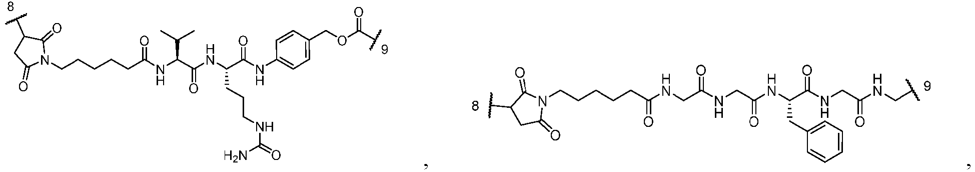 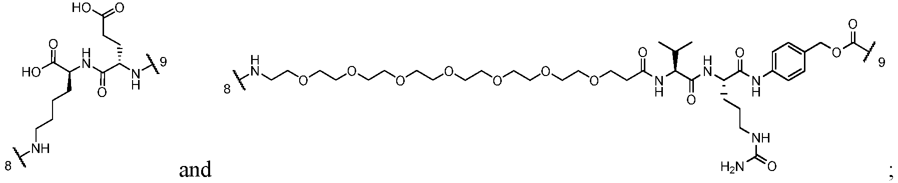 wherein, Position 8 is connected to Aa, and Position 9 is connected to D¹.

In some embodiments, L¹ is selected from the group consisting of the following structures: 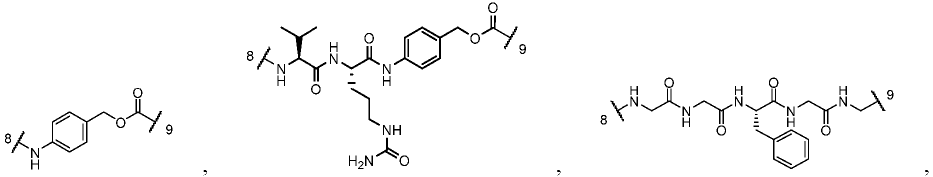 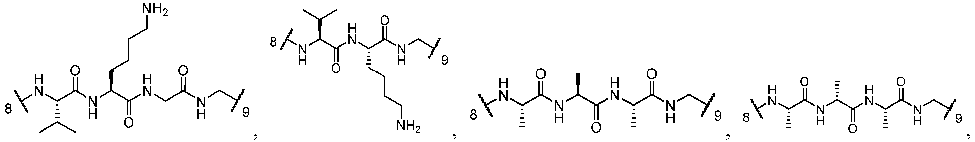 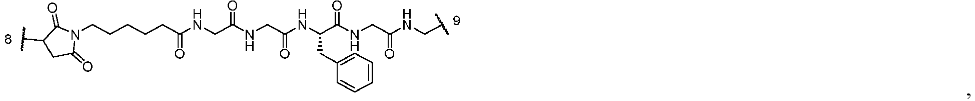 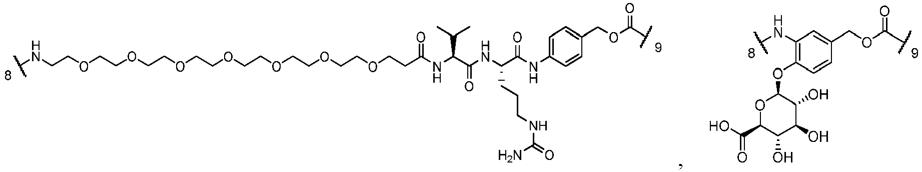 and 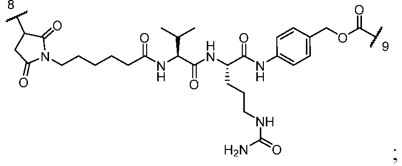 wherein, Position 8 is connected to Aa, and Position 9 is connected to D¹.

In some embodiments, L¹ is selected from the group consisting of the following structures: 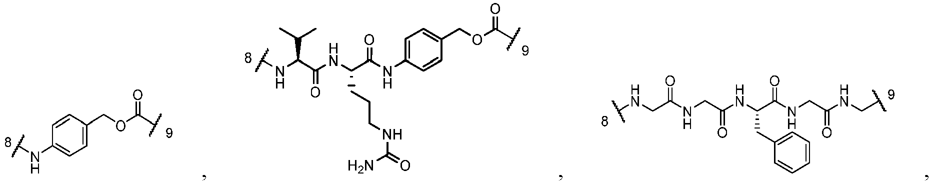 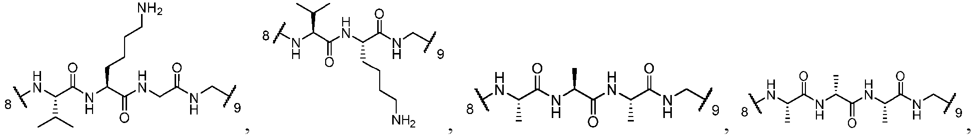 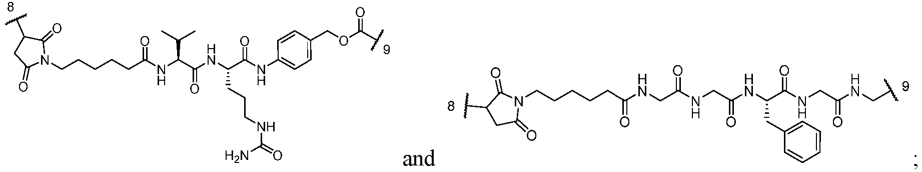 wherein, Position 8 is connected to Aa, and Position 9 is connected to D¹.

In some embodiments, L¹ is selected from the group consisting of the following structures: 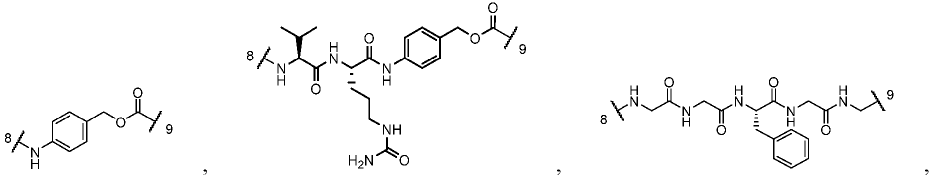 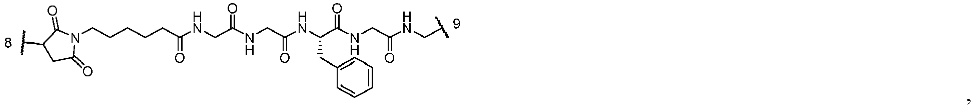 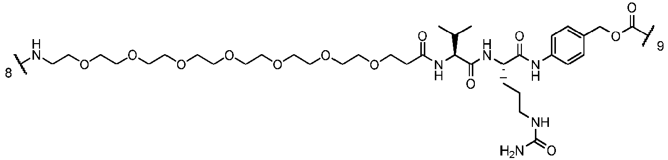 and 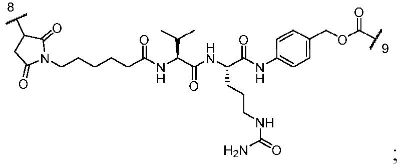 wherein, Position 8 is connected to Aa, and Position 9 is connected to D¹.

In some embodiments, L² is a non-cleavable linker or a cleavable linker, wherein the cleavable linker is cleaved by an enzyme present in a pathological environment, and the enzyme is selected from the group consisting of protease, phosphatase, pyrophosphatase, *β*-glucuronidase, *β*-galactosidase, and sulfatase.

In some embodiments, L² is -L^{d}-L^{e}-L^{f}-, wherein:
L^{d} is a covalent bond or a divalent structure consisting of one or more selected from the following: C₁₋₆ alkylene, -C(O)-(CH₂)_{d}-NH-, 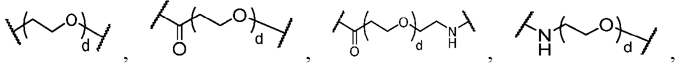 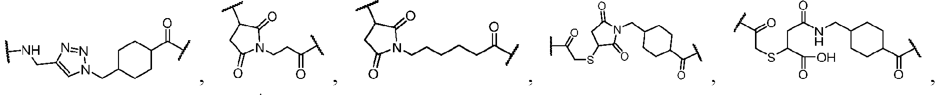 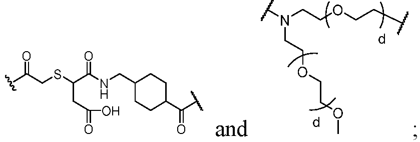 wherein, each d is independently an integer selected from 1 to 12;
L^{e} is a covalent bond or selected from the group consisting of an amino acid fragment, and a peptide fragment formed of two or more amino acids, wherein the amino acid is selected from the group consisting of Val, Cit, Glu, Lys, Arg, Phe, Leu, Gly, Ala and Asn;
L^{f} is a covalent bond, -CH₂-NH- or selected from the group consisting of the following structures:

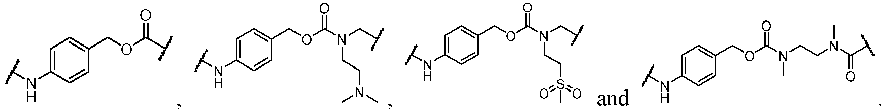

In some embodiments, L² is -L^{d}-L^{e}-L^{f}-, wherein:
L^{d} is a covalent bond or a divalent structure selected from the group consisting of one or more of the following: C₁₋₆ alkylene, -C(O)-(CH₂)_{d}-NH-, 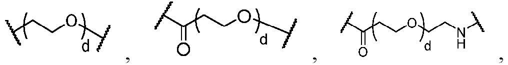 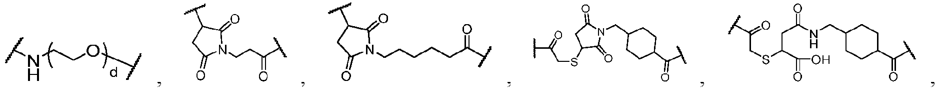 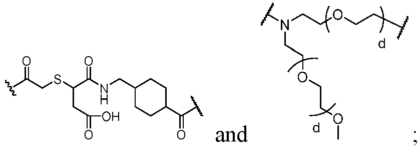 wherein, each d is independently an integer selected from 1 to 12;
L^{e} is a covalent bond or selected from the group consisting of an amino acid fragment, and a peptide fragment formed of two or more amino acids, wherein the amino acid is selected from the group consisting of Val, Cit, Glu, Lys, Arg, Phe, Leu, Gly, Ala and Asn;
L^{f} is a covalent bond, -CH₂-NH- or selected from the group consisting of the following structures:

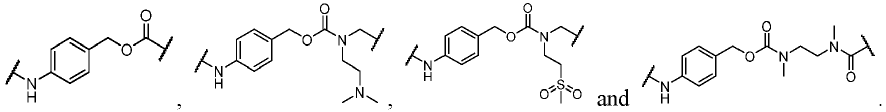

In some embodiments, L^{e} is a covalent bond or selected from the group consisting of the following structures: 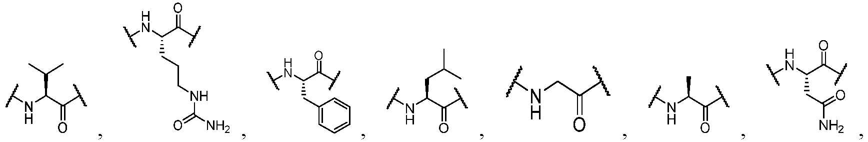 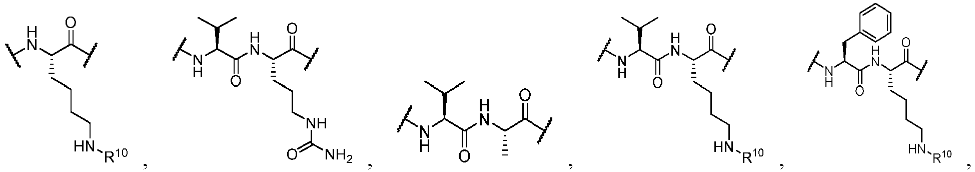 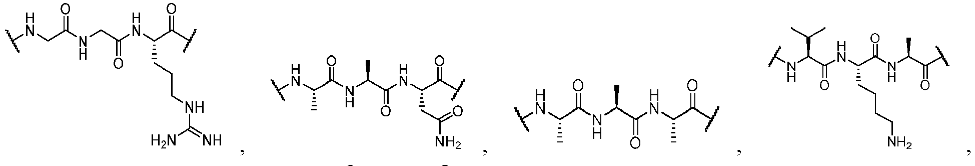 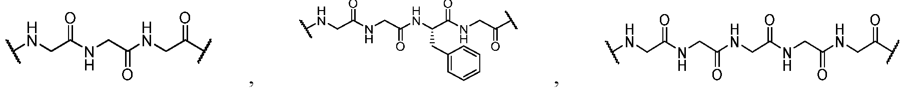 and 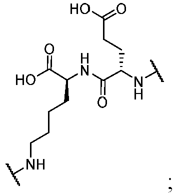 wherein, each R¹⁰ is independently selected from the group consisting of H, acetyl, fluorenyloxycarbonyl, trityl and a polyethylene glycol hydrophilic structural unit; preferably, the polyethylene glycol hydrophilic structural unit is 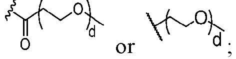 each d is independently an integer selected from 1 to 10.

In some embodiments, L² is selected from the group consisting of the following structures: 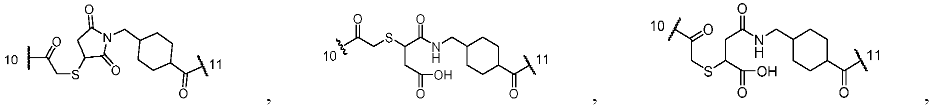 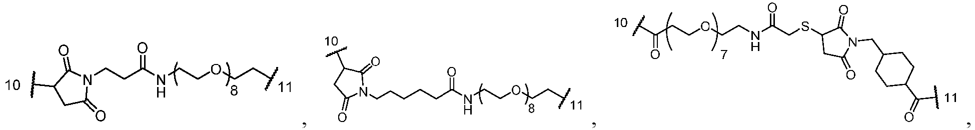 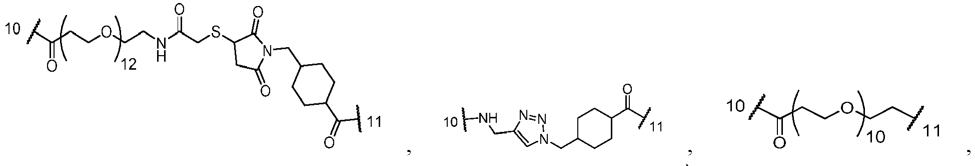 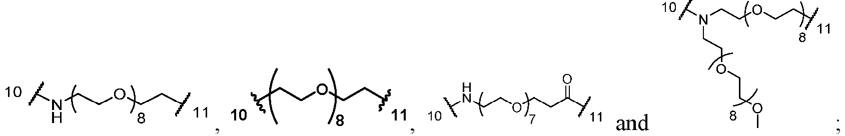 wherein, Position 10 is connected to Aa, and Position 11 is connected to D².

In some embodiments, L² is selected from the group consisting of the following structures: 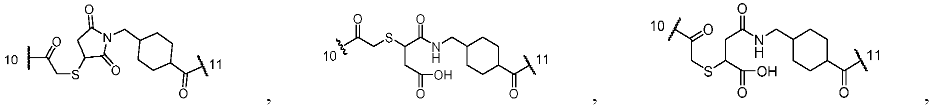 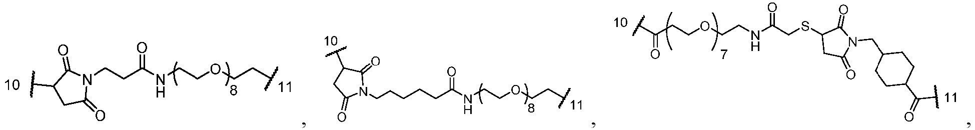 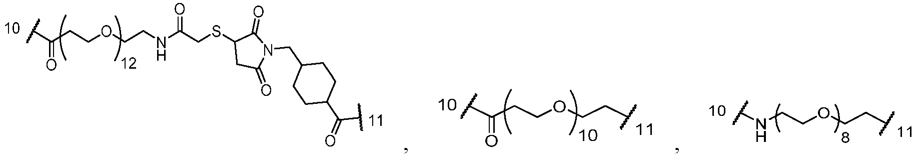 and 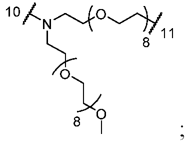 wherein, Position 10 is connected to Aa, and Position 11 is connected to D²;

In some embodiments, L² is selected from the group consisting of the following structures: 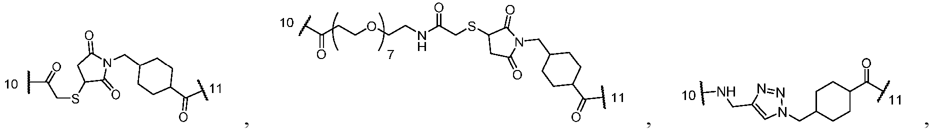 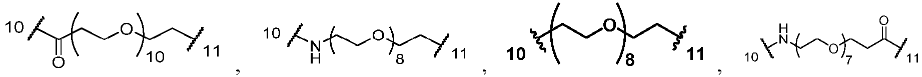 and 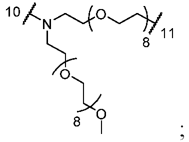 wherein, Position 10 is connected to Aa, and Position 11 is connected to D².

In some embodiments, L² is selected from the group consisting of the following structures: 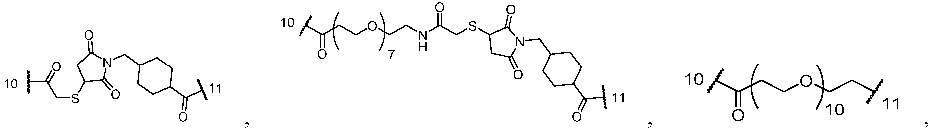 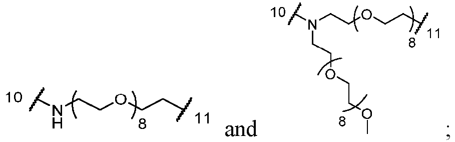 wherein, Position 10 is connected to Aa, and Position 11 is connected to D².

In some embodiments, L² is selected from the group consisting of the following structures: 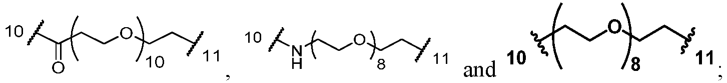 wherein, Position 10 is connected to Aa, and Position 11 is connected to D².

In some embodiments, D¹ is selected from the group consisting of cytotoxic drug fragments, and the cytotoxic drug is selected from the group consisting of tubulin inhibitors, DNA damaging agents and topoisomerase inhibitors, the tubulin inhibitor comprises, but not limited to dolastatin, auristatins, maytansines, tubulysins and cryptomycins, the DNA damaging agent comprises, but not limited to PBDs, duocarmycins and calicheamicins, and the topoisomerase inhibitor comprises, but not limited to camptothecin and derivatives thereof.

In some embodiments, the tubulin inhibitor is selected from the group consisting of dolastatin 10, MMAE, MMAF, maytansine, DM1, DM3, and DM4, and the topoisomerase inhibitor is selected from the group consisting of camptothecin, SN-38, exatecan, topotecan, belotecan, 10-hydroxy-camptothecin, 9-amino-camptothecin, doxorubicin, epirubicin and PNU-159682.

In some embodiments, D¹ is selected from the group consisting of the following structures: 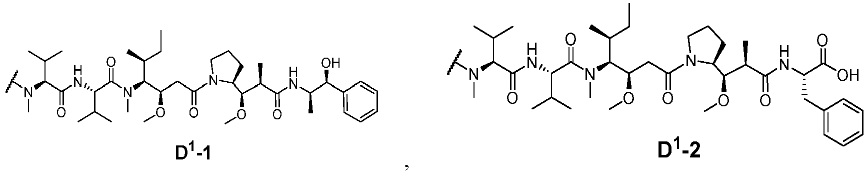 and

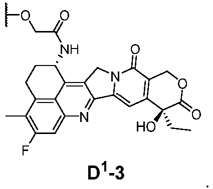

In some embodiments, D² is a TLR agonist fragment.

In some embodiments, the TLR agonist is selected from the group consisting of TLR2 agonists, TLR4 agonists, TLR6 agonists, TLR7 agonists, TLR8 agonists, TLR7/8 agonists, and TLR9 agonists.

In some embodiments, D² is a TLR agonist fragment, and the TLR agonist is selected from the group consisting of TLR7 agonists, TLR8 agonists and TLR7/8 agonists.

In some embodiments, D² is a TLR agonist fragment, and the TLR agonist is a compound represented by Formula (II): 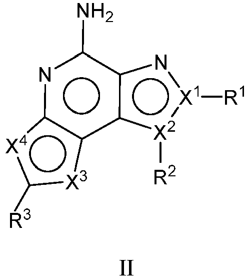 wherein,
X¹ is N or C;
X² is N or C;
and at least one of X¹ and X² is N;
X³ is selected from the group consisting of O, S and N;
X⁴ is selected from the group consisting of O, S, N and CR⁴;
R¹ is selected from the group consisting of C₁₋₆ alkyl and -C₁₋₆ alkylene-O-C₁₋₆ alkyl;
R² is hydrogen or the formula -L³-L⁴-L⁵-L⁶;
L³ is a covalent bond or C₁₋₆ alkylene;
L⁴ is selected from the group consisting of a covalent bond, C₃₋₁₀ cycloalkyl, 3- to 12-membered heterocyclyl, C₆₋₁₀ aryl, and 5- to 10-membered heteroaryl, and the cycloalkyl, heterocyclyl, aryl and heteroaryl is optionally substituted with one or more groups selected from the group consisting of hydrogen, halogen, cyano, C₁₋₆ alkyl, C₁₋₆ alkoxy and -C₁₋₆ alkylene-NH₂;
L⁵ is selected from the group consisting of a covalent bond, -O-, -S-, -C(O)-, -O-C(O)-, -C(O)-O-, -O-C(O)-O-, -NR⁵-, -C(O)-NR⁵-, -NR⁵-C(O)-, -O-C(O)-NR⁵-, -NR⁵-C(O)-O-, -NR⁵-C(O)-NR⁵-, -S(O)ᵣ-NR⁵-, -NR⁵-S(O)ᵣ- and -NR⁵-S(O)ᵣ-NR⁵-;
L⁶ is selected from the group consisting of hydrogen, C₁₋₆ alkyl, -(O-CH₂CH₂)ₙ-O-C₁₋₆ alkyl and -C₁₋₆ alkylene-(O-CH₂CH₂)ₙ-O-C₁₋₆ alkyl, and the alkyl is optionally substituted with one or more groups selected from the group consisting of hydrogen, halogen, hydroxyl, -NH₂, -NH-C(O)-O-C₁₋₆ alkyl, C₁₋₆ alkoxy, carboxyl and -C(O)-O-C₁₋₆ alkyl;
R¹ is hydrogen or the formula -L⁷-L⁸-L⁹-L¹⁰;
L⁷ is selected from the group consisting of a covalent bond and C₁₋₆ alkylene;
L⁸ is selected from the group consisting of a covalent bond, C₃₋₁₀ cycloalkyl, 3- to 12-membered heterocyclyl, C₆₋₁₀ aryl, and 5- to 10-membered heteroaryl, and the cycloalkyl, heterocyclyl, aryl and heteroaryl is optionally substituted with one or more groups selected from the group consisting of hydrogen, halogen, cyano, C₁₋₆ alkyl, C₁₋₆ alkoxy and -C₁₋₆ alkylene-NH₂;
L⁹ is selected from the group consisting of a covalent bond, -O-, -S-, -C(O)-, -O-C(O)-, -C(O)-O-, -O-C(O)-O-, -NR⁶-, -C(O)-NR⁶-, -NR⁶-C(O)-, -O-C(O)-NR⁶-, -NR⁶-C(O)-O-, -NR⁶-C(O)-NR⁶-, -S(O)ₚ-NR⁶-, -NR⁶-S(O)ₚ- and -NR⁶-S(O)ₚ-NR⁶-;
L¹⁰ is selected from the group consisting of hydrogen, C₁₋₆ alkyl, -(O-CH₂CH₂)_{q}-O-C₁₋₆ alkyl, and -C₁₋₆ alkylene-(O-CH₂CH₂)_{q}-O-C₁₋₆ alkyl, and the alkyl is optionally substituted with one or more groups selected from the group consisting of hydrogen, halogen, hydroxyl, -NH₂, -NH-C(O)-O-C₁₋₆ alkyl, C₁₋₆ alkoxy, carboxyl and -C(O)-O-C₁₋₆ alkyl;
R⁴ is selected from the group consisting of hydrogen, halogen, cyano, hydroxyl, C₁₋₆ alkyl, C₁₋₆ haloalkyl, C₁₋₆ alkoxy and C₃₋₁₀ cycloalkyl;
each R⁵ is independently hydrogen or C₁₋₆ alkyl;
each R⁶ is independently hydrogen or C₁₋₆ alkyl;
each r is independently 1 or 2;
each n is independently an integer selected from 1 to 25;
each p is independently 1 or 2;
each q is independently an integer selected from 1 to 25;
D² is connected to L² through R² or R³ in Formula (II).

In some embodiments, each n is independently selected from 1 to 15, preferably, n is selected from the group consisting of 1, 2, 3, 4, 5, 6, 7, 8, 9, 10, 11, 12, 13, 14 and 15.

In some embodiments, each q is independently selected from 1 to 15, preferably, q is selected from the group consisting of 1, 2, 3, 4, 5, 6, 7, 8, 9, 10, 11, 12, 13, 14 and 15.

In some embodiments, X³ is selected from the group consisting of S and N.

In some embodiments, L³ is selected from the group consisting of a covalent bond and C₁₋₃ alkylene.

In some embodiments, L⁴ is selected from the group consisting of a covalent bond, 3- to 12-membered heterocyclyl, C₆₋₁₀ aryl, and 5- to 10-membered heteroaryl, the heterocyclyl, aryl, and heteroaryl are optionally substituted with one or more groups selected from the group consisting of hydrogen, halogen, cyano, C₁₋₃ alkyl, C₁₋₃ alkoxy and -C₁₋₆ alkylene-NH₂.

In some embodiments, L⁵ is selected from the group consisting of a covalent bond, -NR⁵-, -C(O)-NR⁵-, -NR⁵-C(O)-, -O-C(O)-NR⁵-, -NR⁵-C(O)-O-, -NR⁵-C(O)-NR⁵-, -S(O)ᵣ-NR⁵-, -NR⁵-S(O)ᵣ-, and -NR⁵-S(O)ᵣ-NR⁵-, each R⁵ is independently selected from the group consisting of hydrogen and C₁₋₆ alkyl; preferably, L⁵ is selected from the group consisting of a covalent bond and -NR⁵-, and each R⁵ is independently selected from the group consisting of hydrogen and C₁₋₆ alkyl.

In some embodiments, L⁶ is selected from the group consisting of hydrogen, C₁₋₆ alkyl, and -C₁₋₆ alkylene-(O-CH₂CH₂)ₙ-O-C₁₋₆ alkyl, and the alkyl is optionally substituted with one or more groups selected from the group consisting of hydrogen, halogen, hydroxyl, -NH₂, -NH-C(O)-O-C₁₋₆ alkyl, C₁₋₆ alkoxy, carboxyl and -C(O)-O-C₁₋₆ alkyl, and n is 1 to 10.

In some embodiments, L⁸ is selected from the group consisting of a covalent bond and 3- to 12-membered heterocyclyl, and the heterocyclyl is optionally substituted with one or more groups selected from the group consisting of hydrogen, halogen, cyano and C₁₋₆ alkyl.

In some embodiments, L⁹ is a covalent bond.

In some embodiments, L¹⁰ is selected from the group consisting of hydrogen, C₁₋₆ alkyl, and -C₁₋₆ alkylene-(O-CH₂CH₂)_{q}-O-C₁₋₆ alkyl, and q is 1-10.

In some embodiments, R³ is selected from the group consisting of hydrogen and the formula -L⁷-L⁸-L⁹-L¹⁰;
L⁸ is selected from the group consisting of a covalent bond and 3- to 12-membered heterocyclyl, and the heterocyclyl is optionally substituted with one or more groups selected from the group consisting of hydrogen, halogen, cyano and C₁₋₆ alkyl;
L⁹ is a covalent bond;
L¹⁰ is selected from the group consisting of hydrogen, C₁₋₆ alkyl and -C₁₋₆ alkylene-(O-CH₂CH₂)_{q}-O-C₁₋₆ alkyl;
q is selected from 1 to 10, such as 1, 2, 3, 4, 5, 6, 7, 8, 9 and 10.

In some embodiments, X¹ is C;
X² is N;
X³ is S;
X⁴ is selected from CR⁴;
R¹ is selected from C₁₋₆ alkyl;
R² is selected from the formula-L³-L⁴-L⁵-L⁶;
L³ is selected from the group consisting of a covalent bond and C₁₋₆ alkylene;
L⁴ is selected from the group consisting of a covalent bond, 3- to 12-membered heterocyclyl, C₆₋₁₀ aryl and 5- to 10-membered heteroaryl, and the heterocyclyl, aryl and heteroaryl are optionally substituted with one or more groups selected from the group consisting of hydrogen, halogen, cyano, C₁₋₃ alkyl, C₁₋₃ alkoxy and -C₁₋₆ alkylene-NH₂;
L⁵ is selected from the group consisting of a covalent bond and -NR⁵-, and each R⁵ is independently selected from the group consisting of hydrogen and C₁₋₆ alkyl;
L⁶ is selected from the group consisting of hydrogen, C₁₋₆ alkyl and -C₁₋₆ alkylene-(O-CH₂CH₂)ₙ-O-C₁₋₆ alkyl, and the alkyl is optionally substituted with one or more groups selected from the group consisting of hydrogen, halogen, hydroxyl, -NH₂, -NH-C(O)-O-C₁₋₆ alkyl, C₁₋₆ alkoxy, carboxyl and -C(O)-O-C₁₋₆ alkyl.

In some embodiments, D² is selected from the group consisting of the following structures: 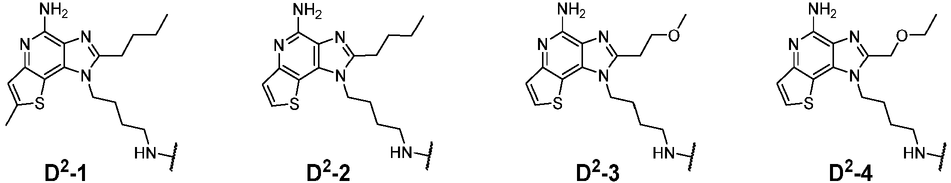 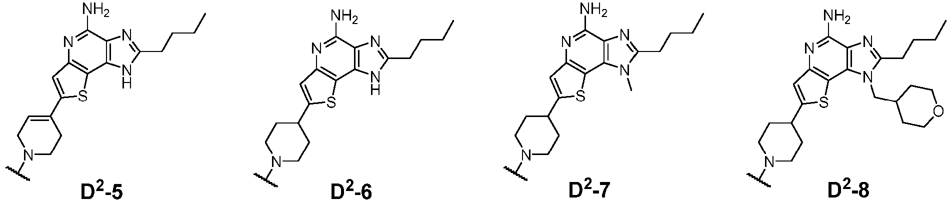 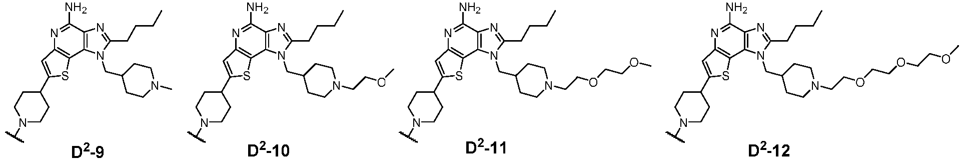 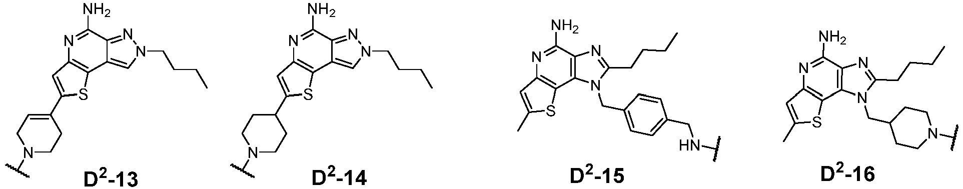 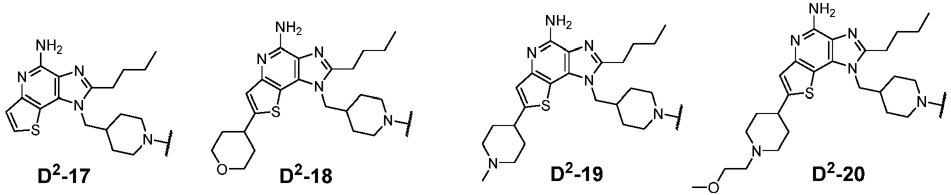 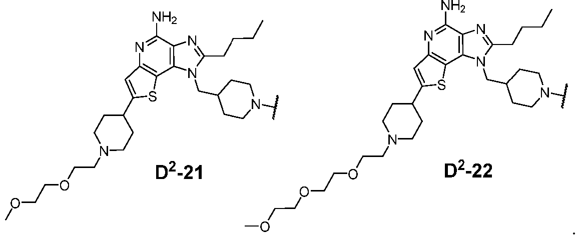 In some embodiments, the compound or pharmaceutically acceptable salt, ester, stereoisomer, tautomer, polymorph, solvate, *N*-oxide, isotopically labeled compound, metabolite or prodrug thereof is selected from the group consisting of the following structures: 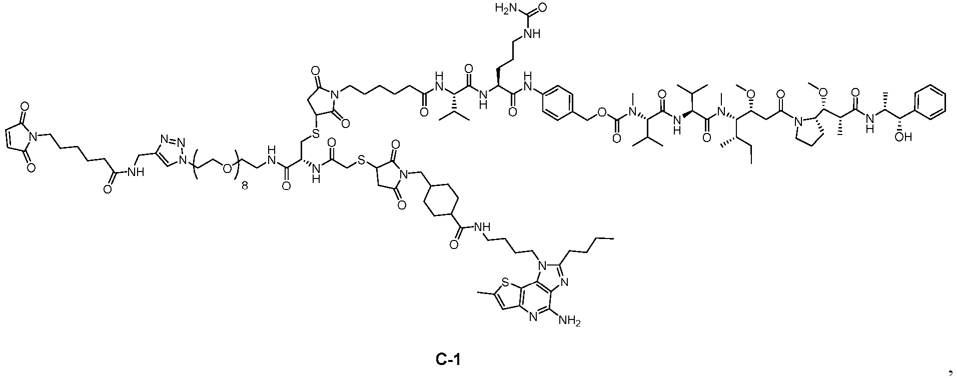 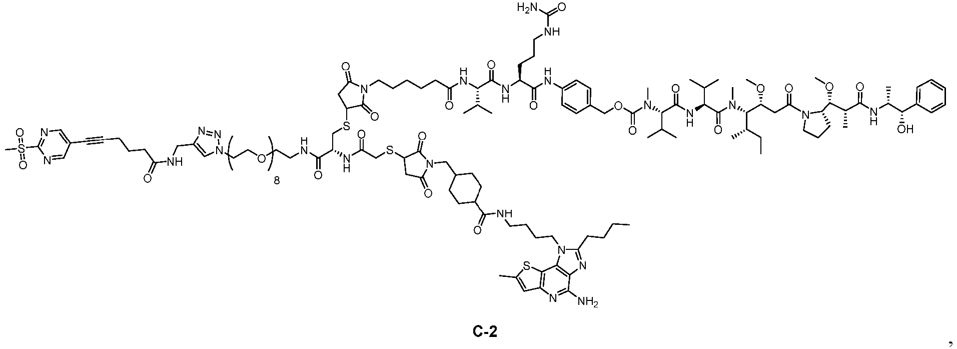 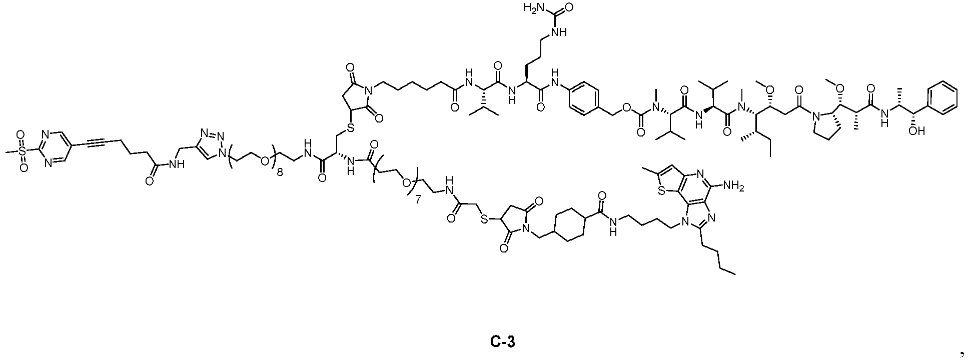 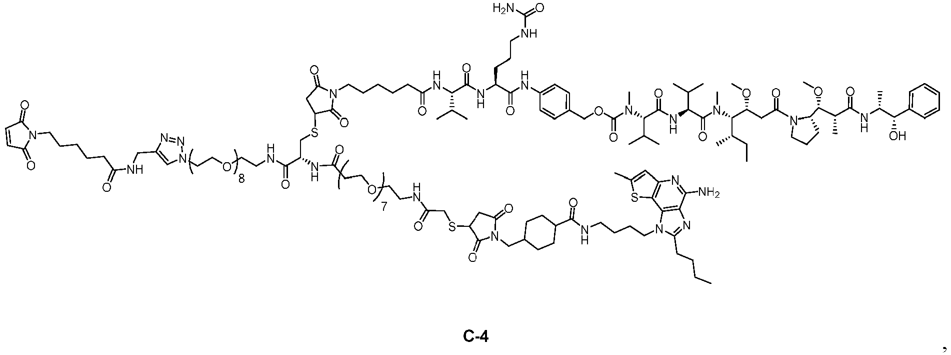 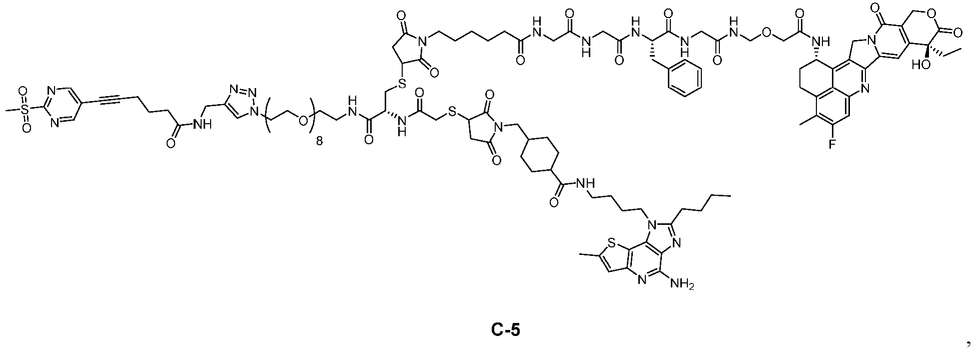 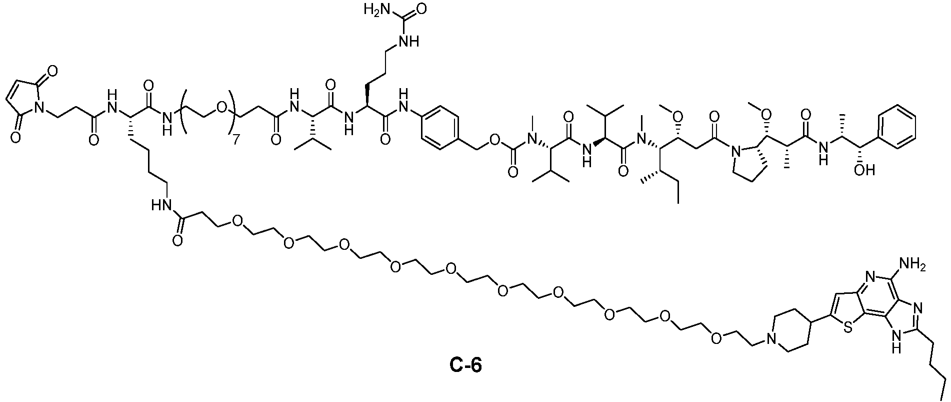 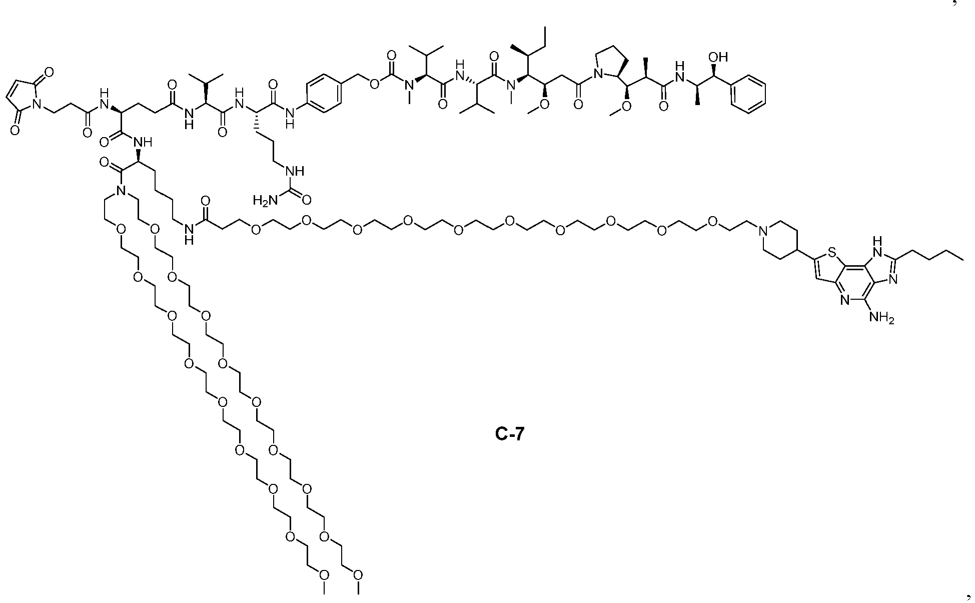 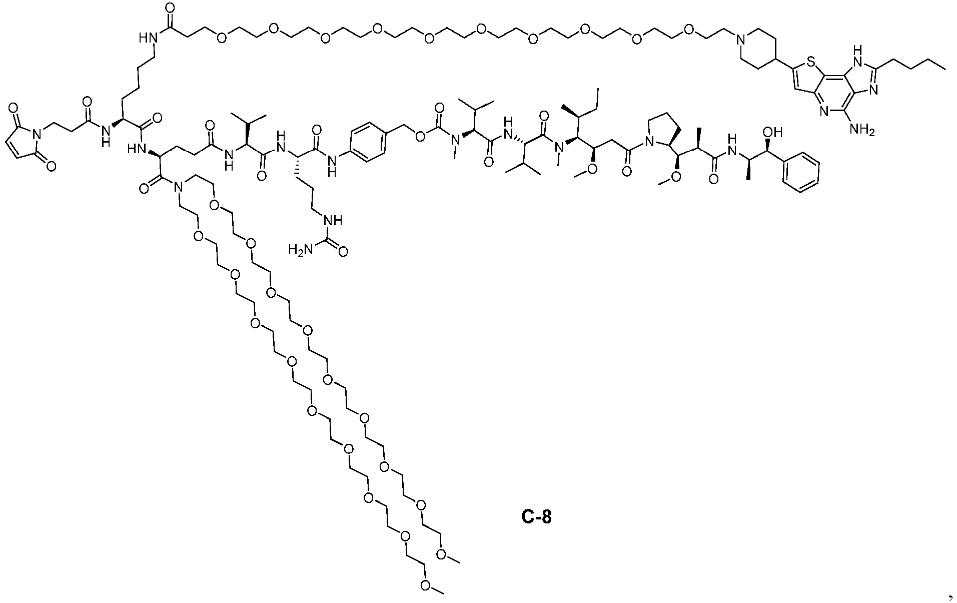 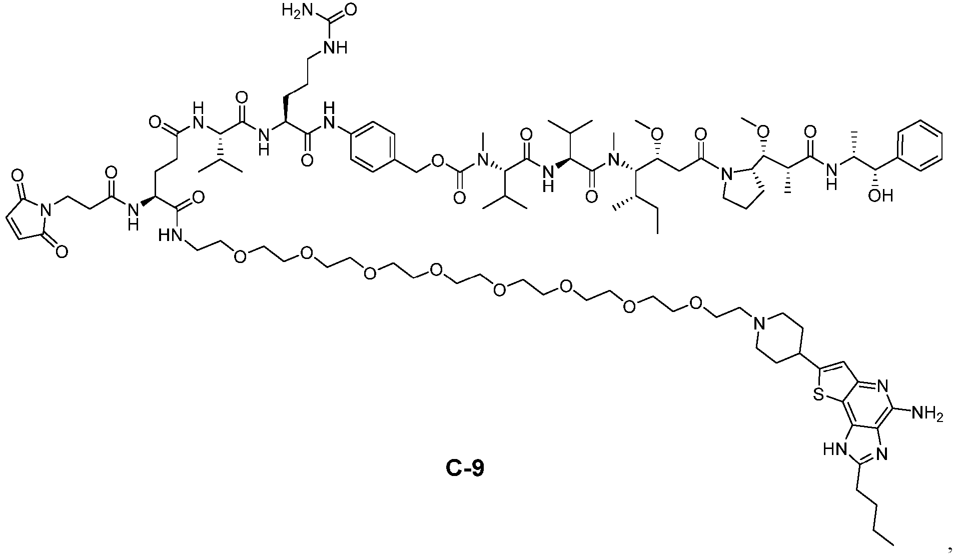 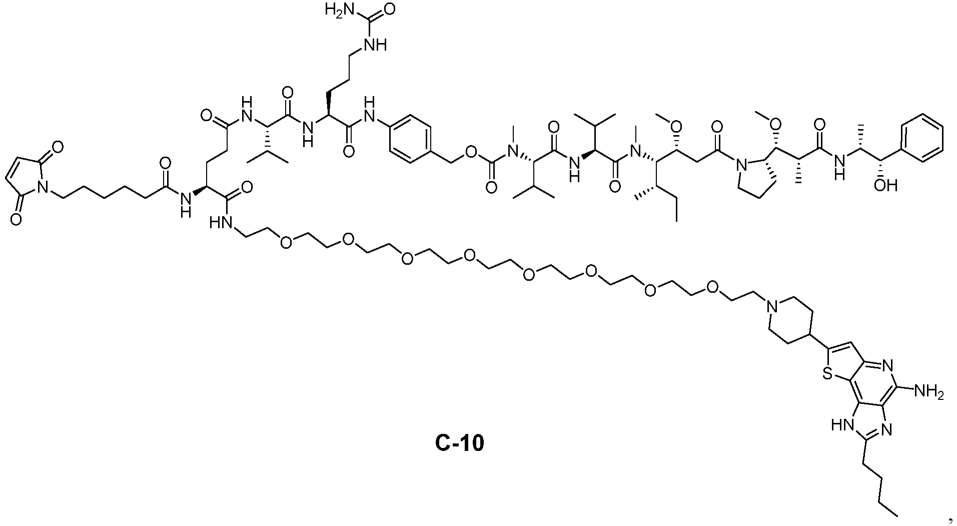 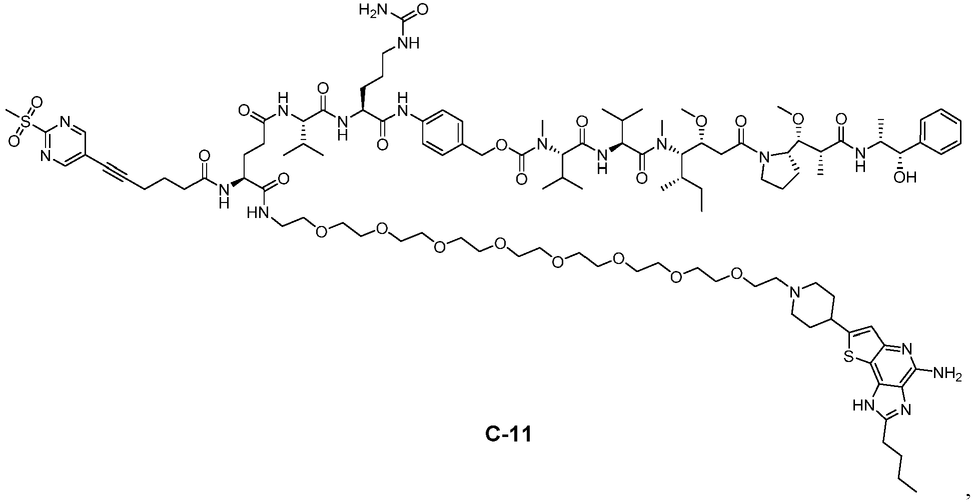 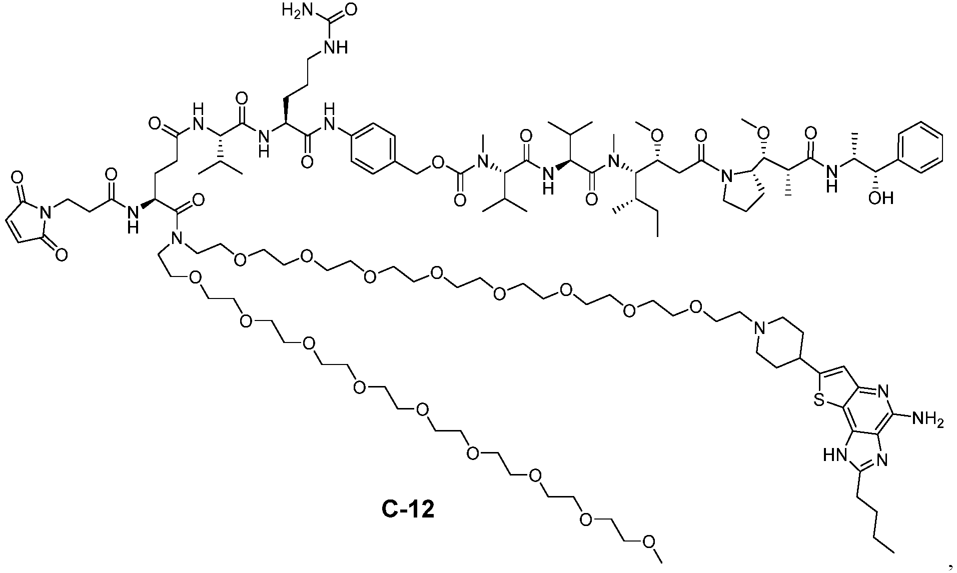 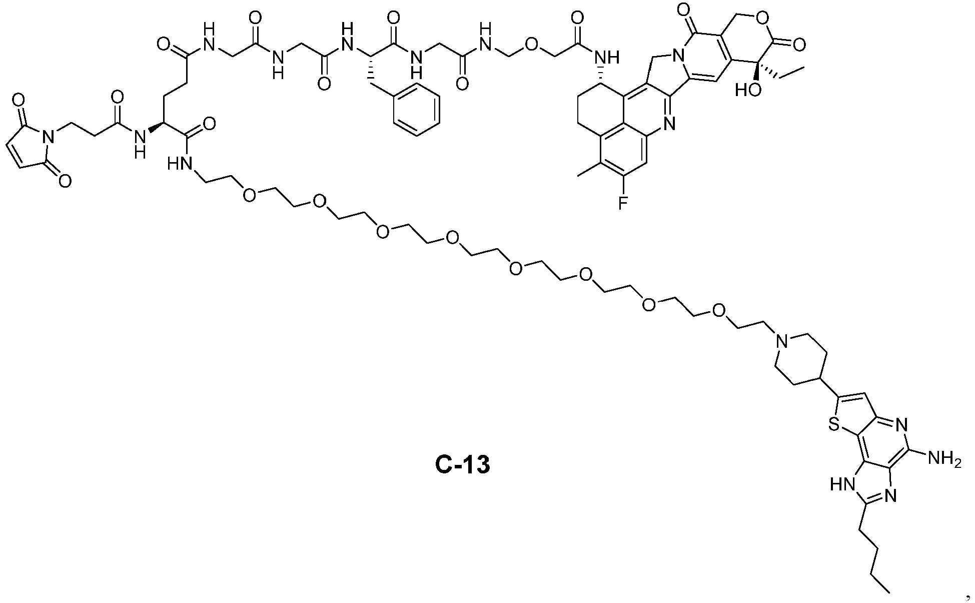 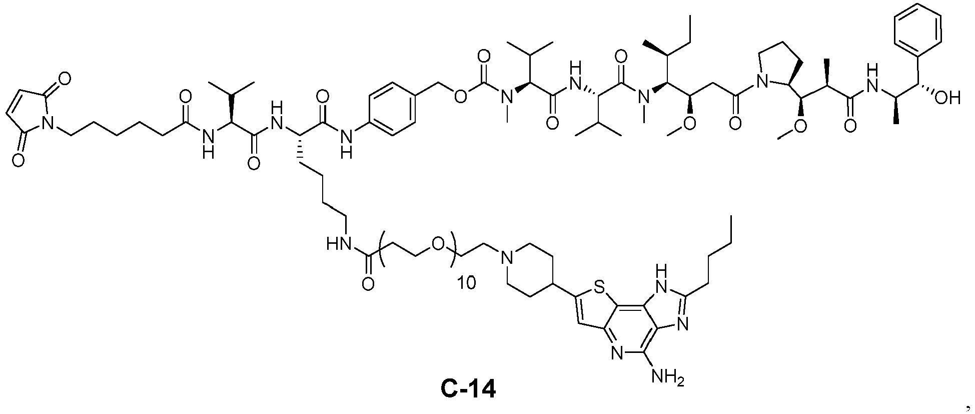 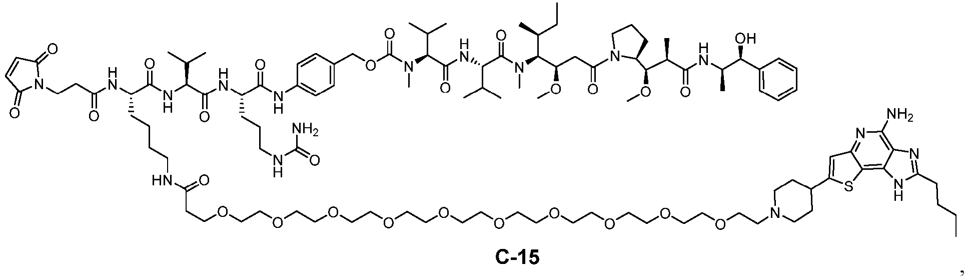 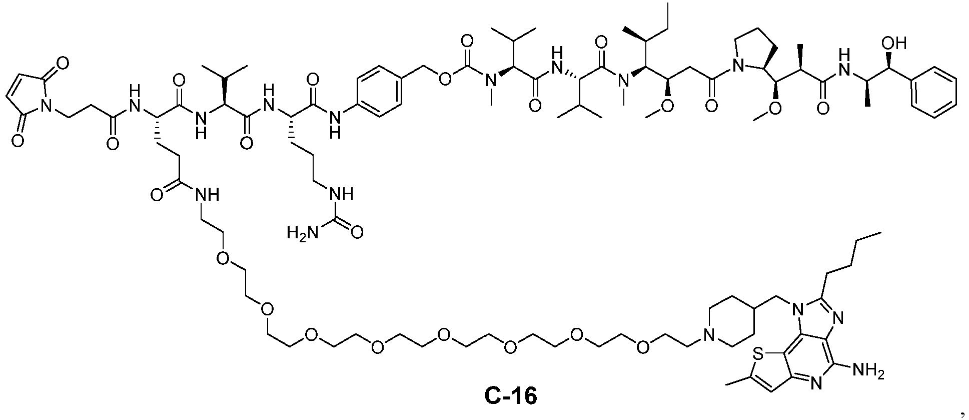 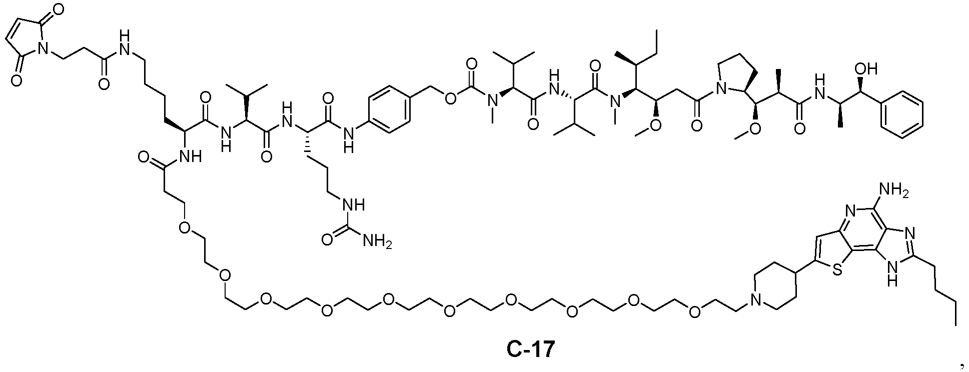 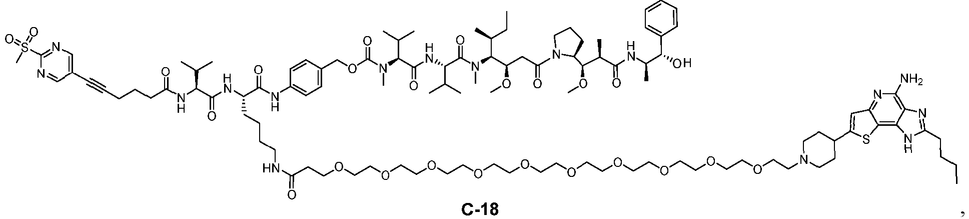 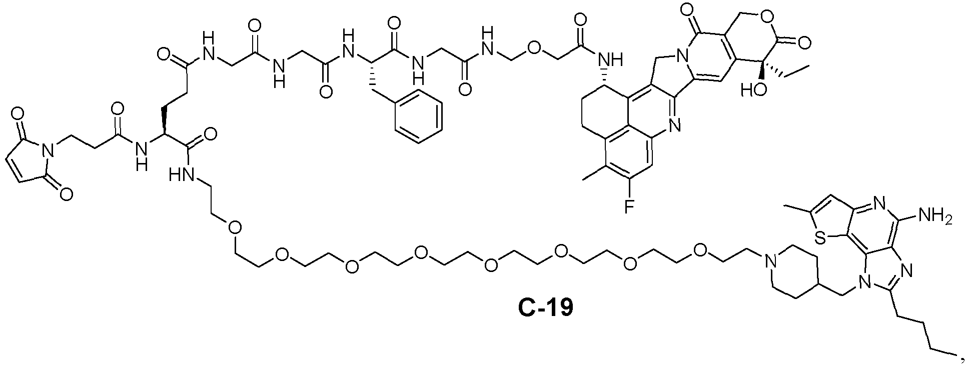 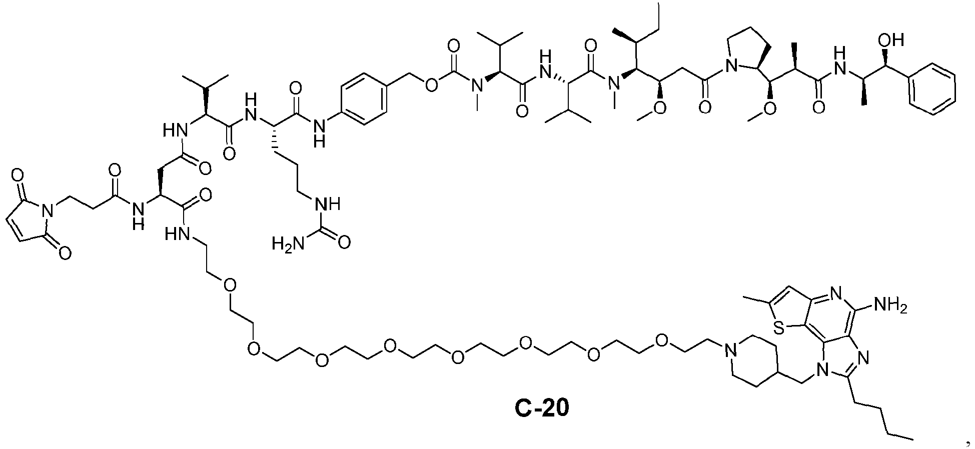 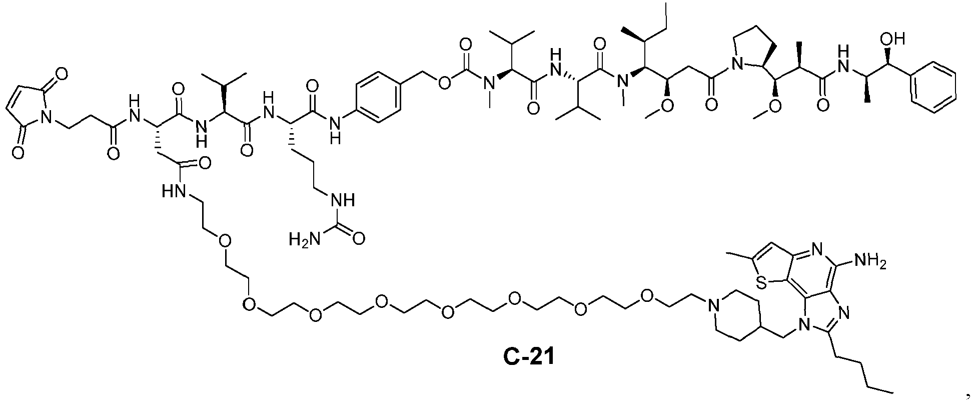 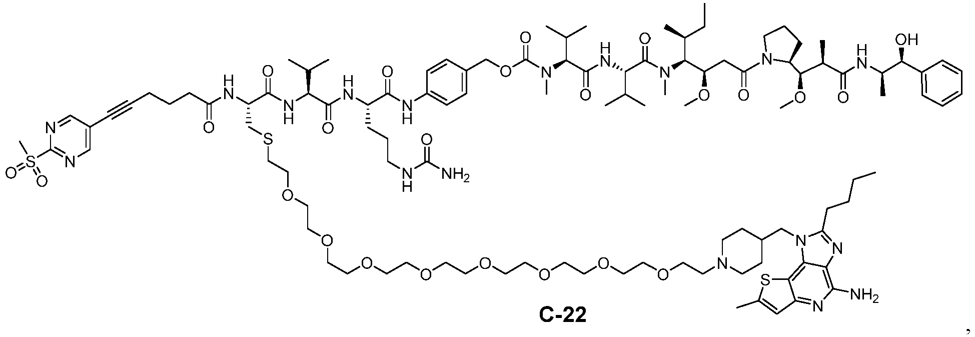 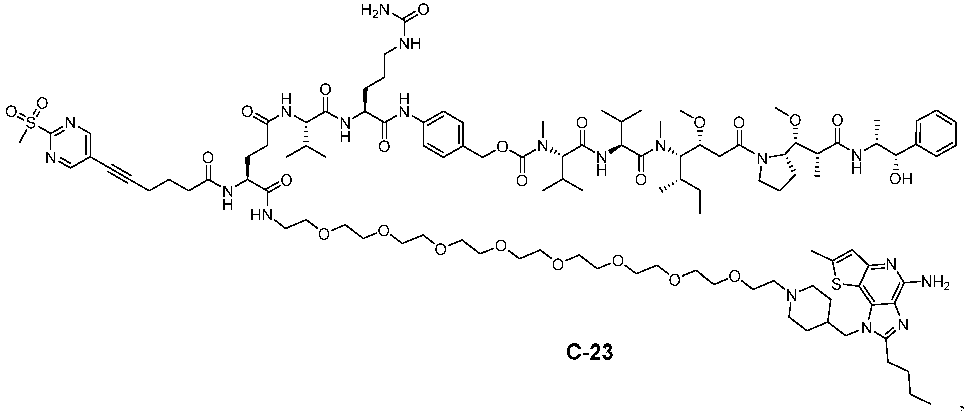 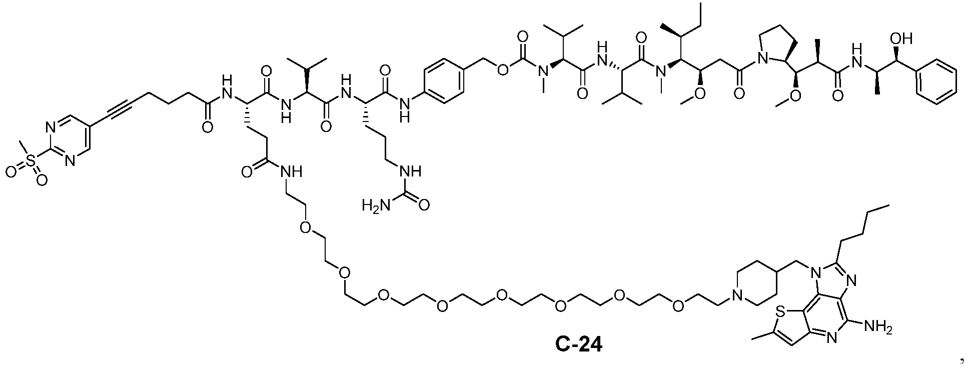 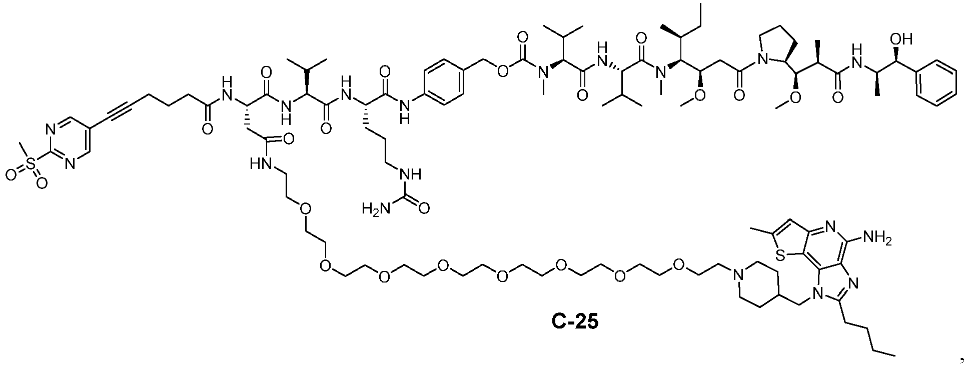 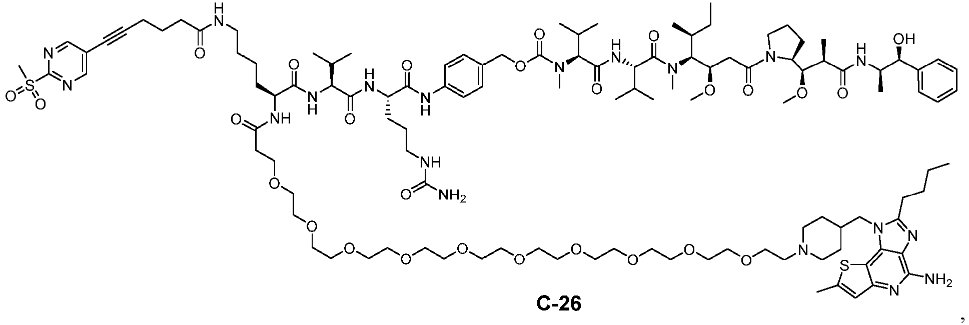 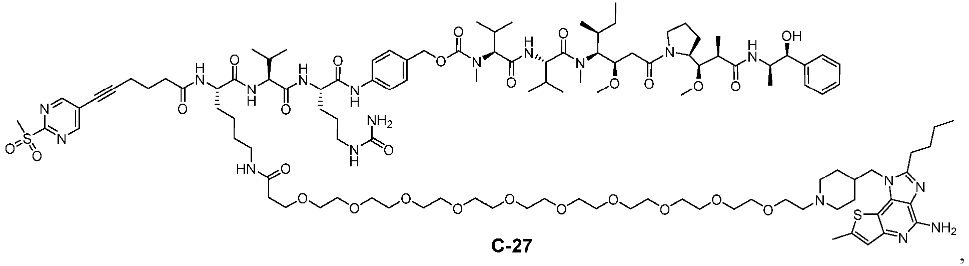 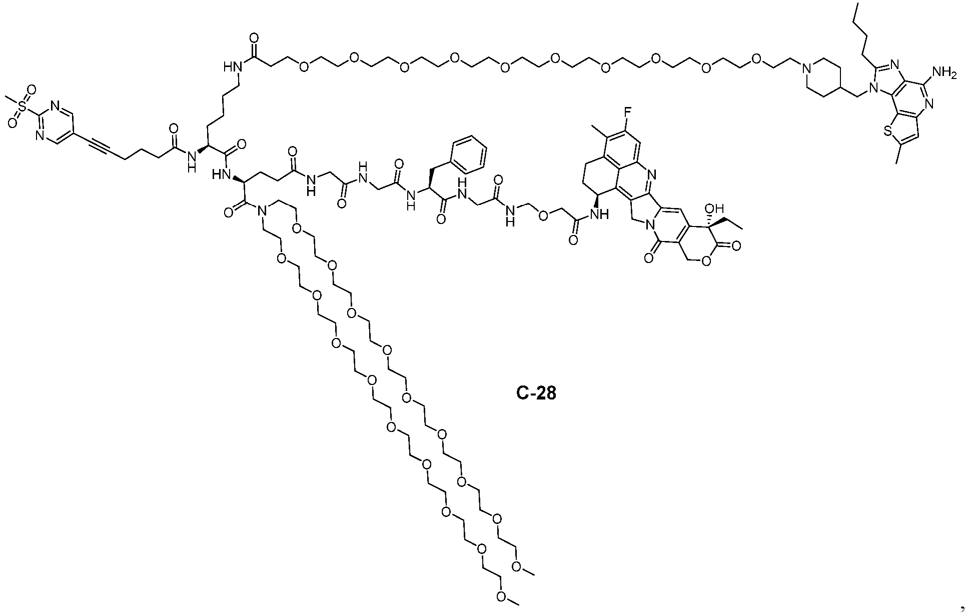 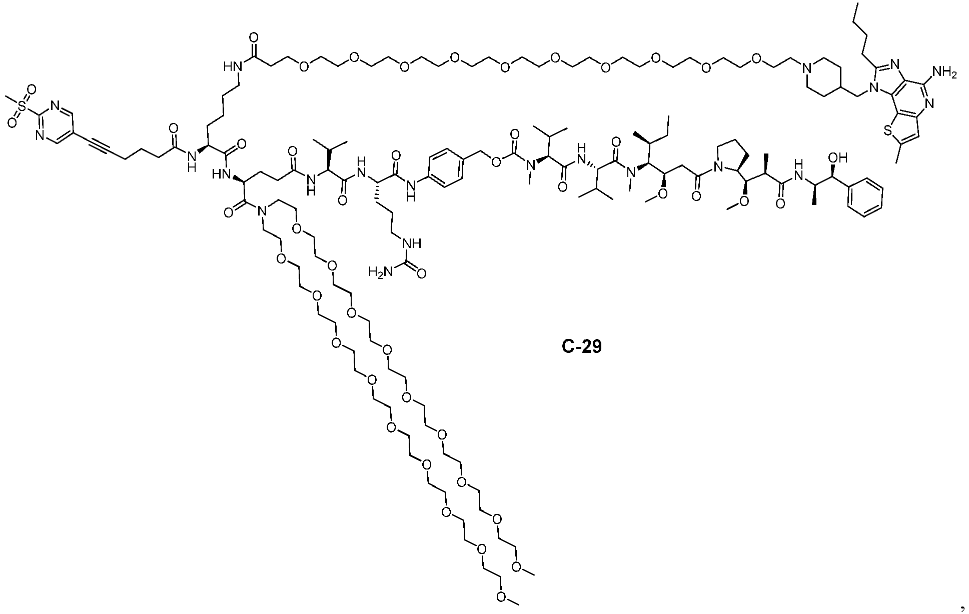 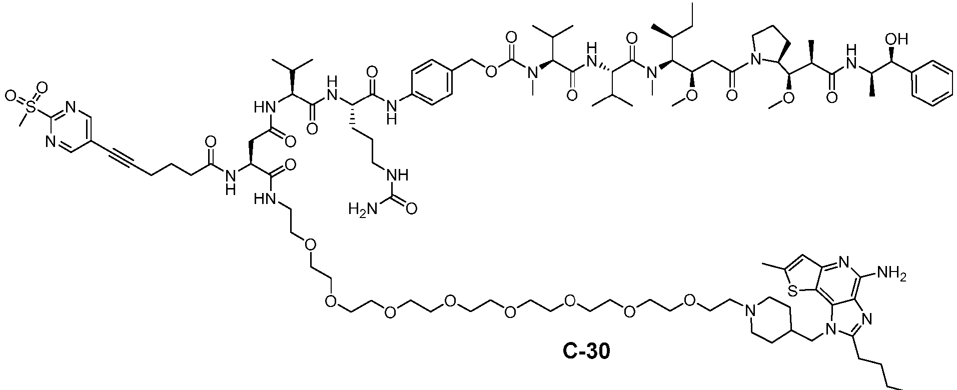 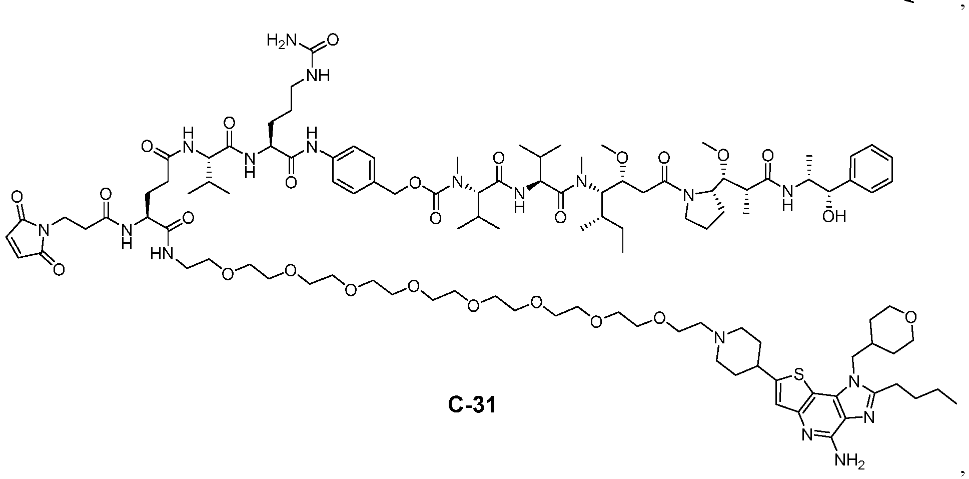 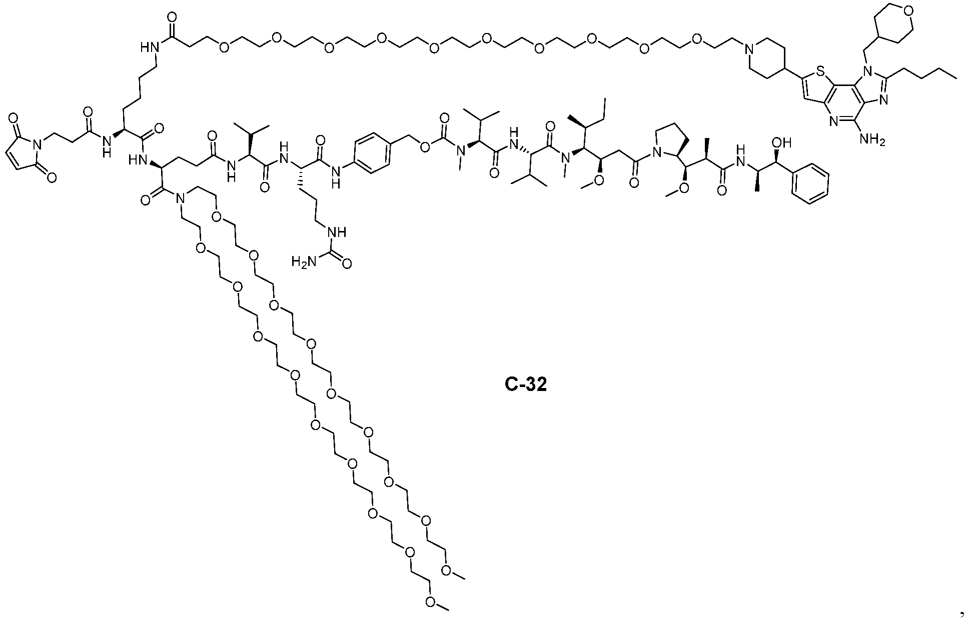 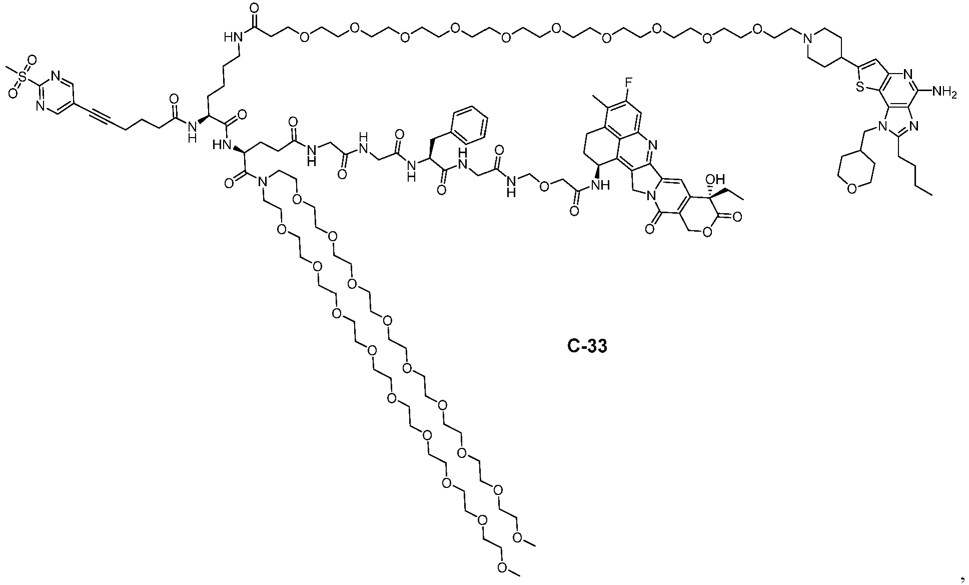 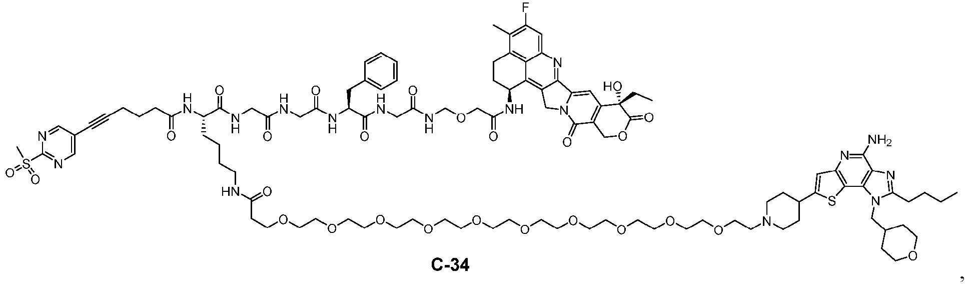 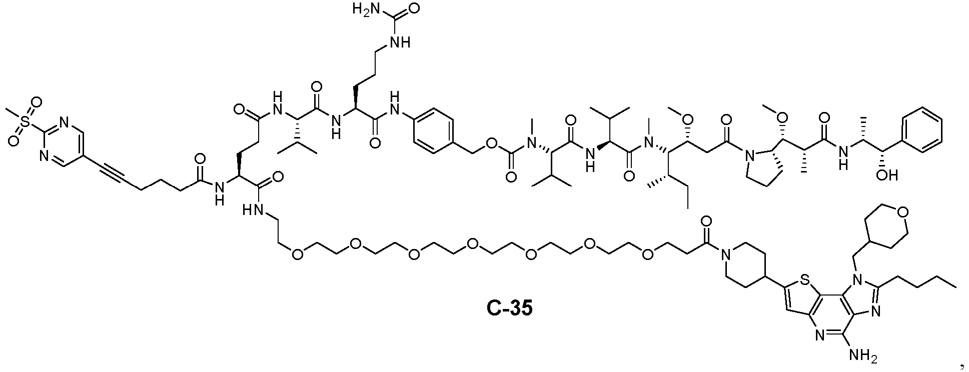 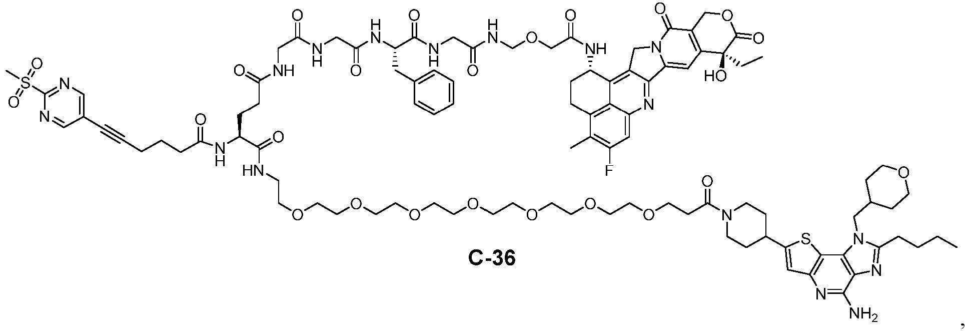 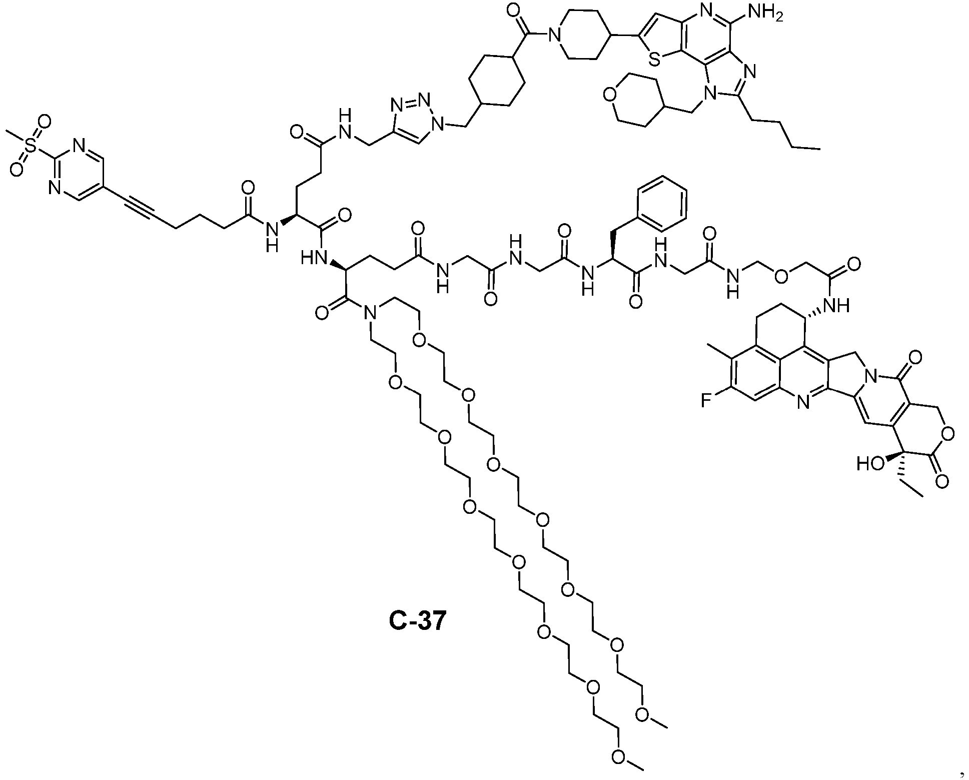 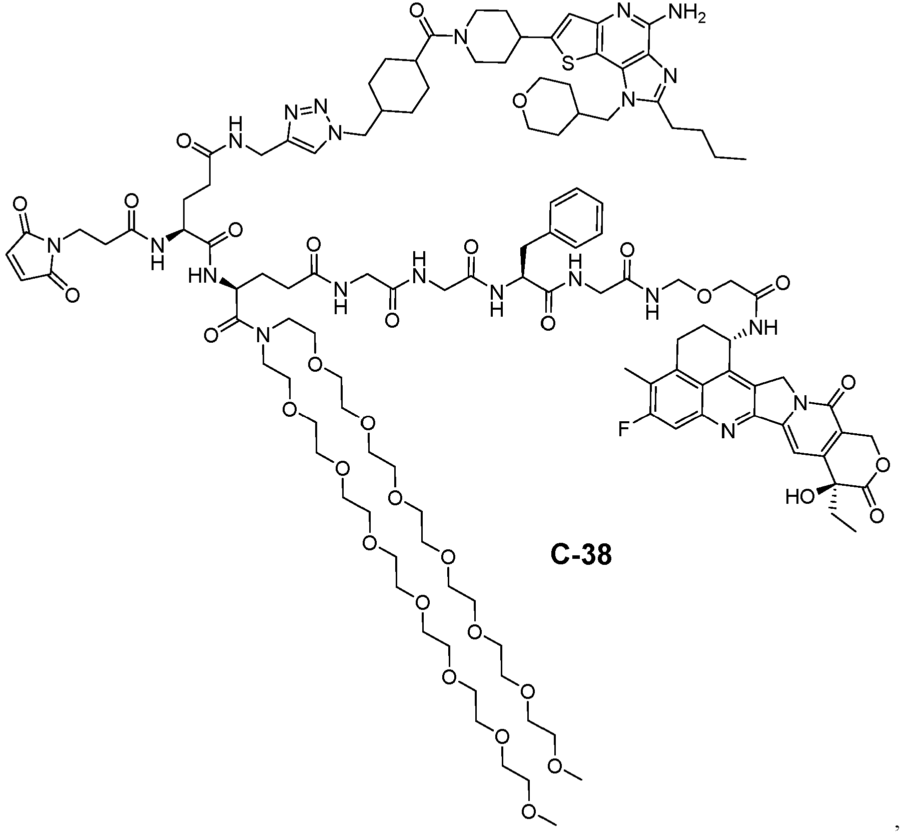 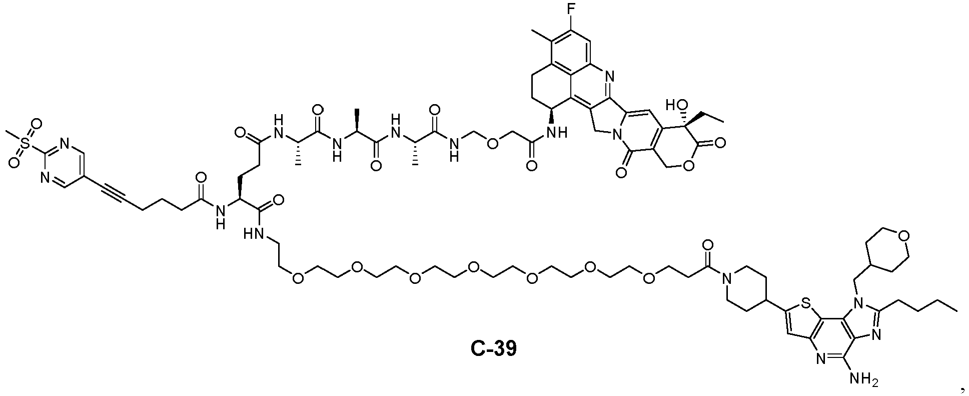 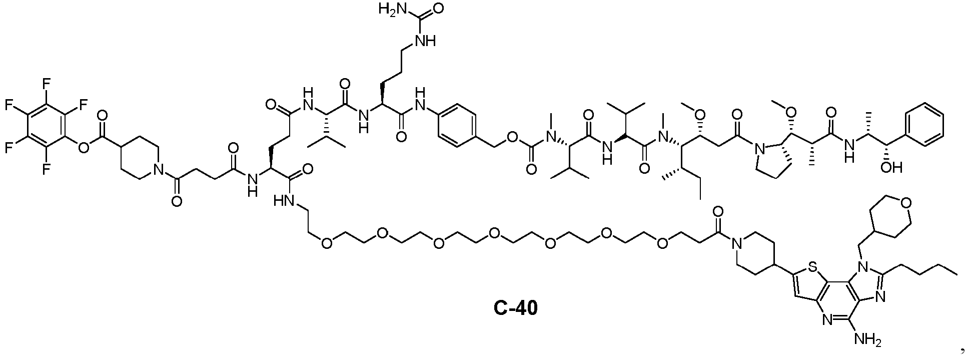 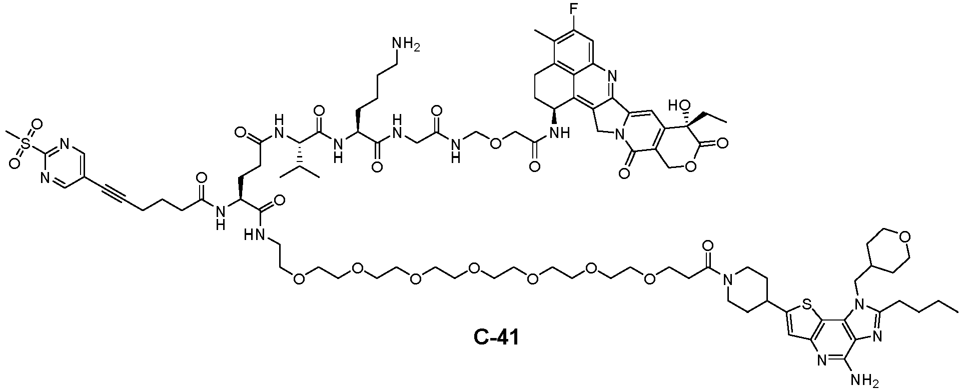 and

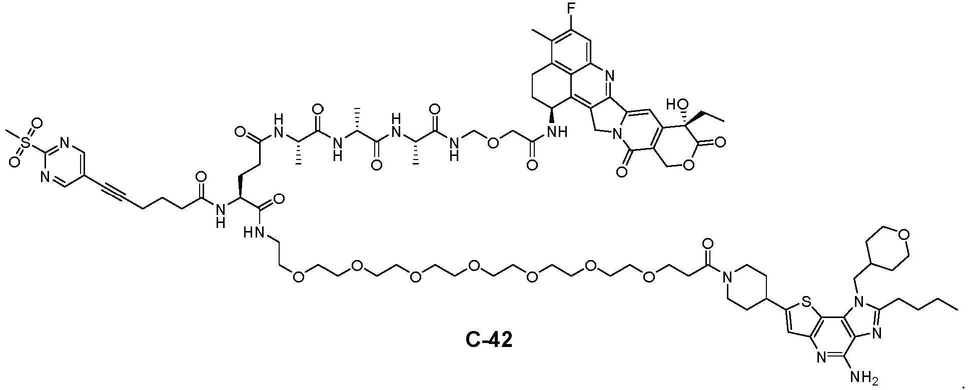

### Pharmaceutical composition

In another aspect, the present invention provides a pharmaceutical composition, which comprises the antibody-drug conjugate, compound (e.g., drug linker, TLR agonist or cytotoxic drug) or pharmaceutically acceptable salt, ester, stereoisomer, tautomer, polymorph, solvate, N-oxide, isotopically labeled compound, metabolite or prodrug thereof according to any one of proceeding paragraphs, and one or more pharmaceutical excipients.

Methods for preparing various pharmaceutical compositions containing certain amount of active ingredients are known or will be apparent to those skilled in the art in light of the disclosure of the present invention. According to the description in REMINGTON'S PHARMACEUTICAL SCIENCES, Martin, E.W., ed., Mack Publishing Company, 19th ed. (1995), the method of preparing the pharmaceutical composition comprises adding an appropriate pharmaceutical excipient.

### Uses

In another aspect, the present invention provides use of the antibody-drug conjugate, compound (e.g., drug linker, TLR agonist or cytotoxic drug) or pharmaceutically acceptable salt, ester, stereoisomer, tautomer, polymorph, solvate, N-oxide, isotopically labeled compound, metabolite or prodrug thereof according to any one of the aforementioned in the manufacture of an antibody-drug conjugate, especially in the manufacture of the antibody-drug conjugate according to any one of the aforementioned.

In another aspect, the present invention provides use of the antibody-drug conjugate, compound (e.g., drug linker, TLR agonist or cytotoxic drug) or pharmaceutically acceptable salt, ester, stereoisomer, tautomer, polymorph, solvate, N-oxide, isotopically labeled compound, metabolite or prodrug thereof, or pharmaceutical composition according to any one of the aforementioned in the manufacture of a medicament for treating and/or preventing a cancer (e.g., a HER2-positive cancer, such as HER2-positive gastric cancer, breast cancer or non-small cell lung cancer).

In another aspect, the present invention provides the antibody-drug conjugate, compound (e.g., drug linker, TLR agonist or cytotoxic drug) or pharmaceutically acceptable salt, ester, stereoisomer, tautomer, polymorph, solvate, N-oxide, isotopically labeled compound, metabolite or prodrug thereof, or pharmaceutical composition according to any one of the aforementioned, for use in treating and/or preventing a cancer (e.g., HER2-positive cancer, such as HER2-positive gastric cancer, breast cancer or non-small cell lung cancer).

In another aspect, the present invention provides a method of treating and/or preventing a cancer (e.g., a HER2-positive cancer, such as HER2-positive gastric cancer, breast cancer, or non-small cell lung cancer), comprising administering to a subject in need thereof an therapeutically and/or prophylactically effective amount of the antibody-drug conjugate, compound (e.g., drug linker, TLR agonist or cytotoxic drug) or pharmaceutically acceptable salt, ester, stereoisomer, tautomer, polymorph, solvate, N-oxide, isotopically labeled compound, metabolite or prodrug thereof, or pharmaceutical composition according to any one of the aforementioned.

### Preparation methods

In another aspect, the present invention provides a method for preparing a compound represented by Formula (III) of the present invention.

When X of the compound of Formula (III) contains a structural fragment 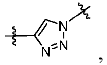 X is 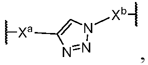 wherein X^{a} and X^{b} are structures connected to two sides of 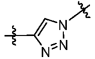 in X; and the present invention provides a method for preparing the compound of Formula (III), which comprises the following steps: 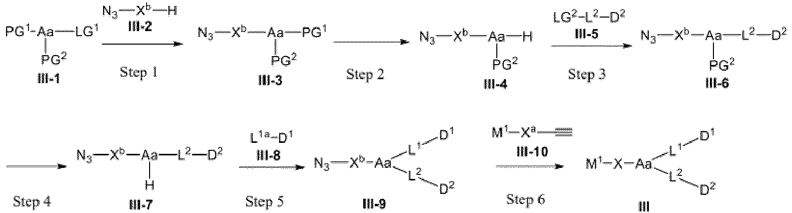
wherein, LG¹ and LG² represent leaving groups, including but not limited to halogen, hydroxyl, succinimide ester, active ester, etc., PG¹ and PG² represent protecting groups, including but not limited to benzyloxycarbonyl (Cbz), tert-butoxycarbonyl (Boc), fluorenyloxycarbonyl (Fmoc), benzyl, acetyl, tert-butyl, etc.; L^{1a} is a precursor structure to form L¹, preferably containing an unsaturated double bond, and capable of connecting to Aa through an addition reaction occurring on the unsaturated double bond;
D¹, D², L¹, L², X, Aa and M¹ are as defined above;

### Step 1: Subjecting Compound III-1 and Compound III-2 to a condensation reaction to obtain Compound III-3;

The reaction is preferably carried out in a suitable organic solvent. The organic solvent may be selected from the group consisting of tetrahydrofuran, 1,4-dioxane, dichloromethane, dimethylsulfoxide, *N*,*N*-dimethylformamide and any combination thereof, preferably *N,N*-dimethylformamide. The reaction is preferably carried out in the presence of a condensing agent. The condensing agent may be selected from 1-hydroxybenzotriazole, 2-ethoxy-1- ethoxycarbonyl-1,2-dihydroquinoline, 2-(7-azobenzotriazole)*-N,N,N',N'*-tetramethylurea hexafluorophosphate, 1-(3-dimethylaminopropyl)-3-ethylcarbodiimide hydrochloride, and benzotriazol-1-yloxytris(dimethylamino)phosphonium hexafluorophosphate, preferably a combination of 1-hydroxybenzotriazole and 1-(3-dimethylaminopropyl)-3-ethylcarbodiimide hydrochloride. The reaction is preferably carried out in the presence of a base. The base may be selected from the group consisting of *N*,*N*-diisopropylethylamine, triethylamine, pyridine, and 4-dimethylaminopyridine, preferably *N,N-*diisopropylethylamine. The reaction is preferably carried out at a suitable temperature, preferably -10 to 60 °C. The condensation reaction is preferably carried out for a suitable time period, such as 1 to 12 hours.

### Step 2: Subjecting Compound III-3 to a deprotection reaction to obtain Compound III-4;

The reaction is preferably carried out in a suitable organic solvent. The organic solvent may be selected from the group consisting of 1,4-dioxane, dichloromethane, tetrahydrofuran and any combination thereof, preferably dichloromethane. The reaction is preferably carried out in the presence of a suitable acid, and the acid may be selected from the group consisting of hydrochloric acid and trifluoroacetic acid, preferably trifluoroacetic acid. The reaction is preferably carried out at a suitable temperature, preferably -10 to 60 °C. The reaction is preferably carried out for a suitable time period, such as 1 to 12 hours.

### Step 3: Subjecting Compound III-4 and Compound III-5 to a condensation reaction to obtain Compound III-6;

The reaction is preferably carried out in a suitable organic solvent. The organic solvent may be selected from the group consisting of tetrahydrofuran, 1,4-dioxane, dichloromethane, dimethylsulfoxide, N,N-dimethylformamide and any combination thereof, preferably *N,N-*dimethylformamide. The reaction is preferably carried out in the presence of a condensing agent. The condensing agent may be selected from 1-hydroxybenzotriazole, 2-ethoxy-1- ethoxycarbonyl-1,2-dihydroquinoline, 2-(7-azobenzotriazole)*-N,N,N',N'*-tetramethylurea hexafluorophosphate, 1-(3-dimethylaminopropyl)-3-ethylcarbodiimide hydrochloride, and benzotriazol-1-yloxytris(dimethylamino)phosphonium hexafluorophosphate, preferably a combination of 1-hydroxybenzotriazole and 1-(3-dimethylaminopropyl)-3-ethylcarbodiimide hydrochloride. The reaction is preferably carried out in the presence of a base. The base may be selected from the group consisting of *N*,*N*-diisopropylethylamine, triethylamine, pyridine, and 4-dimethylaminopyridine, preferably *N,N-*diisopropylethylamine. The reaction is preferably carried out at a suitable temperature, preferably -10 to 60 °C. The condensation reaction is preferably carried out for a suitable time period, such as 1 to 12 hours.

### Step 4: Subjecting Compound III-6 to a deprotection reaction to obtain Compound III-7;

The reaction is preferably carried out in a suitable organic solvent. The organic solvent may be selected from the group consisting of 1,4-dioxane, dichloromethane, tetrahydrofuran and any combination thereof, preferably dichloromethane. The reaction is preferably carried out in the presence of a suitable acid, which may be selected from the group consisting of acetic acid, hydrobromic acid, hydrochloric acid, and trifluoroacetic acid, preferably trifluoroacetic acid. The reaction may be carried out with the addition of triethylsilane as an ion trapping agent. The reaction is preferably carried out at a suitable temperature, preferably -10 to 60 °C. The reaction is preferably carried out for a suitable time period, such as 1 to 12 hours.

### Step 5: Subjecting Compound III-7 and Compound III-8 to an addition reaction to obtain Compound III-9;

The reaction is preferably carried out in a suitable organic solvent. The organic solvent may be selected from the group consisting of 1,4-dioxane, dichloromethane, tetrahydrofuran, dimethylsulfoxide, *N,N-*dimethylformamide and any combination thereof, preferably *N,N*-dimethylformamide. The reaction is preferably carried out in the presence of a base. The base may be selected from the group consisting of *N*,*N*-diisopropylethylamine, triethylamine, pyridine, and 4-dimethylaminopyridine, preferably *N,N-*diisopropylethylamine. The reaction is preferably carried out at a suitable temperature, preferably -10 to 60 °C. The reaction is preferably carried out for a suitable time period, such as 1 to 12 hours.

### Step 6: Subjecting Compound III-9 and Compound III-10 to a cyclization reaction to obtain Compound III;

The reaction is preferably carried out in a suitable organic solvent. The organic solvent may be selected from the group consisting of tetrahydrofuran, 1,4-dioxane, dichloromethane, dimethylsulfoxide, N,N-dimethylformamide, water and any combination thereof, preferably a mixed solvent of dimethylsulfoxide and water. The reaction is preferably carried out in the presence of a metal catalyst. The metal catalyst may be selected from the group consisting of copper sulfate, cuprous iodide, cuprous bromide, and copper acetate, preferably copper sulfate. The reaction is preferably carried out in the presence of ascorbic acid or sodium ascorbate. The reaction is preferably carried out at a suitable temperature, preferably -10 to 60 °C. The condensation reaction is preferably carried out for a suitable time period, such as 1 to 12 hours.

The present invention provides another method for preparing the compound of Formula (III) with X as a covalent bond, comprising the following steps: 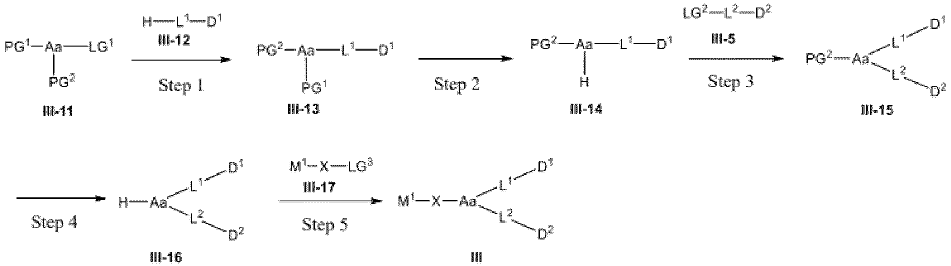
wherein, LG¹, LG² and LG³ represent leaving groups, including but not limited to halogen, hydroxyl, succinimide ester, active ester, etc., PG¹ and PG² represent protecting groups, including but not limited to benzyloxycarbonyl (Cbz), tert-butoxycarbonyl (Boc), fluorenyloxycarbonyl (Fmoc), benzyl, acetyl, tert-butyl, etc.;
D¹, D², L¹, L², Aa and M¹ are as defined above;

### Step 1: Subjecting Compound III-11 and Compound III-12 to a condensation reaction to obtain Compound III-13;

The reaction is preferably carried out in a suitable organic solvent. The organic solvent may be selected from the group consisting of tetrahydrofuran, 1,4-dioxane, dichloromethane, dimethylsulfoxide, *N,N-*dimethylformamide and any combination thereof, preferably *N,N*-dimethylformamide. The reaction is preferably carried out in the presence of a condensing agent. The condensing agent may be selected from 1-hydroxybenzotriazole, 2-ethoxy-1- ethoxycarbonyl-1,2-dihydroquinoline, 2-(7-azobenzotriazole)*-N,N,N',N'*-tetramethylurea hexafluorophosphate, 1-(3-dimethylaminopropyl)-3-ethylcarbodiimide hydrochloride, and benzotriazol-1-yloxytris(dimethylamino)phosphonium hexafluorophosphate, preferably 2-(7-azobenzotriazole)-*N,N,N',N'*-tetramethylurea hexafluorophosphate. The reaction is preferably carried out in the presence of a base. The base may be selected from the group consisting of *N*,*N*-diisopropylethylamine, triethylamine, pyridine, and 4-dimethylaminopyridine, preferably *N,N-*diisopropylethylamine. The reaction is preferably carried out at a suitable temperature, preferably -10 to 60 °C. The condensation reaction is preferably carried out for a suitable time period, such as 1 to 12 hours.

### Step 2: Subjecting Compound III-13 to a deprotection reaction to obtain Compound III-14;

The reaction is preferably carried out in a suitable organic solvent. The organic solvent may be selected from the group consisting of 1,4-dioxane, dichloromethane, tetrahydrofuran and any combination thereof, preferably dichloromethane. The reaction is preferably carried out in the presence of a suitable acid, and the acid may be selected from the group consisting of hydrochloric acid, trifluoroacetic acid and zinc bromide, preferably zinc bromide. The reaction is preferably carried out at a suitable temperature, preferably -10 to 60 °C. The reaction is preferably carried out for a suitable time, such as 1 to 12 hours.

### Step 3: Subjecting Compound III-14 and Compound III-5 to a condensation reaction to obtain Compound III-15;

The reaction is preferably carried out in a suitable organic solvent. The organic solvent may be selected from the group consisting of tetrahydrofuran, 1,4-dioxane, dichloromethane, dimethylsulfoxide, *N*,*N*-dimethylformamide and any combination thereof, preferably *N,N*-dimethylformamide. The reaction is preferably carried out in the presence of a condensing agent. The condensing agent may be selected from 1-hydroxybenzotriazole, 2-ethoxy-1- ethoxycarbonyl-1,2-dihydroquinoline, 2-(7-azobenzotriazole)*-N,N,N',N'*-tetramethylurea hexafluorophosphate, 1-(3-dimethylaminopropyl)-3-ethylcarbodiimide hydrochloride, and benzotriazole-1-yloxytris(dimethylamino)phosphonium hexafluorophosphate, preferably 2-(7-azobenzotriazole)-*N,N,N',N'*-tetramethylurea hexafluorophosphate. The reaction is preferably carried out in the presence of a base. The base may be selected from the group consisting of *N*,*N*-diisopropylethylamine, triethylamine, pyridine, and 4-dimethylaminopyridine, preferably *N,N-*diisopropylethylamine. The reaction is preferably carried out at a suitable temperature, preferably -10 to 60 °C. The condensation reaction is preferably carried out for a suitable time period, such as 1 to 12 hours.

### Step 4: Subjecting Compound III-15 to a deprotection reaction to obtain Compound III-16;

The reaction is preferably carried out in a suitable organic solvent. The organic solvent may be selected from the group consisting of *N,N-*dimethylformamide, dichloromethane, tetrahydrofuran and any combination thereof, preferably *N,N-*dimethylformamide. The reaction is preferably carried out in the presence of a suitable base. The base may be selected from the group consisting of diethylamine, and piperidine, preferably piperidine. The reaction is preferably carried out at a suitable temperature, preferably -10 to 60 °C. The reaction is preferably carried out for a suitable time, such as 1 to 6 hours.

### Step 5: Subjecting Compound III-16 and Compound III-17 to a substitution reaction to obtain Compound III;

The reaction is preferably carried out in a suitable organic solvent. The organic solvent may be selected from the group consisting of dichloromethane, tetrahydrofuran, 1,2-dichloroethane, diethyl ether, *N*,*N*-dimethylformamide and any combination thereof, preferably *N,N*-dimethylformamide. The reaction is preferably carried out in the presence of a base. The base may be selected from the group consisting of *N*,*N*-diisopropylethylamine, triethylamine, pyridine, and 4-dimethylaminopyridine, preferably *N,N-*diisopropylethylamine. The reaction is preferably carried out at a suitable temperature, preferably -10 to 60 °C. The reaction is preferably carried out for a suitable time, such as 1 to 12 hours.

The present invention provides another method for preparing the compound of Formula (III) with X as a covalent bond, comprising the following steps: 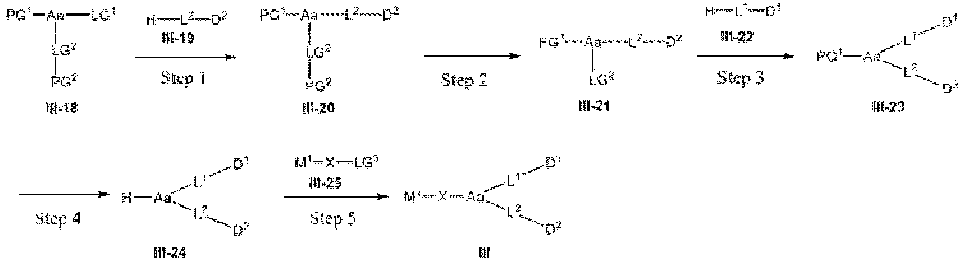
wherein, LG¹, LG² and LG³ represent leaving groups, including but not limited to halogen, hydroxyl, succinimide ester, active ester, etc., PG¹ and PG² represent protecting groups, including but not limited to benzyloxycarbonyl (Cbz), tert-butoxycarbonyl (Boc), fluorenyloxycarbonyl (Fmoc), benzyl, acetyl, tert-butyl, etc.;
D¹, D², L¹, L², Aa and M¹ are as defined above;

### Step 1: Subjecting Compound III-18 and Compound III-19 to a condensation reaction to obtain Compound III-20;

The reaction is preferably carried out in a suitable organic solvent. The organic solvent may be selected from the group consisting of tetrahydrofuran, 1,4-dioxane, dichloromethane, dimethylsulfoxide, *N*,*N*-dimethylformamide and any combination thereof, preferably *N*,*N*-dimethylformamide. The reaction is preferably carried out in the presence of a condensing agent. The condensing agent may be selected from 1-hydroxybenzotriazole, 2-ethoxy-1-ethoxycarbonyl-1,2-dihydroquinoline, 2-(7-azobenzotriazole)-*N,N,N ',N'*- tetramethylurea hexafluorophosphate, 1-(3-dimethylaminopropyl)-3-ethylcarbodiimide hydrochloride, and benzotriazol-1-yloxytris(dimethylamino)phosphonium hexafluorophosphate, preferably 2-(7-azobenzotriazole)-*N,N,N',N'*-tetramethylurea hexafluorophosphate. The reaction is preferably carried out in the presence of a base. The base may be selected from the group consisting of *N*,*N*-diisopropylethylamine, triethylamine, pyridine, and 4-dimethylaminopyridine, preferably *N,N-*diisopropylethylamine. The reaction is preferably carried out at a suitable temperature, preferably -10 to 60 °C. The condensation reaction is preferably carried out for a suitable time period, such as 1 to 12 hours.

### Step 2: Subjecting Compound III-20 to a deprotection reaction to obtain Compound III-21;

The reaction is preferably carried out in a suitable organic solvent. The organic solvent may be selected from the group consisting of 1,4-dioxane, dichloromethane, tetrahydrofuran and any combination thereof, preferably dichloromethane. The reaction is preferably carried out in the presence of a suitable acid, and the acid may be selected from the group consisting of hydrochloric acid and trifluoroacetic acid, preferably trifluoroacetic acid. The reaction is preferably carried out at a suitable temperature, preferably -10 to 60 °C. The reaction is preferably carried out for a suitable time period, such as 1 to 12 hours.

### Step 3: Subjecting Compound III-21 and Compound III-22 to a condensation reaction to obtain Compound III-23;

The reaction is preferably carried out in a suitable organic solvent. The organic solvent may be selected from the group consisting of tetrahydrofuran, 1,4-dioxane, dichloromethane, dimethylsulfoxide, *N*,*N*-dimethylformamide and any combination thereof, preferably *N,N*-dimethylformamide. The reaction is preferably carried out in the presence of a condensing agent. The condensing agent may be selected from 1-hydroxybenzotriazole, 2-ethoxy-1- ethoxycarbonyl-1,2-dihydroquinoline, 2-(7-azobenzotriazole)*-N,N,N',N'*-tetramethylurea hexafluorophosphate, 1-(3-dimethylaminopropyl)-3-ethylcarbodiimide hydrochloride, and benzotriazol-1-yloxytris(dimethylamino)phosphonium hexafluorophosphate, preferably 2-(7-azobenzotriazole)-*N,N,N',N'*-tetramethylurea hexafluorophosphate. The reaction is preferably carried out in the presence of a base. The base may be selected from the group consisting of *N*,*N*-diisopropylethylamine, triethylamine, pyridine, and 4-dimethylaminopyridine, preferably *N,N-*diisopropylethylamine. The reaction is preferably carried out at a suitable temperature, preferably -10 to 60 °C. The condensation reaction is preferably carried out for a suitable time period, such as 1 to 12 hours.

### Step 4: Subjecting Compound III-23 to a deprotection reaction to obtain Compound III-24;

The reaction is preferably carried out in a suitable organic solvent. The organic solvent may be selected from the group consisting of *N,N-*dimethylformamide, dichloromethane, tetrahydrofuran and any combination thereof, preferably *N,N-*dimethylformamide. The reaction is preferably carried out in the presence of a suitable base. The base may be selected from the group consisting of diethylamine and piperidine, preferably piperidine. The reaction is preferably carried out at a suitable temperature, preferably -10 to 60 °C. The reaction is preferably carried out for a suitable time period, such as 1 to 6 hours.

### Step 5: Subjecting Compound III-24 and Compound III-25 to a substitution reaction to obtain Compound III;

The reaction is preferably carried out in a suitable organic solvent. The organic solvent may be selected from the group consisting of dichloromethane, tetrahydrofuran, 1,2-dichloroethane, diethyl ether, N,N-dimethylformamide and any combination thereof, preferably *N,N-*dimethylformamide. The reaction is preferably carried out in the presence of a base. The base may be selected from the group consisting of *N*,*N*-diisopropylethylamine, triethylamine, pyridine, and 4-dimethylaminopyridine, preferably *N,N-*diisopropylethylamine. The reaction is preferably carried out at a suitable temperature, preferably -10 to 60 °C. The reaction is preferably carried out for a suitable time period, such as 1 to 12 hours.

When Aa is a polypeptide fragment formed of two or more amino acids, Aa is -Aa¹-Aa²-, wherein Aa¹ and Aa² are the amino acid fragments constituting Aa; the present invention provides another method for preparing the compound of Formula (III), which comprises the following steps: 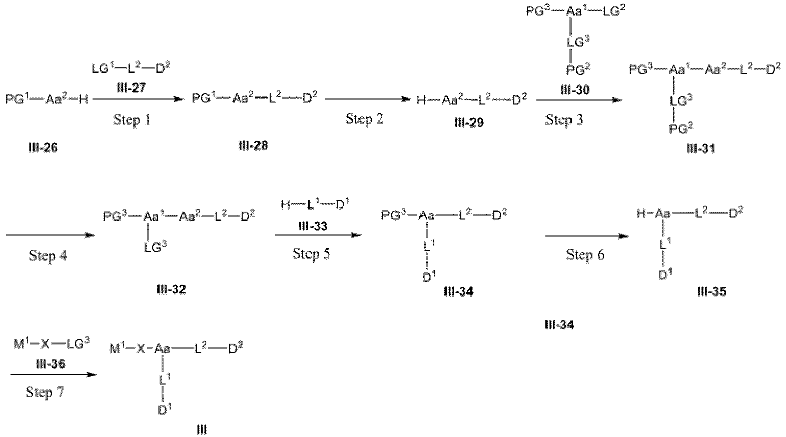
wherein, LG¹, LG² and LG³ represent leaving groups, including but not limited to halogen, hydroxyl, succinimide ester, active ester, etc., PG¹, PG² and PG³ represent protecting groups, including but not limited to benzyloxycarbonyl (Cbz), tert-butoxycarbonyl (Boc), fluorenyloxycarbonyl (Fmoc), benzyl, acetyl, tert-butyl, etc.; Aa¹ and Aa² are the amino acid fragments that constitute Aa;
D¹, D², L¹, L², X, Aa and M¹ are as defined above;

### Step 1: Subjecting Compound III-26 and Compound III-27 to a condensation reaction to obtain Compound III-28;

The reaction is preferably carried out in a suitable organic solvent. The organic solvent may be selected from the group consisting of tetrahydrofuran, 1,4-dioxane, dichloromethane, dimethylsulfoxide, *N*,*N*-dimethylformamide and any combination thereof, preferably *N,N*-dimethylformamide. The reaction is preferably carried out in the presence of a condensing agent. The condensing agent may be selected from 1-hydroxybenzotriazole, 2-ethoxy-1- ethoxycarbonyl-1,2-dihydroquinoline, 2-(7-azobenzotriazole)*-N,N,N',N'*-tetramethylurea hexafluorophosphate, 1-(3-dimethylaminopropyl)-3-ethylcarbodiimide hydrochloride, and benzotriazol-1-yloxytris(dimethylamino)phosphonium hexafluorophosphate, preferably 2-(7-azobenzotriazole)-*N,N,N',N'*-tetramethylurea hexafluorophosphate. The reaction is preferably carried out in the presence of a base. The base may be selected from the group consisting of *N*,*N*-diisopropylethylamine, triethylamine, pyridine, and 4-dimethylaminopyridine, preferably *N,N-*diisopropylethylamine. The reaction is preferably carried out at a suitable temperature, preferably -10 to 60 °C. The condensation reaction is preferably carried out for a suitable time period, such as 1 to 12 hours.

### Step 2: Subjecting Compound III-28 to a deprotection reaction to obtain Compound III-29;

The reaction is preferably carried out in a suitable organic solvent. The organic solvent may be selected from the group consisting of 1,4-dioxane, dichloromethane, tetrahydrofuran and any combination thereof, preferably 1,4-dioxane. The reaction is preferably carried out in the presence of a suitable acid. The acid may be selected from the group consisting of acetic acid, hydrobromic acid, hydrochloric acid, and trifluoroacetic acid, preferably hydrochloric acid. The reaction is preferably carried out at a suitable temperature, preferably -10 to 60 °C. The reaction is preferably carried out for a suitable time period, such as 1 to 12 hours.

### Step 3: Subjecting Compound III-29 and Compound III-30 to a condensation reaction to obtain Compound III-31;

The reaction is preferably carried out in a suitable organic solvent. The organic solvent may be selected from the group consisting of tetrahydrofuran, 1,4-dioxane, dichloromethane, dimethylsulfoxide, *N*,*N*-dimethylformamide and any combination thereof, preferably *N,N*-dimethylformamide. The reaction is preferably carried out in the presence of a condensing agent. The condensing agent may be selected from 1-hydroxybenzotriazole, 2-ethoxy-1- ethoxycarbonyl-1,2-dihydroquinoline, 2-(7-azobenzotriazole)*-N,N,N',N'*-tetramethylurea hexafluorophosphate, 1-(3-dimethylaminopropyl)-3-ethylcarbodiimide hydrochloride, and benzotriazol-1-yloxytris(dimethylamino)phosphonium hexafluorophosphate, preferably 2-(7-azobenzotriazole)-*N,N,N',N'*-tetramethylurea hexafluorophosphate. The reaction is preferably carried out in the presence of a base. The base may be selected from the group consisting of *N*,*N*-diisopropylethylamine, triethylamine, pyridine, and 4-dimethylaminopyridine, preferably *N,N-*diisopropylethylamine. The reaction is preferably carried out at a suitable temperature, preferably -10 to 60 °C. The condensation reaction is preferably carried out for a suitable time period, such as 1 to 12 hours.

### Step 4: Subjecting Compound III-31 to a deprotection reaction to obtain Compound III-32;

The reaction is preferably carried out in a suitable organic solvent. The organic solvent may be selected from the group consisting of 1,4-dioxane, dichloromethane, tetrahydrofuran and any combination thereof, preferably dichloromethane. The reaction is preferably carried out in the presence of a suitable acid, and the acid may be selected from the group consisting of hydrochloric acid and trifluoroacetic acid, preferably trifluoroacetic acid. The reaction is preferably carried out at a suitable temperature, preferably -10 to 60 °C. The reaction is preferably carried out for a suitable time period, such as 1 to 12 hours.

### Step 5: Subjecting Compound III-32 and Compound III-33 to a condensation reaction to obtain Compound III-34;

The reaction is preferably carried out in a suitable organic solvent. The organic solvent may be selected from the group consisting of tetrahydrofuran, 1,4-dioxane, dichloromethane, dimethylsulfoxide, *N*,*N*-dimethylformamide and any combination thereof, preferably *N,N-*dimethylformamide. The reaction is preferably carried out in the presence of a condensing agent. The condensing agent may be selected from 1-hydroxybenzotriazole, 2-ethoxy-1- ethoxycarbonyl-1,2-dihydroquinoline, 2-(7-azobenzotriazole)*-N,N,N',N'*-tetramethylurea hexafluorophosphate, 1-(3-dimethylaminopropyl)-3-ethylcarbodiimide hydrochloride, and benzotriazol-1-yloxytris(dimethylamino)phosphonium hexafluorophosphate, preferably 2-(7-azobenzotriazole)-*N,N,N',N'*-tetramethylurea hexafluorophosphate. The reaction is preferably carried out in the presence of a base. The base may be selected from the group consisting of *N*,*N*-diisopropylethylamine, triethylamine, pyridine, and 4-dimethylaminopyridine, preferably *N,N-*diisopropylethylamine. The reaction is preferably carried out at a suitable temperature, preferably -10 to 60 °C. The condensation reaction is preferably carried out for a suitable time period, such as 1 to 12 hours.

### Step 6: Subjecting Compound III-34 to a deprotection reaction to obtain Compound III-35;

The reaction is preferably carried out in a suitable organic solvent. The organic solvent may be selected from the group consisting of *N,N-*dimethylformamide, dichloromethane, tetrahydrofuran and any combination thereof, preferably *N,N-*dimethylformamide. The reaction is preferably carried out in the presence of a suitable base. The base may be selected from the group consisting of diethylamine and piperidine, preferably piperidine. The reaction is preferably carried out at a suitable temperature, preferably -10 to 60 °C. The reaction is preferably carried out for a suitable time period, such as 1 to 6 hours.

### Step 7: Subjecting Compound III-35 and Compound III-36 to a substitution reaction to obtain Compound III;

The reaction is preferably carried out in a suitable organic solvent. The organic solvent may be selected from the group consisting of dichloromethane, tetrahydrofuran, 1,2-dichloroethane, diethyl ether, *N*,*N*-dimethylformamide and any combination thereof, preferably *N,N*-dimethylformamide. The reaction is preferably carried out in the presence of a base. The base may be selected from the group consisting of *N*,*N*-diisopropylethylamine, triethylamine, pyridine, and 4-dimethylaminopyridine, preferably *N,N-*diisopropylethylamine. The reaction is preferably carried out at a suitable temperature, preferably -10 to 60 °C. The reaction is preferably carried out for a suitable time period, such as 1 to 12 hours.

When Aa is a polypeptide fragment formed of two or more amino acids, Aa is -Aa¹-Aa²-; the present invention provides another method for preparing the compound of Formula (III), which comprises the following steps: 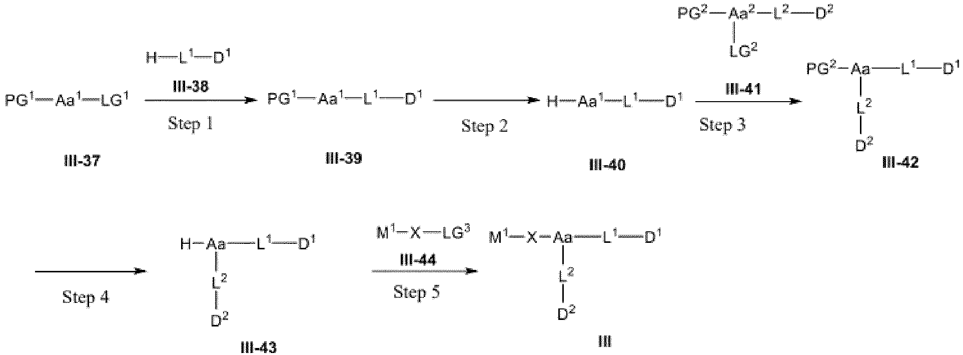
wherein, LG¹, LG² and LG³ represent leaving groups, including but not limited to halogen, hydroxyl, succinimide ester, active ester, etc., PG¹ and PG² represent protecting groups, including but not limited to benzyloxycarbonyl (Cbz), tert-butoxycarbonyl (Boc), fluorenyloxycarbonyl (Fmoc), benzyl, acetyl, tert-butyl, etc.; Aa¹ and Aa² are respectively amino acid fragments, or polypeptide fragments formed of two or more amino acids;
D¹, D², L¹, L², X, Aa and M¹ are as defined above;

### Step 1: Subjecting Compound III-37 and Compound III-38 to a condensation reaction to obtain Compound III-39;

The reaction is preferably carried out in a suitable organic solvent. The organic solvent may be selected from the group consisting of tetrahydrofuran, 1,4-dioxane, dichloromethane, dimethylsulfoxide, *N*,*N*-dimethylformamide and any combination thereof, preferably *N,N*-dimethylformamide. The reaction is preferably carried out in the presence of a condensing agent. The condensing agent may be selected from 1-hydroxybenzotriazole, 2-ethoxy-1- ethoxycarbonyl-1,2-dihydroquinoline, 2-(7-azobenzotriazole)*-N,N,N',N'*-tetramethylurea hexafluorophosphate, 1-(3-dimethylaminopropyl)-3-ethylcarbodiimide hydrochloride, and benzotriazol-1-yloxytris(dimethylamino)phosphonium hexafluorophosphate, preferably 2-(7-azobenzotriazole)-*N,N,N',N'*-tetramethylurea hexafluorophosphate. The reaction is preferably carried out in the presence of a base. The base may be selected from the group consisting of *N*,*N*-diisopropylethylamine, triethylamine, pyridine, and 4-dimethylaminopyridine, with *N,N-*diisopropylethylamine being preferred. The reaction is preferably carried out at a suitable temperature, preferably -10 to 60 °C. The condensation reaction is preferably carried out for a suitable time period, such as 1 to 12 hours.

### Step 2: Subjecting Compound III-39 to a deprotection reaction to obtain Compound III-40;

The reaction is preferably carried out in a suitable organic solvent. The organic solvent may be selected from the group consisting of *N,N-*dimethylformamide, dichloromethane, tetrahydrofuran and any combination thereof, preferably *N,N-*dimethylformamide. The reaction is preferably carried out in the presence of a suitable base, and the base may be selected from the group consisting of diethylamine and piperidine, preferably piperidine. The reaction is preferably carried out at a suitable temperature, preferably -10 to 60 °C. The reaction is preferably carried out for a suitable time period, such as 1 to 6 hours.

### Step 3: Subjecting Compound III-40 and Compound III-41 to a condensation reaction to obtain Compound III-42;

The reaction is preferably carried out in a suitable organic solvent. The organic solvent may be selected from the group consisting of tetrahydrofuran, 1,4-dioxane, dichloromethane, dimethylsulfoxide, *N*,*N*-dimethylformamide and any combination thereof, preferably *N,N*-dimethylformamide. The reaction is preferably carried out in the presence of a condensing agent. The condensing agent may be selected from 1-hydroxybenzotriazole, 2-ethoxy-1- ethoxycarbonyl-1,2-dihydroquinoline, 2-(7-azobenzotriazole)*-N,N,N',N'*-tetramethylurea hexafluorophosphate, 1-(3-dimethylaminopropyl)-3-ethylcarbodiimide hydrochloride, and benzotriazol-1-yloxytris(dimethylamino)phosphonium hexafluorophosphate, preferably 2-(7-azobenzotriazole)-*N,N,N',N'*-tetramethylurea hexafluorophosphate. The reaction is preferably carried out in the presence of a base. The base may be selected from the group consisting of *N*,*N*-diisopropylethylamine, triethylamine, pyridine, and 4-dimethylaminopyridine, preferably *N,N-*diisopropylethylamine. The reaction is preferably carried out at a suitable temperature, preferably -10 to 60 °C. The condensation reaction is preferably carried out for a suitable time period, such as 1 to 12 hours.

### Step 4: Subjecting Compound III-42 to a deprotection reaction to obtain Compound III-43;

The reaction is preferably carried out in a suitable organic solvent. The organic solvent may be selected from the group consisting of 1,4-dioxane, dichloromethane, tetrahydrofuran and any combination thereof, preferably dichloromethane. The reaction is preferably carried out in the presence of a suitable acid, and the acid may be selected from the group consisting of hydrochloric acid, trifluoroacetic acid, and zinc bromide, preferably zinc bromide. The reaction is preferably carried out at a suitable temperature, preferably -10 to 60 °C. The reaction is preferably carried out for a suitable time period, such as 1 to 12 hours.

### Step 5: Subjecting Compound III-43 to a substitution reaction with Compound III-44 to obtain Compound III;

The reaction is preferably carried out in a suitable organic solvent. The organic solvent may be selected from the group consisting of dichloromethane, tetrahydrofuran, 1,2-dichloroethane, diethyl ether, *N*,*N*-dimethylformamide and any combination thereof, preferably *N,N*-dimethylformamide. The reaction is preferably carried out in the presence of a base. The base may be selected from the group consisting of *N*,*N*-diisopropylethylamine, triethylamine, pyridine, and 4-dimethylaminopyridine, preferably *N,N-*diisopropylethylamine. The reaction is preferably carried out at a suitable temperature, preferably -10 to 60 °C. The reaction is preferably carried out for a suitable time period, such as 1 to 12 hours.

### Definition of Terms

Unless otherwise defined below, all technical and scientific terms used herein are intended to have the same meaning as commonly understood by those skilled in the art. The technology as used herein is intended to mean technology as commonly understood in the art, including those variations or equivalents that would be apparent to those skilled in the art. Moreover, the laboratory procedures used herein, such as genomics, nucleic acid chemistry and molecular biology, are routine procedures widely used in the corresponding fields. Although the following terms are believed to be well understood by those skilled in the art, the following definitions are set forth to better explain the present invention.

As used herein, the term "antibody" refers to an immunoglobulin molecule typically composed of two pairs of polypeptide chains, each pair has a light chain (LC) and a heavy chain (HC). Antibody light chains can be classified into κ (kappa) and λ (lambda) light chains. Heavy chains can be classified as µ, δ, γ, α, or ε, and the antibody's isotypes can be defined as IgM, IgD, IgG, IgA, and IgE, respectively. In the light and heavy chains, the variable and constant regions are connected by a "J" region of approximately 12 or more amino acids, and the heavy chain also contains a "D" region of approximately 3 or more amino acids. Each heavy chain consists of a heavy chain variable region (VH) and a heavy chain constant region (CH). The heavy chain constant region consists of 3 domains (CH1, CH2 and CH3). Each light chain consists of a light chain variable region (VL) and a light chain constant region (CL). The light chain constant region consists of one domain, CL. The constant domain is not directly involved in the binding of antibody to antigen, but exhibits a variety of effector functions, such as mediating the interaction of immunoglobulin with host tissue or factor, including the binding of various cells of the immune system (e.g., effector cells) to the first component of the classical complement system (C1q). The VH and VL regions can also be subdivided into regions of high variability called complementarity determining regions (CDRs), interspersed with more conservative regions called framework regions (FRs). Each VH and VL consists of 3 CDRs and 4 FRs arranged from the amino terminus to the carboxyl terminus in the following sequence: FR1, CDR1, FR2, CDR2, FR3, CDR3, FR4. The variable regions (VH and VL) of each heavy chain/light chain pair respectively form an antigen-binding site. The assignment of amino acids to regions or domains can follow various numbering systems known in the art.

The term "complementarity determining region" or "CDR" refers to amino acid residues in a variable region of an antibody that are responsible for antigen binding. The variable regions of heavy chain and light chain each contains three CDRs, named CDR1, CDR2 and CDR3. The precise boundaries of these CDRs can be defined according to various numbering systems known in the art, such as the Kabat numbering system (Kabat et al., Sequences of Proteins of Immunological Interest, 5th Ed. Public Health Service, National Institutes of Health, Bethesda, Md., 1991), the Chothia numbering system (Chothia & Lesk (1987) J. Mol. Biol. 196:901-917; Chothia et al. (1989) Nature 342:878-883), the IMGT numbering system (Lefranc et al. al., Dev. Comparat. Immunol. 27:55-77, 2003), or the AbM numbering system (Martin ACR, Cheetham JC, Rees AR (1989) Modeling antibody hypervariable loops: A combined algorithm. Proc Natl Acad Sci USA 86:9268-9272). For a given antibody, one skilled in the art will readily identify the CDRs defined by each numbering system. Moreover, the correspondence between different numbering systems is well known to those skilled in the art (e.g., see Lefranc et al., Dev. Comparat. Immunol. 27:55-77, 2003).

In the present invention, the CDRs contained in the antibody or antigen-binding fragment thereof can be determined according to various numbering systems known in the art, for example, by the Kabat, Chothia, IMGT or AbM numbering system. In certain embodiments, the antibody or antigen-binding fragment thereof contains CDRs defined by the Chothia numbering system.

The term "framework region" or "FR" residue refers to those amino acid residues in an antibody variable region other than the CDR residue as defined above.

The term "antigen-binding fragment" of an antibody refers to a polypeptide that is a fragment of the antibody, such as a fragment of a full-length antibody, that retains the ability to specifically bind to the same antigen that the full-length antibody binds and/or competes with the full-length antibody for specific binding to the antigen, which is also called "antigen-binding moiety". See generally, Fundamental Immunology, Ch. 7 (Paul, W., ed., 2nd ed., Raven Press, N.Y. (1989), which is incorporated herein by reference in its entirety for all purposes. An antigen-binding fragment of an antibody can be obtained by recombinant DNA technology or by enzymatic or chemical cleavage of an intact antibody. Non-limiting examples of an antigen-binding fragment include Fab, Fab', F(ab)'₂, F(ab)'₃, Fd, Fv, scFv, di-scFv, (scFv)₂, disulfide-stabilized Fv ("dsFv"), single domain antibody (sdAb, nanobody) and such polypeptide, which contains at least a portion of an antibody that is sufficient to confer specific antigen-binding ability to the polypeptide. Engineered antibody variants are reviewed in Holliger et al., 2005; Nat Biotechnol, 23: 1126-1136.

The term "Fd" refers to an antibody fragment consisting of VH and CH1 domains; the term "dAb" refers to an antibody fragment consisting of VH domain (Ward et al., Nature 341:544 546 (1989)); the term "Fab" refers to an antibody fragment consisting of VL, VH, CL and CH1 domains; the term "F(ab')₂" refers to an antibody fragment comprising two Fab connected by a disulfide bridge on hinge region; the term "Fab'" refers to a fragment that is obtained by reducing the disulfide bond connecting the two heavy chain fragments in F(ab')₂, and consists of a complete light chain and a Fd of heavy chain (consisting of VH and CH1 domains).

The term "Fv" refers to an antibody fragment consisting of the VL and VH domains of a single arm of an antibody. Fv is generally considered to be the smallest antibody fragment that can form a complete antigen-binding site. It is generally believed that six CDRs confer the antigen-binding specificity to an antibody. However, even a variable region (e.g., a Fd, which comprises only three antigen-specific CDRs) can recognize and bind to antigen, although its affinity may be lower than that of an intact binding site.

The term "Fc" refers to an antibody fragment formed with the second and third constant regions of a first heavy chain of an antibody and the second and third constant regions of a second heavy chain through disulfide bonding. Fc fragment of antibody has various functions but does not participate in antigen binding.

The term "scFv" refers to a single polypeptide chain comprising VL and VH domains, in which the VL and VH are connected via a linker (see, for example, Bird et al., Science 242:423-426 (1988); Huston et al., Proc. Natl. Acad. Sci. USA 85:5879-5883 (1988); and Pluckthun, The Pharmacology of Monoclonal Antibodies, Vol. 113, Roseburg and Moore, eds., Springer-Verlag, New York, pp. 269-315 (1994)). Such scFv molecules may have the general structure as follows: NH₂-VL-linker-VH-COOH or NH₂-VH-linker-VL-COOH. Suitable linkers in the prior art consist of repeated GGGGS amino acid sequences or variants thereof. For example, a linker having the amino acid sequence (GGGGS)₄ can be used, but variants thereof can also be used (Holliger et al. (1993), Proc. Natl. Acad. Sci. USA 90: 6444-6448). Other linkers may be used in the present invention are described by Alfthan et al. (1995), Protein Eng. 8:725-731, Choi et al. (2001), Eur. J. Immunol. 31:94-106, Hu et al. (1996), Cancer Res. 56:3055-3061, Kipriyanov et al. (1999), J. Mol. Biol. 293:41-56, and Roovers et al. (2001), Cancer Immunol. In some cases, a disulfide bond may also exist between VH and VL of scFv. In certain embodiments, VH and VL domains can be positioned relative to each other in any suitable arrangement. For example, scFv comprises NH₂-VH-VH-COOH, NH₂-VL-VL-COOH.

Each of the above antibody fragments retains the ability to specifically bind to the same antigen that the full-length antibody binds, and/or competes with the full-length antibody for specific binding to the antigen.

As used herein, when the term "antibody" is mentioned, it includes not only intact antibody but also an antigen-binding fragment of the antibody, unless the context clearly indicates otherwise.

An antigen-binding fragment of an antibody (e.g., the above-described antibody fragment) can be obtained from a given antibody (e.g., the antibody provided by the present invention) using conventional techniques known to those skilled in the art (e.g., recombinant DNA technology or enzymatic or chemical fragmentation methods), and the antigen-binding fragment of the antibody is screened for specificity in the same manner as for the intact antibody.

The term "murine antibody" refers to an antibody obtained by the following method: fusing B cells of immunized mouse with myeloma cells, selecting murine hybrid fusion cells that can both proliferate indefinitely and secrete antibody, and then performing screening, antibody preparation and antibody purification; or refers to an antibody secreted by plasma cells after B cells differentiate and proliferate after the antigen invades the mouse body.

The term "humanized antibody" refers to a non-human antibody that has been genetically engineered and whose amino acid sequence has been modified to increase the sequence homology to a human antibody. Generally speaking, all or part of the CDR regions of a humanized antibody comes from a non-human antibody (donor antibody), and all or part of the non-CDR regions (e.g., variable region FR and/or constant region) comes from a human immunoglobulin (receptor antibodies). Humanized antibodies usually retain the expected properties of the donor antibody, including but not limited to, antigen specificity, affinity, reactivity, ability to increase immune cell activity, ability to enhance immune response, etc. The donor antibody may be a mouse, rat, rabbit, or non-human primate (e.g., cynomolgus monkey) antibody with desired properties (e.g., antigen specificity, affinity, reactivity, ability to increase immune cell activity, and/or ability to enhance immune responses).

The twenty conventional amino acids involved herein have been prepared following conventional usage. See, for example, Immunology-A Synthesis (2nd Edition, E. S. Golub and D. R. Gren, Eds., Sinauer Associates, Sunderland, Mass. (1991)), which is incorporated herein by reference. In the present invention, amino acids are generally represented by one-letter and three-letter abbreviations well known in the art. For example, alanine can be represented by A or Ala.

The terms "comprise", "include", "have", "contain", or "involve" and other variations herein are inclusive or open-ended and do not exclude other unrecited elements or method steps.

The term "alkyl" refers to a straight-chain or branched saturated hydrocarbonyl obtained by removing one hydrogen atom, such as "C₁₋₂₀ alkyl", "C₁₋₁₀ alkyl", "C₁₋₆ alkyl", "C₁₋₄ alkyl", "C₁₋₃ alkyl", etc. Specific examples include but are not limited to: methyl, ethyl, n-propyl, isopropyl, n-butyl, isobutyl, sec-butyl, tert-butyl, n-pentyl, isopentyl, 2-methylbutyl, neopentyl, 1-ethylpropyl, n-hexyl, isohexyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, 1,2-dimethylpropyl, etc.

The term "alkylene" refers to a straight-chain or branched saturated hydrocarbonyl obtained by removing 2 hydrogen atoms, such as "C₁₋₂₀ alkylene", "C₁₋₁₀ alkylene", "C₃₋₁₀ alkylene", "C₅₋₈ alkylene", "C₁₋₆ alkylene", "C₁₋₄ alkylene", "C₁₋₃ alkylene", etc. Specific examples include but are not limited to: methylene, ethylene, 1,3-propylene, 1,4-butylene, 1,5-pentylene or 1,6-hexylene, etc.

The term "cycloalkyl" refers to a saturated cyclic saturated hydrocarbonyl, including, but not limited to, monocycloalkyl and bicycloalkyl (e.g., spirocycloalkyl, fused cycloalkyl and bridged cycloalkyl). The term "C₃₋₁₀ cycloalkyl" refers to a cycloalkyl having 3 to 10 ring-forming carbon atoms, such as cyclopropyl, cyclobutyl, cyclopentyl, cyclohexyl, and the like.

The term "heterocyclyl" refers to a saturated or partially saturated cyclic structure containing at least one ring member selected from the group consisting of N, O and S. Specific examples include but are not limited to 3- to 12-membered heterocyclyl, 3- to 8-membered heterocyclyl, 5- to 6-membered heterocyclyl, etc., such as tetrahydrofuryl, pyrrolidinyl, piperidinyl, tetrahydropyranyl, etc.

The term "heteroaryl" refers to an aromatic cyclic structure containing at least one ring member selected from the group consisting of N, O and S. Specific examples include but are not limited to 5- to 10-membered heteroaryl, 5- to 6-membered heteroaryl, etc., such as furyl, thienyl, pyrrolyl, thiazolyl, isothiazolyl, thiadiazolyl, oxazolyl, isooxazolyl, oxadiazolyl, imidazolyl, pyrazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazolyl, pyridyl, pyrimidinyl, pyridazinyl, pyrazinyl, 1,2,3-triazinyl, 1,3,5-triazinyl, 1,2,4,5-tetrazinyl, etc.

The term "aryl" refers to a group obtained by removing a hydrogen atom from a carbon atom of the aromatic nucleus of an aromatic hydrocarbon molecule, such as C₆₋₁₀ aryl. Specific examples include but are not limited to phenyl, naphthyl, anthracenyl, etc.

The term "fragment" or "structural fragment" as used herein refers to the remaining part of a compound molecule after losing one or more atoms or radicals. For example, "cytotoxic drug fragment" refers to the remaining part of a cytotoxic drug obtained after losing a hydrogen atom or hydroxyl from the cytotoxic drug described herein and connecting to the linker in an antibody-drug conjugate. In some specific embodiments, the cytotoxic drug fragment is represented by D¹. Likewise, "TLR agonist fragment" refers to the remaining part of a TLR agonist described herein that is obtained by losing one or more atoms or atom groups from the TLR and connecting to a linker in an antibody-drug conjugate. In some specific embodiments, the TLR agonist fragment is represented by D².

If a substituent or value is described as being "independently selected from" a group of groups or values, each substituent or value is selected independently from the other. Thus, each substituent or value may be the same or different from another (other) substituent or value.

The present invention also includes all pharmaceutically acceptable isotopically labeled compounds that are identical to the compounds of the present invention except that one or more atoms are substituted with atoms that have the same atomic number but an atomic mass or mass number different from the atomic mass that predominates in nature. Examples of isotopes suitable for inclusion in the compounds of the present invention include, but are not limited to, isotopes of hydrogen (e.g., ²H, ³H, deuterium D, tritium T); isotopes of carbon (e.g., ¹¹C, ¹³C, and ¹⁴C); isotopes of chlorine (e.g., ³⁷Cl); isotopes of fluorine (e.g., ¹⁸F); isotopes of iodine (e.g., ¹²³I and ¹²⁵I); isotopes of nitrogen (e.g., ¹³N and ¹⁵N); isotopes of oxygen (e.g., ¹⁵O, ¹⁷O and ¹⁸O); isotopes of phosphorus (e.g., ³²P); and sulfur isotopes (e.g., ³⁵S). Certain isotopically labeled compounds of the present invention (e.g., those incorporated with radioactive isotopes) may be used in drug and/or substrate tissue distribution studies (e.g., assays). The radioactive isotopes tritium (i.e., ³H) and carbon-14 (i.e., ¹⁴C) are particularly useful for this purpose because they are easy to be incorporated and detected. Substitution with positron-emitting isotopes such as ¹¹C, ¹⁸F, ¹⁵O, and ¹³N can be used to examine substrate receptor occupancy in positron emission tomography (PET) studies. Isotopically labeled compounds of the present invention may be prepared by methods similar to those described in the accompanying schemes and/or examples and those in preparations by using appropriate isotopically labeled reagents in place of the previously employed non-labeled reagents. Pharmaceutically acceptable solvates of the present invention include those in which the crystallization solvent may be isotopically substituted, for example, D₂O, acetone-*d*₆ or DMSO-*d*₆.

The term "stereoisomer" refers to an isomer formed due to at least one asymmetric center. In compounds with one or more (e.g., 1, 2, 3 or 4) asymmetric centers, it may give rise to racemic mixtures, single enantiomers, diastereomeric mixtures and individual diastereomers. Certain individual molecules may also exist as geometric isomers (cis/trans). Similarly, compounds of the present invention may exist as mixtures of two or more structurally distinct forms in rapid equilibrium (often referred to as tautomers). Representative examples of tautomers include keto-enol tautomers, phenol-ketone tautomers, nitroso-oxime tautomers, and imine-enamine tautomers, etc.. It is to be understood that the scope of the present application encompasses all such isomers or mixtures in any proportion (e.g., 60%, 65%, 70%, 75%, 80%, 85%, 90%, 95%, 96%, 97%, 98%, 99%).

The present invention encompasses all possible crystalline forms or polymorphs of the compounds of the present invention, which may be a single polymorph or a mixture of more than one polymorph in any proportion.

It will also be understood that certain compounds of the present invention may exist in free form for therapeutic use, or, where appropriate, as pharmaceutically acceptable derivatives thereof. In the present invention, pharmaceutically acceptable derivatives include, but are not limited to: pharmaceutically acceptable salts, solvates, metabolites or prodrugs that, upon administration to a patient in need thereof, can directly or indirectly provide the compounds of the present invention or metabolites or residues thereof. Therefore, when reference is made herein to "a compound of the present invention", it is also intended to encompass the various derivative forms of the compound described above.

The pharmaceutically acceptable salts of the compounds of the present invention include acid addition salts and base addition salts thereof. Suitable acid addition salts are formed from acids that form pharmaceutically acceptable salts. Suitable base addition salts are formed from bases that form pharmaceutically acceptable salts. For a review of suitable salts, see Stahl and Wermuth, "Handbook of Pharmaceutical Salts: Properties, Selection, and Use" (Wiley-VCH, 2002). Methods for preparing the pharmaceutically acceptable salts of the compounds of the present invention are known to those skilled in the art.

The compounds of the present invention may exist in the form of solvates, preferably hydrates, wherein the compounds of the present invention comprise a polar solvent as a structural element of the crystal lattice of the compounds. The amount of polar solvent, especially water, may be present in a stoichiometric or non-stoichiometric ratio.

Those skilled in the art will understand that because nitrogen requires an available lone pair of electrons to oxidize to form an oxide, not all nitrogen-containing heterocycles are capable of forming N-oxides; and those skilled in the art will recognize nitrogen-containing heterocycles that are capable of forming N-oxides. Those skilled in the art will also recognize that tertiary amines are capable of forming N-oxides. Synthetic methods for the preparation of N-oxides of heterocycles and tertiary amines are well known to those skilled in the art and comprise using peroxyacids such as peracetic acid and m-chloroperoxybenzoic acid (MCPBA), hydrogen peroxide, alkyl hydroperoxides such as tert-butyl hydroperoxide, sodium perborate and dioxirane such as dimethyldioxirane to oxidize heterocycles and tertiary amines. These methods for preparing N-oxides have been extensively described and reviewed in the literature, see for example: T. L. Gilchrist, Comprehensive Organic Synthesis, vol. 7, pp 748-750; A. R. Katritzky and A. J. Boulton, Eds., Academic Press; and G. W. H. Cheeseman and E. S. G. Werstiuk, Advances in Heterocyclic Chemistry, vol. 22, pp 390-392; A. R. Katritzky and A. J. Boulton, Eds., Academic Press.

Also included within the scope of the present invention are metabolites of the compounds of the present invention, i.e., substances formed in the body upon administration of the compounds of the present invention. Such products may result, for example, from oxidation, reduction, hydrolysis, amidation, deamidation, esterification, enzymatic hydrolysis, etc. of the administered compounds. Accordingly, the present invention comprises metabolites of the compounds of the present invention, including compounds prepared by contacting a compound of the present invention with a mammal for a time sufficient to produce metabolites thereof.

The present invention further comprises within its scope the prodrugs of the compounds of the present invention, which are certain derivatives of the compounds of the present invention that may themselves have little or no pharmacological activity and can be converted by, for example, hydrolytic cleavage, into the compounds of the present invention having the desired activity after being administered in or on the body. Typically, such prodrugs will be functional derivatives of the compounds that can be readily converted into the compounds with desired therapeutic activity in the body. Additional information on the use of prodrugs can be found in "Pro-drugs as Novel Delivery Systems," Volume 14, ACS Symposium Series (T. Higuchi and V. Stella) and "Bioreversible Carriers in Drug Design," Pergamon Press, 1987 (Edited by E. B. Roche, American Pharmaceutical Association). The prodrugs of the present invention may be prepared, for example, by using certain moieties known to those skilled in the art as "pro-moieties" (e.g., those described in "Design of Prodrugs", H. Bundgaard (Elsevier, 1985)) to replace appropriate functional groups present in the compounds of the present invention.

As used herein, 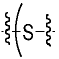 in the antibody-drug conjugate of the present invention indicates, when the targeting moiety is an antibody or antigen-binding fragment, a specific connection method between the sulfhydryl in the antibody or antigen-binding fragment and the linker.

As used herein, 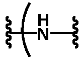 in the antibody-drug conjugate of the present invention indicates, when the targeting moiety is an antibody or antigen-binding fragment, a specific connection method between the amino group in the antibody or antigen-binding fragment and the linker.

Antibody-drug conjugates are also characterized by the average loading of the drug moiety (e.g., cytotoxic drug and TLR agonist) to the antibody-binding moiety, which is often referred to as drug-to-antibody ratio (DAR) of the conjugated sample. For example, from the LC-MS data of reduced and deglycosylated samples, and based on the average loading of LC and HC chains, the average DAR of the ADC can be calculated. The DAR of a given antibody-drug conjugate sample represents the average number of drug (payload) molecules attached to a tetrameric antibody containing two light chains and two heavy chains.

Although the drug-to-antibody ratio has an exact value (e.g., m in Formula (I)) for a particular antibody-drug conjugate molecule, it is understood that, when used to describe samples containing many molecules, this value will often be an average value, which is due to some degree of non-uniformity typically associated with the coupling step. The average loading of an antibody-drug conjugate sample is referred to herein as drug-to-antibody ratio or "DAR".

In some embodiments, the DAR value (drug/antibody ratio of a conjugate sample) of the antibody-drug conjugate is 1 to 10, for example: 1 to 2, 1 to 3, 1 to 4, 1 to 5, 1 to 6, 1 to 7, 1 to 8, 1 to 9, 1 to 10, 2 to 3, 2 to 4, 2 to 5, 2 to 6, 2 to 7, 2 to 8, 2 to 9, 2 to 10,3 to 4,3 to 5,3 to 6,3 to 7,3 to 8,3 to 9,3 to 10,4 to 5,4 to 6,4 to 7,4 to 8,4 to 9,4 to 10, 5 to 6, 5 to 7, 5 to 8, 5 to 9, 5 to 10, 6 to 7, 6 to 8, 6 to 9, 6 to 10, 7 to 8, 7 to 9, 7 to 10, 8 to 9, 8 to 10, or 9 to 10, preferably 3 to 8, for example, 3.0 to 3.5, 3.0 to 4.0, 3.0 to 4.5, 3.0 to 5.0, 3.0 to 5.5, 3.0 to 6.0, 3.5 to 4.0, 3.5 to 4.5, 3.5 to 5.0, 3.5 to 5.5, 3.5 to 6.0, 3.5 to 6.5, 3.5 to 7.0, 3.5 to 7.5, 3.5 to 8.0, 4.0 to 4.5, 4.0 to 5.0, 4.0 to 5.5, 4.0 to 6.0, 4.0 to 6.5, 4.0 to 7.0, 4.0 to 7.5, 4.0 to 8.0, 4.5 to 5.0, 4.5 to 5.5, 4.5 to 6.0, 4.5 to 6.5, 4.5 to 7.0, 4.5 to 7.5, 4.5 to 8.0, 5.0 to 5.5, 5.0 to 6.0, 5.0 to 6.5, 5.0 to 7.0, 5.0 to 7.5, 5.0 to 8.0, 5.5 to 6.0, 5.5 to 6.5, 5.5 to 7.0, 5.5 to 7.5, 5.5 to 8.0, 6.0 to 6.5, 6.0 to 7.0, 6.0 to 7.5, 6.0 to 8.0, 6.5 to 7.0, 6.5 to 7.5, 6.5 to 8.0, 7.0 to 7.5, 7.0 to 8.0, or 7.5 to 8.0.

In the present invention, "treatment" generally refers to the partial or complete stabilization or cure of a disease and/or the side effects resulting from the disease. As used herein, "treatment" encompasses any treatment to a patient's disease, which comprises: (a) inhibiting a symptom of the disease, i.e., preventing its progression; or (b) alleviating a symptoms of the disease, i.e., causing regression of the disease or symptom.

In the present invention, "prevention" refers to inhibiting and delaying the onset of a disease, including not only prevention before the development of the disease, but also prevention of the recurrence of the disease after treatment.

In the present invention, "subject" refers to a vertebrate animal. In some embodiments, the vertebrate animal refers to a mammal. The mammal includes, but is not limited to, livestock (e.g., cattle), pet (e.g., cat, dog, and horse), primate, mouse, and rat. In some embodiments, the mammal is a human.

In the present invention, "effective amount" refers to an amount effective in the necessary dosage and time to achieve the desired therapeutic effect. The "therapeutically effective amount" may vary depending on factors such as the disease state, age, sex and weight of an individual and the ability of an active ingredient to elicit a desired response in the individual. A therapeutically effective amount also encompasses an amount of an active ingredient in which the therapeutically beneficial effects outweigh any toxic or harmful consequences. In cancer treatment, a therapeutically effective amount of a drug can reduce the number of cancer cells; reduce the size of a tumor; inhibit (i.e., slow down to a certain extent, preferably stop) the infiltration of cancer cells into surrounding organs; inhibit (i.e., slow down to a certain extent, preferably stop) tumor metastasis; inhibit tumor growth to a certain extent; and/or alleviate one or more symptoms related to cancer to a certain extent.

### Specific Models for Carrying Out the present Invention

In order to make the purpose and technical solutions of the present invention clearer, the embodiments of the present invention will be described in detail below with reference to the examples. However, those skilled in the art will understand that the following examples are only used to illustrate the present invention and should not be regarded as limiting the scope of the present invention. If specific conditions are not indicated in the examples, conventional conditions or conditions recommended by the manufacturer are carried out accordingly. If the manufacturers of the reagents or instruments used are not indicated, they were all commercially available conventional products.

The structures of the compounds were determined by nuclear magnetic resonance (¹H NMR) or mass spectrometry (MS). The measuring instrument for ¹H NMR was JEOL Eclipse 400 nuclear magnetic instrument. The measuring solvent was deuterated methanol (CD₃OD), deuterated chloroform (CDCl₃) or hexadeuterated dimethylsulfoxide (DMSO-*d*₆). The internal standard was tetramethylsilane (TMS), and the chemical shifts (δ) were given in parts per million (ppm).

The measuring instrument for MS was Agilent (ESI) mass spectrometer, manufacturer: Agilent, model: Agilent 6120B.

Preparation method for high performance liquid chromatography:
Instrument model: Agilent 1260, chromatographic column: Waters SunFire Prep C18 OBD (19 mm×150 mm×5.0 µm); column temperature: 25°C; flow rate: 20.0 mL/min; detection wavelength: 214 nm; elution gradient: (0 min: 10% A, 90% B; 16.0 min: 90% A, 10% B); mobile phase A: acetonitrile; mobile phase B: 0.05% formic acid aqueous solution.

The thin layer chromatography silica gel plate (TLC) as used was aluminum plate (20 × 20 cm) produced by Merck, and the specification of the silica gel plate for TLC separation and purification was GF 254 (1 mm) produced in Yantai.

The reaction was monitored by thin layer chromatography (TLC) or LC-MS; the solvent system used for development comprised: dichloromethane/methanol system, n-hexane/ethyl acetate system, and petroleum ether/ethyl acetate system, and the volume ratio of the solvents can be adjusted according to the polarity of the compound or by adding triethylamine, etc.

Preparation method for reverse column chromatography:

### Preparation Method A:

Instrument model: Biotage rapid medium-pressure preparative chromatography, chromatographic column: Agela C18 reverse column (Spherical; 20-35µm; 100A); chromatographic column temperature: 25°C; flow rate: 28.0 mL/min; detection wavelength: 220 nm; mobile phase A: acetonitrile; mobile phase B: water;

### Preparation Method B:

Instrument model: Biotage rapid medium-pressure preparative chromatography, chromatographic column: Agela C18 reverse column (Spherical; 20-35µm; 100A); chromatographic column temperature: 25°C; flow rate: 28.0 mL/min; detection wavelength: 220 nm; mobile phase A: acetonitrile; mobile phase B: 0.05% formic acid aqueous solution;

### Preparation Method C:

Instrument model: Biotage rapid medium-pressure preparative chromatography, chromatographic column: Agela C18 reverse column (Spherical; 20-35µm; 100A); chromatographic column temperature: 25°C; flow rate: 28.0 mL/min; detection wavelength: 220 nm; mobile phase A: acetonitrile; mobile phase B: 0.05% ammonium bicarbonate aqueous solution;

Microwave reaction was carried out by using Biotage Initiator + (400 W, RT ~ 300°C) microwave reactor.

200-300 mesh silica gel was usually used as the carrier of column chromatography. The eluent system comprised: dichloromethane/methanol system, as well as petroleum ether/ethyl acetate system. The volume ratio of the solvents was adjusted according to the polarity of the compound or by adding a small amount of triethylamine.

Unless otherwise specified in the examples, the reaction temperature was room temperature (20°C to 35°C).

The reagents used in the present invention were purchased from Acros Organics, Aldrich Chemical Company, Tebo Chemical and other companies.

In general preparation methods, examples, and intermediate preparations examples, the meanings of abbreviations were as follows.

| Abbreviation | Meaning | Abbreviation | Meaning |
|---|---|---|---|
| TLC | Thin layer chromatography | DMSO-*d*₆ | Hexadeuterated dimethylsulfoxide |
| LC-MS | Liquid chromatography-mass spectrometry | s | singlet |
| DMSO | Dimethylsulfoxide | d | doublet |
| TCEP | Tris(2-carboxyethyl)phosphine | t | triplet |
| EDTA | Ethylenediaminetetraacetic acid | q | quartet |
| MS | Mass spectrum | dd | double doublet |
| NMR | Nuclear magnetic resonance | m | multiplet |
| TMS | Tetramethylsilane | br | broad |
| CD₃OD | Deuterated methanol | *J* | Coupling constant |
| CDCl₃ | Deuterated chloroform | Hz | hertz |

### 1. Examples of compounds

### Intermediate Preparation Example 1: Preparation of 2-((1-((4-((4-(4-amino-2-butyl-7-methyl-1H-imidazo[4,5-d]thieno[3,2-b]pyridin-1-yl)butyl)carbamoyl)cyclohexyl)methyl)-2,5-dioxopyrrol idin-3-yl)thio)acetic acid (Compound Int1)

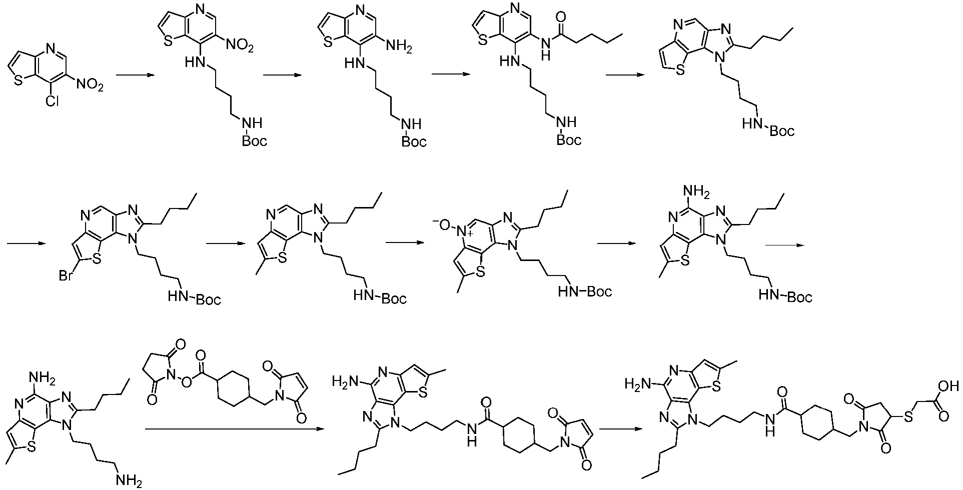

### Step 1: Preparation of tert-butyl (4-((6-nitrothieno[3,2-b]pyridin-7-yl)amino)butyl)carbamate

7-Chloro-6-nitrothieno[3,2-*b*]pyridine (10.0 g, 46.59 mmol) and N-tert-butoxycarbonyl-1,4-butanediamine (10.53 g, 55.91 mmol) were dissolved in N,N-dimethylformamide (150 mL), cooled in an ice-water bath, added with N,N-diisopropylethylamine (12.04 g, 93.18 mmol) at 0°C, and warmed slowly to room temperature and stirred for 6 hours. The reaction solution was poured into water (1500 mL) and extracted three times with ethyl acetate (450 mL). The organic phases were combined, washed three times with saturated brine (150 mL), dried over anhydrous sodium sulfate and concentrated to obtain the title compound of this step (16.61 g, yield: 97.3%).

MS m/z (ESI): 367.1 [M+H]⁺.

### Step 2: Preparation of tert-butyl 4-((6-aminothieno[3,2-b]pyridin-7-yl)amino)butyl)carbamate

Tert-butyl (4-((6-nitrothieno[3,2-*b*]pyridin-7-yl)amino)butyl)carbamate (16.61 g, 49.37 mmol) was dissolved in a mixed solvent of tetrahydrofuran (100 mL) and methanol (100 mL), added with 10% palladium on carbon (2.0 g, 11.97 mmol), subjected to gas replacement with hydrogen three times. The reaction system was stirred at room temperature for 12 hours, then subjected to suction filtration. The filter cake was washed with methanol (30 mL), and the filtrate was concentrated to obtain the title compound of this step (14.47 g, yield: 87.1%).

MS m/z (ESI): 337.1 [M+H]⁺.

### Step 3: Preparation of tert-butyl (4-((6-pentanamidothieno[3,2-b]pyridin-7-yl)amino)butyl)carbamate

Tert-butyl (4-((6-aminothieno[3,2-*b*]pyridin-7-yl)amino)butyl)carbamate (14.47 g, 43.01 mmol) and *N*,*N*-diisopropylethylamine (11.12 g, 86.02 mmol) were dissolved in tetrahydrofuran (250 mL), cooled in an ice-water bath, added slowly with n-pentanoyl chloride (7.78 g, 64.52 mmol) in a dropwise manner at 0°C, slowly warmed to room temperature and stirred for 4 hours. Water (100 mL) was added to the reaction system to quench the reaction. The reaction system was concentrated to remove tetrahydrofuran as much as possible. The residue was added with water (300 mL) and extracted three times with ethyl acetate (300 mL). The organic phases were combined, washed three times with saturated brine (150 mL), dried over anhydrous sodium sulfate, and concentrated to obtain the title compound of this step (16.72 g, yield: 92.4%).

MS m/z (ESI): 421.0 [M+H]⁺.

### Step 4: Preparation of tert-butyl (4-(2-butyl-1H-imidazo[4,5-d]thieno[3,2-b]pyridin-1-yl)butyl)carbamate

Tert-butyl (4-((6-pentanamidothieno[3,2-*b*]pyridin-7-yl)amino)butyl)carbamate (16.72 g, 39.76 mmol) and *o*-chlorobenzoic acid (3.11 g, 19.88 mmol) were added to toluene (300 mL), heated to reflux, and reacted under stirring and separating water for 4 hours. After the reaction system was cooled to room temperature, the solvent was removed by concentration. The residue was purified by silica gel column chromatography (eluent: 100% ethyl acetate) to obtain the title compound of this step (14.76 g, yield: 92.2%).

MS m/z (ESI): 403.2 [M+H]⁺.

### Step 5: Preparation of tert-butyl (4-(7-bromo-2-butyl-1H-imidazo[4,5-d]thieno[3,2-b]pyridin-1-yl)butyl)carbamate

Tert-butyl (4-(2-butyl-1H-imidazo[4,5-*d*]thieno[3,2-*b*]pyridin-1-yl)butyl)carbamate (5.41 g, 13.44 mmol) was completely dissolved in a mixed solvent of *N*,*N*-dimethylformamide (75 mL) and glacial acetic acid (25 mL), cooled in an ice-water bath, added with *N*-bromosuccinimide (4.78 g, 26.88 mmol) slowly in batches to the reaction solution at 0°C, slowly warmed to room temperature and stirred for 24 hours. The reaction solution was poured into water (300 mL), and extracted three times with ethyl acetate (300 mL). The organic phases were combined, washed three times with saturated sodium bicarbonate aqueous solution (150 mL), washed three times with saturated brine (150 mL), dried over anhydrous sodium sulfate and concentrated. The residue was purified by silica gel column chromatography (eluent: ethyl acetate/petroleum ether = 1/1 (v/v)) to obtain the title compound of this step (4.95 g, yield: 76.5%).

MS m/z (ESI): 481.0 [M+H]⁺.

### Step 6: Preparation of tert-butyl (4-(2-butyl-7-methyl-1H-imidazo[4,5-d]thieno[3,2-b]pyridin-1-yl)butyl)carbamate

Tert-butyl (4-(7-bromo-2-butyl-1*H*-imidazo[4,5-*d*]thieno[3,2-b]pyridin-1-yl)butyl)carbamate (1.0 g, 2.08 mmol), trimethylboroxine (50 wt.% solution in tetrahydrofuran, 2.62 g, 10.4 mmol), potassium carbonate (575.0 mg, 4.16 mmol), and [1,1'-bis(diphenylphosphino)ferrocene]dichloropalladium (307.3 mg, 0.42 mmol) were added in sequence to a mixed solvent of 1,4-dioxane (10 mL) and water (1 mL). After the reaction system was bubbled with nitrogen for 5 minutes, it was placed under microwave, heated to 100°C and stirred for 2 hours. After the reaction system was cooled to room temperature, the solvent was removed by concentration. The residue was purified by silica gel column chromatography (eluent: 100% ethyl acetate) to obtain the title compound of this step (0.65 g, yield: 75.0%).

MS m/z (ESI): 417.1 [M+H]⁺.

### Step 7: 1-(4-((tert-butoxycarbonyl)amino)butyl)-2-butyl-7-methyl-1H-imidazo[4,5-d] thieno[3,2-b]pyridine-5-oxide

Tert-butyl (4-(2-butyl-7-methyl-1*H*-imidazo[4,5-*d*]thieno[3,2-*b*]pyridin-1-yl)butyl) carbamate (0.65 g, 1.56 mmol) was dissolved in dichloromethane (10 mL), cooled in an ice-water bath, added slowly with *m*-chloroperoxybenzoic acid (538.5 mg, 3.12 mmol) in batches to the reaction solution at 0°C, slowly warmed to room temperature and stirred for 4 hours. The reaction solution was poured into water (100 mL) and extracted three times with dichloromethane (60 mL). The organic phases were combined, washed twice with saturated sodium bicarbonate aqueous solution (30 mL), washed twice with saturated sodium sulfite aqueous solution (30 mL), washed three times with saturated brine (45 mL), dried over anhydrous sodium sulfate and concentrated to obtain the title compound of this step (640 mg, yield: 94.8%).

MS m/z (ESI): 433.2 [M+H]⁺.

### Step 8: Preparation of tert-butyl (4-(4-amino-2-butyl-7-methyl-1H-imidazo[4,5-d]thieno[3,2-b]pyridin-1-yl)butyl)carbamate

1-(4-((Tert-butoxycarbonyl)amino)butyl)-2-butyl-7-methyl-1*H*-imidazo[4,5-d*]*thieno[3,2-*b*]pyridine-5-oxide (640 mg, 1.48 mmol) was dissolved in a mixed solvent of dichloromethane (10 mL) and ammonia (5 mL), cooled in an ice-water bath, added slowly with *p*-toluenesulfonyl chloride (564.1 mg, 2.96 mmol) in batches to the reaction solution at 0°C, slowly warmed to room temperature and stirred for 12 hours. The reaction system was added with water (100 mL) and extracted three times with dichloromethane (60 mL). The organic phases were combined, washed three times with saturated brine (45 mL), dried over anhydrous sodium sulfate, and concentrated. The residue was purified by silica gel column chromatography (eluent: ethyl acetate/methanol = 15/1 (v/v)) to obtain the title compound of this step (266 mg, yield: 41.6%).

MS m/z (ESI): 432.2 [M+H]⁺.

### Step 9: Preparation of 1-(4-aminobutyl)-2-butyl-7-methyl-1H-imidazo[4,5-d]thieno[3,2-b]pyridin-4-amine (Compound 1)

Tert-butyl (4-(4-amino-2-butyl-7-methyl-1H-imidazo[4,5-d]thieno[3,2-b]pyridin-1-yl)butyl) carbamate (266 mg, 0.62 mmol) was dissolved in a mixed solvent of methanol (10 mL) and hydrogen chloride in 1,4-dioxane (4N, 5 mL), and stirred at room temperature for 12 hours. The solvent was removed by concentration. A mixed solvent of dichloromethane and methanol (volume ratio: 4:1, 10 mL) and a saturated sodium bicarbonate aqueous solution (2 mL) were added in sequence to the residue. After stirring at room temperature for 5 minutes, the solvent was removed by concentration. The obtained residue was purified by high-pressure liquid chromatography to obtain the title compound (130 mg, yield: 63.3%).
MS m/z (ESI): 332.1 [M+H]⁺.
¹H NMR (400 MHz, DMSO-d₆): δ 8.34 (s, 2H), 6.98 (d, *J =* 1.2 Hz, 1H), 6.06 (s, 2H), 4.24 (t, *J =* 8.0 Hz, 2H), 2.86 (t, *J =* 8.0 Hz, 2H), 2.78 (t, *J =* 8.0 Hz, 2H), 2.56 (d, *J =* 1.2 Hz, 1H), 1.83-1.75 (m, 4H), 1.62-1.58 (m, 2H), 1.47-1.40 (m, 2H), 0.96 (t, *J =* 8.0 Hz, 3H).

### Step 10: Preparation of N-(4-(4-amino-2-butyl-7-methyl-1H-imidazo[4,5-d]thieno[3,2-b]pyridin-1-yl)butyl)-4-((2,5-dioxo-2,5-di hydro-1H-pyrrol-1-yl)methyl)cyclohexane-1-carboxamide

1-(4-Aminobutyl)-2-butyl-7-methyl-1*H*-imidazo[4,5-*d*]thieno[3,2-*b*]pyridin-4-amine (200 mg, 0.60 mmol) and *N*,*N*-diisopropylethylamine (155 mg, 1.2 mmol) were added to *N*,*N*-dimethylformamide (2 mL), added with 2,5-dioxopyrrolidin-1-yl-4-((2,5-dioxo-2,5-dihydro-1H-pyrrol-1-yl)methyl)cyclohexane-1-carboxylate (240 mg, 0.72 mmol) in dichloromethane (1 mL) in a dropwise manner, and the resulting reaction mixture was stirred at 25°C for 12 hours. The reaction system was added with water, extracted three times with dichloromethane. The organic phases were collected, washed with saturated brine, dried over anhydrous sodium sulfate, and filtered. The filtrate was concentrated to obtain a crude product, which was subjected to purification by silica gel column chromatography (eluent: dichloromethane/methanol= 10/1 (v/v)) to obtain the title compound of this step (282 mg, yield: 85.4%).

MS m/z (ESI): 551.3 [M+H]⁺.

### Step 11: Preparation of 2-((1-((4-((4-(4-amino-2-butyl-7-methyl-1H-imidazo[4,5-d]thieno[3, 2-b]pyridin-1-yl)butyl)carbamoyl)cyclohexyl)methyl)-2,5-dioxopyrrolidin-3-yl)thio)acetic acid (Int1)

*N*-(4-(4-Amino-2-butyl-7-methyl-1*H*-imidazo[4,5-*d*]thieno[3,2-*b*]pyridin-1-yl)butyl)-4-((2,5-dioxo-2 ,5-dihydro-1*H*-pyrrol-1-yl)methyl)cyclohexane-1-carboxamide (282 mg, 0.51 mmol) and thioglycolic acid (94 mg, 1.02 mmol) were added to dichloromethane (50 mL), and the resulting reaction mixture was stirred at 25°C for 30 minutes. The reaction solution was concentrated to obtain a yellow solid, which was slurried with methyl tert-butyl ether to obtain the title compound of this step (237 mg, yield: 72.3%).

MS m/z (ESI): 643.3 [M+H]⁺.

### Intermediate Preparation Example 2: Preparation of N-(tert-butyl)-2-butyl-7-(piperidin-4-yl)-1H-imidazo[4,5-d]thieno[3,2-b]pyridin-4-amine (Compound Int2)

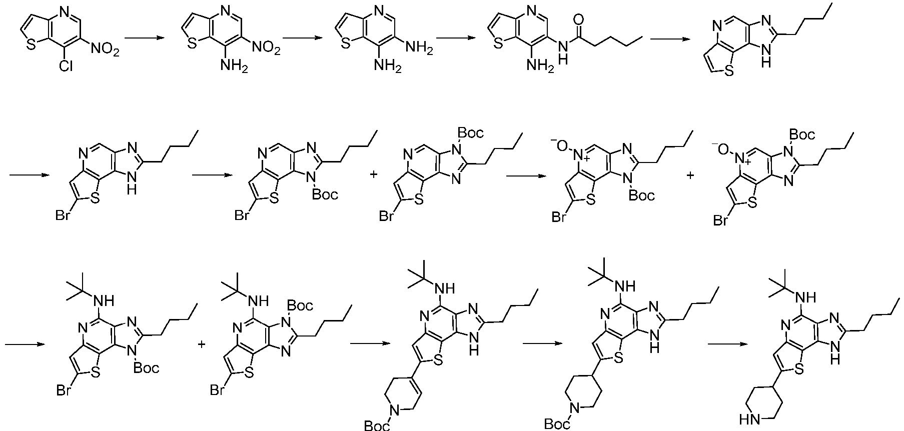

### Step 1: Preparation of 6-nitrothieno[3,2-b]pyridin-7-amine

7-Chloro-6-nitrothieno[3,2-*b*]pyridine (2.0 g, 9.23 mmol) was dissolved in methanol (20 mL), added with ammonia in methanol solution (7 N, 26.4 mL, 184.8 mmol), heated to 50°C and stirred for 4 hours. After the reaction system was cooled to room temperature, the solvent was removed by concentration to obtain the title compound of this step (1.73 g, yield: 96.1%).

MS m/z (ESI): 196.1 [M+H]⁺.

### Step 2: Preparation of thieno[3,2-b]pyridine-6,7-diamine

The synthetic route of Intermediate Preparation Example 1 was adopted. The reaction raw material tert-butyl (4-((6-nitrothieno[3,2-*b*]pyridin-7-yl)amino)butyl)carbamate in step 2 was replaced with 6-nitrothieno[3,2-*b*]pyridin-7-amine to obtain the title compound in this step (1.4 g, yield: 98.4%).

MS m/z (ESI): 166.1 [M+H]⁺.

### Step 3: Preparation of N-(7-aminothieno[3,2-b]pyridin-6-yl)pentanamide

The synthetic route of Intermediate Preparation Example 1 was adopted. The reaction raw material tert-butyl (4-((6-aminothieno[3,2-*b*]pyridin-7-yl)amino)butyl)carbamate in step 3 was replaced with thieno[3,2-*b*]pyridine-6,7-diamine to obtain the title compound of this step (2.5 g, yield: 97.6%).

MS m/z (ESI): 250.1 [M+H]⁺.

### Step 4: Preparation of 2-butyl-1H-imidazo[4,5-d]thieno[3,2-b]pyridine

*N*-(7-Aminothieno[3,2-*b*]pyridin-6-yl)pentanamide (2.5 g, 8.48 mmol) was added to ethanol (30 mL), followed by an addition of sodium hydroxide (1.7 g, 42.4 mmol) ) at room temperature, the resulting mixture was heated to 50°C and stirred for 6 hours. The reaction system was quenched by water (150 mL), adjusted pH to 6 by dilute hydrochloric acid, extracted three times with ethyl acetate (150 mL). The organic phases were combined, washed twice with saturated sodium bicarbonate solution (60 mL), washed twice with saturated brine (60 mL), dried over anhydrous sodium sulfate, and concentrated to obtain the title compound of this step (1.5 g, yield: 76.5%).

MS m/z (ESI): 232.0 [M+H]⁺.

### Step 5: Preparation of 7-bromo-2-butyl-1H-imidazo[4,5-d]thieno[3,2-b]pyridine

The synthetic route of Intermediate Preparation Example 1 was adopted. The reaction raw material tert-butyl (4-(2-butyl-1*H*-imidazo[4,5-*d*]thiophene[3,2-*b*]pyridin-1-yl)butyl)carbamate in step 5 was replaced with 2-butyl-1*H*-imidazo[4,5-*d*]thieno[3,2-*b*]pyridine to obtain the title compound of this step (1.4 g, yield: 65.7%).

MS m/z (ESI): 310.0 [M+H]⁺.

### Step 6: Preparation of mixture of tert-butyl 7-bromo-2-butyl-1H-imidazo[4,5-d]thieno[3,2-b]pyridine-1-carboxylate and tert-butyl 7-bromo-2-butyl-3H-imidazo[4,5-d]thieno[3,2-b]pyridine-3-carboxylate

7-Bromo-2-butyl-1H-imidazo[4,5-d]thieno[3,2-b]pyridine (1 g, 3.20 mmol) and 4-dimethylaminopyridine (40 mg, 0.32 mmol) were dissolved in tetrahydrofuran (10 mL), added with triethylamine (0.65 g, 6.40 mmol) and di-tert-butyl dicarbonate (1.1 g, 4.8 mmol) in sequence at room temperature, heated to 40°C and stirred for 12 hours. The reaction solution was poured into water (100 mL) and extracted three times with dichloromethane (60 mL). The organic phases were combined, washed three times with saturated brine (30 mL), dried over anhydrous sodium sulfate and concentrated. The residue was purified by silica gel column chromatography (eluent: ethyl acetate/petroleum ether = 3/1 (v/v)) to obtain the mixture of the title compounds of this step (1.0 g, yield: 76.7%).

MS m/z (ESI): 410.0 [M+H]⁺.

### Step 7: Preparation of mixture of 7-bromo-1-(tert-butoxycarbonyl)-2-butyl-1H-imidazo[4,5-d]thieno[3,2-b]pyridine-5-oxide and 7-bromo-3-(tert-butoxycarbonyl)-2-butyl-3H-imidazo[4,5-d]thieno[3,2-b]pyridine-5-oxide

The mixture of tert-butyl 7-bromo-2-butyl-1H-imidazo[4,5-*d*]thieno[3,2-b]pyridine- 1-carboxylate and tert-butyl 7-bromo-2-butyl-3*H*-imidazo[4,5-*d*]thieno[3,2-*b*]pyridine-3-carboxylate (1.0 g, 2.43 mmol) was dissolved in dichloromethane (10 mL), cooled in an ice-water bath, slowly added with *m*-chloroperbenzoic acid (0.98 g, 4.86 mmol) in batches to the reaction solution at 0°C, slowly heated to room temperature and stirred for 4 hours. The reaction solution was poured into water (100 mL) and extracted three times with dichloromethane (60 mL). The organic phases were combined, washed twice with saturated sodium bicarbonate aqueous solution (30 mL), washed twice with saturated sodium sulfite aqueous solution (30 mL), washed three times with saturated brine (45 mL), dried over anhydrous sodium sulfate and concentrated to obtain the mixture of the title compounds of this step (1.0 g, yield: 90.9%).

MS m/z (ESI): 426.0 [M+H]⁺.

### Step 8: Preparation of mixture of tert-butyl 7-bromo-2-butyl-4-(tert-butylamino)-1H-imidazo[4,5-d]thieno[3,2-b]pyridine-1-carboxylate and tert-butyl 7-bromo-2-butyl-4-(tert-butylamino)-3H-imidazo[4,5-d]thieno[3,2-b]pyridine-3-carboxylate

The mixture of 7-bromo-1-(tert-butoxycarbonyl)-2-butyl-1*H*-imidazo[4,5-d]thieno [3,2-*b*]pyridine-5-oxide and 7-bromo-3-(tert-butoxycarbonyl)-2-butyl-3*H*-imidazo[4,5-*d*]thieno [3,2-*b*]pyridine-5-oxide (1.0 g, 2.34 mmol) and tert-butylamine (0.85 g, 11.7 mmol) were dissolved in dichloromethane (20 mL), cooled in an ice-water bath, slowly added with *p*-toluenesulfonyl chloride (0.89 g, 4.68 mmol) in batches to the reaction solution at 0°C, heated to room temperature and stirred for 12 hours. The reaction system was added with water (100 mL), extracted three times with dichloromethane (60 mL). The organic phases were combined, washed three times with saturated brine (45 mL), dried over anhydrous sodium sulfate. and concentrated. The residue was purified by silica gel column chromatography (eluent: ethyl acetate/petroleum ether = 5/1 (v/v)) to obtain the mixture of the title compounds of this step (0.6 g, yield: 53.1%).

MS m/z (ESI): 481.2 [M+H]⁺.

### Step 9: Preparation of tert-butyl 4-(2-butyl-4-(tert-butylamino)-1H-imidazo[4,5-d]thieno [3,2-b]pyridin-7-yl)-3,6-dihydropyridine-1(2H)-carboxylate

The mixture of tert-butyl 7-bromo-2-butyl-4-(tert-butylamino)-1*H*-imidazo[4,5-d]thieno [3,2-*b*]pyridine-1-carboxylate and tert-butyl 7-bromo-2-butyl-4-(tert-butylamino)-3*H*-imidazo [4,5-*d*]thieno[3,2-b]pyridine-3-carboxylate (1 g, 2.1 mmol) was added to a mixed solution of 1,4-dioxane (20 mL) and water (2 mL), then added with tert-butyl 4-(4,4,5,5-tetramethyl-1,3,2-dioxaborolan-2-yl)-5,6-dihydropyridine-1(2*H*)-carboxylate (0.85 g, 2.73 mmol) and potassium carbonate (0.6 g, 4.2 mmol), subjected to gas replacement with nitrogen three times, added with 1,1'-bis(diphenylphosphino)ferrocene palladium dichloride (76 mg, 0.1 mmol), heat to 90°C under microwave and reacted for 2 hours. After the reaction was completed, the reaction system was cooled to room temperature and diluted with water, then extracted with dichloromethane. The organic phases were combined and washed with brine. The crude product obtained by concentrating the combined organic phase was purified by silica gel column chromatography (eluent: petroleum ether/ethyl acetate = 3/1 (v/v)) to obtain the title compound of this step (0.8 g, yield: 80.0%).

MS m/z (ESI): 484.3 [M+H]⁺.

### Step 10: Preparation of tert-butyl 4-(2-butyl-4-(tert-butylamino)-1H-imidazo[4,5-d] thieno[3,2-b]pyridin-7-yl)piperidine-1-carboxylate

Under nitrogen, to a solution of tert-butyl 4-(2-butyl-4-(tert-butylamino)-1*H*-imidazo[4,5-*d*] thieno[3,2-*b*]pyridin-7-yl)-3,6-dihydropyridine-1(2*H*)-carboxylate (0.8 g, 1.66 mmol) in methanol (20 mL), 10% palladium on carbon (50 mg) was added, subjected to gas replacement with hydrogen three times, and stirred at room temperature for 24 hours. After the reaction was completed, the palladium on carbon was removed by filtration. The filtrate was concentrated. The obtained solid was dried under vacuum to obtain the title compound of this step (0.78 g, yield: 97.5%).

MS m/z (ESI): 486.3 [M+H]⁺.

### Step 11: Preparation of N-(tert-butyl)-2-butyl-7-(piperidin-4-yl)-1H-imidazo[4,5-d] thieno[3,2-b]pyridine -4-amine (Int2)

To a solution of tert-butyl 4-(2-butyl-4-(tert-butylamino)-1*H*-imidazo[4,5-*d*]thieno[3,2-*b*] pyridin-7-yl)piperidine-1-carboxylate (0.78 g, 1.61 mmol) in methanol (10 mL), hydrogen chloride in 1,4-dioxane (2 mL, 4 M, 8 mmol) was added, and reacted at room temperature for 12 hours. After the reaction was completed, the reaction solution was concentrated. The obtained solid was dried under vacuum to obtain the title compound of this step (0.57 g, yield: 91.9%).

MS m/z (ESI): 386.2 [M+H]⁺.

### Intermediate Preparation Example 3: Preparation of Compound Int3

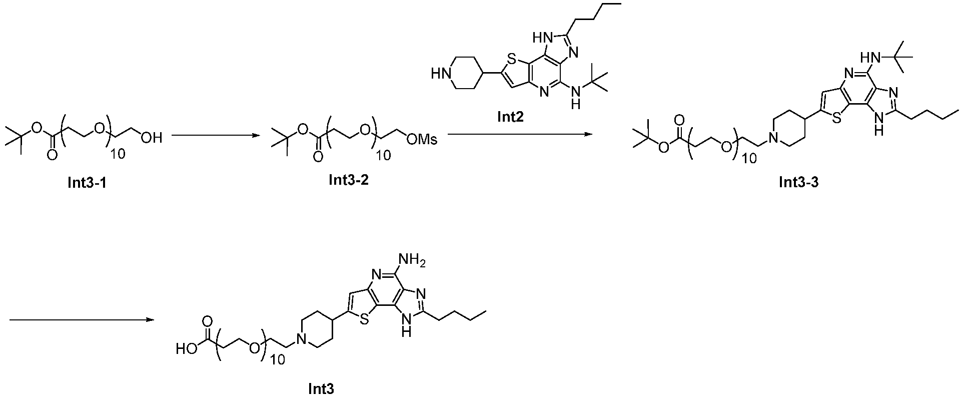

### Step 1: Preparation of Compound Int3-2

To a solution of Compound **Int3-1** (2 g, 3.41 mmol) in dichloromethane (30 mL), triethylamine (0.69 g, 6.82 mmol) was added, followed by an addition of methylsulfonyl chloride (0.58 g, 5.12 mmol) in ice-water bath, and stirred at room temperature overnight. After the reaction was completed, the reaction solution was diluted with dichloromethane, then washed with sodium carbonate solution and brine in sequence, and the organic phase was concentrated to obtain the title compound of this step (2.2 g, yield: 95.6%).

MS m/z (ESI): 682.4 [M+NH₄]⁺.

### Step 2: Preparation of Compound Int3-3

To a solution of Compound **Int2** (1 g, 2.59 mmol) in *N*,*N*-dimethylformamide (15 mL), Compound **Int3-2** (2.2 g, 3.37 mmol) and N,N-diisopropylethylamine (0.88 g, 6.74 mmol) were added, the reaction mixture was reacted at 90°C for 24 hours. After the reaction was completed, the reaction mixture was diluted with water and extracted with dichloromethane. The organic phases were combined and washed with brine. The crude product obtained by concentrating the combined organic phase was purified by silica gel column chromatography (eluent: dichloromethane/methanol=10/1 (v/v)) to obtain the title compound of this step (1.2 g, yield: 37.5%).

MS m/z (ESI): 954.4 [M+H]⁺.

### Step 3: Preparation of Compound Int3

To a solution of Compound **Int3-3** (1.2 g, 1.26 mmol) in 1,4-dioxane (10 mL), a solution of hydrogen chloride in 1,4-dioxane (3.2 mL, 4 M, 12.6 mmol) was added. The reaction mixture was reacted at 90°C for 12 hours. After the reaction was completed, the reaction solution was concentrated to obtain the crude title compound of this step (1 g, yield: 90.9%).

MS m/z (ESI): 842.4 [M+H]⁺.

### Intermediate Preparation Example 4: Preparation of 6-(2,5-dioxy-2,5-dihydro-1H-pyrrol-1-yl)-N-(prop-2-yn-1-yl)hexanamide

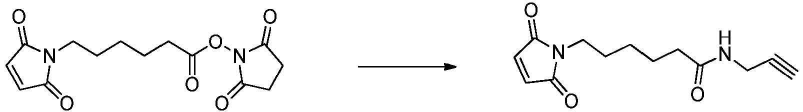

*N*-Succinimidyl 6-(maleimido)hexanoate (631.58 mg, 2.05 mmol) was dissolved in *N*,*N*-dimethylformamide (4 mL), added with propargylamine (124.12 mg, 2.25 mmol) and triethylamine (414.87 mg, 4.1 mmol), stirred at 25°C for 12 hours, concentrated to obtain a crude product, which was then purified by silica gel column chromatography (eluent: dichloromethane/methanol=20/1 (v/v)) to obtain the title compound of this step (256 mg, yield: 50.2%).

MS m/z (ESI): 249.1 [M+H]⁺.

### Intermediate Preparation Example 5: Preparation of Compound Int5

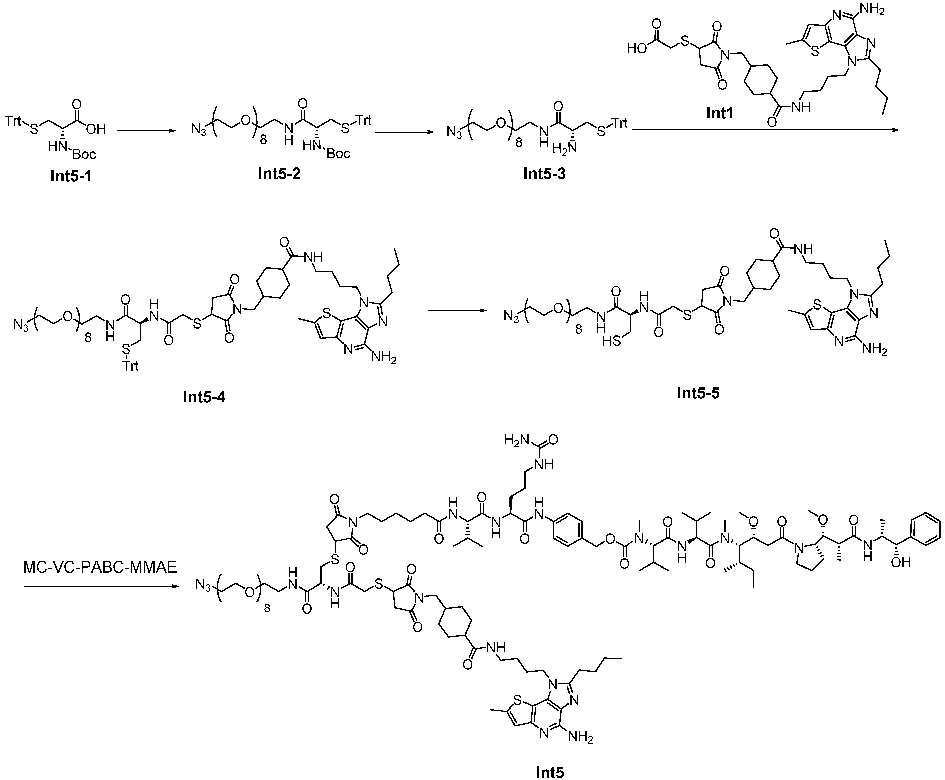

### Step 1: Preparation of Compound Int5-2

*N*-Tert-butoxycarbonyl-*S*-trityl-*L*-cysteine (384 mg, 0.83 mmol) and 26-azido-3,6,9,12,15,18,21,24-octoxohexacosane-1-amine (435 mg, 0.99 mmol) were dissolved in *N*,*N*-dimethylformamide (4 mL), added with 1-hydroxybenzotriazole (167.58 mg, 1.24 mmol), *N*,*N*-diisopropylethylamine (320.43 mg, 2.48 mmol) and 1-(3-dimethylaminopropyl)-3-ethylcarbodiimide hydrochloride (238 mg, 1.24 mmol)) under ice bath, stirred at 20°C for 3 hours, and the reaction solution was added dropwise into water, extracted with dichloromethane, the organic phase was dried and concentrated to obtain the title compound of this step (0.69 g, yield: 78.4%).

MS m/z (ESI): 884.4 [M+H]⁺.

### Step 2: Preparation of Compound Int5-3

Compound **Int5-2** (0.69 g, 0.78 mmol) was dissolved in dichloromethane (10 mL), added dropwise with trifluoroacetic acid (10 mL), stirred at room temperature for 2 hours, concentrated, and diluted with dichloromethane (20 mL). The organic phase was washed with sodium bicarbonate solution, dried and concentrated to obtain the title compound of this step (0.6 g, yield: 98%).

MS m/z (ESI): 784.4 [M+H]⁺.

### Step 3: Preparation of Compound Int5-4

Compound **Int1** (136 mg, 211 mmol) and Compound **Int5-3** (182 mg, 233 mmol) were dissolved in *N*,*N*-dimethylformamide (4 mL), added with 1-hydroxybenzotriazole (43.2 mg, 0.32 mmol), *N*,*N*-diisopropylethylamine (81.6 mg, 0.63 mmol) and 1-(3-dimethylaminopropyl)-3-ethylcarbodiimide hydrochloride (61.3 mg, 0.32 mmol) under ice bath, stirred at 20°C for 3 hours, the reaction solution was added dropwise into water, extracted with dichloromethane, and the organic phase was dried and concentrated to obtain the title compound of this step (0.23 g, yield: 95.8%).

MS m/z (ESI): 1408.6 [M+H]⁺.

### Step 4: Preparation of Compound Int5-5

Compound **Int5-4** (0.23 g, 0.16 mmol) was dissolved in dichloromethane (2 mL) and triethylsilane (0.52 mL), added dropwise with trifluoroacetic acid (1 mL), stirred at room temperature for 1 hour, concentrated, and diluted with dichloromethane (20 mL). The organic phase was washed with sodium bicarbonate solution, dried and concentrated to obtain the title compound of this step (0.15 g, yield: 78.9%).

MS m/z (ESI): 1166.5 [M+H]⁺.

### Step 5: Preparation of Compound Int5

Compound **Int5-5** (0.15 g, 0.13 mmol) was dissolved in *N*,*N*-dimethylformamide (5 mL), added with MC-VC-PABC-MMAE (0.17 g, 0.13 mmol) and N,N-diisopropylethylamine (50.3 mg, 0.39 mmol), stirred at room temperature for 1 hour, added with ammonium chloride aqueous solution (10 mL), and extracted three times with dichloromethane (10 mL). The organic phase was dried and concentrated to obtain a crude product, which was then purified by productive thin layer silica gel plate (eluent: dichloromethane/methanol = 10/1 (v/v)) to obtain the title compound of this step (150 mg, yield: 47.0%).

MS m/z (ESI): 1241.7 [M/2+H]⁺.

### Intermediate Preparation Example 6: Preparation of Compound Int6

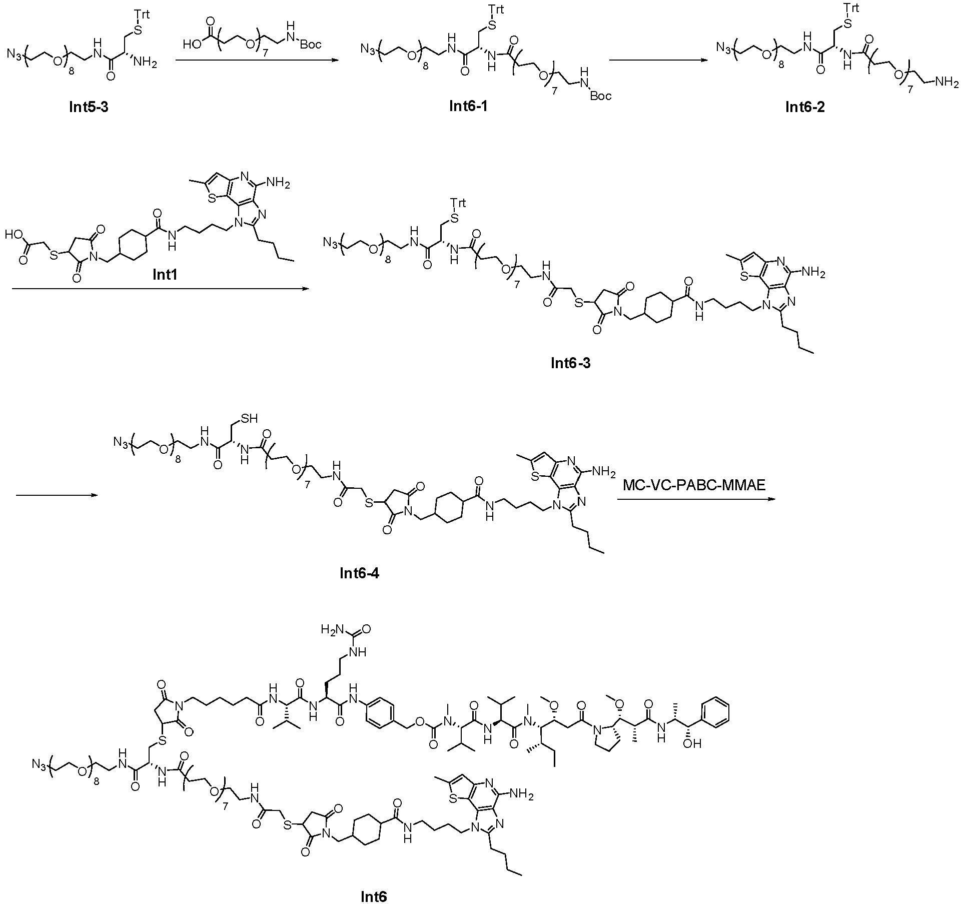

### Step 1: Preparation of Compound Int6-1

Compound **Int5-3** (192 mg, 0.244 mmol) and *N*-tert-butoxycarbonyl-heptaethylene glycol-carboxylic acid (110.53 mg, 0.222 mmol) were dissolved in *N*,*N*-dimethylformamide (3 mL), added with 1-hydroxybenzotriazole (45 mg, 0.333 mmol), *N*,*N*-diisopropylethylamine (85.9 mg, 0.666 mmol) and 1-(3-dimethylaminopropyl)-3-ethylcarbodiimide hydrochloride (63.8 mg, 0.333 mmol) under ice bath, and stirred at 20°C for 6 hours. The reaction solution was dropped into water (10 mL) and extracted three times with dichloromethane (10 mL). The organic phase was dried and concentrated to obtain a crude product, which was then purified by silica gel column chromatography (eluent: dichloromethane/methanol = 10/1 (v/v)) to obtain the title compound of this step (145 mg, yield: 51.6%).

MS m/z (ESI): 1263.6 [M+H]⁺.

### Step 2: Preparation of Compound Int6-2

Compound **Int6-1** (145 mg, 0.11 mmol) was dissolved in dichloromethane (2 mL), added dropwise with trifluoroacetic acid (1 mL), stirred at room temperature for 2 hours, concentrated, and diluted with dichloromethane (10 mL). The organic phase was washed with sodium bicarbonate solution, dried and concentrated to obtain the title compound of this step (130 mg, yield: 97%).

MS m/z (ESI): 1163.6 [M+H]⁺.

### Step 3: Preparation of Compound Int6-3

Compound **Int1** (65 mg, 0.10 mmol) and Compound **Int6-2** (130 mg, 0.11 mmol) were dissolved in *N*,*N*-dimethylformamide (5 mL), added with 1-hydroxybenzotrizoate (20.3 mg, 0.15 mmol), *N*,*N*-diisopropylethylamine (38.7 mg, 0.3 mmol) and 1-(3-dimethylaminopropyl)-3-ethylcarbodiimide hydrochloride (28.8 mg, 0.15 mmol) under ice bath, and stirred at 20°C for 3 hours. The reaction solution was dropped into water (10 mL) and extracted three times with dichloromethane (10 mL). The organic phase was dried and concentrated to obtain a crude product, which was then purified by silica gel column chromatography (eluent: dichloromethane/methanol=10/1 (v/v)) to obtain the title compound of this step (110 mg, yield: 60.8%).

MS m/z (ESI): 1787.8 [M+H]⁺.

### Step 4: Preparation of Compound Int6-4

Compound **Int6-3** (110 mg, 0.06 mmol) was dissolved in dichloromethane (2 mL) and triethylsilane (0.4 mL), added dropwise with trifluoroacetic acid (1 mL), stirred at room temperature for 1 hour, concentrated, and diluted with dichloromethane (20 mL). The organic phase was washed with sodium bicarbonate solution, dried and concentrated to obtain the title compound of this step (95 mg, yield: 100%).

MS m/z (ESI): 1545.7 [M+H]⁺.

### Step 5: Preparation of Compound Int6

Compound **Int6-4** (95 mg, 0.06 mmol) was dissolved in *N*,*N-*dimethylformamide (25 mL), added with MC-VC-PABC-MMAE (79 mg, 0.06 mmol) and *N*,*N*-diisopropylethylamine (23.2 mg, 0.18 mmol), stirred at room temperature for 1 hour, added with ammonium chloride aqueous solution (10 mL), and extracted three times with dichloromethane (10 mL). The organic phase was dried and concentrated to obtain a crude product, which was then purified by preparative thin-layer silica gel plate (eluent: dichloromethane/methanol = 8/1 (v/v)) to obtain the title compound of this step (73 mg, yield: 41.4%).

MS m/z (ESI): 1431.3 [M/2+H]⁺.

### Intermediate Preparation Example 7: Preparation of Compound Int7

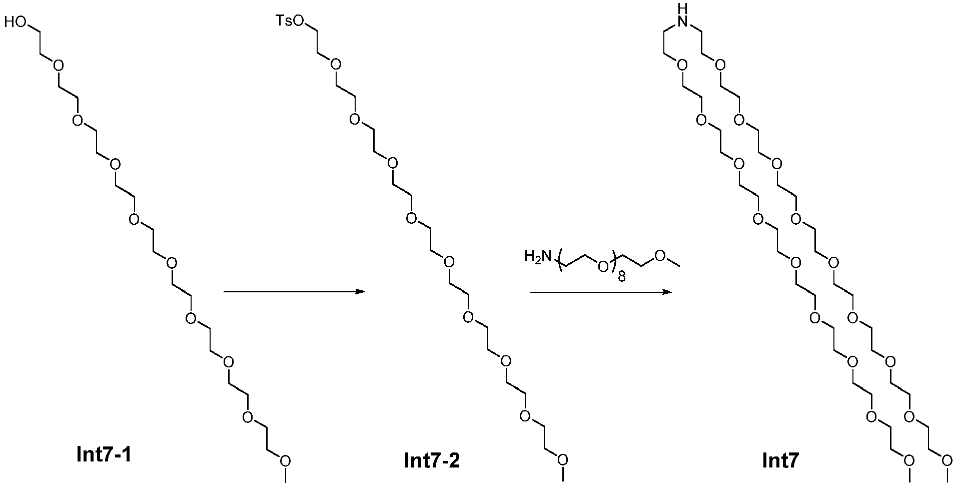

### Step 1: Preparation of Compound Int7-2

2,5,8,11,14,17,20,23,26-nonaoxaoctacosan-28-ol (2 g, 4.43 mmol) and *p*-toluenesulfonyl chloride (1.07 g, 5.32 mmol) were added to dichloromethane (10 mL), cooled to 0 to 10 °C, added with potassium hydroxide (1.26 g, 22.17 mmol), and reacted at 25°C for 12 hours. The reaction solution was concentrated, and the residue obtained was purified by silica gel column chromatography (eluent: dichloromethane/methanol = 20/1 (v/v)) to obtain the title compound of this step (2.5 g, yield: 91.9 %).

MS m/z (ESI): 583.5 [M+H]⁺.

### Step 2: Preparation of Compound Int7

Compound **Int7-2** (1.30 g, 2.12 mmol), 2,5,8,11,14,17,20,23,26- nonaoxaoctacosane-28-amine (1 g, 2.22 mmol), *N*,*N*-diisopropylethylamine (828.80 mg, 6.35 mmol, 1.06 mL) and potassium iodide (35.48 mg, 211.63 µmol) were added in sequence to *N*,*N*-dimethylformamide (15 mL), and reacted at 70°C for 24 hours. The reaction solution was concentrated, and the residue obtained was purified by silica gel column chromatography (eluent: dichloromethane/methanol = 19/1 (v/v)) to obtain the title compound of this step (1.5 g, yield: 71.9 %).

MS m/z (ESI): 838.3 [M+H]⁺.

### Intermediate Preparation Example 8: Preparation of Compound Int8

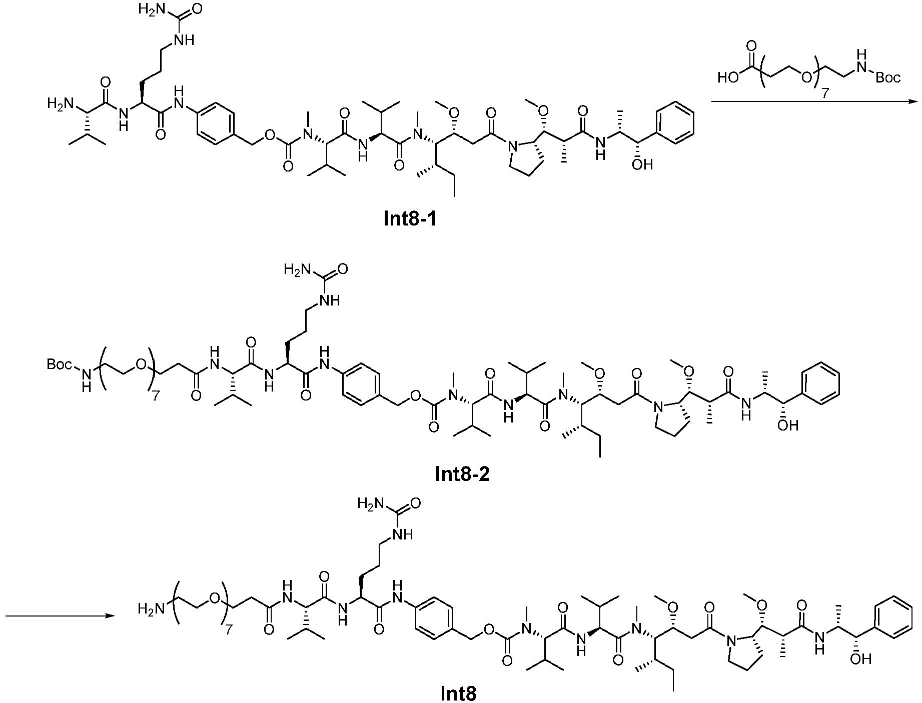

### Step 1: Preparation of Compound Int8-2

Compound **Int8-1** (155 mg, 131.07 µmol) and N-tert-butoxycarbonyl-hepta(ethylene glycol)-carboxylic acid (68.65 mg, 131.07 µmol) were dissolved in anhydrous *N*,*N*-dimethylformamide (4 mL), added with N,N-diisopropylethylamine (42.78 mg, 327.68 µmol) and 2-(7-azobenzotriazole)-*N*,*N*,*N*',*N*'-tetramethylurea hexafluorophosphate (75.53 mg, 196.61 µmol), and reacted at 25°C for 5 hours. The reaction solution was concentrated, and the residue obtained was purified by silica gel column chromatography (eluent: dichloromethane/methanol = 20/1 (v/v)) to obtain the title compound of this step (193 mg, yield: 87.3%).

MS m/z (ESI): 1603.0 [M+H]⁺.

### Step 2: Preparation of Compound Int8

Under nitrogen protection, Compound **Int8-2** (165 mg, 97.79 µmol) and zinc bromide (139.08 mg, 586.72 µmol) were added to anhydrous dichloromethane (2 mL) in sequence, and reacted at 25°C for 2 hours. The reaction solution was concentrated to obtain the crude product of the title compound in this step, which was used directly in the next step.

MS m/z (ESI): 1502.9 [M+H]⁺.

### Intermediate Preparation Example 9: Preparation of Compound Int9

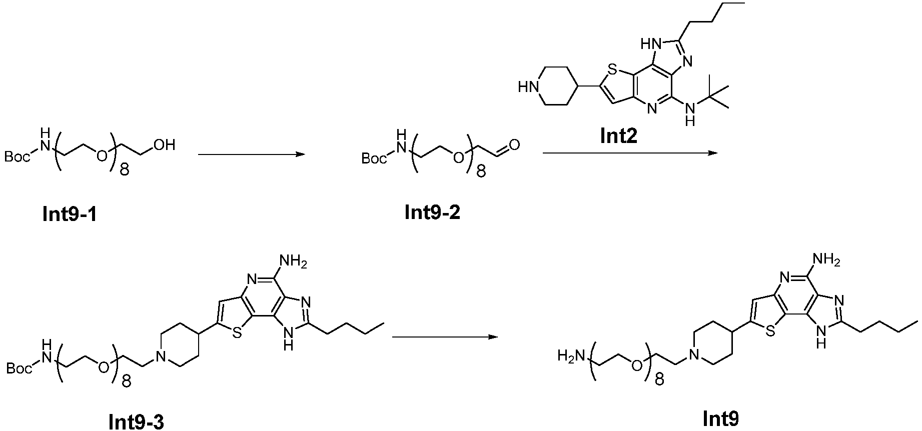

### Step 1: Preparation of Compound Int9-2

Dess-Martin oxidant (1.23 g, 29.3 mmol) was added to a solution of Compound **Int9-1** (1 g, 19.5 mmol) in dichloromethane (20 mL), and stirred at room temperature overnight. The reaction solution was diluted with dichloromethane, added with saturated sodium thiosulfate solution (10 mL) and sodium bicarbonate solution (10 mL) in sequence, stirred for 20 minutes, subjected to liquid separation, extracted twice with dichloromethane. The organic phases were combined and washed with brine. The crude product obtained by concentrating the combined organic phase was purified by silica gel column chromatography (eluent: dichloromethane/methanol = 12/1 (v/v)) to obtain the title compound of this step (0.9 g, yield: 90.9 %).

MS m/z (ESI): 512.3 [M+H]⁺.

### Step 2: Preparation of Compound Int9-3

To a solution of Compound **Int2** (0.5 g, 1.29 mmol) in dichloroethane (10 mL), Compound **Int9-2** (0.86 g, 1.68 mmol), glacial acetic acid (0.16 g, 2.60 mmol) and sodium cyanoborohydride (0.42 g, 6.45 mmol) were added in sequence, and reacted at room temperature for 12 hours. The reaction solution was poured into water and extracted with dichloromethane. The organic phases were combined and washed with brine. The crude product obtained by concentrating the combined organic phase was purified by silica gel column chromatography (eluent: dichloromethane/methanol=10/1 (v/ v)) to obtain the title compound of this step (0.6 g, yield: 52.6%).

MS m/z (ESI): 825.5 [M+H]⁺.

### Step 3: Preparation of Compound Int9

To a solution of Compound **Int9-3** (0.6 g, 0.68 mmol) in 1,4-dioxane (4 mL), a solution of hydrogen chloride in 1,4-dioxane (1.7 mL, 4 M, 6.8 mmol) was added, and reacted at 70°C for 12 hours. The crude product obtained by concentrating the reaction solution was purified by reverse-phase column chromatography (preparation method A, elution gradient: A%=15% to 25%) to obtain the title compound of this step (0.3 g, yield: 61.2%).

MS m/z (ESI): 725.4 [M+H]⁺.

### Intermediate Preparation Example 10: Preparation of Compound Int10

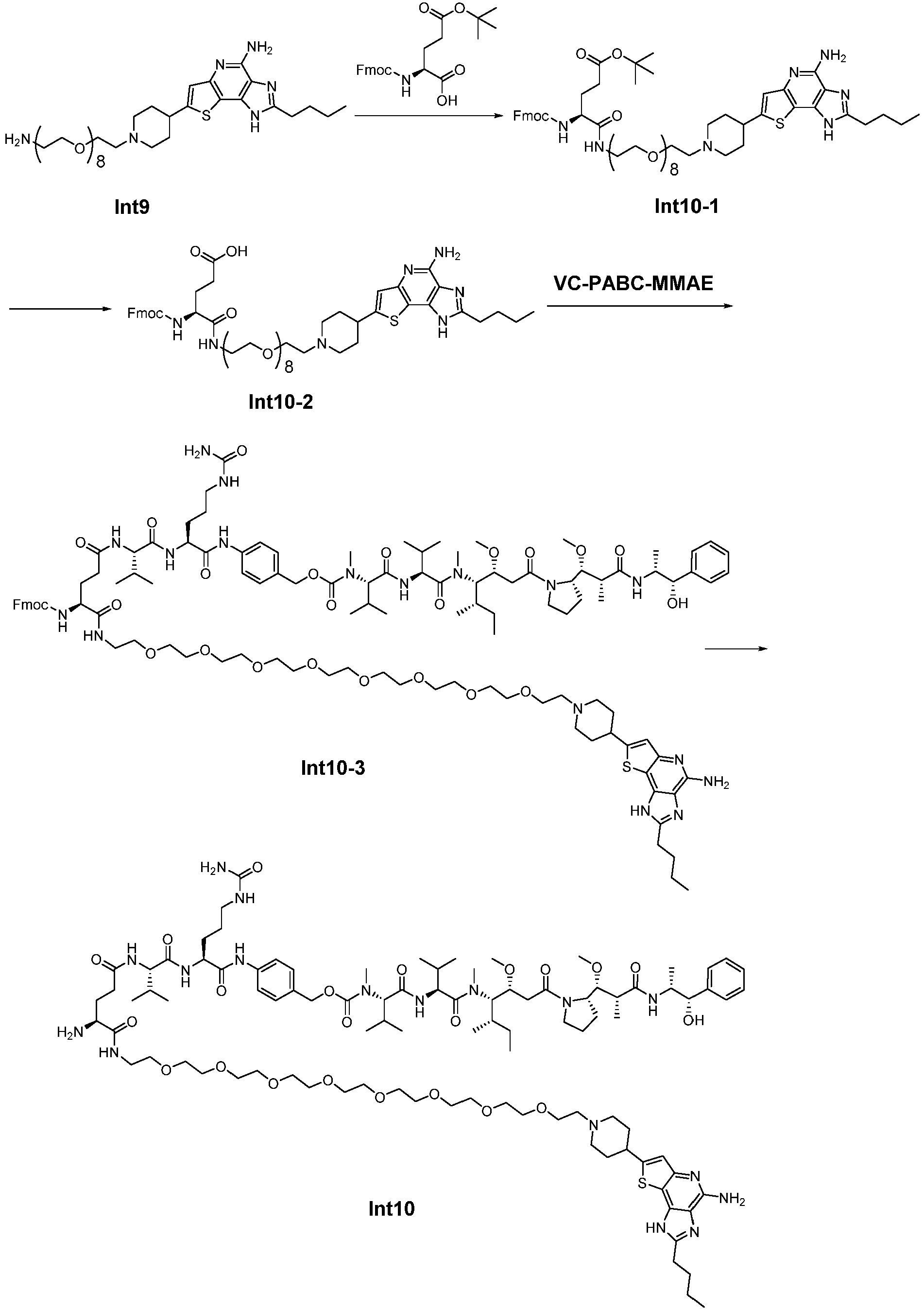

### Step 1: Preparation of Compound Int10-1

Compound **Int9** (186 mg, 243.74 µmol), (*S*)-2-((((9*H*-fluoren-9-yl)methoxy)carbonyl)amino)-5-(tert-butoxy)-5-oxopentanoic acid (131 mg, 292.49 µmol), *N*,*N*-diisopropylethylamine (66.32 mg, 487.48 µmol) and 2-(7-azobenzotriazole)-*N*,*N*,*N*',*N*'-tetramethylurea hexafluorophosphate (136.58 mg, 341.24 µmol) were added to anhydrous *N*,*N*-dimethylformamide (3 mL) in sequence, and reacted at 0°C for 1 hour. The reaction solution was directly purified by reversed-phase column chromatography (preparation method A, elution gradient: A%=25% to 35%) to obtain the title compound of this step (270 mg, yield: 92.9%).

MS m/z (ESI): 1132.6 [M+H]⁺.

### Step 2: Preparation of Compound Int10-2

Compound **Int10-1** (340 mg, 285.23 µmol) and trifluoroacetic acid (2 mL) were added to dichloromethane (6 mL) and reacted at 25°C for 2 hours. The reaction solution was concentrated. The residue obtained was purified by silica gel column chromatography (eluent: dichloromethane/methanol = 4/1 (v/v)) to obtain the title compound of this step (250 mg, yield: 77.4%).

MS m/z (ESI): 1076.6 [M+H]⁺.

### Step 3: Preparation of Compound Int10-3

Compound **Int10-2** (150 mg, 132.40 µmol), VC-PABC-MMAE (156.57 mg, 132.40 µmol), *N*,*N*-diisopropylethylamine (25.93 mg, 198.60 µmol) and 2-(7-azobenzotriazole)*-N,N,N',N'-*tetramethylurea hexafluorophosphate (61.02 mg, 158.88 µmol) were added to anhydrous *N*,*N*-dimethylformamide (5 mL) in sequence, and reacted at 0°C for 1 hour. The reaction solution was directly purified by reversed-phase column chromatography (preparation method B, elution gradient: A%=35% to 45%) to obtain the title compound of this step (250 mg, yield: 82.2%).

MS m/z (ESI): 1091.1 [M/2+H]⁺.

### Step 4: Preparation of Compound Int10

Compound **Int10-3** (250 mg, 8.86 µmol) and piperidine (37.45 mg, 435.44 µmol) were added to *N*,*N*-dimethylformamide (4 mL), and reacted at 25°C for 2 hours. The reaction solution was directly purified by reversed-phase column chromatography (preparation method A, elution gradient: A%=20% to 30%) to obtain the title compound of this step (185 mg, yield: 82.4%).

MS m/z (ESI): 980.2 [M/2+H]⁺.

### Intermediate Preparation Example 11: Preparation of Compound Int11

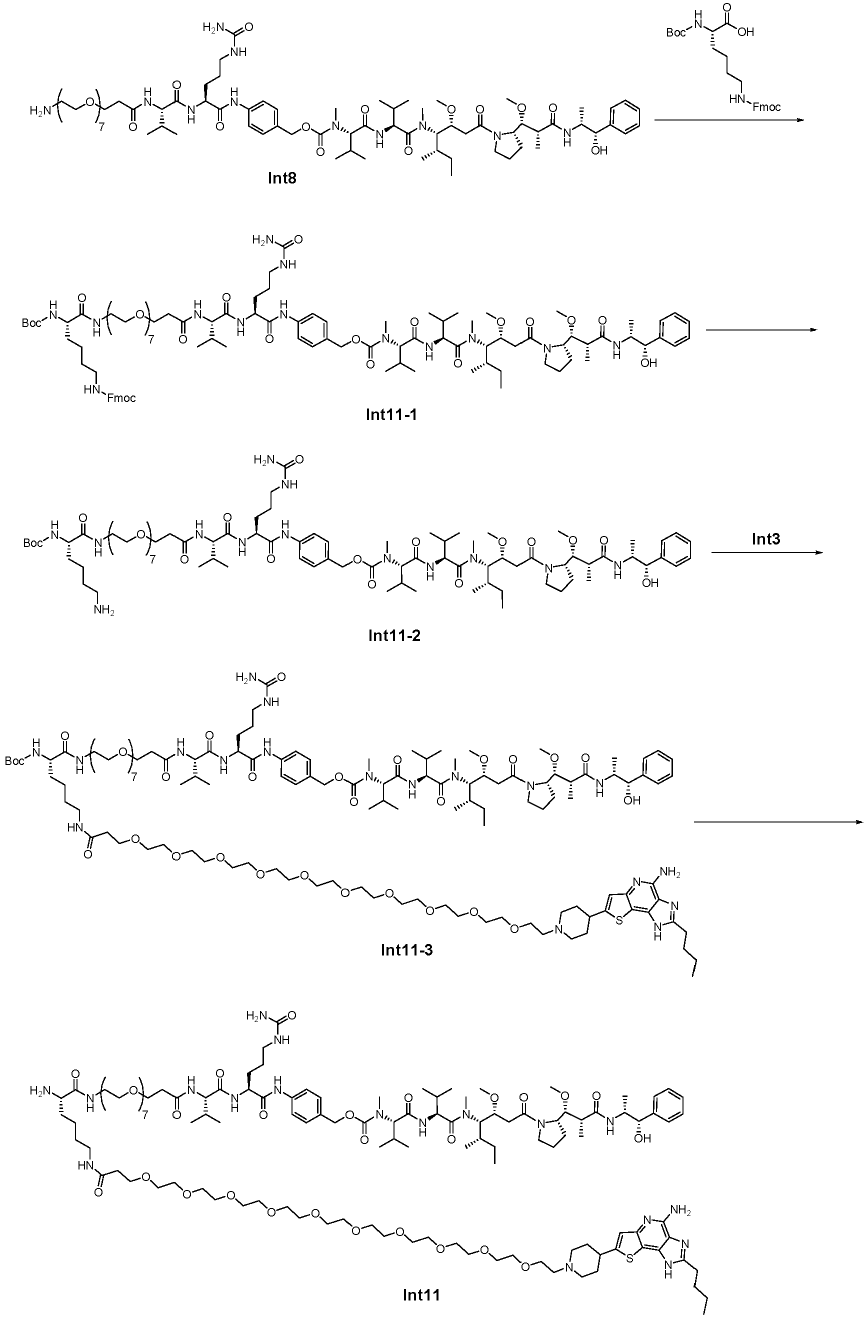

### Step 1: Preparation of Compound Int11-1

Compound **Int8** (114 mg, 72.06 µmol) and *N*⁶-(((9*H*-fluoren-9-yl)methoxy)carbonyl)-*N*²-(tert-butoxycarbonyl)-*L*-lysine (37.43 mg, 72.06 µmol) were dissolved in anhydrous *N*,*N*-dimethylformamide (2 mL), to which *N*,*N*-diisopropylethylamine (23.52 mg, 180.16 µmol) and 2-(7-azobenzotriazole)-*N*,*N*,*N*',*N*'-tetramethylurea hexafluorophosphate (41.53 mg, 108.09 µmol) were added, and the reaction was carried out at 25°C for 5 hours. The reaction solution was concentrated. The residue obtained was purified by silica gel column chromatography (eluent: dichloromethane/methanol = 50/1 (v/v)) to obtain the title compound of this step (110 mg, yield: 70.4%).

MS m/z (ESI): 1953.1 [M+H]⁺.

### Step 2: Preparation of Compound Int11-2

Compound **Int11-1** (100 mg, 46.07 µmol) was dissolved in dichloromethane (3 mL), added with diethylamine (67.40 mg, 921.47 µmol), and reacted at 20°C for 10 hours. The reaction solution was concentrated. The residue obtained was purified by silica gel column chromatography (eluent: dichloromethane/methanol = 20/1 (v/v)) to obtain the title compound of this step (40 mg, yield: 42.8%).

MS m/z (ESI): 1731.1 [M+H]⁺.

### Step 3: Preparation of Compound Int11-3

Compound **Int11-2** (100 mg, 54.88 µmol) and Compound **Int3** (46.64 mg, 54.88 µmol) were added to anhydrous *N*,*N*-dimethylformamide (2 mL), added with *N*,*N-*diisopropylethylamine (10.75 mg, 82.31 µmol) and 2-(7-azobenzotriazole)-N,N,N',N'-tetramethylurea hexafluorophosphate (25.29 mg, 65.85 µmol), and reacted at 25°C for 5 hours. The reaction solution was purified by preparative high-performance liquid chromatography to obtain the title compound of this step (60 mg, yield: 40.7%).

MS m/z (ESI): 1277.8 [M/2+H]⁺.

### Step 4: Preparation of Compound Int11

Under nitrogen protection, Compound **Int11**-3 (45 mg, 7.04 µmol) and zinc bromide (6.68 mg, 28.18 µmol) were added to anhydrous dichloromethane (2 mL) in sequence and reacted at 25°C for 2 hours. The reaction solution was concentrated to obtain the crude product of the title compound in this step, which was used directly in the next step.

MS m/z (ESI): 1227.8 [M/2+H]⁺.

### Intermediate Preparation Example 12: Preparation of Compound Int12

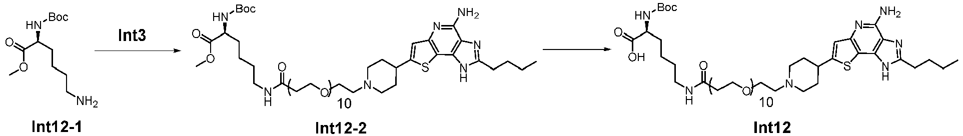

### Step 1: Preparation of Compound Int12-2

Compound **Int3** (150 mg, 169.23 µmol) and *N*-tert-butoxycarbonyllysine methyl ester hydrochloride (46.37 mg, 169.23 µmol) were dissolved in anhydrous N,N-dimethylformamide (4 mL), then added with *N*,*N*-diisopropylethylamine (44.18 mg, 338.46 µmol) and 2-(7-azobenzotriazole)-*N*,*N*,*N*',*N*'-tetramethylurea hexafluorophosphate (78.01 mg, 203.08 µmol) in sequence, and reacted at 25°C for 2 hours. The reaction solution was concentrated under reduced pressure. The obtained residue was purified by silica gel column chromatography (eluent: dichloromethane/methanol = 6/1 (v/v)) to obtain the title compound of this step (160 mg, yield: 95.8%).

MS m/z (ESI): 1084.6 [M+H]⁺.

### Step 2: Preparation of Compound Int12

Compound **Int12-2** (185 mg, 162.08 µmol) was dissolved in a mixed solvent of tetrahydrofuran (1 mL), methanol (1 mL) and water (1 mL), added with lithium hydroxide monohydrate (28.63 mg, 648.31 µmol), and reacted at 25°C for 3 hours. The reaction solution was concentrated, added with *N*,*N*-dimethylformamide (1 mL). The obtained solution was purified by reverse-phase column chromatography (preparation method A, elution gradient: A%=25% to 35%) to obtain the title compound of this step (160 mg, yield: 87.6%).

MS m/z (ESI): 1070.6 [M+H]⁺.

### Intermediate Preparation Example 13: Preparation of Compound Int13

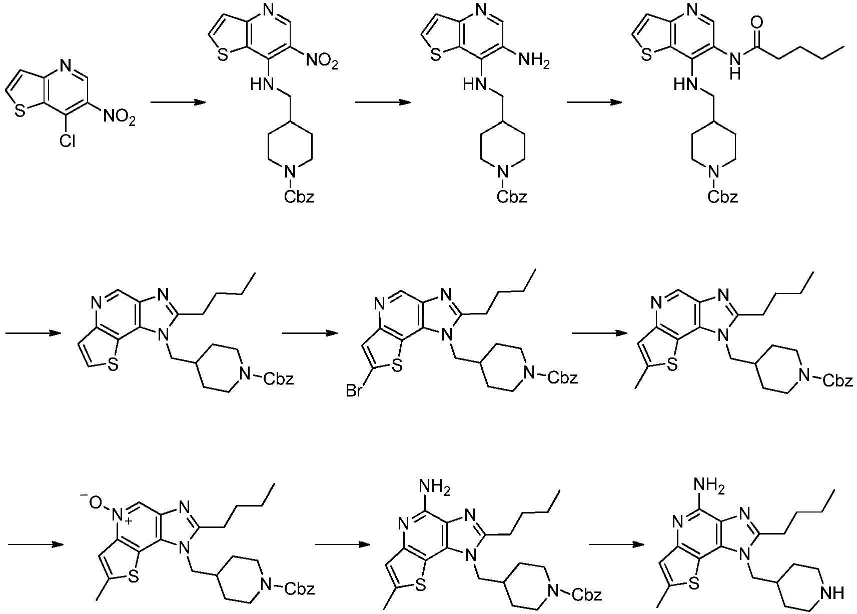

### Step 1: Preparation of benzyl 4-(((6-nitrothieno[3,2-b]pyridin-7-yl)amino)methyl)piperidine-1-carboxylate

7-Chloro-6-nitrothieno[3,2-*b*]pyridine (1.0 g, 4.67 mmol) and benzyl 4-(aminomethyl)tetrahydro-1(2H)-pyridine-carboxylate (1.38 g, 5.60 mmol) were dissolved in *N*,*N*-dimethylformamide (10 mL), cooled in an ice-water bath, added with *N*,*N*-diisopropylethylamine (1.08 g, 8.4 mmol) to the reaction solution at 0°C, slowly heated to room temperature and stirred for 6 hours. The reaction solution was poured into water (100 mL) and extracted three times with ethyl acetate (50 mL). The organic phases were combined, washed three times with saturated brine (50 mL), dried over anhydrous sodium sulfate, and concentrated to obtain the title compound of this step (1.91 g, yield: 96.3%).

MS m/z (ESI): 427.0 [M+H]⁺.

### Step 2: Preparation of benzyl 4-(((6-aminothieno[3,2-b]pyridin-7-yl)amino)methyl) piperidine-1-carboxylate

Benzyl 4-(((6-nitrothieno[3,2-*b*]pyridin-7-yl)amino)methyl)piperidine-1-carboxylate (1.91 g, 4.47 mmol) was completely dissolved in anhydrous ethanol (30 mL), and then added with water (5 mL), cooled down in an ice-water bath, added with sodium hydrosulfite (0.8 g, 45 mmol) to the reaction solution at 0°C, slowly heated to room temperature and stirred for 4 hours. The reaction solution was poured into water (100 mL), stirred to precipitate a solid, and filtered. The solid was washed with water, and dried to obtain the title compound of this step (1.5 g, yield: 84.2%).

MS m/z (ESI): 397.1 [M+H]⁺.

### Step 3: Preparation of benzyl 4-(((6-pentanamidothieno[3,2-b]pyridin-7-yl)amino)methyl) piperidine-1-carboxylate

Benzyl 4-(((6-aminothieno[3,2-b]pyridin-7-yl)amino)methyl)piperidine-1-carboxylate (1.5 g, 3.78 mmol) and *N*,*N*-diisopropylethylamine (0.97 g, 7.57 mmol) were dissolved in tetrahydrofuran (20 mL), cooled in an ice-water bath, slowly added dropwise with *n*-pentanoyl chloride (0.68 g, 5.67 mmol) to the reaction solution at 0°C, and slowly heated to room temperature and stirred for 4 hours. Water (10 mL) was added to the reaction system to quench the reaction. The reaction system was concentrated to remove tetrahydrofuran as much as possible. The obtained residue was added with water (50 mL) and extracted three times with ethyl acetate (30 mL). The organic phases were combined, washed three times with saturated brine (50 mL), dried over anhydrous sodium sulfate, and concentrated to obtain the title compound of this step (1.63 g, yield: 90%).

MS m/z (ESI): 481.1 [M+H]⁺.

### Step 4: Preparation of benzyl 4-((2-butyl-1H-imidazo[4,5-d]thieno[3,2-b]pyridin-1-yl) methyl)piperidine-1-carboxylate

Benzyl 4-(((6-pentanamidothieno[3,2-*b*]pyridin-7-yl)amino)methyl)piperidine-1-carboxylate (1.63 g, 3.38 mmol) and *o*-chlorobenzoic acid (260 mg, 1.7 mmol) were added to toluene (50 mL), heated to reflux, and reacted under stirring and separating water for 4 hours. After the reaction system was cooled to room temperature, the solvent was removed by concentration. The residue obtained was purified by silica gel column chromatography (eluent: 100% ethyl acetate) to obtain the title compound of this step (1.39 g, yield: 89.3%).

MS m/z (ESI): 463.1 [M+H]⁺.

### Step 5: Preparation of benzyl 4-((7-bromo-2-butyl-1H-imidazo[4,5-d]thieno[3,2-b] pyridin-1-yl)methyl)piperidine-1-carboxylate

Benzyl 4-((2-butyl-1H-imidazo[4,5-d]thieno[3,2-b]pyridin-1-yl)methyl)piperidine-1- carboxylate (1.1 g, 2.37 mmol) was completely dissolved in a mixed solvent of *N*,*N*-dimethylformamide (9 mL) and glacial acetic acid (3 mL), cooled in an ice-water bath, slowly added with *N*-bromosuccinimide (0.63 g, 3.55 mmol) in batches to the reaction solution at 0°C, slowly warmed to room temperature and stirred for 24 hours. The reaction solution was poured into water (100 mL) and extracted three times with ethyl acetate (30 mL). The organic phases were combined, washed three times with saturated sodium bicarbonate aqueous solution (50 mL), washed three times with saturated brine (50 mL), dried over anhydrous sodium sulfate, and concentrated. The residue was purified by silica gel column chromatography (eluent: ethyl acetate/petroleum ether = 1/1 (v/v)) to obtain the title compound of this step (0.87 g, yield: 68.5%).

MS m/z (ESI): 541.1 [M+H]⁺.

### Step 6: Preparation of benzyl 4-((2-butyl-7-methyl-1H-imidazo[4,5-d]thieno[3,2-b] pyridin-1-yl)methyl)piperidine-1-carboxylate

Benzyl 4-((7-bromo-2-butyl-1*H*-imidazo[4,5-*d*]thieno[3,2-*b*]pyridin-1-yl)methyl)piperidine-1-carboxylate (0.87 g, 1.61 mmol), trimethylboroxine (50 wt.% solution in tetrahydrofuran, 2.0 g, 8.05 mmol), potassium carbonate (0.67 g, 4.83 mmol), and 1,1'-bis(diphenylphosphine) ferrocene palladium dichloride (0.23 g, 0.32 mmol) were added in sequence to a mixed solvent of 1,4-dioxane (10 mL) and water (1 mL), the reaction system was bubbled with nitrogen for 5 minutes, heated to 100°C under microwave and stirred for 2 hours. After the reaction system was cooled to room temperature, the solvent was removed by concentration. The residue was purified by silica gel column chromatography (eluent: 100% ethyl acetate) to obtain the title compound of this step (0.54 g, yield: 71.0%).

MS m/z (ESI): 477.1 [M+H]⁺.

### Step 7: Preparation of 1-((1-((benzyloxy)carbonyl)piperidin)-4-yl)methyl)-2-butyl-7-methyl-1H-imidazo[4,5-d]thieno[3,2-b]pyridine-5-oxide

Benzyl 4-((2-butyl-7-methyl-1*H*-imidazo[4,5-d]thieno[3,2-*b*]pyridin-1-yl)methyl)piperidine-1-carboxylate (0.5 g, 1.04 mmol) was dissolved in dichloromethane (10 mL), cooled in an ice-water bath, and slowly added with m-chloroperoxybenzoic acid (0.42 g, 2.05 mmol) to the reaction solution at 0°C, slowly warmed to room temperature and stirred for 4 hours. The reaction solution was poured into water (100 mL) and extracted three times with dichloromethane (20 mL). The organic phases were combined, washed twice with saturated sodium bicarbonate aqueous solution (30 mL), washed twice with saturated sodium sulfite aqueous solution (30 mL), and washed three times with saturated brine (30 mL), dried over anhydrous sodium sulfate, and concentrated to obtain the title compound of this step (0.52g, yield: 100%).

MS m/z (ESI): 493.2 [M+H]⁺.

### Step 8: Preparation of benzyl 4-((4-amino-2-butyl-7-methyl-1H-imidazo[4,5-d]thieno[3,2-b] pyridin-1-yl)methyl)piperidine-1-carboxylate

1-((1-((Benzyloxy)carbonyl)piperidin)-4-yl)methyl)-2-butyl-7-methyl-1*H*-imidazo[4,5-*d*]thieno[3,2-b]pyridine-5-oxide (0.52 g, 1.04 mmol) was dissolved in a mixed solvent of dichloromethane (10 mL) and ammonia water (5 mL), and cooled in an ice-water bath, *p*-toluenesulfonyl chloride (0.4 g, 2.09 mmol) was slowly added in batches to the reaction solution at 0°C, slowly warmed to room temperature and stirred for 12 hours. The reaction system was added with water (100 mL) and extracted three times with dichloromethane (20 mL). The organic phases were combined, washed three times with saturated brine (30 mL), dried over anhydrous sodium sulfate, and concentrated. The residue was purified by silica gel column chromatography (eluent: ethyl acetate/methanol = 20/1 (v/v)) to obtain the title compound of this step (230 mg, yield: 45%).

MS m/z (ESI): 492.2 [M+H]⁺.

### Step 9: Preparation of 2-butyl-7-methyl-1-(piperidin-4-ylmethyl)-1H-imidazo[4,5-d]thieno [3,2-b]pyridine-4-amine (Int13)

Benzyl 4-((4-amino-2-butyl-7-methyl-1*H*-imidazo[4,5-*d*]thieno[3,2-*b*]pyridin-1-yl)methyl) piperidine-1-carboxylate (150 mg, 0.31 mmol) was added to trifluoroacetic acid (6 mL), heated to 50°C and stirred for 3 hours. The solvent was removed by concentration. The residue was added with a mixed solvent of dichloromethane and methanol (volume ratio: 4:1, 10 mL) and saturated sodium bicarbonate aqueous solution (10 mL) in sequence, stirred at room temperature for 5 minutes, then concentrated to remove the solvent. The obtained residue was purified by preparative high performance liquid chromatography to obtain the title compound of this step (22 mg, yield: 19.9%).
MS m/z (ESI): 358.1 [M+H]⁺.
¹H NMR (400 MHz, DMSO-*d₆*): δ 8.24 (s, 1H), 6.99-6.98 (m, 1H), 6.11 (br, 2H), 4.14 (d, *J =* 8.0 Hz, 2H), 3.26-3.23 (m, 2H), 2.88-2.76 (m, 4H), 2.57-2.55 (m, 3H), 2.19-2.12(m, 1H), 1.87-1.76 (m, 2H), 1.68-1.62(m, 2H), 1.56-1.39 (m, 4H), 0.96 (t, *J =* 8.0 Hz, 3H).

### Intermediate Preparation Example 14: Preparation of Compound Int14

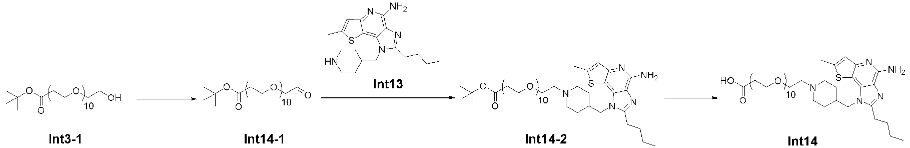

### Step 1: Preparation of Compound Int14-1

Compound **Int3-1** (1 g, 1.7 mmol) and Dess-Martin oxidant (1.08 g, 2.56 mmol) were added to dichloromethane (15 mL) and reacted at 25°C for 10 hours. Saturated sodium thiosulfate solution (5 mL) and saturated sodium bicarbonate aqueous solution (10 mL) were added to the reaction solution, stirred until clear, then extracted three times with dichloromethane, washed with saturated brine, dried over anhydrous sodium sulfate, and filtered. The filtrate was concentrated. The residue obtained was purified by silica gel column chromatography (eluent: dichloromethane/methanol = 10/1 (v/v)) to obtain the title compound of this step (900 mg, yield: 91.3%).

MS m/z (ESI): 585.5 [M+H]⁺.

### Step 2: Preparation of Compound Int14-2

Compound **Int14-1** (472.48 mg, 799.99 µmol), Compound **Int13** (191 mg, 533.33 µmol), sodium cyanoborohydride (99.08 mg, 1576.7 µmol) and glacial acetic acid (51.72 mg, 853.33 µmol) were added in sequence to a mixed solvent of 1,2-dichloroethane (6 mL) and methanol (3 mL) and reacted at 25°C for 4 hours. The crude product obtained by concentrating the reaction solution was dissolved in methanol (3 mL) and purified by reverse-phase column chromatography (preparation method B, elution gradient: A%=15% to 25%) to obtain the title compound of this step (250 mg, yield: 50.6%).

MS m/z (ESI): 926.5 [M+H]⁺.

### Step 3: Preparation of Compound Int14

Compound **Int14-2** (250 mg, 269.83 µmol) was dissolved in dioxane hydrochloride solution (6 mL, 4M) and reacted at 25°C for 2 hours. The reaction solution was concentrated to obtain the title compound of this step (203 mg, yield: 86.5%).

MS m/z (ESI): 870.4 [M+H]⁺.

### Intermediate Preparation Example 15: Preparation of Compound Int15

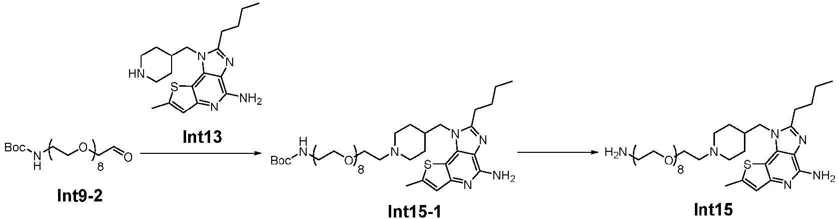

### Step 1: Preparation of Compound Int15-1

Compound **Int9-2** (390.27 mg, 794.96 µmol), Compound **Int13** (200 mg, 529.98 µmol), sodium cyanoborohydride (159.79 mg, 2.52 mmol) and glacial acetic acid (7.88 mg, 1.51 mmol) were added sequentially to a mixed solvent of 1,2-dichloroethane (6 mL) and methanol (3 mL) and reacted at 25°C for 4 hours. The crude product obtained by concentrating the reaction solution was dissolved in methanol (3 mL) and purified by reverse-phase column chromatography (preparation method B, elution gradient: A%=25% to 35%) to obtain the title compound of this step (330 mg, yield: 76.8%).

MS m/z (ESI): 853.5 [M+H]⁺.

### Step 2: Preparation of Compound Int15

Compound **Int15-1** (330 mg, 386.82 µmol) was dissolved in dioxane hydrochloride solution (6 mL, 4M) and reacted at 25°C for 5 hours. The reaction solution was concentrated to obtain the title compound of this step (250 mg, yield: 86.2%).

MS m/z (ESI): 753.4 [M+H]⁺.

### Intermediate Preparation Example 16: Preparation of Compound Int16

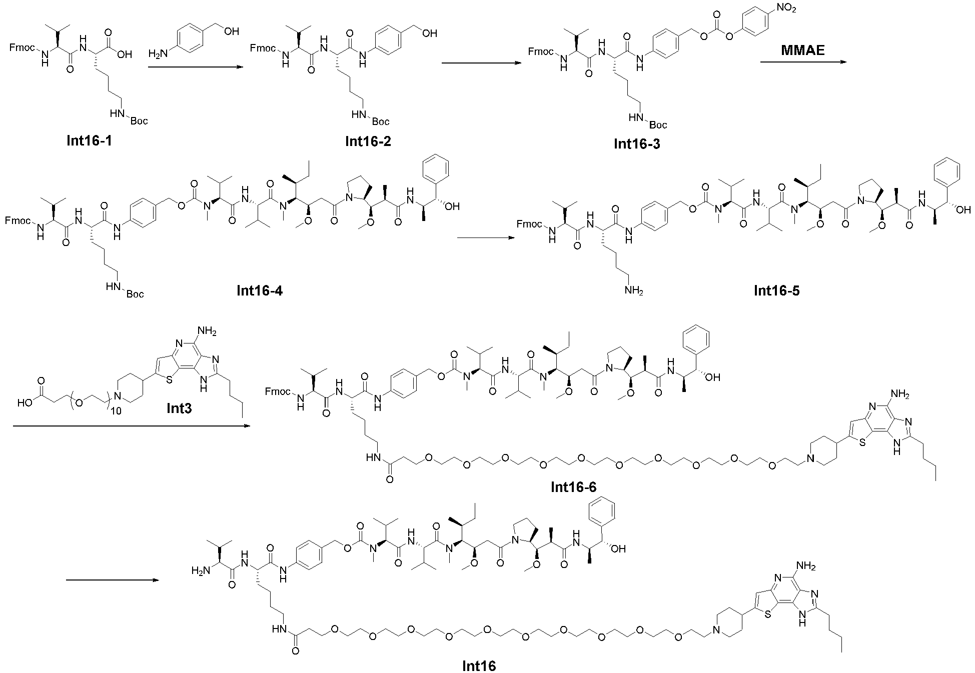

### Step 1: Preparation of Compound Int16-2

Compound **Int16-1** (270 mg, 428.06 µmol), p-aminobenzyl alcohol (106.50 mg, 856.13 µmol) and 2-ethoxy-1-ethoxycarbonyl-1,2-dihydroquinoline (213.60 mg, 856.13 µmol) were added to anhydrous methanol (1 mL) and anhydrous dichloromethane (3 mL) and stirred at room temperature overnight. The residue obtained by concentrating the reaction solution was purified by reverse-phase column chromatography (preparation method B, elution gradient: A%=45% to 55%) to obtain the title compound of this step (130 mg, yield: 40.62%).

MS m/z (ESI): 673.3 [M+H]⁺.

### Step 2: Preparation of Compound Int16-3

Compound **Int16-2** (125 mg, 167.21 µmol), bis(*p*-nitrophenyl) carbonate (77.07 mg, 250.81 µmol) and *N*,*N*-diisopropylethylamine (54.47 mg, 418.02 µmol) were added to N,N-dimethylformamide (2 mL) in sequence, and stirred at room temperature for 5 hours. The reaction solution was directly purified by reversed-phase column chromatography (preparation method B, elution gradient: A%=65%-75%) to obtain the title compound of this step (95 mg, yield: 64.42%).

¹H-NMR (400MHz, DMSO-*d₆*): δ 10.14 (br, 1H), 8.31 (d, *J* = 8Hz, 2H), 8.12 (d, *J* = 8Hz, 1H), 7.90 (d, *J = 4Hz,* 2H), 7.76-7.72 (m, 2H), 7.65 (d, *J =* 8Hz, 2H), 7.58 (d, *J* = 8Hz, 2H), 7.48-7.39 (m, 5H), 7.32 (t,*J* = 8Hz, 2H), 6.76 (br, 1H), 5.24 (s, 2H), 4.38-4.22 (m, 4H), 3.92 (t, *J =* 8Hz, 1H), 2.88 (d, *J =* 4Hz, 2H), 2.01- 1.96 (m, 1H), 1.68-1.59 (m, 2H), 1.40-1.24 (m, 15H), 0.89-0.85 (m, 6H)

MS m/z (ESI): 838.3 [M+H]⁺.

### Step 3: Preparation of Compound Int16-4

Monomethyl auristatin E (77 mg, 101.88 µmol), Compound **Int16-3** (94.85 mg, 101.88 µmol), 1-hydroxybenzotriazole (2.09 mg, 15.28 µmol) and *N*,*N*-diisopropylethylamine (26.55 mg, 203.77 µmol) were added to *N*,*N*-dimethylformamide (1 mL), and stirred at 35°C for 5 hours. The reaction solution was directly purified by reversed-phase column chromatography (preparation method B, elution gradient: A%=50% to 60%) to obtain the title compound of this step (65 mg, yield: 42.78%).

MS m/z (ESI): 1416.8 [M+H]⁺.

### Step 4: Preparation of Compound Int16-5

Compound **Int16-4** (30 mg, 19.06 µmol) and anhydrous zinc bromide (21.68 mg, 95.29 µmol) were added to anhydrous dichloromethane (2 mL) and stirred at 35°C for 2 hours. The reaction solution was diluted with ethyl acetate (10 mL) and washed with saturated brine (3 mL*2), the organic phase was dried over anhydrous sodium sulfate and filtered, and the filtrate was concentrated to obtain the title compound of this step (31 mg, yield: 98.84 %).

MS m/z (ESI): 1316.8 [M+H]⁺.

### Step 5: Preparation of Compound Int16-6

Compound **Int3** (20 mg, 21.38 µmol), Compound **Int16-5** (31.0 mg, 21.38 µmol), *N*,*N*-diisopropylethylamine (5.57 mg, 42.75 µmol) and 2-(7-azobenzotriazole)-*N*,*N*,*N*',*N*'-tetramethylurea hexafluorophosphate (9.85 mg, 25.65 µmol) were added to *N*,*N*-dimethylformamide (1 mL), and stirred at room temperature for 1 hour. The reaction solution was directly purified by reversed-phase column chromatography (preparation method B, elution gradient: A%=40%-50%) to obtain the title compound of this step (29 mg, yield: 57.04%).

MS m/z (ESI): 1070.6 [M/2+H]⁺.

### Step 6: Preparation of Compound Int16

Compound **Int16-6** (29 mg, 12.19 µmol) and piperidine (2.10 mg, 24.38 µmol) were added to *N*,*N*-dimethylformamide (1 mL), and stirred at room temperature for 2 hours. After the reaction solution was concentrated, methyl *tert*-butyl ether (5 mL) was added, treated with ultrasonic for 5 minutes, then allowed to stand, filtered, and the solid was dried to obtain the title compound of this step (22 mg, yield: 94.06%).

MS m/z (ESI): 959.6 [M/2+H]⁺.

### Intermediate Preparation Example 17: Preparation of Compound Int17

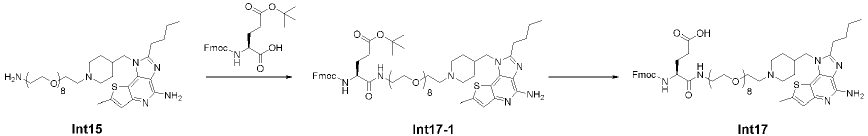

### Step 1: Preparation of Compound Int17-1

(*S*)-2-((((9*H*-Fluoren-9-yl)methoxy)carbonyl)amino)-5-(tert-butoxy)-5-oxopentanoic acid (85.29 mg, 200.46 µmol), *N*,*N*-diisopropylethylamine (45.2 mg, 346.26 µmol), 2-(7-azobenzotriazole)-*N*,*N*,*N*',*N*'-tetramethylurea hexafluorophosphate (103.94 mg, 270.22 µmol) and Compound **Int15** (150 mg, 182.24 µmol) were added to anhydrous *N*,*N*-dimethylformamide (4 mL) in sequence, and reacted at 0°C for 2 hours. The reaction solution was directly purified by reversed-phase column chromatography (preparation method B, elution gradient: A%=25% to 35%) to obtain the title compound of this step (170 mg, yield: 79.8%).

MS m/z (ESI): 1160.6 [M+H]⁺.

### Step 2: Preparation of Compound Int17

Compound **Int17-1** (170 mg, 138.15 µmol) was dissolved in a mixed solvent of anhydrous dichloromethane (6 mL) and trifluoroacetic acid (3 mL) and reacted at 25°C for 2 hours. The reaction solution was concentrated to obtain the title compound of this step (133 mg, yield: 86.3%).

MS m/z (ESI): 1104.6 [M+H]⁺.

### Intermediate Preparation Example 18: Preparation of Compound Int18

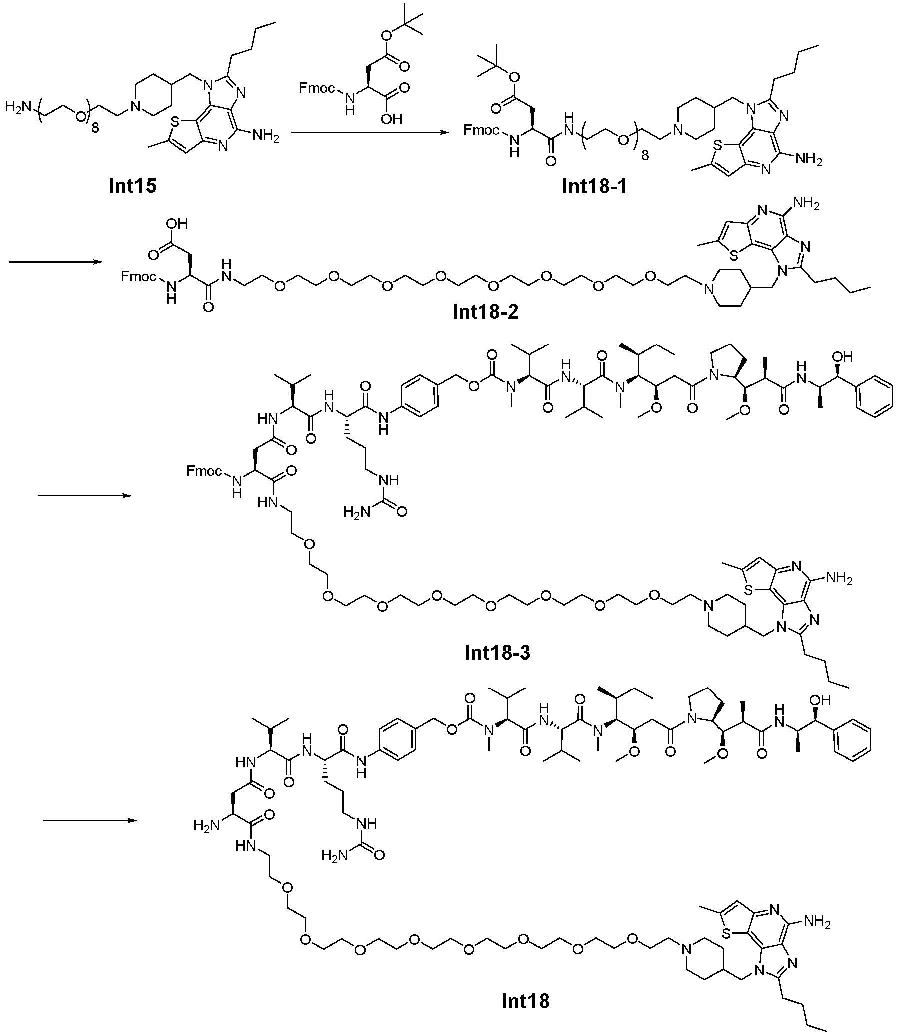

### Step 1: Preparation of Compound Int18-1

Compound **Int15** (60 mg, 79.78 µmol), (*S*)-2-((((9*H*-fluoren-9-yl)methoxy)carbonyl)amino)-4-(tert-butoxy)-4-oxobutyric acid (32.78 mg, 79.78 µmol), *N*,*N*-diisopropylethylamine (25.73 mg, 199.46 µmol) and 2-(7-azobenzotriazole)-*N*,*N*,*N*',*N*'-tetramethylurea hexafluorophosphate (36.38 mg, 95.73 µmol) were added to *N*,*N*-dimethylformamide (1 mL) in sequence, and stirred at room temperature for 1 hour. The reaction solution was directly purified by reversed-phase column chromatography (preparation method B, elution gradient: A%=35%-40%) to obtain the title compound of this step (52 mg, yield: 56.94%).

MS m/z (ESI): 1146.7 [M+H]⁺.

### Step 2: Preparation of Compound Int18-2

Compound **Int18-1** (50 mg, 43.60 µmol) and 48% hydrobromic acid aqueous solution (0.5 mL) were added to acetonitrile (1 mL) in sequence and stirred at room temperature for 3 hours. The reaction solution was concentrated to obtain the title compound of this step (35 mg, yield: 73.42%).

MS m/z (ESI): 1090.6 [M+H]⁺.

### Step 3: Preparation of Compound Int18-3

Compound **Int18-2** (35 mg, 32.09 mmol), VC-PABC-MMAE (36.04 mg, 32.09 µmol), *N*,*N*-diisopropylethylamine (10.34 mg, 80.22 µmol) and 2-(7-azobenzotriazole)-*N*,*N*,*N*',*N*'-tetramethylurea hexafluorophosphate (14.63 mg, 38.50 µmol) were added to *N*,*N*-dimethylformamide (1 mL) in sequence, and stirred at room temperature for 2 hours. The reaction solution was directly purified by reversed-phase column chromatography (preparation method B, elution gradient: A%=40%-45%) to obtain the title compound of this step (63 mg, yield: 89.31%).

MS m/z (ESI): 1098.1 [M/2+H]⁺.

### Step 4: Preparation of Compound Int18

Compound **Int18-3** (63 mg, 28.7 µmol) and piperidine (4.82 mg, 57.40 µmol) were added to *N*,*N*-dimethylformamide (1 mL), and stirred at room temperature for 2 hours. The reaction solution was directly purified by reversed-phase column chromatography (preparation method B, elution gradient: A%=25% to 35%) to obtain the title compound of this step (28 mg, yield: 49.45%).

MS m/z (ESI): 1973.2 [M+H]⁺.

### Intermediate Preparation Example 19: Preparation of Compound Int19

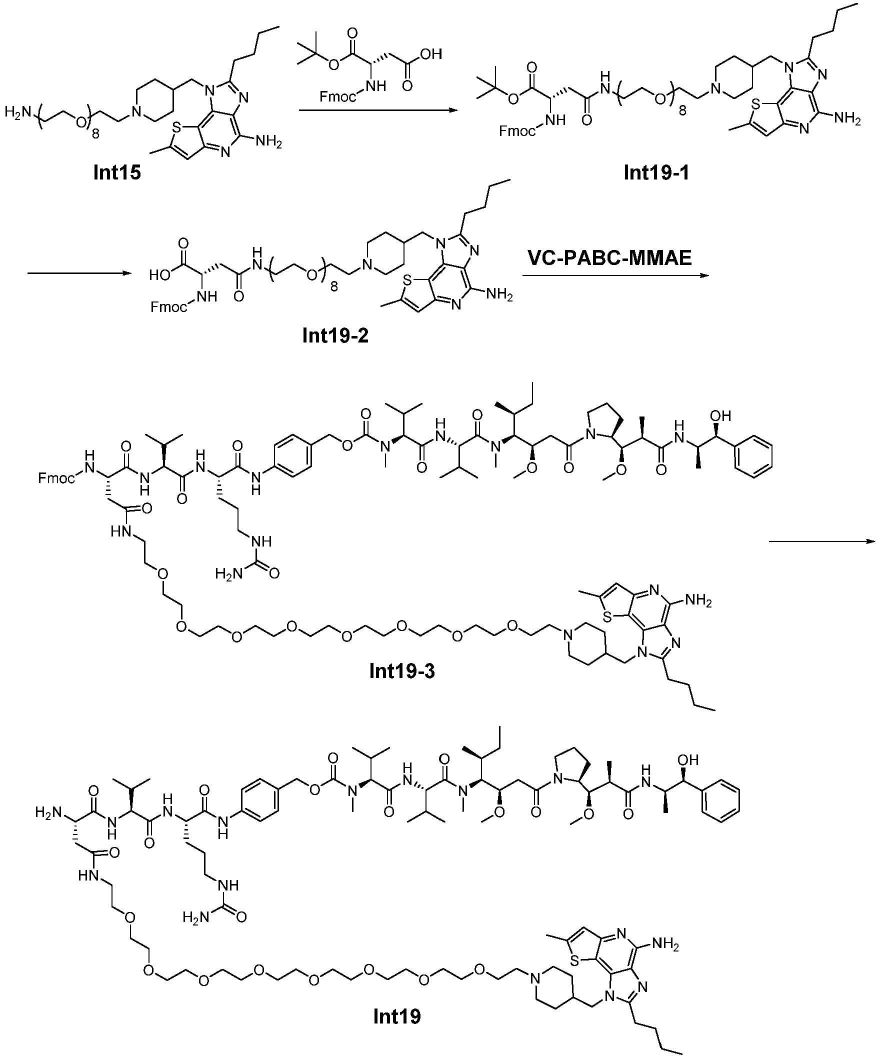

### Step 1: Preparation of Compound Int19-1

(*S*)-3-((((9*H*-Fluoren-9-yl)methoxy)carbonyl)amino)-4-(tert-butoxy)-4-oxobutyric acid (35.52 mg, 86.33 µmol), *N*,*N*-diisopropylethylamine (14.16 mg, 109.56 µmol), 2-(7-azobenzotriazole)-*N*,*N*,*N*',*N*'-tetramethylurea hexafluorophosphate (38.88 mg, 102.25 µmol) and Compound **Int15** (65 mg, 86.33 µmol) were added to anhydrous *N*,*N*-dimethylformamide (5 mL) in sequence, and reacted at 0°C for 2 hours. The reaction solution was directly purified by reversed-phase column chromatography (preparation method B, elution gradient: A%=25% to 35%) to obtain the title compound of this step (50 mg, yield: 53.2%).

MS m/z (ESI): 1146.6 [M+H]⁺.

### Step 2: Preparation of Compound Int19-2

Compound **Int19-1** (50 mg, 43.18 µmol) was dissolved in a mixed solvent of anhydrous dichloromethane (3 mL) and trifluoroacetic acid (1.5 mL) and reacted at 25°C for 4 hours. The reaction solution was directly purified by reversed-phase column chromatography (preparation method B, elution gradient: A%=35% to 45%) to obtain the title compound of this step (45 mg, yield: 89.2%).

MS m/z (ESI): 1090.5 [M+H]⁺.

### Step 3: Preparation of Compound Int19-3

Compound **Int19-2** (41.53 mg, 38.09 µmol), *N*,*N*-diisopropylethylamine (6.82 mg, 52.37 µmol), 2-(7-azobenzotriazole)-*N*,*N*,*N*',*N*'-tetramethylurea hexafluorophosphate (18.07 mg, 47.05 µmol) and VC-PBAC-MMAE (42.8 mg, 38.09 µmol) were added to anhydrous *N*,*N*-dimethylformamide (3 mL) in sequence, and reacted at 0°C for 2 hours. The reaction solution was directly purified by reversed-phase column chromatography (preparation method B, elution gradient: A%=20% to 25%) to obtain the title compound of this step (52 mg, yield: 65.4%).

MS m/z (ESI): 1098.1 [M/2+H]⁺.

### Step 4: Preparation of Compound Int19

Compound **Int19-3** (52 mg, 23.69 µmol) and piperidine (6.52 mg, 76.54 µmol) were added to N,N-dimethylformamide (2 mL) and reacted at 25°C for 2 hours. The reaction solution was directly purified by reversed-phase column chromatography (preparation method B, elution gradient: A%=35% to 45%) to obtain the title compound of this step (31 mg, yield: 82.9%).

MS m/z (ESI): 658.4 [M/3+H]⁺.

### Intermediate Preparation Example 20: Preparation of Compound Int20

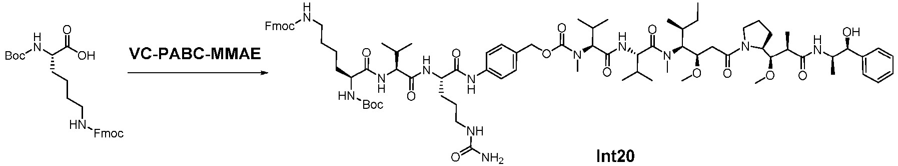

VC-PABC-MMAE (150 mg, 133.52 µmol), *N*,*N*-diisopropylethylamine (22.43 mg, 173.58 µmol), 2-(7-azobenzotriazole)-*N*,*N*,*N*',*N*'-tetramethylurea hexafluorophosphate (66 mg, 173.58 µmol) and *N*⁶-(((9*H*-fluoren-9-yl)methoxy)carbonyl)-*N*²-((tert-butoxy)carbonyl)-*L*-lysine (62.56 mg, 133.52 µmol) were added to anhydrous N,N-dimethylformamide (5 mL) in sequence and reacted at 0°C for 1 hour. The reaction solution was directly purified by reversed-phase column chromatography (preparation method B, elution gradient: A%=60% to 70%) to obtain the title compound of this step (170 mg, yield: 85.1%).

MS m/z (ESI): 1573.9 [M+H]⁺.

### Intermediate Preparation Example 21: Preparation of Compound Int21

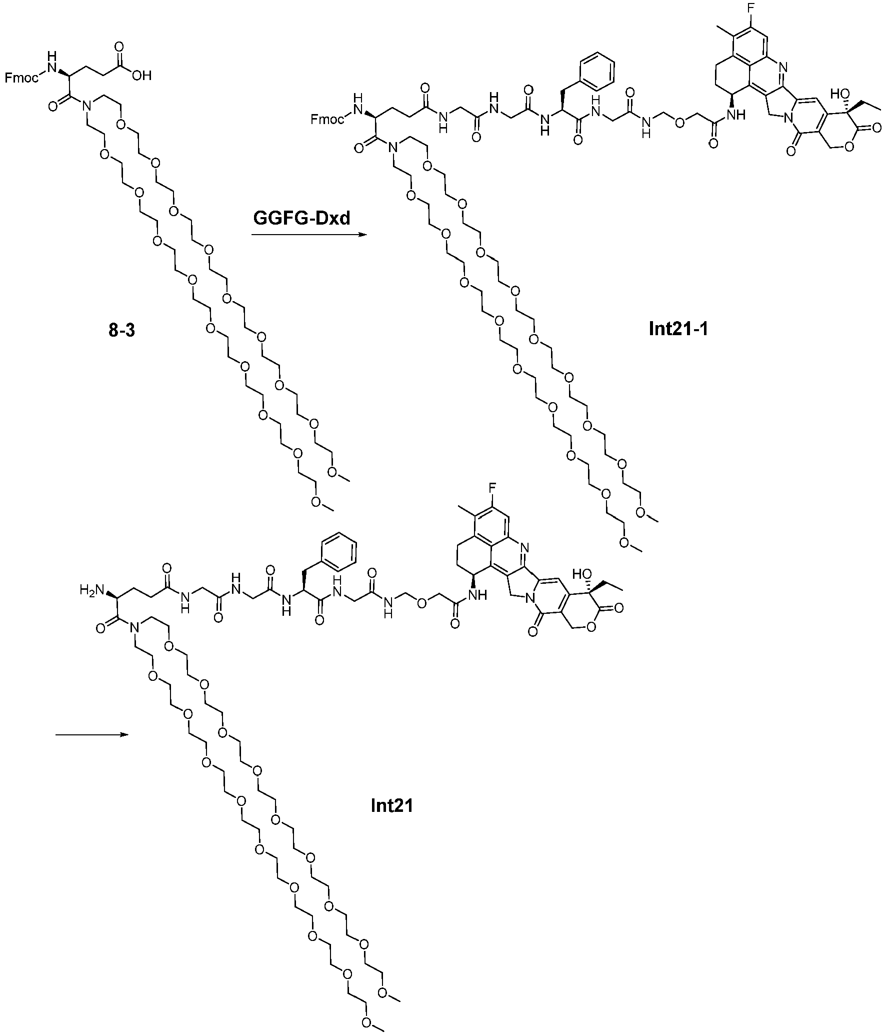

### Step 1: Preparation of Compound Int21-1

Compound **8-3** (86 mg, 72.3 µmol), *N*,*N*-diisopropylethylamine (12.15 mg, 94 µmol), 2-(7-azobenzotriazole)-*N*,*N*,*N*',*N*'-tetramethylurea hexafluorophosphate (35.74 mg, 94 µmol) and GGFG-Dxd (60.8 mg, 72.3 µmol) were added to anhydrous N,N-dimethylformamide (3 mL) in sequence, and reacted at 0°C for 2 hours. The reaction solution was directly purified by reversed-phase column chromatography (preparation method B, elution gradient: A%=25% to 35%) to obtain the title compound of this step (60 mg, yield: 43.4%).

MS m/z (ESI): 1006.5 [M/2+H]⁺.

### Step 2: Preparation of Compound Int21

Compound **Int21-1** (60 mg, 29.82 µmol) and diethylamine (10.46 mg, 141.64 µmol) were added to *N*,*N*-dimethylformamide (2 mL) and reacted at 25°C for 2 hours. The reaction solution was directly purified by reversed-phase column chromatography (preparation method B, elution gradient: A%=15% to 25%) to obtain the title compound of this step (45 mg, yield: 88.8%).

MS m/z (ESI): 1789.9 [M+H]⁺.

### Intermediate Preparation Example 22: Preparation of Compound Int22

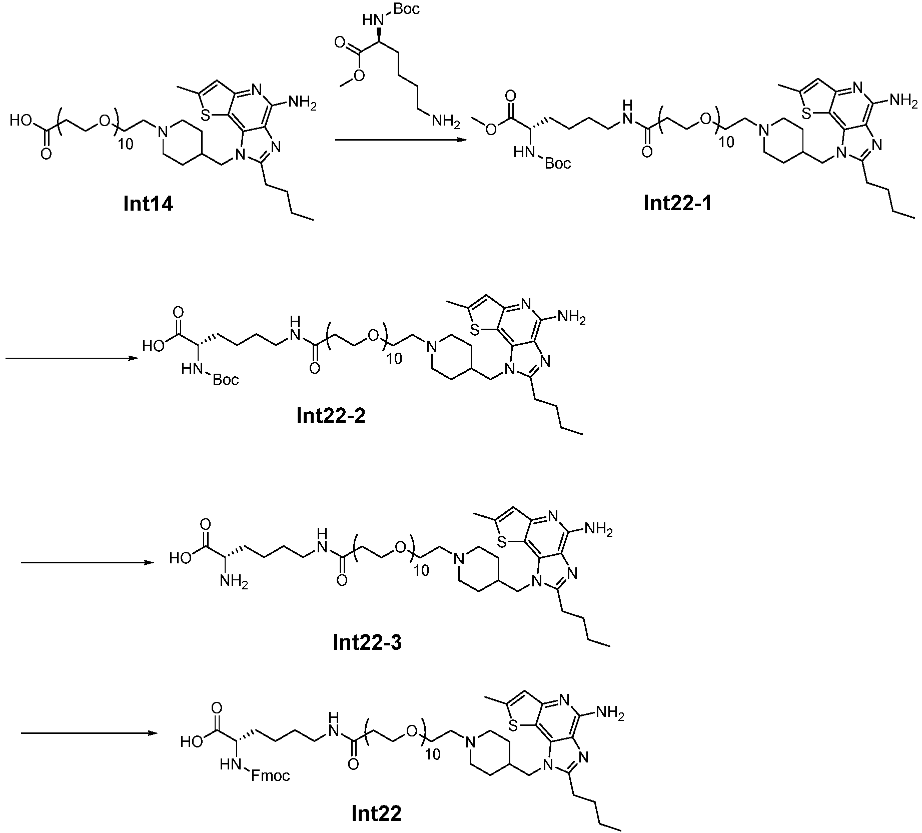

### Step 1: Preparation of Compound Int22-1

Compound **Int14** (280 mg, 321.84 µmol), *N*,*N*-diisopropylethylamine (68.42 mg, 524.08 µmol), 2-(7-azobenzotriazole)-*N*,*N*,*N*',*N*'-tetramethylurea hexafluorophosphate (114.8 mg, 350 µmol) and *N*-tert-butoxycarbonyllysine methyl ester hydrochloride (92 mg, 350 µmol) were added to anhydrous *N*,*N*-dimethylformamide (5 mL) in sequence, and reacted at 0°C for 2 hours. The reaction solution was directly purified by reversed-phase column chromatography (preparation method B, elution gradient: A%=25% to 35%) to obtain the title compound of this step (300 mg, yield: 83.8%).

MS m/z (ESI): 1112.6 [M+H]⁺.

### Step 2: Preparation of Compound Int22-2

Compound **Int22-1** (300 mg, 269.68 µmol) was dissolved in a mixed solvent of methanol (6 mL) and water (2 mL), then added with lithium hydroxide (56.58 mg, 1348.4 µmol), and reacted at 25°C for 2 hours. The reaction solution was directly purified by reversed-phase column chromatography (preparation method B, elution gradient: A%=25% to 35%) to obtain the title compound of this step (280 mg, yield: 94.5%).

MS m/z (ESI): 1098.6 [M+H]⁺.

### Step 3: Preparation of Compound Int22-3

Compound **Int22-2** (280 mg, 254.93 µmol) was dissolved in a mixed solvent of anhydrous dichloromethane (6 mL) and trifluoroacetic acid (3 mL) and reacted at 25°C for 2 hours. The reaction solution was directly purified by reversed-phase column chromatography (preparation method B, elution gradient: A%=25% to 35%) to obtain the title compound of this step (180 mg, yield: 74.5%).

MS m/z (ESI): 998.6 [M+H]⁺.

### Step 4: Preparation of Compound Int22

Compound **Int22-3** (180 mg, 178.51 µmol) and 9-fluorenylmethyl-N-succinimidyl carbonate (91.24 mg, 267.76 µmol) were dissolved in anhydrous dichloromethane (3 mL), then added with *N*,*N*-diisopropylethylamine (46.14 mg, 357.02 µmol), and reacted at 25°C for 2 hours. The reaction solution was directly purified by reversed-phase column chromatography (preparation method B, elution gradient: A%=35% to 45%) to obtain the title compound of this step (180 mg, yield: 82.9%).

MS m/z (ESI): 1220.6 [M+H]⁺.

### Intermediate Preparation Example 23: Preparation of Compound Int23

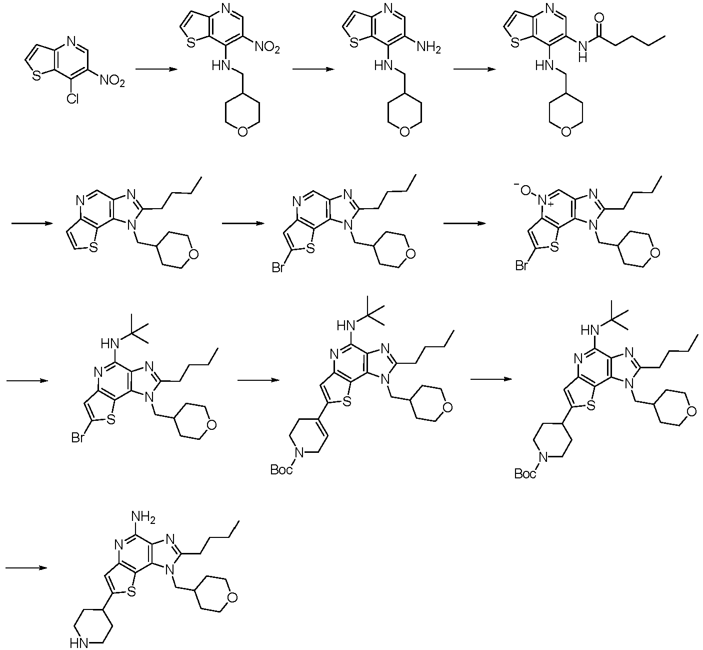

### Step 1: Preparation of 6-nitro-N-((tetrahydro-2H-pyran-4-yl)methyl)thieno[3,2-b] pyridine-7-amine

7-Chloro-6-nitrothieno[3,2-*b*]pyridine (2 g, 9.34 mmol) and 4-aminomethyltetrahydropyran (1.29 g, 11.2 mmol) were dissolved in *N*,*N*-dimethylformamide (20 mL), cooled in an ice-water bath, added with *N*,*N*-diisopropylethylamine (1.81 g, 14 mmol) to the reaction solution at 0°C, slowly heated to room temperature and stirred for 6 hours. The reaction solution was poured into water (100 mL) and extracted three times with ethyl acetate (50 mL). The organic phases were combined, washed three times with saturated brine (50 mL), dried over anhydrous sodium sulfate and concentrated to obtain the title compound of this step (2.52 g, yield: 92.3%).

MS m/z (ESI): 294.1 [M+H]⁺.

### Step 2: Preparation of N⁷-((tetrahydro-2H-pyran-4-yl)methyl)thieno[3,2-b]pyridine- 6,7-diamine

6-Nitro-*N*-((tetrahydro-2H-pyran-4-yl)methyl)thieno[3,2-*b*]pyridin-7-amine (2.52 g, 8.53 mmol) was dissolved in tetrahydrofuran (50 mL) and methanol (50 mL), added with 10% palladium on carbon (0.2 g), and replaced with hydrogen three times, the reaction system was stirred at room temperature for 12 hours, and filtered with suction, the filter cake was washed with methanol (30 mL), and the filtrate was concentrated to obtain the title compound of this step (2.1 g, yield: 93.7%).

MS m/z (ESI): 264.1 [M+H]⁺.

### Step 3: Preparation of N-(7-(((tetrahydro-2H-pyran-4-yl)methyl)amino)thieno[3,2-b] pyridin-6-yl)pentanamide

*N*⁷-((Tetrahydro-2*H*-pyran-4-yl)methyl)thieno[3,2-*b*]pyridine-6,7-diamine (2.1 g, 7.98 mmol) and N,N-diisopropylethylamine (2.1 g, 15.9 mmol) were dissolved in tetrahydrofuran (40 mL), cooled in an ice-water bath, added dropwise with n-pentanoyl chloride (1.45 g, 11.97 mmol) to the reaction solution at 0°C, heated slowly to room temperature, and stirred for 4 hours. Water (10 mL) was added to the reaction system to quench the reaction. The reaction system was concentrated to remove tetrahydrofuran as much as possible. The residue was added with water (50 mL) and extracted three times with ethyl acetate (30 mL). The organic phases were combined, washed three times with saturated brine (50 mL), dried over anhydrous sodium sulfate and concentrated to obtain the title compound of this step (2.8 g, yield: 100%).

MS m/z (ESI): 348.1 [M+H]⁺.

### Step 4: Preparation of 2-butyl-1-((tetrahydro-2H-pyran-4-yl)methyl)-1H-imidazo[4,5-d] thieno[3,2-b]pyridine

*N*-(7-(((tetrahydro-2*H*-pyran-4-yl)methyl)amino)thieno[3,2-*b*]pyridin-6-yl)pentanamide (2.8 g, 7.98 mmol) was added to ethanol (30 mL), added with sodium hydroxide (1.3g, 31.9 mmol) at room temperature, heated to 50°C and stirred for 6 hours. Water (150 mL) was added to the reaction system to quench the reaction. The reaction system was adjusted with hydrochloric acid to pH = 6 and extracted three times with ethyl acetate (100 mL). The organic phases were combined, washed twice with saturated sodium bicarbonate solution (60 mL), and washed twice with saturated brine (60 mL), dried over anhydrous sodium sulfate and concentrated to obtain the title compound of this step (1.9 g, yield: 72.2%).

MS m/z (ESI): 330.1 [M+H]⁺.

### Step 5: Preparation of 7-bromo-2-butyl-1-((tetrahydro-2H-pyran-4-yl)methyl)-1H-imidazo[4,5-d]thieno[3,2-b]pyridine

2-Butyl-1-((tetrahydro-2*H*-pyran-4-yl)methyl)-1*H*-imidazo[4,5-*d*]thieno[3,2-*b*]pyridine (1.9 g, 5.75 mmol) was completely dissolved in a mixed solvent of *N*,*N*-dimethylformamide (20 mL) and glacial acetic acid (6 mL), cooled in an ice-water bath, slowly added with *N*-bromosuccinimide (1.54 g, 8.63 mmol) in batches to the reaction solution at 0°C, slowly heated to room temperature and stirred for 24 hours. The reaction solution was poured into water (100 mL) and extracted three times with ethyl acetate (30 mL). The organic phases were combined, washed three times with saturated sodium bicarbonate aqueous solution (50 mL), washed three times with saturated brine (50 mL), dried over anhydrous sodium sulfate and concentrated, and the residue was purified by silica gel column chromatography (eluent: ethyl acetate/petroleum ether = 1/1 (v/v)) to obtain the title compound of this step (1.64 g, yield: 70%).

MS m/z (ESI): 408.0 [M+H]⁺.

### Step 6: Preparation of 7-bromo-2-butyl-1-((tetrahydro-2H-pyran-4-yl)methyl)-1H-imidazo [4,5-d]thieno[3,2-b]pyridine-5-oxide

7-Bromo-2-butyl-1-((tetrahydro-2H-pyran-4-yl)methyl)-1*H*-imidazo[4,5-*d*]thieno[3,2-*b*]pyridine (1.64 g, 4.0 mmol) was dissolved in dichloromethane (30 mL), cooled in an ice-water bath, slowly added with m-chloroperoxybenzoic acid (1.63 g, 8.0 mmol) in batches to the reaction solution at 0°C, slowly heated to room temperature and stirred for 4 hours. The reaction solution was poured into water (100 mL) and extracted three times with dichloromethane (30 mL). The organic phases were combined, washed twice with saturated sodium bicarbonate aqueous solution (30 mL), washed twice with saturated sodium sulfite aqueous solution (30 mL), washed three times with saturated brine (30 mL), dried over anhydrous sodium sulfate and concentrated to obtain the title compound (1.7 g, yield: 100%).

MS m/z (ESI): 424.0 [M+H]⁺.

### Step 7: Preparation of 7-bromo-N-(tert-butyl)-2-butyl-1-((tetrahydro-2H-pyran-4-yl)methyl)-1H-imidazo[4,5-d]thieno[3,2-b]pyridine-4-amine

7-Bromo-2-butyl-1-((tetrahydro-2*H*-pyran-4-yl)methyl)-1*H*-imidazo[4,5-*d*]thieno[3,2-*b*]pyridine-5-o xide (1.7 g, 4.0 mmol) and *tert*-butylamine (0.73 g, 20 mmol) were dissolved in dichloromethane (20 mL), cooled in an ice-water bath, slowly added with *p*-toluenesulfonyl chloride (1.53 g, 8.0 mmol) in batches to the reaction solution at 0°C, slowly warmed to room temperature and stirred for 12 hours. The reaction system was added with water (100 mL) and extracted three times with dichloromethane (30 mL). The organic phases were combined, washed three times with saturated brine (30 mL), dried over anhydrous sodium sulfate and concentrated, and the residue was purified by silica gel column chromatography (eluent: ethyl acetate/petroleum ether = 5/1 (v/v)) to obtain the title compound of this step (1.1 g, yield: 57.3%).

MS m/z (ESI): 479.0 [M+H]⁺.

### Step 8: Preparation of tert-butyl 4-(2-butyl-4-(tert-butylamino)-1-((tetrahydro-2H-pyran-4-yl) methyl)-1H-imidazo[4,5-d]thieno[3,2-b]pyridin-7-yl)-3,6-dihydropyridine-1(2H)-carboxylate

7-Bromo-*N*-(tert-butyl)-2-butyl-1-((tetrahydro-2*H*-pyran-4-yl)methyl)-1*H*-imidazo[4,5-*d*]thieno[3,2-*b*]pyridin-4-amine (0.8 g, 1.71 mmol), tert-butyl 4-(4,4,5,5-tetramethyl-1,3,2-dioxaborolan-2-yl)-3,6-dihydropyridine-1(2*H*)-carboxylate (0.8 g, 2.56 mmol), potassium carbonate (4.73 g, 3.4 mmol) and 1,1'-bis(diphenylphosphine) ferrocene palladium dichloride (62 mg, 0.08 mmol) were added to a mixed solvent of 1,4-dioxane (10 mL) and water (2 mL). The reaction system was bubbled with nitrogen for 5 minutes, then heated to 100°C and stirred for 2 hours. After the reaction system was cooled to room temperature, the solvent was removed by concentration, and the residue was purified by silica gel column chromatography (eluent: petroleum ether/ethyl acetate = 1/3 (v/v)) to obtain the title compound of this step (0.5 g, yield: 50.5%).

MS m/z (ESI): 582.3 [M+H]⁺.

### Step 9: Preparation of tert-butyl 4-(2-butyl-4-(tert-butylamino)-1-((tetrahydro-2H-pyran-4-yl)methyl)-1H-imidazo[4,5-d]thieno[3,2-b]pyridin-7-yl)piperidine-1-carboxylate

Tert-butyl 4-(2-butyl-4-(tert-butylamino)-1-((tetrahydro-2*H*-pyran-4-yl)methyl)-1*H*-imidazo [4,5-*d*]thieno[3,2-*b*]pyridin-7-yl)-3,6-dihydropyridine-1(2*H*)-carboxylate (0.5 g, 0.86 mmol), ammonium formate (1.08 g, 17.2 mmol) and 10% palladium on carbon (20 mg, 0.019 mmol) were added to absolute ethanol (10 mL) in sequence, slowly heated to 80°C and stirred for 12 hours. The reaction system was cooled to room temperature, then filtered with suction, the filter cake was washed with ethanol (15 mL), the filtrate was concentrated, and the residue was purified by silica gel column chromatography (eluent: dichloromethane/methanol=10/1 (v/v))) to obtain the title compound of this step (0.40 g, yield: 79.7%).

MS m/z (ESI): 584.3 [M+H]⁺.

### Step 10: Preparation of 2-butyl-7-(piperidin-4-yl)-1-((tetrahydro-2H-pyran-4-yl)methyl)-1H-imidazo[4,5-d]thieno[3,2-b]pyridin-4-amine (Int23)

Tert-butyl 4-(2-butyl-4-(tert-butylamino)-1-((tetrahydro-2*H*-pyran-4-yl)methyl)-1*H*-imidazo [4,5-*d*]thieno[3,2-*b*]pyridin-7-yl)piperidine-1-carboxylate (0.3 g, 0.51 mmol) was dissolved in methanol (3 mL), added with concentrated hydrochloric acid (0.5 mL) at room temperature, heated to 50°C and stirred for 4 hours. The reaction system was cooled to room temperature, then concentrated to remove the solvent, and the residue was purified by preparative high-performance liquid chromatography to obtain the title compound of this step (0.19 g, yield: 87.1%).
MS m/z (ESI): 428.3 [M+H]⁺.
¹H NMR (400 MHz, CD3OD) δ: 7.11 (s, 1H), 4.23 (d, *J =* 7.6 Hz, 2H), 3.95-3.92 (m, 2H), 3.47-3.44 (m, 2H), 3.15-3.08 (m, 2H), 2.98-2.94 (m, 2H), 2.31-2.15 (m, 3H), 2.02-1.86 (m, 4H), 1.57-1.49 (m, 5H), 1.37-1.26 (m, 4H), 1.03 (t, *J =* 7.6 Hz, 3H).

### Intermediate Preparation Example 24: Preparation of Compound Int24

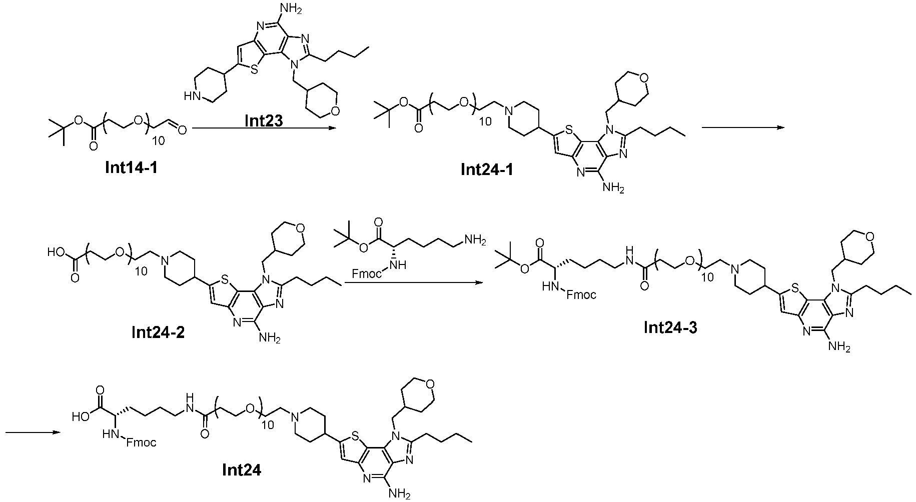

### Step 1: Preparation of Compound Int24-1

Compound **Int14-1** (472.48 mg, 799.99 µmol), Compound **Int23** (250 mg, 533.33 µmol), sodium cyanoborohydride (99.08 mg, 1576.7 µmol) and glacial acetic acid (51.72 mg, 853.33 µmol) were added in sequence to a mixed solvent of 1,2-dichloroethane (6 mL) and methanol (3 mL) and reacted at 25°C for 4 hours. The crude product obtained by concentrating the reaction solution was dissolved in methanol (3 mL) and purified by reverse-phase column chromatography (preparation method B, elution gradient: A%=20% to 30%) to obtain the title compound of this step (475 mg, yield: 89.4%).

MS m/z (ESI): 996.6 [M+H]⁺.

### Step 2: Preparation of Compound Int24-2

Compound **Int24-1** (475 mg, 476.76 µmol) was dissolved in dioxane hydrochloride solution (6 mL, 4M) and reacted at 25°C for 2 hours. The reaction solution was concentrated to obtain the title compound of this step (400 mg, yield: 99.3%).

MS m/z (ESI): 940.6 [M+H]⁺.

### Step 3: Preparation of Compound Int24-3

Compound **Int24-2** (280 mg, 297.81 µmol), *N*,*N*-diisopropylethylamine (116.63 mg, 893.44 µmol), 2-(7-azobenzotriazole)-*N*,*N*,*N*',*N*'-tetramethylurea hexafluorophosphate (160.13 mg, 416.94 µmol) and (((9*H*-fluoren-9-yl)methoxy)carbonyl)-*L*-lysine tert-butyl ester (133.08 mg, 313.48 µmol) were added in sequence to anhydrous N,N-dimethylformamide (5 mL), and reacted at 0°C for 2 hours. The reaction solution was directly purified by reversed-phase column chromatography (preparation method B, elution gradient: A%=25% to 35%) to obtain the title compound of this step (248 mg, yield: 61.8%).

MS m/z (ESI): 1346.7 [M+H]⁺.

### Step 4: Preparation of Compound Int24

Compound **Int24-3** (94.74 mg, 70.34 µmol) was dissolved in a mixed solvent of anhydrous dichloromethane (4 mL) and trifluoroacetic acid (2 mL) and reacted at 25°C for 2 hours. The reaction solution was concentrated to obtain the title compound of this step (70 mg, yield: 81.2%).

MS m/z (ESI): 1290.7 [M+H]⁺.

### Intermediate Preparation Example 25: Preparation of Compound Int25

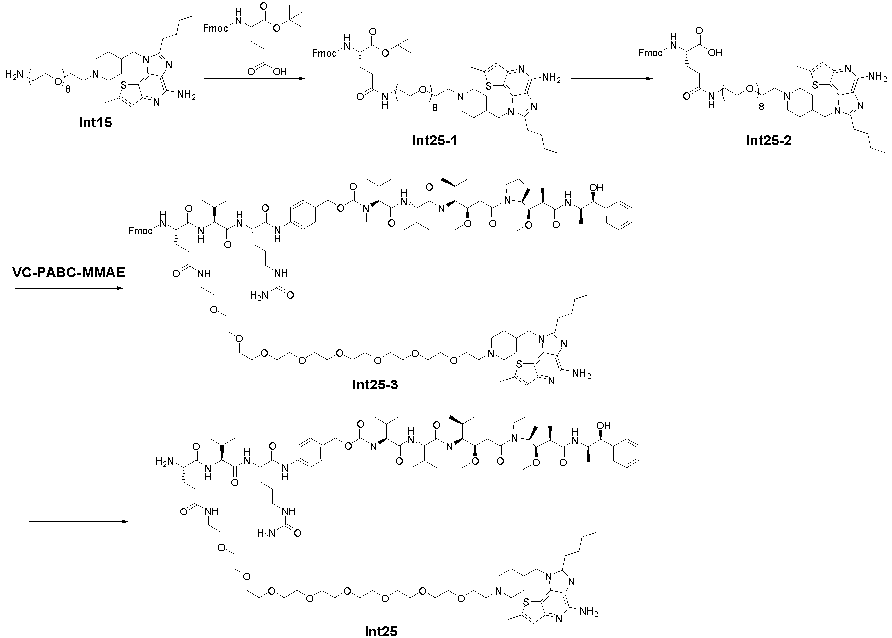

### Step 1: Preparation of Compound Int25-1

(*S*)-2-((((9*H*-fluoren-9-yl)methoxy)carbonyl)amino)-5-(tert-butoxy)-5-oxopentanoic acid (37.29 mg, 87.65 µmol), *N*,*N*-diisopropylethylamine (14.16 mg, 109.56 µmol), 2-(7-azobenzotriazole)-*N*,*N*,*N*',*N*'-tetramethylurea hexafluorophosphate (38.88 mg, 102.25 µmol) and Compound **Int15** (55 mg, 73.04 µmol) were added in sequence to anhydrous N,N-dimethylformamide (5 mL), and reacted at 0°C for 2 hours. The reaction solution was directly purified by reversed-phase column chromatography (preparation method B, elution gradient: A%=25% to 35%) to obtain the title compound of this step (80 mg, yield: 99.4%).

MS m/z (ESI): 1160.6 [M+H]⁺.

### Step 2: Preparation of Compound Int25-2

Compound **Int25-1** (50 mg, 43.09 µmol) was dissolved in a mixed solvent of anhydrous dichloromethane (3 mL) and trifluoroacetic acid (1.5 mL) and reacted at 25°C for 4 hours. The reaction solution was directly purified by reversed-phase column chromatography (preparation method B, elution gradient: A%=35% to 45%) to obtain the title compound of this step (45 mg, yield: 62.2%).

MS m/z (ESI): 1104.6 [M+H]⁺.

### Step 3: Preparation of Compound Int25-3

Compound **Int25-2** (45 mg, 40.74 µmol), *N*,*N*-diisopropylethylamine (7.58 mg, 58.07 µmol), 2-(7-azobenzotriazole)-*N*,*N*,*N*',*N*'-tetramethylurea hexafluorophosphate (20.81 mg, 54.19 µmol) and VC-PABC-MMAE (45.78 mg, 40.74 µmol) were added to anhydrous *N*,*N-*dimethylformamide (4 mL), and reacted at 0°C for 2 hours. The reaction solution was directly purified by reversed-phase column chromatography (preparation method B, elution gradient: A%=20% to 30%) to obtain the title compound of this step (30 mg, yield: 35.1%).

MS m/z (ESI): 1105.2 [M/2+H]⁺.

### Step 4: Preparation of Compound Int25

Compound **Int25-3** (30 mg, 13.44 µmol) and diethylamine (4.58 mg, 53.76 µmol) were added to *N*,*N*-dimethylformamide (2 mL) and reacted at 25°C for 2 hours. The reaction solution was directly purified by reversed-phase column chromatography (preparation method B, elution gradient: A%=35% to 45%) to obtain the title compound of this step (25 mg, yield: 93.6%).

MS m/z (ESI): 994.1 [M/2+H]⁺.

### Intermediate Preparation Example 26: Preparation of Compound Int26

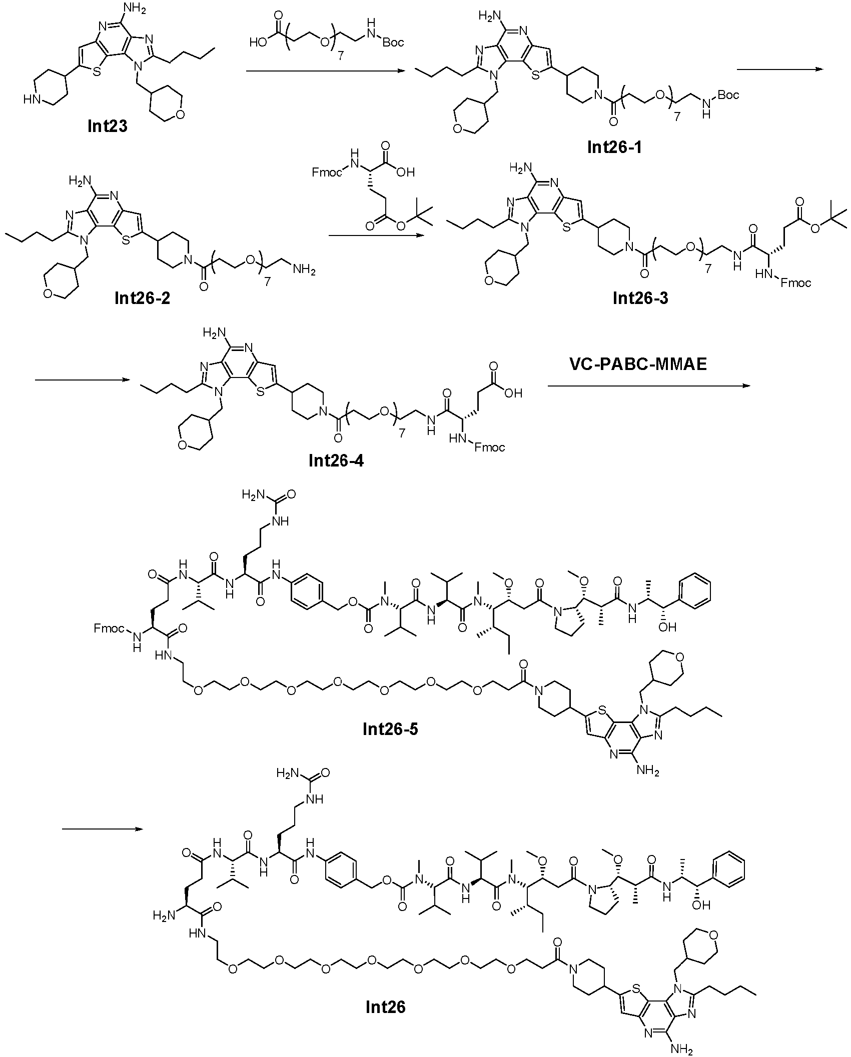

### Step 1: Preparation of Compound Int26-1

*N*-Tert-butoxycarbonyl-hepta(ethylene glycol)-carboxylic acid (450 mg, 895.34 µmol), *N*,*N*-diisopropylethylamine (466.66 mg, 3.58 mmol), 2-(7-azobenzotriazole)-*N*,*N*,*N*',*N*'- tetramethylurea hexafluorophosphate (412.4 mg, 1.07 mmol) and Compound **Int23** (419.69 mg, 895.34 µmol) were added in sequence to anhydrous *N*,*N*-dimethylformamide (6 mL), and reacted at 0°C for 2 hours. The reaction solution was directly purified by reversed-phase column chromatography (preparation method B, elution gradient: A%=55% to 65%) to obtain the title compound of this step (800 mg, yield: 98.5%).

MS m/z (ESI): 907.5 [M+H]⁺.

### Step 2: Preparation of Compound Int26-2

Compound **Int26-1** (800 mg, 881.87 µmol) was dissolved in dioxane hydrochloride solution (6 mL, 4M) and reacted at 25°C for 2 hours. The reaction solution was concentrated to obtain the title compound of this step (610 mg, yield: 90.2%).

MS m/z (ESI): 807.5 [M+H]⁺.

### Step 3: Preparation of Compound Int26-3

(*S*)-2-((((9*H*-Fluoren-9-yl)methoxy)carbonyl)amino)-5-(tert-butoxy)-5-oxopentanoic acid (276.97 mg, 644.45 µmol), *N*,*N*-diisopropylethylamine (209.93 mg, 1610 µmol), 2-(7-azobenzotriazole)-*N*,*N*,*N*',*N*'-tetramethylurea hexafluorophosphate (296.84 mg, 773.34 µmol) and Compound **Int26-**2 (604 mg, 644.45 µmol) were added in sequence to anhydrous *N*,*N*-dimethylformamide (4 mL), and reacted at 0°C for 2 hours. The reaction solution was directly purified by reversed-phase column chromatography (preparation method B, elution gradient: A%=25% to 35%) to obtain the title compound of this step (747.9 mg, yield: 95.6%).

MS m/z (ESI): 1214.6 [M+H]⁺.

### Step 4: Preparation of Compound Int26-4

Compound **Int26-3** (460 mg, 378.75 µmol) was dissolved in a mixed solvent of anhydrous dichloromethane (6 mL) and trifluoroacetic acid (3 mL) and reacted at 25°C for 2 hours. The reaction solution was concentrated to obtain the title compound of this step (400 mg, yield: 91.2%).

MS m/z (ESI): 1158.6 [M+H]⁺.

### Step 5: Preparation of Compound Int26-5

Compound **Int26-4** (113.74 mg, 91.19 µmol), *N*,*N*-diisopropylethylamine (23.24 mg, 178.03 µmol), 2-(7-azobenzotriazole)-*N*,*N*,*N*',*N*'-tetramethylurea hexafluorophosphate (51.28 mg, 133.52 µmol) and VC-PABC-MMAE (95 mg, 84.56 µmol) were added to anhydrous *N*,*N*-dimethylformamide (4 mL), and reacted at 0°C for 2 hours. The reaction solution was directly purified by reversed-phase column chromatography (preparation method B, elution gradient: A%=55% to 65%) to obtain the title compound of this step (170 mg, yield: 93.5%).

MS m/z (ESI): 1132.2 [M/2+H]⁺.

### Step 6: Preparation of Compound Int26

Compound **Int26-5** (170 mg, 75.09 µmol) and diethylamine (27.74 mg, 375.47 µmol) were added to *N*,*N*-dimethylformamide (4 mL), and reacted at 25°C for 2 hours. The reaction solution was directly purified by reversed-phase column chromatography (preparation method B, elution gradient: A%=55% to 65%) to obtain the title compound of this step (110 mg, yield: 71.9%).

MS m/z (ESI): 1021.2 [M/2+H]⁺.

### Intermediate Preparation Example 27: Preparation of Compound Int27

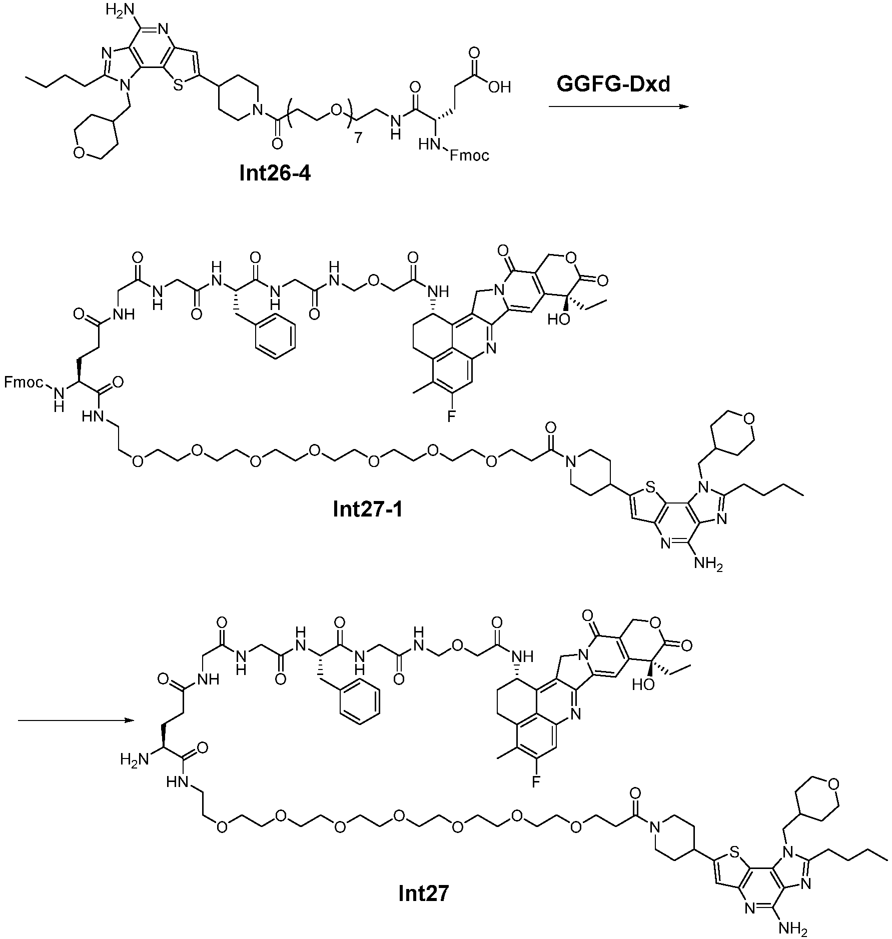

### Step 1: Preparation of Compound Int27-1

Compound **Int26-4** (440 mg, 379.83 µmol), *N*,*N*-diisopropylethylamine (98.18 mg, 759.66 µmol), 2-(7-azobenzotriazole)-*N*,*N*,*N*',*N*'-tetramethylurea hexafluorophosphate (173.31 mg, 455.8 µmol) and GGFG-Dxd (319.38 mg, 379.83 µmol) were added to anhydrous *N*,*N*-dimethylformamide (6 mL), and reacted at 0°C for 2 hours. The reaction solution was directly purified by reversed-phase column chromatography (preparation method B, elution gradient: A%=25% to 35%) to obtain the title compound of this step (250 mg, yield: 35.0%).

MS m/z (ESI): 991.2 [M/2+H]⁺.

### Step 2: Preparation of Compound Int27

Compound **Int27-1** (205 mg, 103.47 µmol) and diethylamine (36.31 mg, 491.49 µmol) were added to *N*,*N*-dimethylformamide (2 mL), and reacted at 25°C for 2 hours. The reaction solution was directly purified by reversed-phase column chromatography (preparation method B, elution gradient: A%=25% to 35%) to obtain the title compound of this step (160 mg, yield: 93.1%).

MS m/z (ESI): 586.9 [M/3+H]⁺.

### Intermediate Preparation Example 28: Preparation of Compound Int28

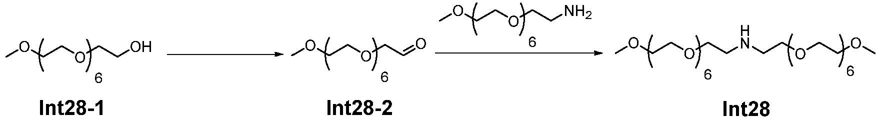

### Step One: Preparation of Compound Int28-2

Hepta(ethylene glycol) monomethyl ether (1 g, 2.91 mmol) and Dess-Martin oxidant (1.87 g, 4.36 mmol) were added to dichloromethane (15 mL), and reacted at 25°C for 10 hours. Saturated sodium thiosulfate solution (5 mL) and saturated sodium bicarbonate aqueous solution (10 mL) were added to the reaction solution, stirred until clear, then extracted three times with dichloromethane, washed with saturated brine, dried over anhydrous sodium sulfate, and filtered. The filtrate was concentrated. The crude product obtained was purified by silica gel column chromatography (eluent: dichloromethane/methanol = 15/1 (v/v)) to obtain the title compound of this step (890 mg, yield: 90.4%).

MS m/z (ESI): 339.2 [M+H]⁺.

### Step 2: Preparation of Compound Int28

Compound **Int28-2** (600 mg, 1.78 mmol), sodium cyanoborohydride (143.5 mg, 2.26 mmol) and glacial acetic acid (137.13 mg, 2.26 mmol) were added to a mixed solvent of 1,2-dichloroethane (6 mL) and methanol (4 mL), stirred evenly at 25°C, and then 2,5,8,11,14,17,20-heptaoxadocosan-22-amine (592.33 mg, 1.74 mmol) was dissolved in 1,2-dichloroethane (6 mL), and added dropwise into the above system within 3 hours by using a constant pressure dropping funnel. The filtrate was concentrated. The crude product obtained was purified by silica gel column chromatography (eluent: dichloromethane/methanol = 17/3 (v/v)) to obtain the title compound of this step (650 mg, yield: 65.2%).

MS m/z (ESI): 662.3 [M+H]⁺.

### Intermediate Preparation Example 29: Preparation of Compound Int29

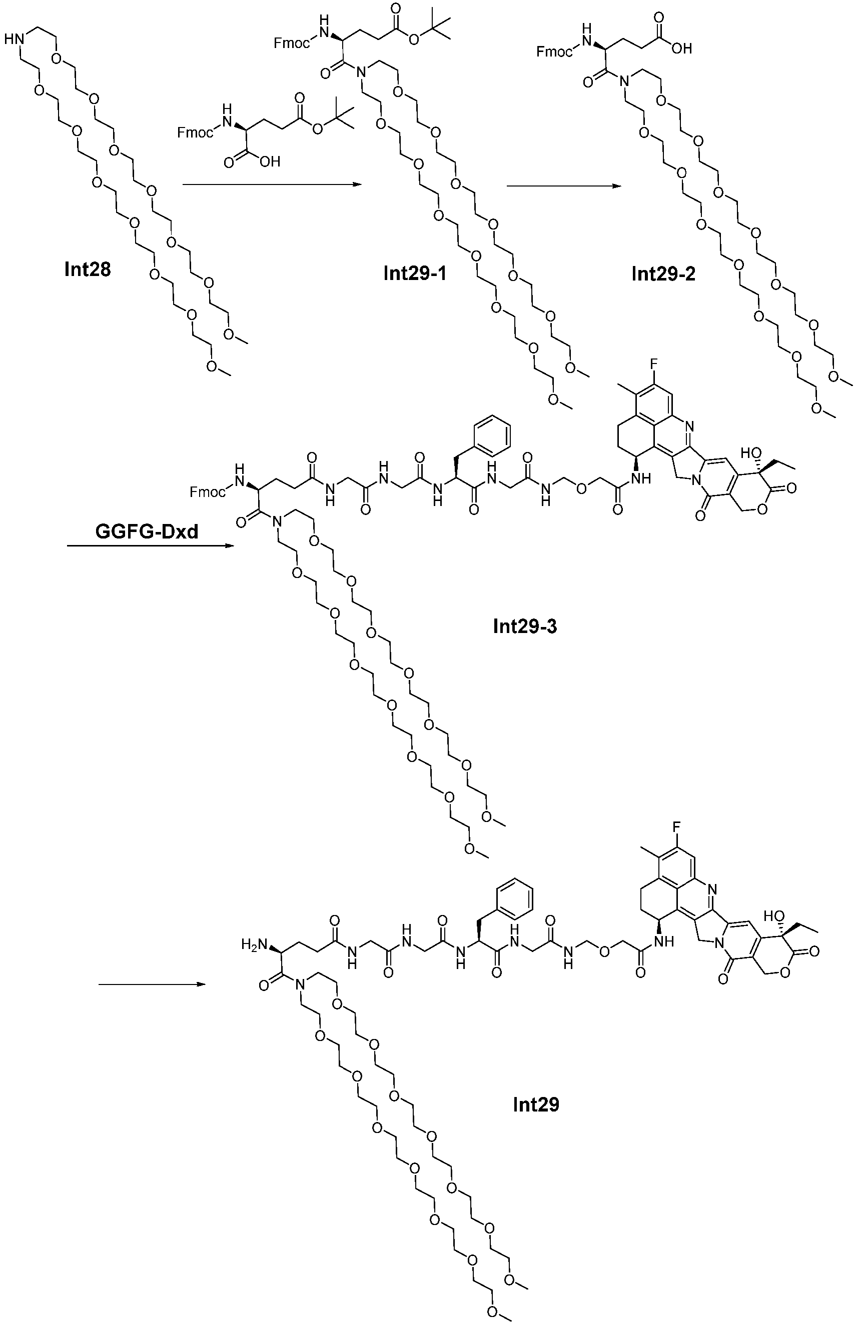

### Step 1: Preparation of Compound Int29-1

(*S*)-2-((((9*H*-Fluoren-9-yl)methoxy)carbonyl)amino)-5-(tert-butoxy)-5-oxopentanoic acid (496.25 mg, 1.17 mmol), *N*,*N*-diisopropylethylamine (188.43 mg, 1.39 mmol), 2-(7-azobenzotriazole)-*N*,*N*,*N*',*N*'-tetramethylurea hexafluorophosphate (554.36 mg, 1.39 mmol) and Compound **Int28** (650 mg, 982.14 µmol) were added to anhydrous *N*,*N*-dimethylformamide (5 mL), and reacted at 0°C for 2 hours. The reaction solution was directly purified by reversed-phase column chromatography (preparation method B, elution gradient: A%=25% to 35%) to obtain the title compound of this step (650 mg, yield: 65.8%).

MS m/z (ESI): 1069.6 [M+H]⁺.

### Step 2: Preparation of Compound Int29-2

Compound **Int29-1** (235.79 mg, 220.51 µmol) was dissolved in dioxane hydrochloride solution (5 mL, 4M) and reacted at 25°C for 2 hours. The reaction solution was concentrated to obtain the title compound of this step (180 mg, yield: 84.8%).

MS m/z (ESI): 1013.5 [M+H]⁺.

### Step 3: Preparation of Compound Int29-3

Compound **Int29-2** (151.82 mg, 149.85 µmol), *N*,*N*-diisopropylethylamine (25.18 mg, 194.8 µmol), 2-(7-azobenzotriazole)-*N*,*N*,*N*',*N*'-tetramethylurea hexafluorophosphate (62.67 mg, 164.83 µmol) and GGFG-Dxd (126 mg, 149.85 µmol) were added to anhydrous *N*,*N*-dimethylformamide (5 mL), and reacted at 0°C for 2 hours. The reaction solution was directly purified by reversed-phase column chromatography (preparation method B, elution gradient: A%=25% to 35%) to obtain the title compound of this step (160 mg, yield: 61.2%).

MS m/z (ESI): 1835.8 [M+H]⁺.

### Step 4: Preparation of Compound Int29

Compound **Int29-3** (160 mg, 87.16 µmol) and diethylamine (63.74 mg, 871.6 µmol) were added to *N*,*N*-dimethylformamide (2 mL), and reacted at 25°C for 2 hours. The reaction solution was directly purified by reversed-phase column chromatography (preparation method B, elution gradient: A%=25% to 35%) to obtain the title compound of this step (145 mg, yield: 596.7%).

MS m/z (ESI): 1613.8 [M+H]⁺.

### Intermediate Preparation Example 30: Preparation of Compound Int30

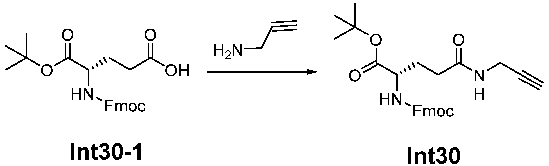

(*S*)-2-((((9*H*-Fluoren-9-yl)methoxy)carbonyl)amino)-5-(tert-butoxy)-5-oxopentanoic acid (400 mg, 930.73 µmol), *N*,*N*-diisopropylethylamine (243 mg, 1.86 mmol), 2-(7-azobenzotriazole)-*N*,*N*,*N*',*N*'-tetramethylurea hexafluorophosphate (500.45 mg, 1.3 mmol) and propargylamine (77.67 mg, 1.4 mmol) were added to anhydrous *N*,*N*-dimethylformamide (4 mL), and reacted at 25°C for 3 hours. The crude product obtained by concentrating the reaction solution was purified by silica gel column chromatography (eluent: petroleum ether/ethyl acetate = 3/2 (v/v)) to obtain the title compound of this step (420 mg, yield: 97.7%).

MS m/z (ESI): 463.2 [M+H]⁺.

### Intermediate Preparation Example 31: Preparation of Compound Int31

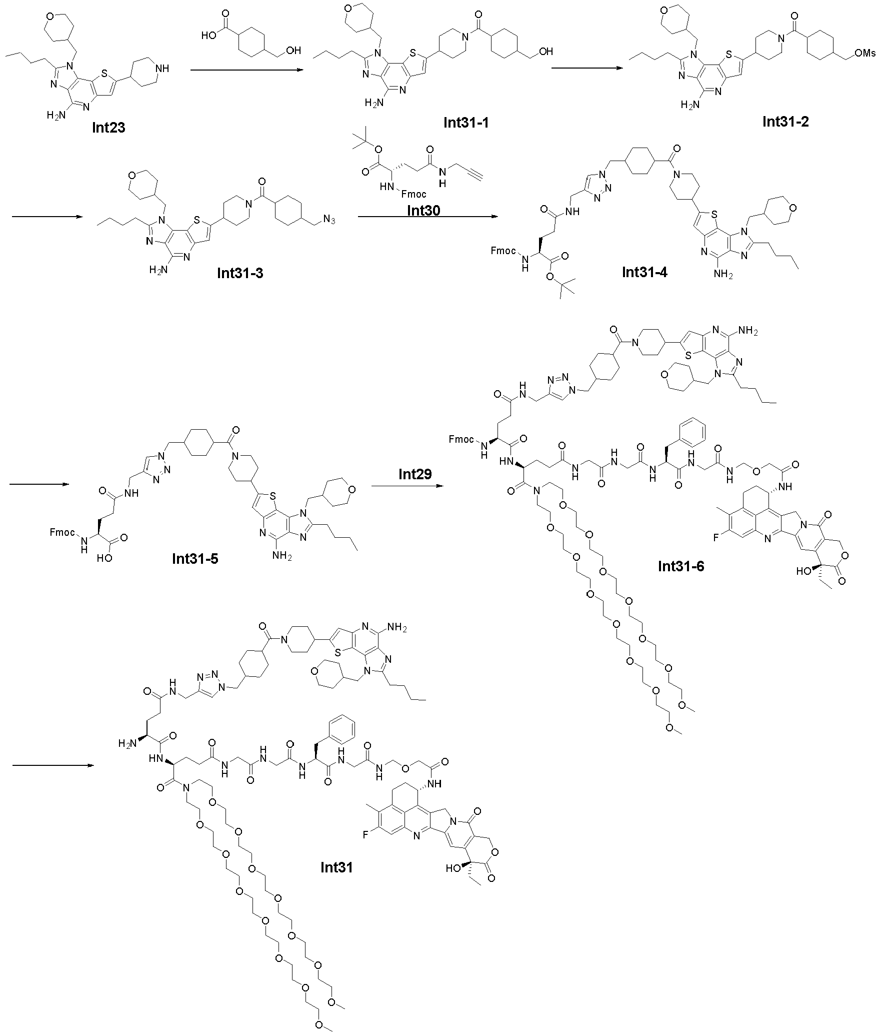

### Step 1: Preparation of Compound Int31-1

4-Hydroxymethyl-1-cyclohexanecarboxylic acid (125.94 mg, 788.14 µmol), *N*,*N*-diisopropylethylamine (292.42 mg, 2.26 mmol, 373.94 µL), 2-(7-azobenzotriazole)-*N*,*N*,*N*',*N*'-tetramethylurea hexafluorophosphate (315.45 mg, 788.14 µmol) and Compound **Int23** (350 mg, 754.2 µmol) were added to anhydrous N,N-dimethylformamide (4 mL), and reacted at 25°C for 3 hours. The reaction solution was directly purified by reversed-phase column chromatography (preparation method B, elution gradient: A%=35% to 45%) to obtain the title compound of this step (380 mg, yield: 93.4%).

MS m/z (ESI): 568.3 [M+H]⁺.

### Step 2: Preparation of Compound Int31-2

Compound **Int31-1** (325 mg, 503.19 µmol) was dissolved in anhydrous pyridine (5 mL), and methylsulfonyl chloride (65.43 mg, 1006.39 µmol) was added in batches within 1 hour. After the addition, the reaction was carried out at 25°C for 3 hours. The reaction solution was concentrated, then diluted with *N*,*N*-dimethylformamide (4 mL), and directly purified by reversed-phase column chromatography (preparation method B, elution gradient: A%=60% to 70%) to obtain the title compound of this step (325 mg, yield: 82.5%).

MS m/z (ESI): 646.3 [M+H]⁺.

### Step 3: Preparation of Compound Int31-3

Compound **Int31-2** (325 mg, 503.19 µmol) was dissolved in anhydrous *N*,*N*-dimethylformamide (5 mL), then added with sodium azide (65.43 mg, 1006.39 µmol), and reacted at 80°C for 4 hours. The reaction solution was added with water (20 mL), extracted with ethyl acetate (20 mL*3) and washed with saturated brine, the organic phase was dried over anhydrous magnesium sulfate and filtered, and the filtrate was concentrated to obtain the title compound of this step (260 mg, yield: 91.9%).

MS m/z (ESI): 593.3 [M+H]⁺.

### Step 4: Preparation of Compound Int31-4

Compound **Int31-3** (230.69 mg, 389.16 µmol), Compound **Int30** (180 mg, 389.16 µmol), sodium ascorbate (154.19 mg, 778.32 µmol) and anhydrous copper sulfate (291.5 mg, 1170 µmol) were added to a mixed solvent of water (0.25 mL), dimethylsulfoxide (2 mL) and tetrahydrofuran (1 mL) and reacted at 0°C for 3 hours. The residue obtained by concentrating the reaction solution was purified by reverse-phase column chromatography (preparation method B, elution gradient: A%=25% to 35%) to obtain the title compound of this step (405 mg, yield: 98.8%).

MS m/z (ESI): 1055.5 [M+H]⁺.

### Step 5: Preparation of Compound Int31-5

Compound **Int31-4** (190 mg, 180.04 µmol) was dissolved in a mixed solvent of anhydrous dichloromethane (6 mL) and trifluoroacetic acid (2 mL) and reacted at 25°C for 2 hours. The residue obtained by concentrating the reaction solution was purified by reverse-phase column chromatography (preparation method B, elution gradient: A%=20% to 30%) to obtain the title compound of this step (150 mg, yield: 84.7%).

MS m/z (ESI): 999.6 [M+H]⁺.

### Step 6: Preparation of Compound Int31-6

Compound **Int31-5** (24.77 mg, 24.79 µmol), *N*,*N*-diisopropylethylamine (4.16 mg, 32.22 µmol), 2-(7-azobenzotriazole)-*N*,*N*,*N*',*N*'-tetramethylurea hexafluorophosphate (11.31 mg, 29.75 µmol) and Compound **Int29** (40 mg, 24.79 µmol) were added to anhydrous *N*,*N*-dimethylformamide (3 mL), and reacted at 0°C for 2 hours. The reaction solution was directly purified by reversed-phase column chromatography (preparation method B, elution gradient: A%=35% to 45%) to obtain the title compound of this step (50 mg, yield: 81.8%).

MS m/z (ESI): 1297.6 [M/2+H]⁺.

### Step 7: Preparation of Compound Int31

Compound **Int31-6** (50 mg, 19.26 µmol) and diethylamine (6.76 mg, 91.52 µmol) were added to *N*,*N*-dimethylformamide (2 mL) and reacted at 25°C for 2 hours. The reaction solution was directly purified by reversed-phase column chromatography (preparation method B, elution gradient: A%=35% to 45%) to obtain the title compound of this step (25 mg, yield: 57.6%).

MS m/z (ESI): 1186.6 [M/2+H]⁺.

### Intermediate Preparation Example 32: Preparation of Compound Int32

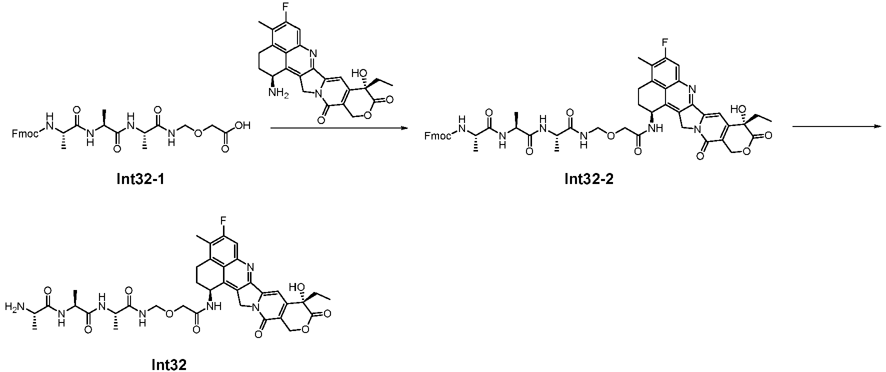

### Step 1: Preparation of Compound Int32-2

Compound **Int32-1** (75 mg, 138.75 µmol), *N,N*-diisopropylethylamine (26.9 mg, 208.12 µmol), 2-(7-azobenzotriazole)-*N,N,N',N'*-tetramethylurea hexafluorophosphate (65.58 mg, 180.36 µmol) and exatecan mesylate (73.75 mg, 138.75 µmol) were added to anhydrous *N,N*-dimethylformamide (5 mL), and reacted at 0°C for 2 hours. The reaction solution was directly purified by reversed-phase column chromatography (preparation method B, elution gradient: A%=25% to 35%) to obtain the title compound of this step (115 mg, yield: 91.1%).

MS m/z (ESI): 958.3 [M+H]⁺.

### Step 2: Preparation of Compound Int32

Compound **Int32-2** (115 mg, 120.04 µmol) and diethylamine (42.13 mg, 570.2 µmol) were added to *N,N*-dimethylformamide (4 mL) and reacted at 25°C for 2 hours. The reaction solution was directly purified by reversed-phase column chromatography (preparation method B, elution gradient: A%=20% to 30%) to obtain the title compound of this step (75 mg, yield: 89.4%).

MS m/z (ESI): 736.3 [M+H]⁺.

### Intermediate Preparation Example 33: Preparation of Compound Int33

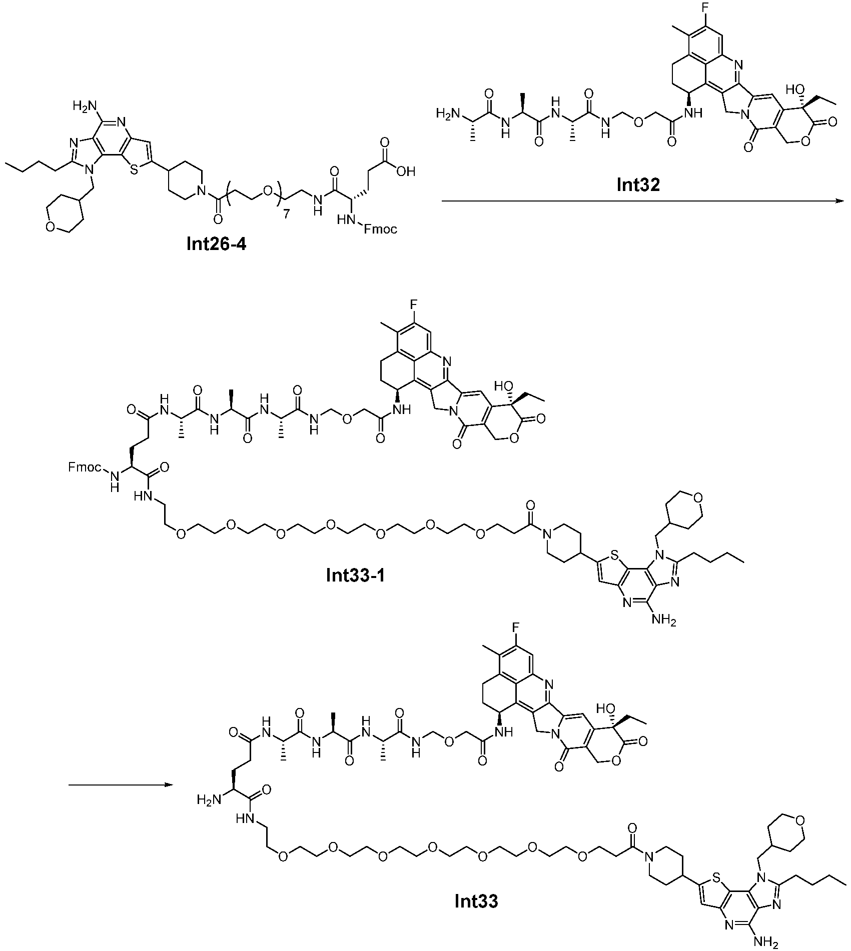

### Step 1: Preparation of Compound Int33-1

Compound **Int26-4** (75.54 mg, 61.78 µmol), *N,N*-diisopropylethylamine (15.97 mg, 123.56 µmol), 2-(7-azobenzotriazole)-*N,N,N',N'*-tetramethylurea hexafluorophosphate (30.54 mg, 80.32 µmol) and Compound **Int32** (50 mg, 67.96 µmol) were added to anhydrous *N,N*-dimethylformamide (3 mL), and reacted at 0°C for 2 hours. The reaction solution was directly purified by reversed-phase column chromatography (preparation method B, elution gradient: A%=25% to 35%) to obtain the title compound of this step (85 mg, yield: 77.2%).

MS m/z (ESI): 1875.9 [M+H]⁺.

### Step 2: Preparation of Compound Int33

Compound **Int33-1** (85 mg, 45.3 µmol) and diethylamine (15.9 mg, 215.2 µmol) were added to *N,N*-dimethylformamide (2 mL) and reacted at 25°C for 2 hours. The reaction solution was directly purified by reversed-phase column chromatography (preparation method B, elution gradient: A%=45% to 55%) to obtain the title compound of this step (70 mg, yield: 98.3%).

MS m/z (ESI): 1653.8 [M+H]⁺.

### Intermediate Preparation Example 34: Preparation of Compound Int34

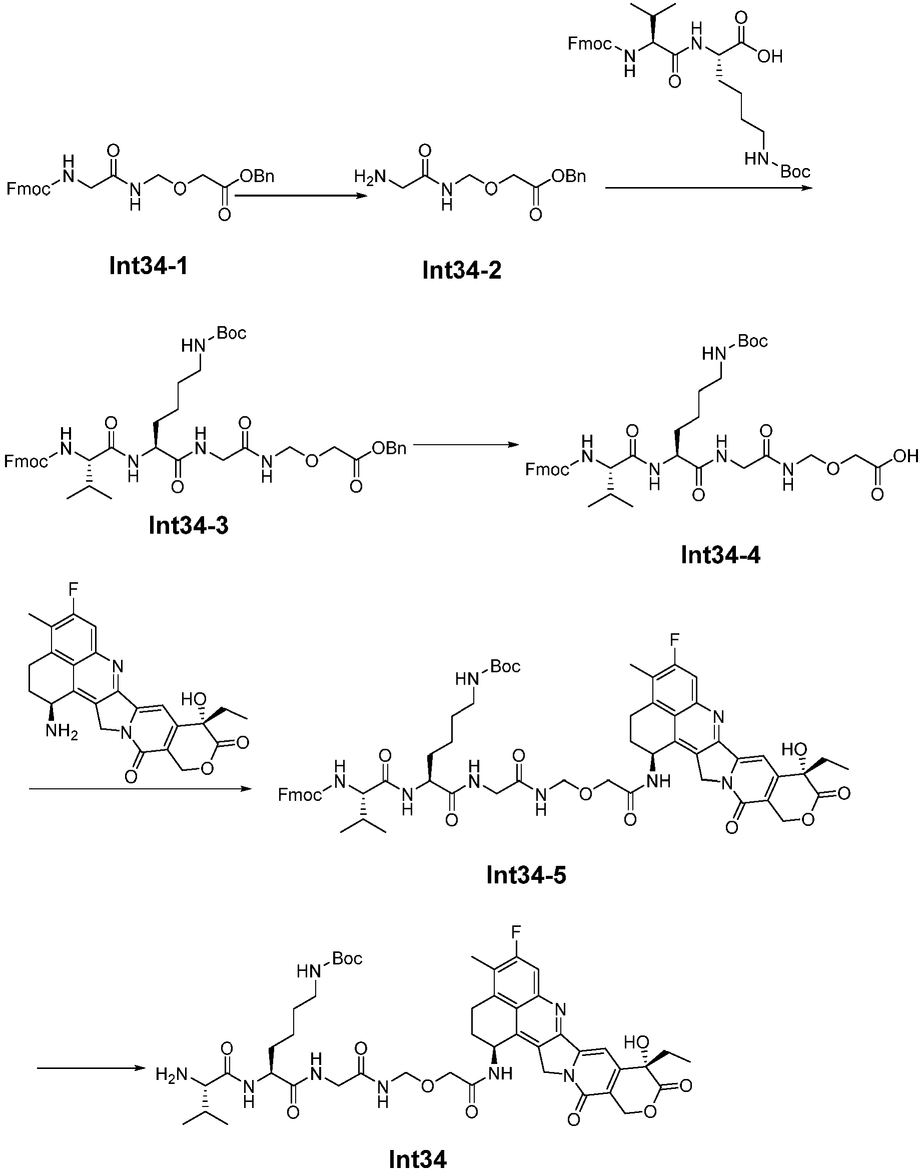

### Step 1: Preparation of Compound Int34-2

Compound **Int34-1** (800 mg, 1.67 mmol) and piperidine (287.11 mg, 3.34 mmol) were added to *N,N*-dimethylformamide (15 mL) and reacted at room temperature for 2 hours. After the reaction solution was concentrated, methyl tert-butyl ether (20 mL) was added, treated with ultrasonic for 5 minutes, allowed to stand, and filtered, and the solid was dried to obtain the title compound of this step (315 mg, yield: 73.47%).

MS m/z (ESI): 253.1 [M+H]⁺.

### Step 2: Preparation of Compound Int34-3

Compound **Int34-2** (90 mg, 321.09 µmol), *N*²-((((9*H*-fluoren-9-yl)methoxy)carbonyl)-*L*-valine)-*N⁶*-(tert-butoxycarbonyl)-*L*-lysine (202.53 mg, 321.09 µmol), *N,N*-diisopropylethylamine (104.60 mg, 802.72 µmol), 1-hydroxybenzotriazole (49.23 mg, 353.20 µmol) and 1-(3-dimethylaminopropyl)-3-ethylcarbodiimide hydrochloride (77.43 mg, 401.36 µmol) were added to N,N-dimethylformamide (3 mL) and reacted at room temperature for 3 hours. The reaction solution was directly purified by reversed-phase column chromatography (preparation method B, elution gradient: A%=50% to 55%) to obtain the title compound of this step (142 mg, yield: 47.43%).

MS m/z (ESI): 802.4 [M+H]⁺.

### Step 3: Preparation of Compound Int34-4

Compound **Int34-3** (142 mg, 159.37 µmol), ammonium formate (50.75 mg, 796.84 µmol) and 10% palladium on carbon (15 mg) were added to anhydrous methanol (4 mL) and reacted at room temperature for 3 hours. The reaction solution was directly purified by reversed-phase column chromatography (preparation method B, elution gradient: A%=30%-35%) to obtain the title compound of this step (101 mg, yield: 80.13%).

MS m/z (ESI): 712.4 [M+H]⁺.

### Step 4: Preparation of Compound Int34-5

Compound **Int34-4** (95 mg, 133.46 µmol),exatecan mesylate (70.94 mg, 133.46 µmol), *N,N*-diisopropylethylamine (43.04 mg, 333.66 µmol) and bis(2-oxo-3-oxazolidinyl)phosphonic chloride (50.85 mg, 200.20 µmol) were added to *N,N*-dimethylformamide (1 mL) and reacted at room temperature for 3 hours. The reaction solution was directly purified by reversed-phase column chromatography (preparation method B, elution gradient: A%=45% to 55%) to obtain the title compound of this step (105 mg, yield: 69.67%).

MS m/z (ESI): 1129.5 [M+H]⁺.

### Step 5: Preparation of Compound Int34

Compound **Int34-5** (105 mg, 92.98 µmol) and piperidine (15.83 mg, 185.97 µmol) were added to *N,N*-dimethylformamide (1 mL) and reacted at room temperature for 2 hours. The reaction solution was directly purified by reversed-phase column chromatography (preparation method A, elution gradient: A%=27% to 33%) to obtain the title compound of this step (46 mg, yield: 54.54%).

MS m/z (ESI): 907.4 [M+H]⁺.

### Intermediate Preparation Example 35: Preparation of Compound Int35

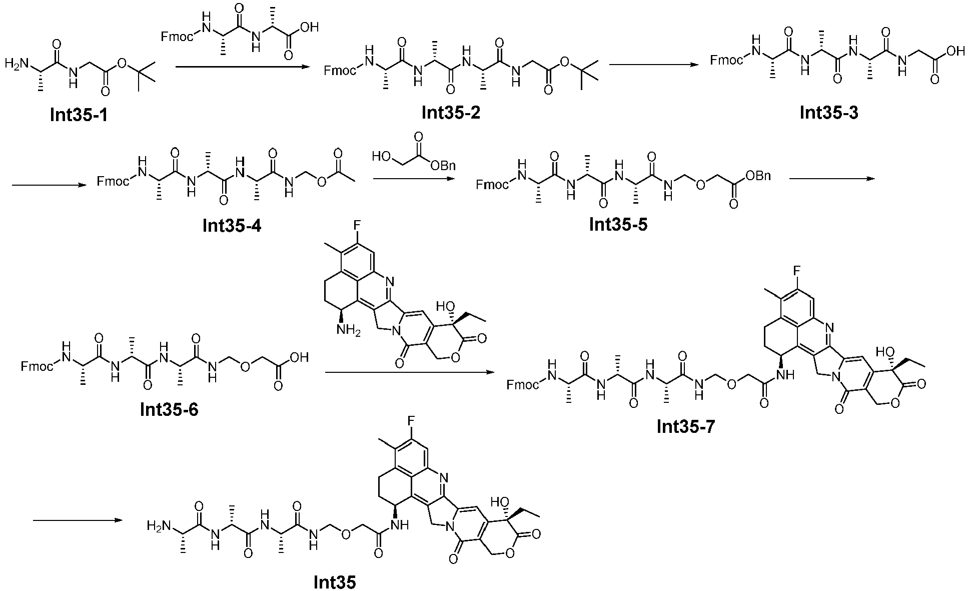

### Step 1: Preparation of Compound Int35-2

(((9*H*-Fluoren-9-yl)methoxy)carbonyl)-*L*-alanine-*D*-alanine (537.38 mg, 1.33 mmol), *N,N*-diisopropylethylamine (345.07 mg, 3.67 µmol), 2-(7-azobenzotriazole)-*N,N,N',N'-* tetramethylurea hexafluorophosphate (641.18 mg, 16.84 mmol) and Compound **Int35-1** (300 mg, 1.33 mmol) were added to anhydrous *N,N-*dimethylformamide (6 mL) and reacted at 0°C for 2 hours. The reaction solution was directly purified by reversed-phase column chromatography (preparation method B, elution gradient: A%=45% to 55%) to obtain the title compound of this step (400 mg, yield: 52.9%).

MS m/z (ESI): 567.3 [M+H]⁺.

### Step 2: Preparation of Compound Int35-3

Compound **Int35-2** (400 mg, 635.32 µmol) was dissolved in dioxane hydrochloride solution (4 mL, 4M) and reacted at 25°C for 3 hours. The reaction solution was concentrated to obtain the title compound of this step (310 mg, yield: 95.6%).

MS m/z (ESI): 511.2 [M+H]⁺.

### Step 3: Preparation of Compound Int35-4

Under nitrogen protection, Compound **Int35-3** (350 mg, 651.27 µmol), lead tetraacetate (437.51 mg, 976.91 µmol), copper acetate (13.13 mg, 64.13 µmol) and glacial acetic acid (59.26 mg, 976.91 µmol) were added in sequence to anhydrous *N,N*-dimethylformamide (6 mL), and reacted at 60°C for 0.5 hours. The reaction solution was directly purified by reversed-phase column chromatography (preparation method B, elution gradient: A%=45% to 55%) to obtain the title compound of this step (285 mg, yield: 83.4%).

MS m/z (ESI): 547.2 [M+Na]⁺.

### Step 4: Preparation of Compound Int35-5

Compound **Int35-4** (280 mg, 533.76 µmol) and benzyl glycolate (373.47 mg, 2.14 mmol) were added to a mixed solvent of dichloromethane (6 mL) and trifluoroacetic acid (2 mL), and reacted at 25°C for 0.5 hours. The crude product obtained by concentrating the reaction solution was purified by silica gel column chromatography (eluent: dichloromethane/methanol = 7/3 (v/v)) to obtain the title compound of this step (30 mg, yield: 98.1%).

MS m/z (ESI): 653.3 [M+Na]⁺.

### Step 5: Preparation of Compound Int35-6

Compound **Int35-5** (160 mg, 237.84 µmol), 10% palladium on carbon (28.89 mg) and ammonium acetate (89.99 mg, 1430 µmol) were added to methanol (6 mL) and reacted at 25°C for 3 hours. The reaction solution was filtered through diatomaceous earth, and the filtrate was concentrated to obtain the title compound of this step (30 mg, yield: 23.3%).

MS m/z (ESI): 541.2 [M+H]⁺.

### Step 6: Preparation of Compound Int35-7

Compound **Int35-6** (30 mg, 55.49 µmol), *N,N*-diisopropylethylamine (11.09 mg, 85.84 µmol), 2-(7-azobenzotriazole)-*N,N,N',N'*-tetramethylurea hexafluorophosphate (22.85 mg, 60.08 µmol) and exatecan mesylate (32.45 mg, 61.05 µmol) were added to anhydrous *N,N*-dimethylformamide (3 mL), and reacted at 0°C for 2 hours. The reaction solution was directly purified by reversed-phase column chromatography (preparation method B, elution gradient: A%=45% to 55%) to obtain the title compound of this step (45 mg, yield: 89.1%).

MS m/z (ESI): 958.4 [M+H]⁺.

### Step 7: Preparation of Compound Int35

Compound **Int35-7** (39.8 mg, 41.55 µmol) and diethylamine (15.35 mg, 207.74 µmol) were added to *N,N*-dimethylformamide (2 mL) and reacted at 25°C for 2 hours. The reaction solution was directly purified by reversed-phase column chromatography (preparation method B, elution gradient: A%=55% to 65%) to obtain the title compound of this step (30 mg, yield: 98.1%).

MS m/z (ESI): 736.2 [M+H]⁺.

### Example1-1: Preparation of Compound C-1

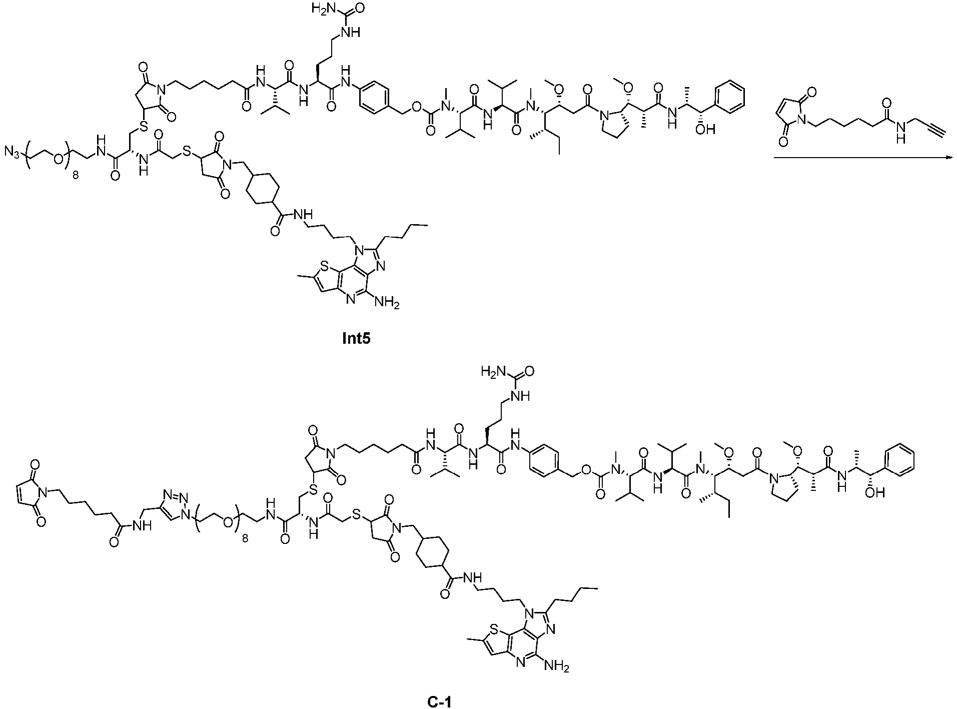

Compound **Int5** (50 mg, 0.02 mmol) was dissolved in DMSO (6 mL) and water (0.6 mL), added with 6-(2,5-dioxo-2,5-dihydro-1*H*-pyrrol-1-yl)-*N*-(prop-2-yn-1-yl)hexanoamide (5.99 mg, 0.024 mmol), sodium ascorbate (5.94 mg, 0.03 mmol), and anhydrous copper sulfate (6.4 mg, 0.04 mmol) under nitrogen protection, stirred at room temperature for 1 hour, and added with water and ethyl acetate for extraction, the organic phase was dried and concentrated to obtain a crude product that was purified by preparative high performance liquid chromatography to obtain the title compound of this step (15 mg, yield: 27.2%).

MS m/z (ESI): 1365.7 [M/2+H]⁺.

### Example1-2: Preparation of Compound C-2

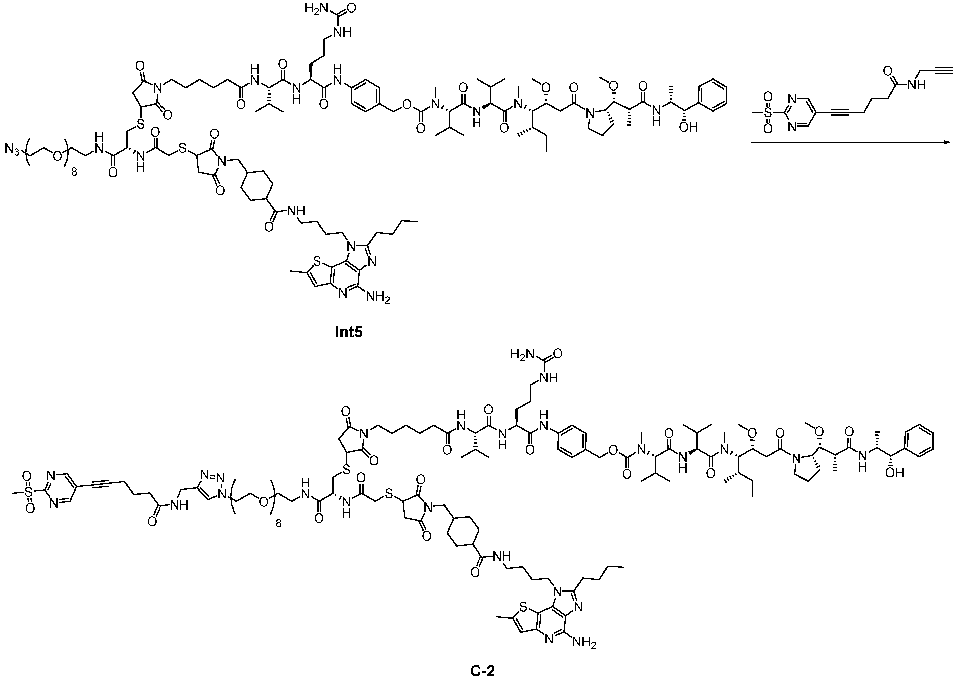

The synthetic route of Example 1-1 was adopted. The reaction raw material 6-(2,5-dioxo-2,5-dihydro-1*H*-pyrrol-1-yl)-*N*-(prop-2-yn-1-yl)hexanamide was replaced with 6-(2-(methylsulfonyl)pyrimidin-5-yl)-*N*-(prop-2-yn-1-yl)hexa-5-ynamide to obtain the title compound of this step (3 mg, yield: 5.6%).

MS m/z (ESI): 1394.2 [M/2+H]⁺.

### Example1-3: Preparation of Compound C-3

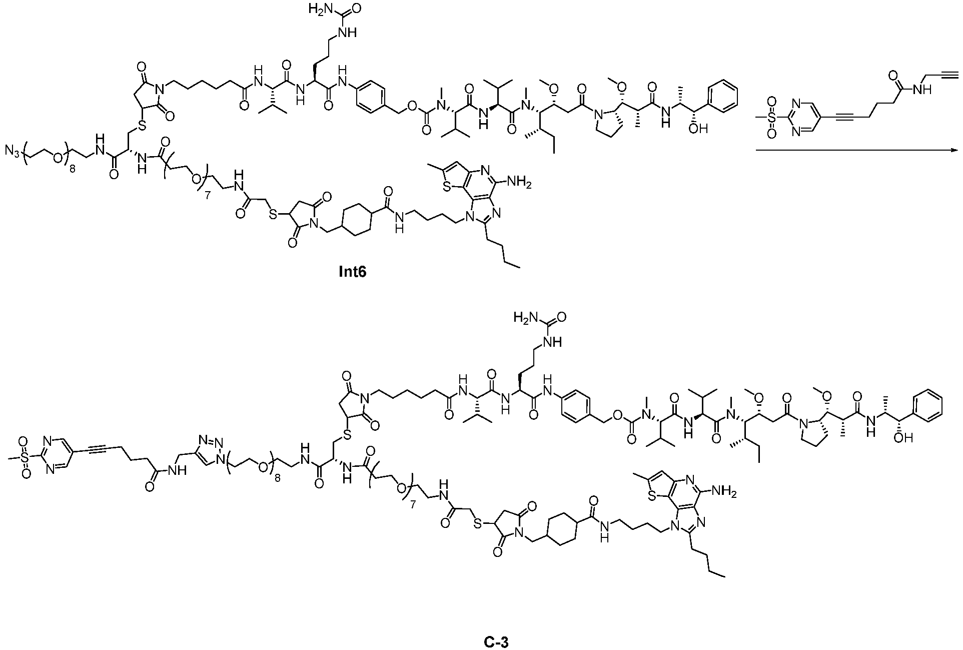

The synthetic route of Example 1-1 was adopted. The reaction raw material Compound **Int5** was replaced with Compound **Int6,** and 6-(2,5-dioxo-2,5-dihydro-1*H*-pyrrol-1-yl)-*N*-(prop-2-yn-1-yl) hexanamide was replaced with 6-(2-(methylsulfonyl)pyrimidin-5-yl)-*N*-(prop-2-yn-1-yl) hexa-5-ynamide to obtain the title compound of this step (5 mg, yield: 8%).

MS m/z (ESI): 1583.8 [M/2+H]⁺.

### Example1-4: Preparation of Compound C-4

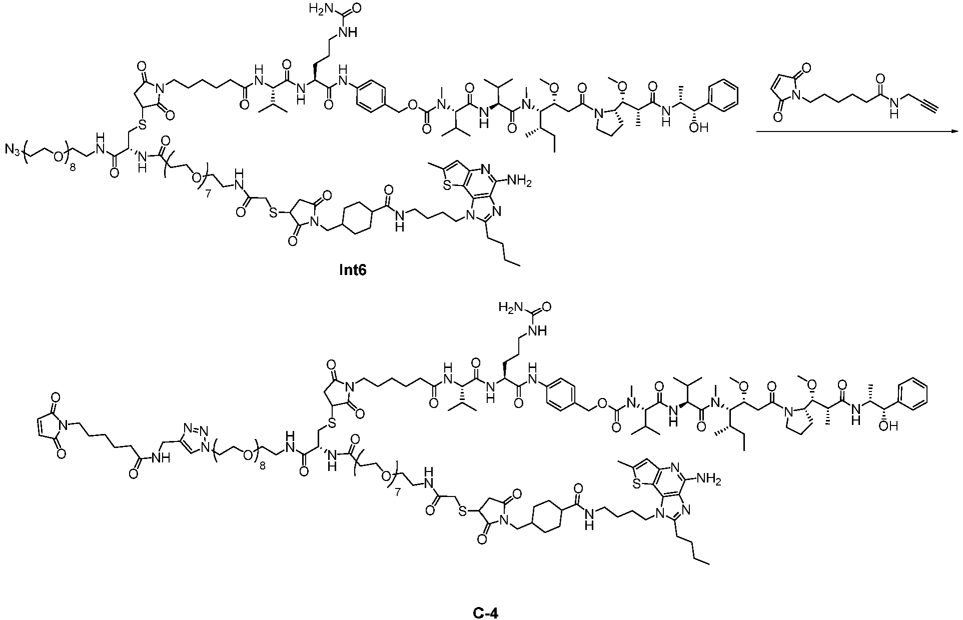

The synthetic route of Example 1-1 was adopted, and the reaction raw material Compound Int5 was replaced with Compound Int6 to obtain the title compound of this step (10 mg, yield: 6%).

MS m/z (ESI): 1555.3 [M/2+H]⁺.

### Example1-5: Preparation of Compound C-5

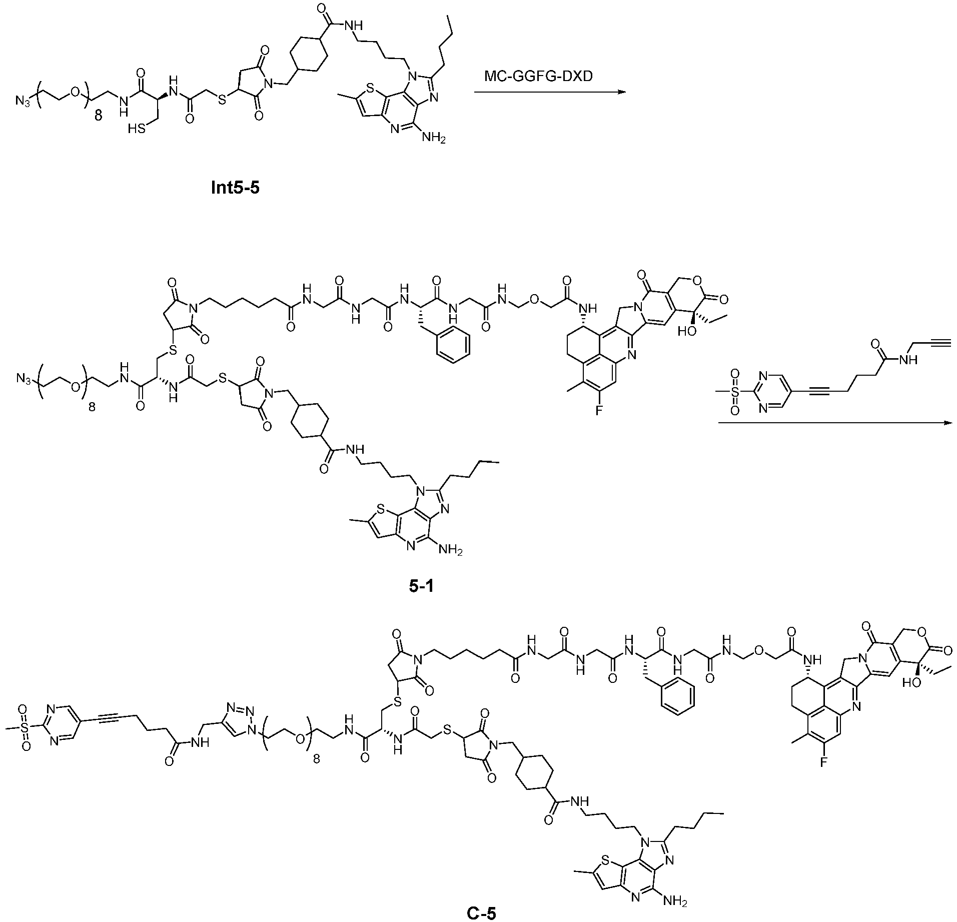

### Step 1: Preparation of Compound 5-1

The synthetic route of Compound Int5 was adopted, and the reaction raw material MC-VC-PABC-MMAE was replaced with MC-GGFG-Dxd to obtain the title compound of this step (30 mg, yield: 21.3%).

MS m/z (ESI): 1100.5 [M/2+H]⁺.

### Step 2: Preparation of Compound C-5

Compound **5-1** (30 mg, 0.014 mmol) was dissolved in DMSO (3 mL) and water (0.3 mL), added with 6-(2-(methylsulfonyl)pyrimidin-5-yl)-*N*-(prop-2-yn-1-yl)hexa-5-ynamide (4.99 mg, 0.016 mmol), sodium ascorbate (4.16 mg, 0.021 mmol) and anhydrous copper sulfate (4.5 mg, 0.028 mmol) under nitrogen protection, stirred at room temperature for 1 hour, and added with water and ethyl acetate for extraction, the organic phase was dried and concentrated to obtain a crude product that was purified by preparative high-performance liquid chromatography to obtain the title compound of this step (3 mg, yield: 8.8%).

MS m/z (ESI): 1252.0 [M/2+H]⁺.

### Example1-6: Preparation of Compound C-6

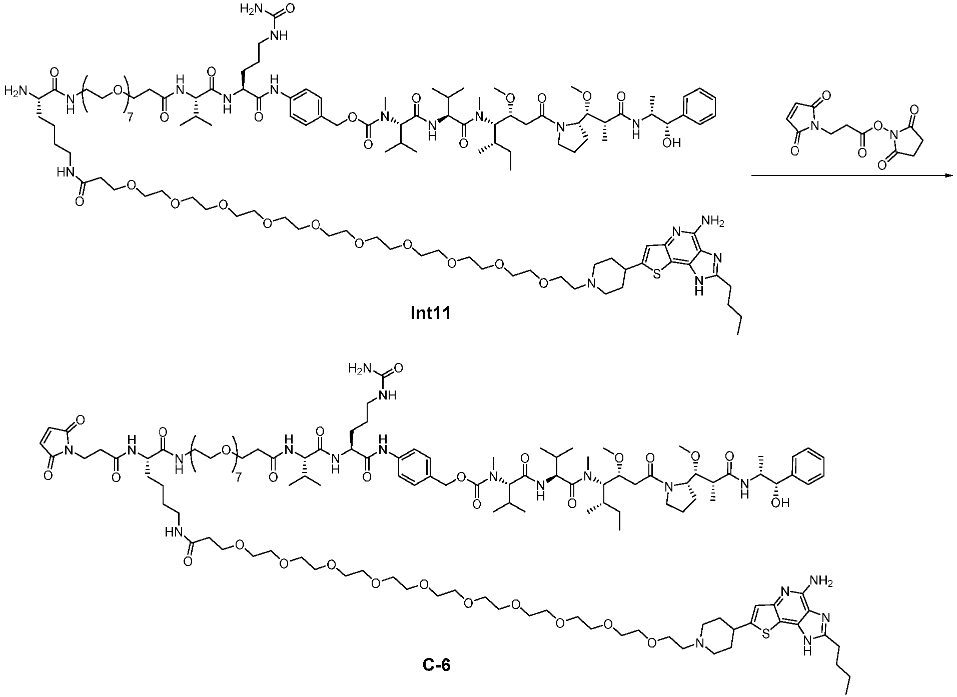

Compound **Int11** (20 mg, 7.74 µmol),3-maleimidopropionic acid hydroxysuccinimide ester (2.17 mg, 7.74 µmol) and *N,N*-diisopropylethylamine (1.52 mg, 11.61 µmol) were added in sequence to anhydrous *N,N*-dimethylformamide (1.5 mL), and reacted at 25°C for 3 hours. The reaction solution was purified by preparative high-performance liquid chromatography to obtain the title compound of this step (0.64 mg, yield: 3%).

MS m/z (ESI): 1303.3 [M/2+H]⁺.

### Example1-7: Preparation of Compound C-7

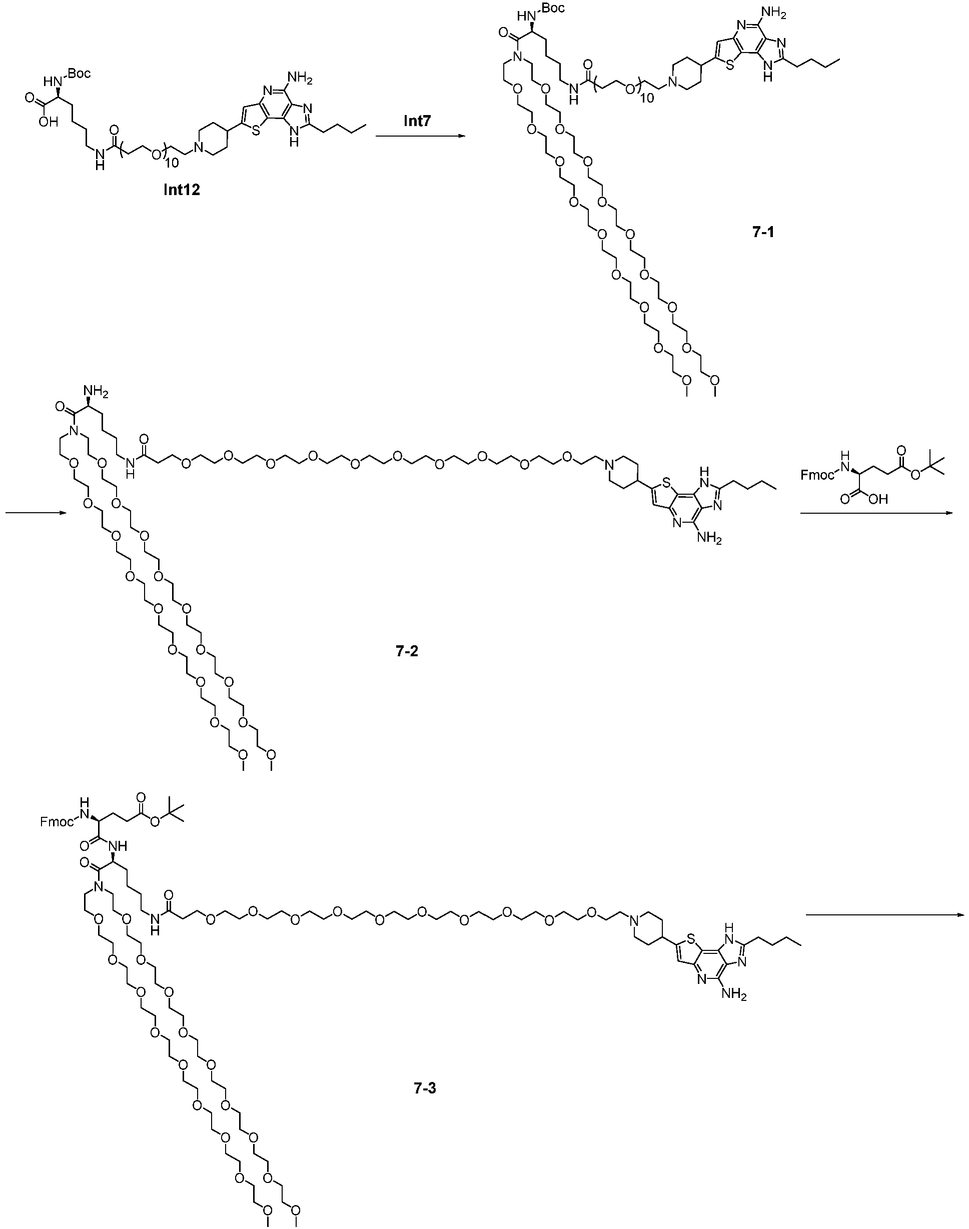

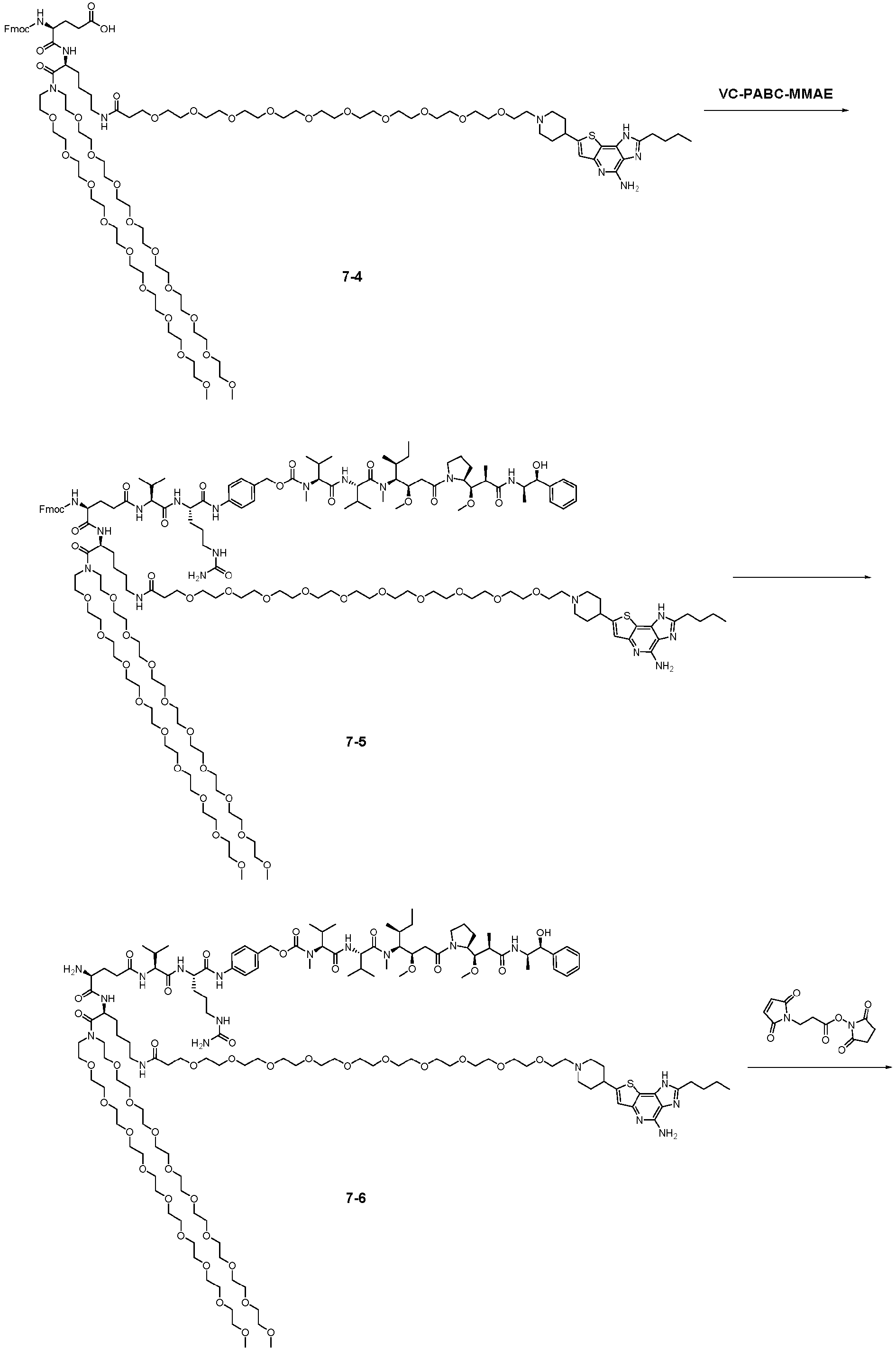

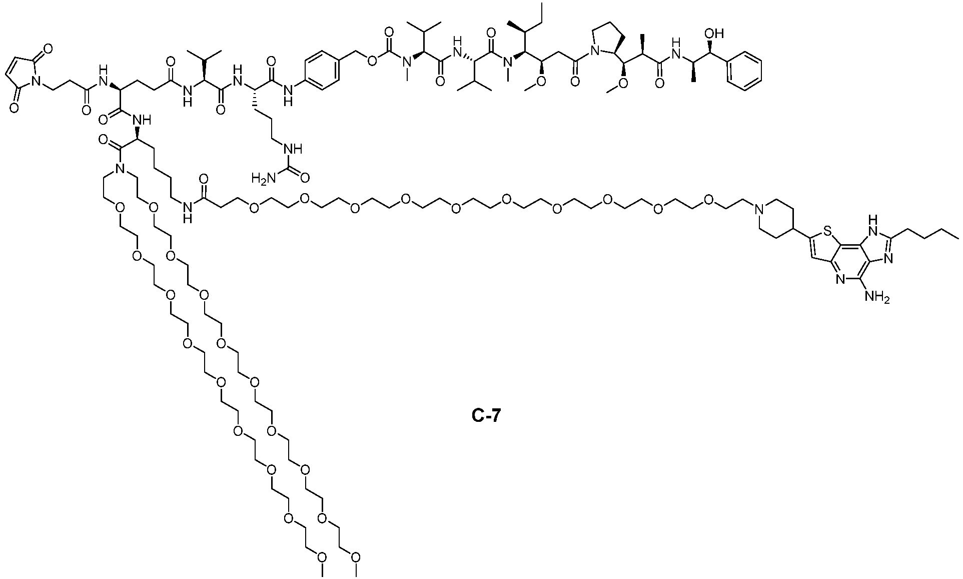

### Step 1: Preparation of Compound 7-1

Compound **Int12** (115 mg, 102.07 µmol) and Compound **Int7** (135.06 mg, 153.11 µmol) were dissolved in anhydrous *N,N-*dimethylformamide (4 mL), added with *N,N-*diisopropylethylamine (27.77 mg, 204.14 µmol) and 2-(7-azobenzotriazole)-*N,N,N',N'*-tetramethylurea hexafluorophosphate (61.29 mg, 153.11 µmol) in sequence, and reacted at 0°C for 1 hour. The crude product obtained by concentrating the reaction solution was purified by reverse-phase column chromatography (preparation method A, elution gradient: A%=40%-50%) to obtain the title compound of this step (125 mg, yield: 58.3%).

MS m/z (ESI): 945.6 [M/2+H]⁺.

### Step 2: Preparation of Compound 7-2

Compound **7-1** (125 mg, 59.51 µmol) was dissolved in hydrogen chloride in 1,4-dioxane solution (2 mL, 4M) under nitrogen protection, and reacted at 25°C for 2 hours. The residue obtained by concentrating the reaction solution was dissolved in dichloromethane (15 mL) and concentrated again under reduced pressure to remove the solvent, and this was repeated three times to obtain the title compound of this step (140 mg, yield: 90.4%).

MS m/z (ESI): 895.6 [M/2+H]⁺.

### Step 3: Preparation of Compound 7-3

Compound **7-2** (95 mg, 50.41 µmol), (*S*)-2-((((9*H*-fluoren-9-yl)methoxy)carbonyl)amino)-5-(tert-butoxy)-5-oxopentanoic acid (135.06 mg, 153.11 µmol), *N,N*-diisopropylethylamine (13.72 mg, 100.82 µmol) and 2-(7-azobenzotriazole)*-N,N,N',N*'-tetramethylurea hexafluorophosphate (30.27 mg, 75.62 µmol) were added in sequence to anhydrous *N,N-*dimethylformamide (4 mL), and reacted at 0°C for 1 hour. The reaction solution was directly purified by reversed-phase column chromatography (preparation method A, elution gradient: A%=40%-50%) to obtain the title compound of this step (120 mg, yield: 97.5%).

MS m/z (ESI): 1099.0 [M/2+H]⁺.

### Step 4: Preparation of Compound 7-4

Compound **7-3** (110 mg, 45.05 µmol) and trifluoroacetic acid (2 mL) were dissolved in dichloromethane (6 mL) and reacted at 25°C for 2 hours. The residue obtained by concentrating the reaction solution was purified by reverse-phase column chromatography (preparation method B, elution gradient: A%=25% to 35%) to obtain the title compound of this step (76 mg, yield: 74.8%).

MS m/z (ESI): 1071.1 [M/2+H]⁺.

### Step 5: Preparation of Compound 7-5

Compound **7-4** (60 mg, 26.62 µmol), VC-PABC-MMAE (43.19 mg, 34.60 µmol), *N,N*-diisopropylethylamine (5.21 mg, 39.92 µmol) and 2-(7-azobenzotriazole)-*N,N,N',N'*-tetramethylurea hexafluorophosphate (14.31 mg, 37.26 µmol) were dissolved in anhydrous *N,N-*dimethylformamide (2 mL), and reacted at 0°C for 1 hour. The reaction solution was directly purified by reversed-phase column chromatography (preparation method B, elution gradient: A%=35% to 45%) to obtain the title compound of this step (65 mg, yield: 71.5%).

MS m/z (ESI): 1623.5 [M/2+H]⁺.

### Step 6: Preparation of Compound 7-6

Compound **7-5** (65 mg, 19.02 µmol) and piperidine (3.27 mg, 129.16 µmol) were added to *N,N*-dimethylformamide (2 mL) in sequence, and reacted at 25°C for 2 hours. The reaction solution was directly purified by reversed-phase column chromatography (preparation method A, elution gradient: A%=25% to 35%) to obtain the title compound of this step (55.6 mg, yield: 91.8%).

MS m/z (ESI): 1008.6 [M/3+H]⁺.

### Step 7: Preparation of Compound C-7

Compound **7-6** (58 mg, 18.22 µmol),3-maleimidopropionic acid hydroxysuccinimide ester (5.10 mg, 18.22 µmol) and *N,N*-diisopropylethylamine (3.57 mg, 27.32 µmol) were dissolved in anhydrous *N,N*-dimethylformamide (1.5 mL), and reacted at 25°C for 3 hours. The reaction solution was purified by preparative high-performance liquid chromatography to obtain the title compound of this step (28.33 mg, yield: 46.5%).

MS m/z (ESI): 1587.9 [M/2+H]⁺.

### Example1-8: Preparation of Compound C-8

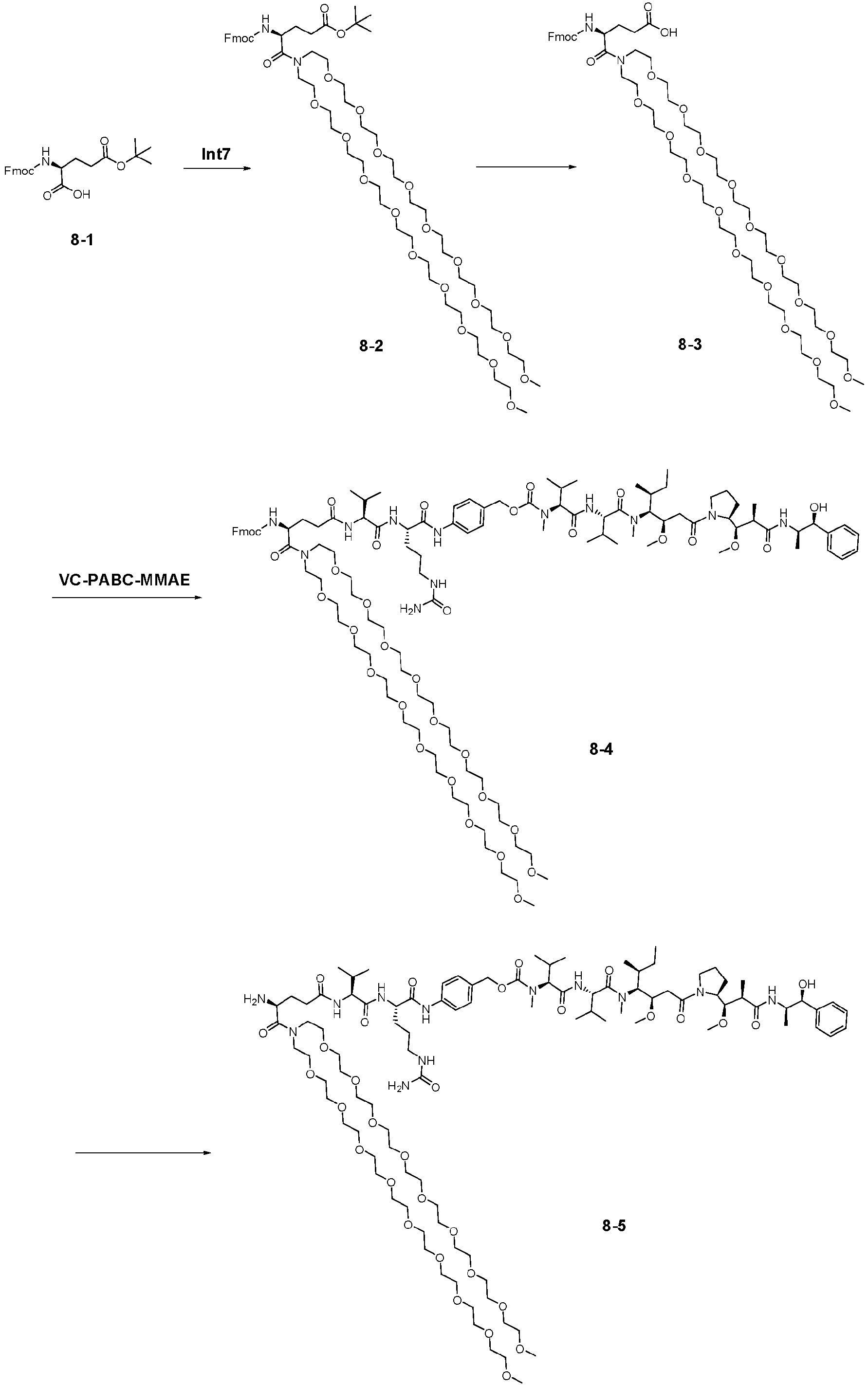

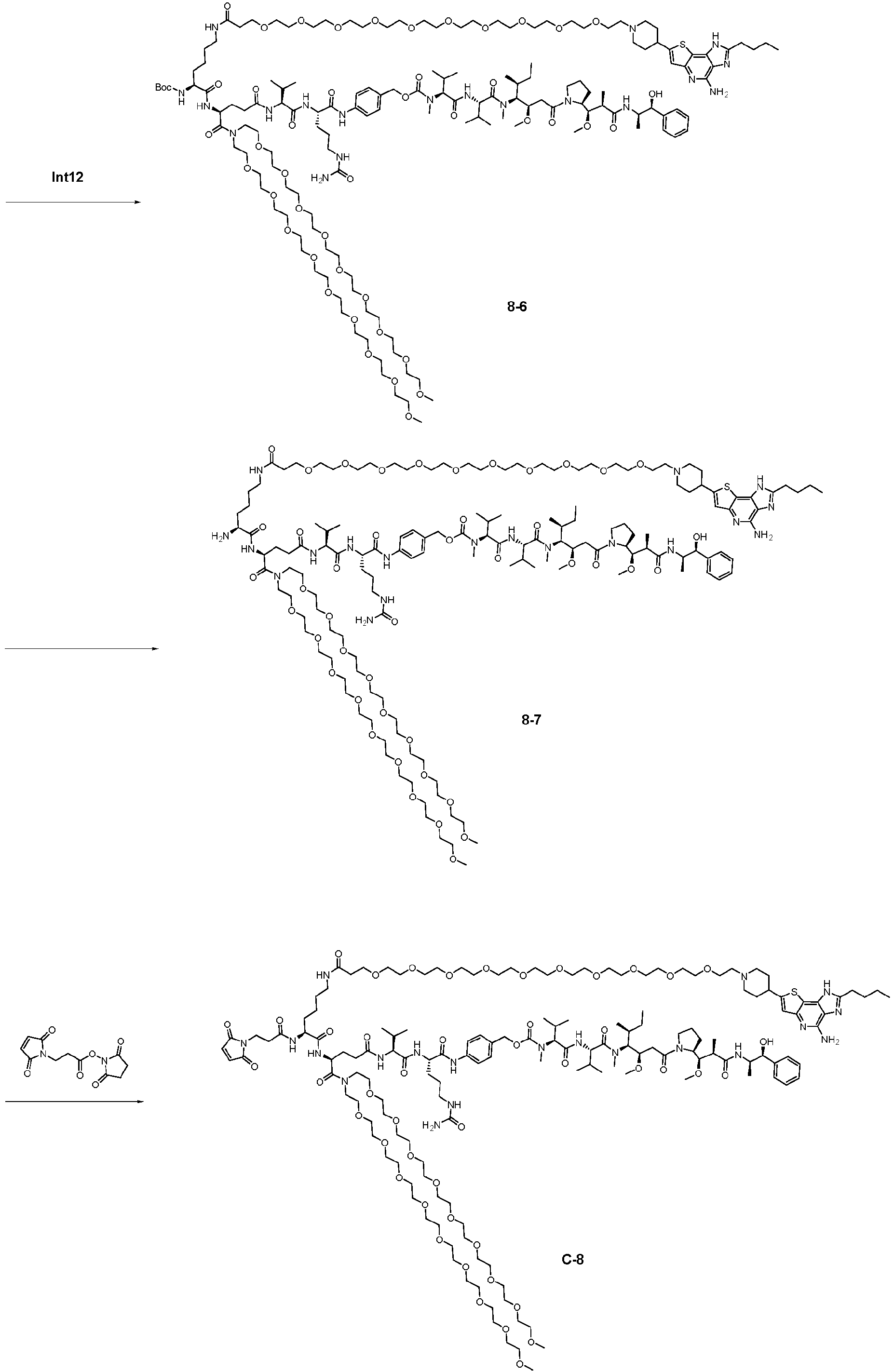

### Step 1: Preparation of Compound 8-2

Compound **Int7** (300 mg, 340.08 µmol), (*S*)-2-((((9*H*-fluoren-9-yl)methoxy)carbonyl) amino)-5-(tert-butoxy)-5-oxopentanoic acid (228.47 mg, 425.47 µmol), *N,N-*diisopropylethylamine (92.53 mg, 680.17 µmol) and 2-(7-azobenzotriazole)-*N,N,N',N'-* tetramethylurea hexafluorophosphate (204.22 mg, 510.13 µmol) were dissolved in anhydrous *N,N*-dimethylformamide (4 mL) in sequence, and reacted at 25°C for 2 hours. The reaction solution was concentrated. The residue obtained was purified by silica gel column chromatography (eluent: dichloromethane/methanol = 9/1 (v/v)) to obtain the title compound of this step (371 mg, yield: 78.8%).

MS m/z (ESI): 1245.7 [M+H]⁺.

### Step 2: Preparation of Compound 8-3

Compound **8-2** (350 mg, 252.91 µmol) and trifluoroacetic acid (2 mL) were added to dichloromethane (6 mL), and reacted at 25°C for 2 hours. The residue obtained by concentrating the reaction solution was purified by reverse-phase column chromatography (preparation method A, elution gradient: A%=35% to 45%) to obtain the title compound of this step (250 mg, yield: 74.8%).

MS m/z (ESI): 1189.6 [M+H]⁺.

### Step 3: Preparation of Compound 8-4

Compound **8-3** (105 mg, 83.87 µmol), VC-PABC-MMAE (110.85 mg, 83.87 µmol), *N,N*-diisopropylethylamine (19.71 mg, 150.96 µmol) and 2-(7-azobenzotriazole)-*N,N,N',N'-*tetramethylurea hexafluorophosphate (45.11 mg, 117.44 µmol) were dissolved in anhydrous *N,N-*dimethylformamide (4 mL) in sequence, and reacted at 0°C for 2 hours. The reaction solution was directly purified by reversed-phase column chromatography (preparation method A, elution gradient: A%=45% to 55%) to obtain the title compound of this step (170 mg, yield: 79.5%).

MS m/z (ESI): 1147.7 [M/2+H]⁺.

### Step 4: Preparation of Compound 8-5

Compound **8-4** (156 mg, 64.58 µmol) and piperidine (11.11 mg, 129.16 µmol) were added to *N,N-*dimethylformamide (3 mL) and reacted at 25°C for 2 hours. The reaction solution was directly purified by reversed-phase column chromatography (preparation method A, elution gradient: A%=35% to 45%) to obtain the title compound of this step (125 mg, yield: 88.7%).

MS m/z (ESI): 1036.7 [M/2+H]⁺.

### Step 5: Preparation of Compound 8-6

Compound **8-5** (120.05mg, 55.03 µmol), Compound **Int12** (62 mg, 55.03 µmol), *N,N*-diisopropylethylamine (10.78 mg, 82.54µmol) and 2-(7-azobenzotriazole)-*N,N,N',N'*-tetramethylurea hexafluorophosphate (25.36 mg, 66.02 µmol) were added in sequence and dissolved in anhydrous *N,N*-dimethylformamide (4 mL), and reacted at 0°C for 1 hour. The reaction solution was directly purified by reversed-phase column chromatography (preparation method B, elution gradient: A%=35% to 45%) to obtain the title compound of this step (170 mg, yield: 89.0%).

MS m/z (ESI): 1562.6 [M/2+H]⁺.

### Step 6: Preparation of Compound 8-7

Compound **8-6** (160 mg, 48.64 µmol) and zinc bromide (46.12 mg, 194.67 µmol) were added to anhydrous dichloromethane (2 mL) in sequence under nitrogen protection and reacted at 25°C for 2 hours. The residue obtained by concentrating the reaction solution was purified by reverse-phase column chromatography (preparation method B, elution gradient: A%=30% to 40%) to obtain the title compound of this step (140 mg, yield: 90.4%).

MS m/z (ESI): 1512.4 [M/2+H]⁺.

### Step 7: Preparation of Compound C-8

Compound **8-7** (80 mg, 125.13 µmol), 3-maleimidopropionic acid hydroxysuccinimide ester (7.04 mg, 25.13 µmol) and *N,N*-diisopropylethylamine (4.92 mg, 37.69 µmol, 6.29 µL) were added to anhydrous *N,N*-dimethylformamide (1.5 mL) in sequence, and reacted at 25°C for 3 hours. The reaction solution was purified by preparative high-performance liquid chromatography to obtain the title compound of this step (19.4 mg, yield: 23.1%).

MS m/z (ESI): 1587.9 [M/2+H]⁺.

### Example1-9: Preparation of Compound C-9

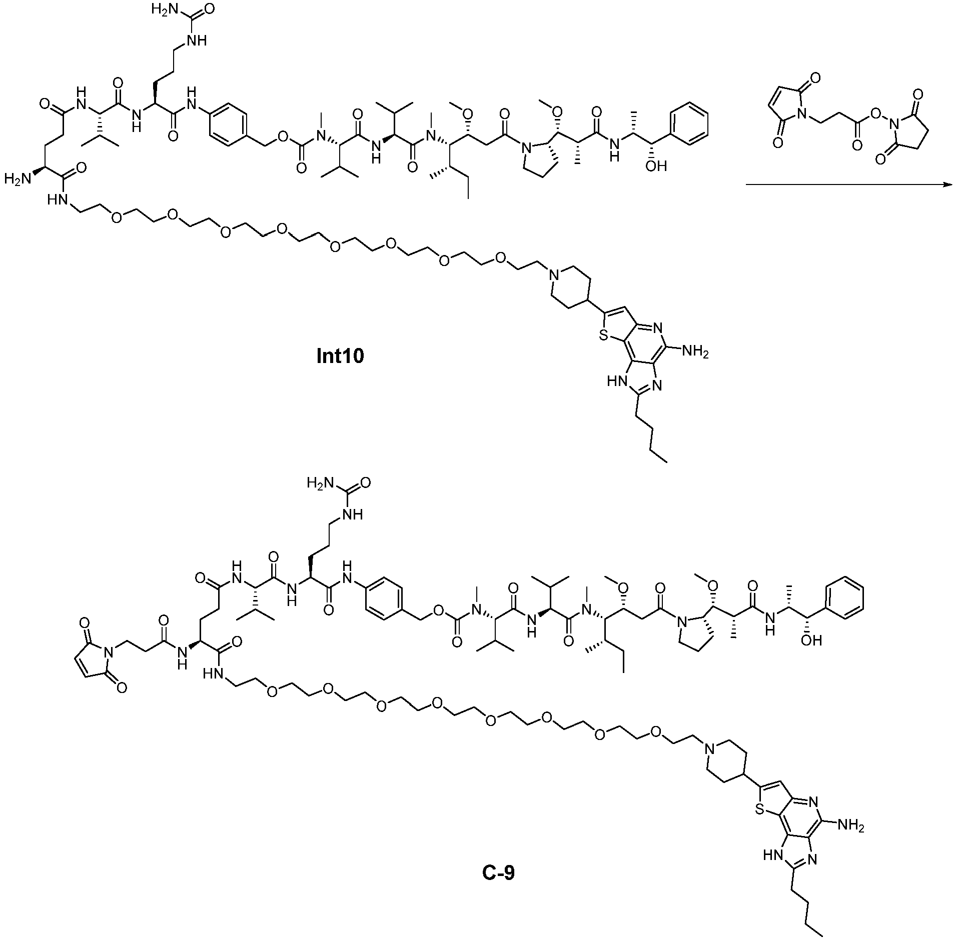

Compound **Int10** (50 mg, 24.24 µmol), 3-maleimidopropionic acid hydroxysuccinimide ester (20.38 mg, 72.72 µmol) and *N,N*-diisopropylethylamine (4.75 mg, 36.36 µmol) were added to anhydrous *N,N*-dimethylformamide (2 mL) in sequence, and reacted at 0°C for 2 hours. The reaction solution was purified by preparative high-performance liquid chromatography to obtain the title compound of this step (11 mg, yield: 20.2%).

MS m/z (ESI): 1055.5 [M/2+H]⁺.

### Example1-10: Preparation of Compound C-10

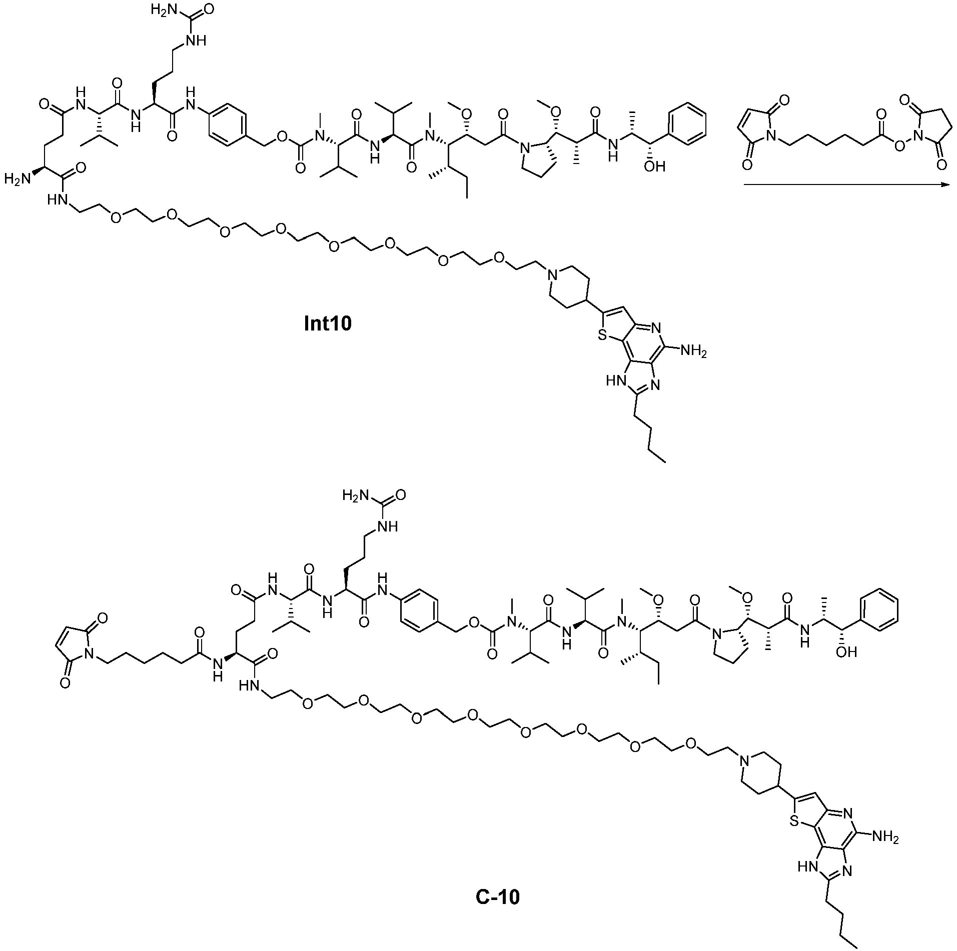

Compound **Int10** (60 mg, 29.09 µmol), 6-(maleimido)hexanoic acid succinimide ester (9.44 mg, 09.09 µmol) and *N,N*-diisopropylethylamine (45.70 mg, 43.63 µmol) were dissolved in anhydrous *N,N-*dimethylformamide (2 mL) in sequence, and reacted at 0°C for 2 hours. The reaction solution was purified by preparative high-performance liquid chromatography to obtain the title compound of this step (11 mg, yield: 16.3%).

MS m/z (ESI): 1076.6 [M/2+H]⁺.

### Example1-11: Preparation of Compound C-11

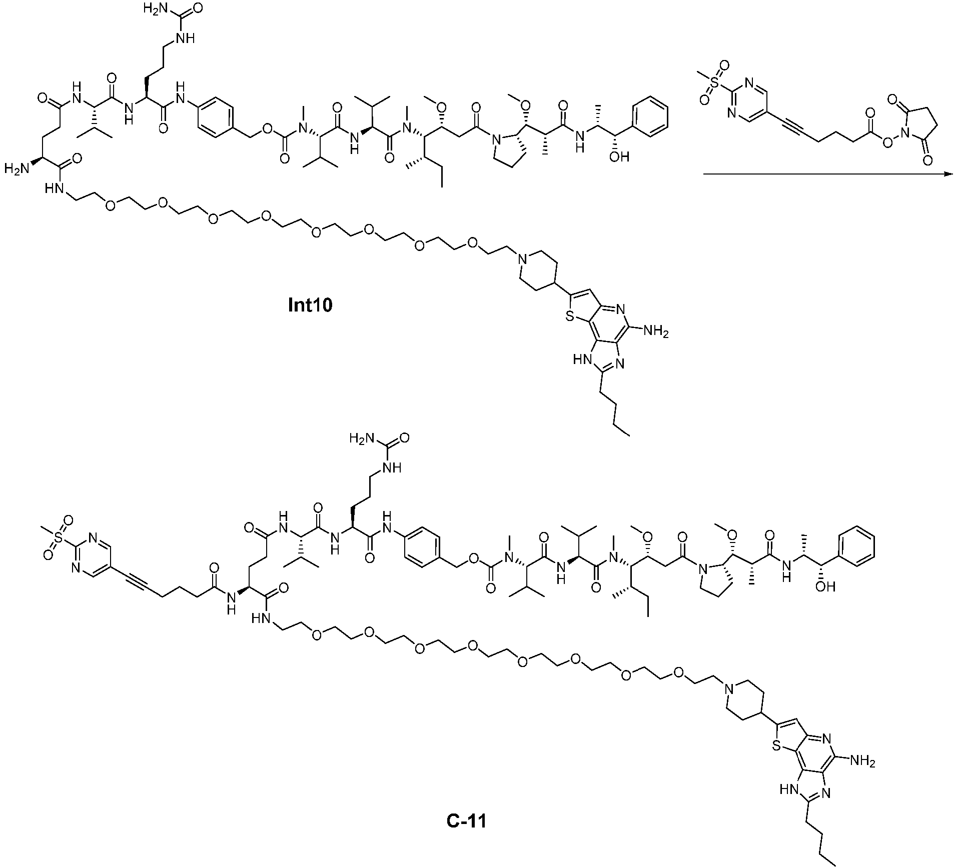

Compound **Int10** (60 mg, 29.09 µmol), 2,5-dioxopyrrolidin-1-yl-6-(2-(methylsulfonyl)pyrimidin-5-yl)hexa-5-ynolate (22.38 mg, 58.18 µmol), *N,N*-diisopropylethylamine (5.7 mg, 43.63 µmol), and 1-hydroxybenzotrizoate (5.09 mg, 14.55 µmol) were added to anhydrous *N,N*-dimethylformamide (2 mL) in sequence, and reacted at 25°C for 2 hours. The reaction solution was purified by preparative high-performance liquid chromatography to obtain the title compound of this step (4 mg, yield: 5.8%).

MS m/z (ESI): 1105.1 [M/2+H]⁺.

### Example 1-12: Preparation of Compound C-14

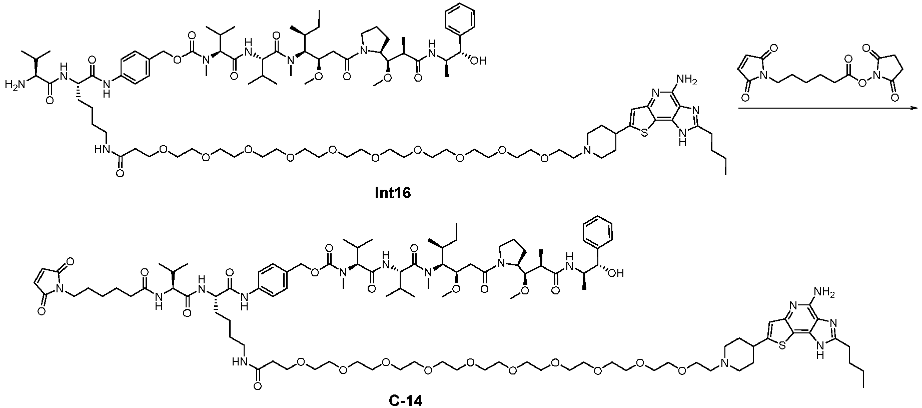

### Step 1: Preparation of Compound C-14

Compound **Int16** (22 mg, 10.32 µmol), 6-(maleimido)hexanoic acid succinimide ester (4.82 mg, 15.48 µmol) and *N,N-*diisopropylethylamine (3.36 mg, 25.80 µmol) were added to *N,N*-dimethylformamide (1 mL) in sequence, and reacted at room temperature for 2 hours. The reaction solution was directly purified by reversed-phase column chromatography (preparation method B, elution gradient: A%=25%-30%) to obtain the title compound of this step (12.73 mg, yield: 54.31%).

MS m/z (ESI): 1056.1 [M/2+H]⁺.

### Example 1-13: Preparation of Compound C-15

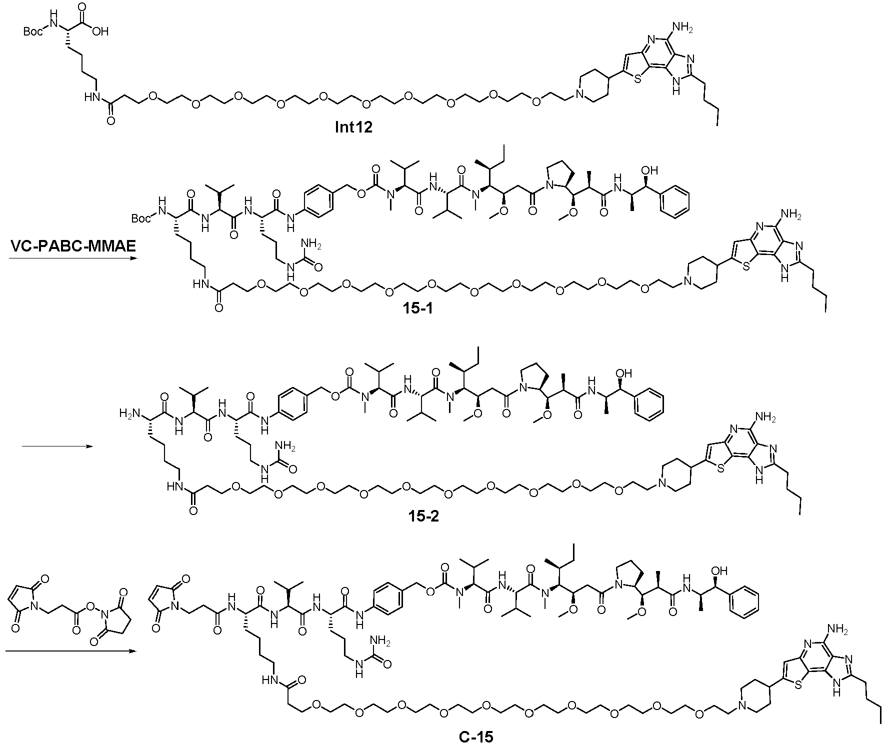

### Step 1: Preparation of Compound 15-1

Compound **Int12** (50 mg, 42.04 µmol), VC-PABC-MMAE (52.48 mg, 42.04 µmol), *N,N*-diisopropylethylamine (13.70 mg, 105.11 µmol) and 2-(7-azobenzotriazole)-*N,N,N',N*'-tetramethylurea hexafluorophosphate (19.37 mg, 50.45 µmol) were added to *N,N*-dimethylformamide (1.5 mL) in sequence, and reacted at room temperature for 1.5 hours. The reaction solution was directly purified by reversed-phase column chromatography (preparation method B, elution gradient: A%=45% to 55%) to obtain the title compound of this step (41 mg, yield: 40.34%).

MS m/z (ESI): 1088.2 [M/2+H]⁺.

### Step 2: Preparation of Compound 15-2

Compound **15-1** (38 mg, 15.72 µmol) and anhydrous zinc bromide (17.88 mg, 78.59 µmol) were added to anhydrous dichloromethane (3 mL) and reacted at room temperature for 5 hours. The crude product obtained by concentrating the reaction solution was purified by reverse-phase column chromatography (preparation method B, elution gradient: A%=20% to 30%) to obtain the title compound of this step (30 mg, yield: 82.76%).

MS m/z (ESI): 1038.1 [M/2+H]⁺.

### Step 3: Preparation of Compound C-15

Compound **15-2** (30 mg, 13.01 µmol), 3-maleimidopropionic acid hydroxysuccinimide ester (5.25 mg, 19.51 µmol) and *N,N-*diisopropylethylamine (2.54 mg, 19.51 µmol) were added to *N,N*-dimethylformamide (1 mL) in sequence, and reacted at room temperature for 1.5 hours. The reaction solution was directly purified by reversed-phase column chromatography (preparation method B, elution gradient: A%=25% to 40%) to obtain the title compound of this step (9.81 mg, yield: 31.52%).

MS m/z (ESI): 1113.7 [M/2+H]⁺.

### Example1-14: Preparation of Compound C-16

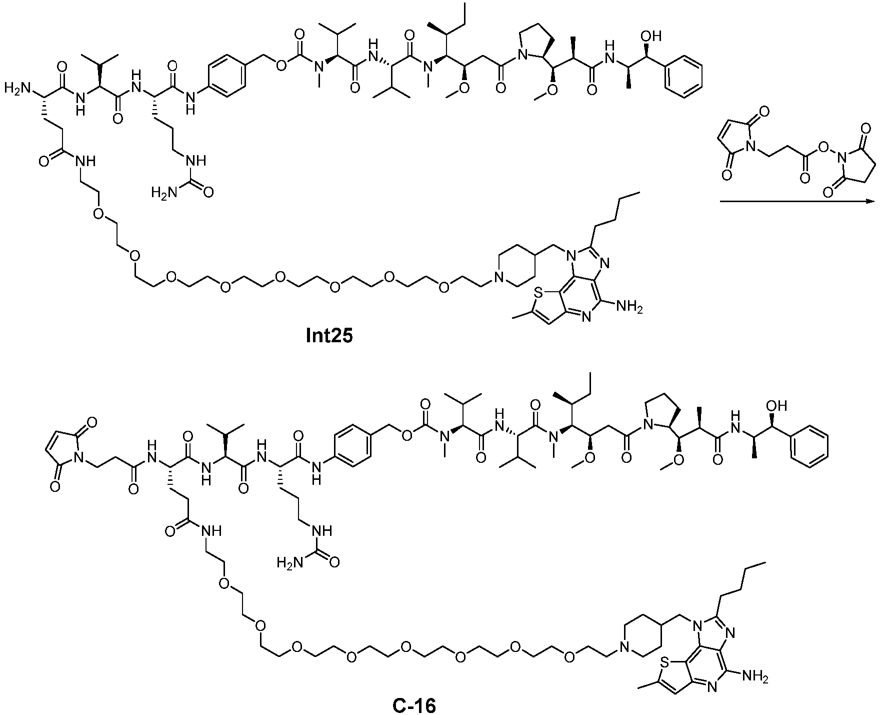

Compound Int25 (25 mg, 12.58 µmol), 3-maleimidopropionic acid hydroxysuccinimide ester (10.05 mg, 37.74 µmol) and *N,N*-diisopropylethylamine (2.34 mg, 17.92 µmol) were added to anhydrous *N,N*-dimethylformamide (2 mL) in sequence, and reacted at 0°C for 2 hours. The reaction solution was purified by preparative high-performance liquid chromatography to obtain the title compound of this step (8 mg, yield: 31.3%).

MS m/z (ESI): 1069.6 [M/2+H]⁺.

### Example 1-15: Preparation of Compound C-17

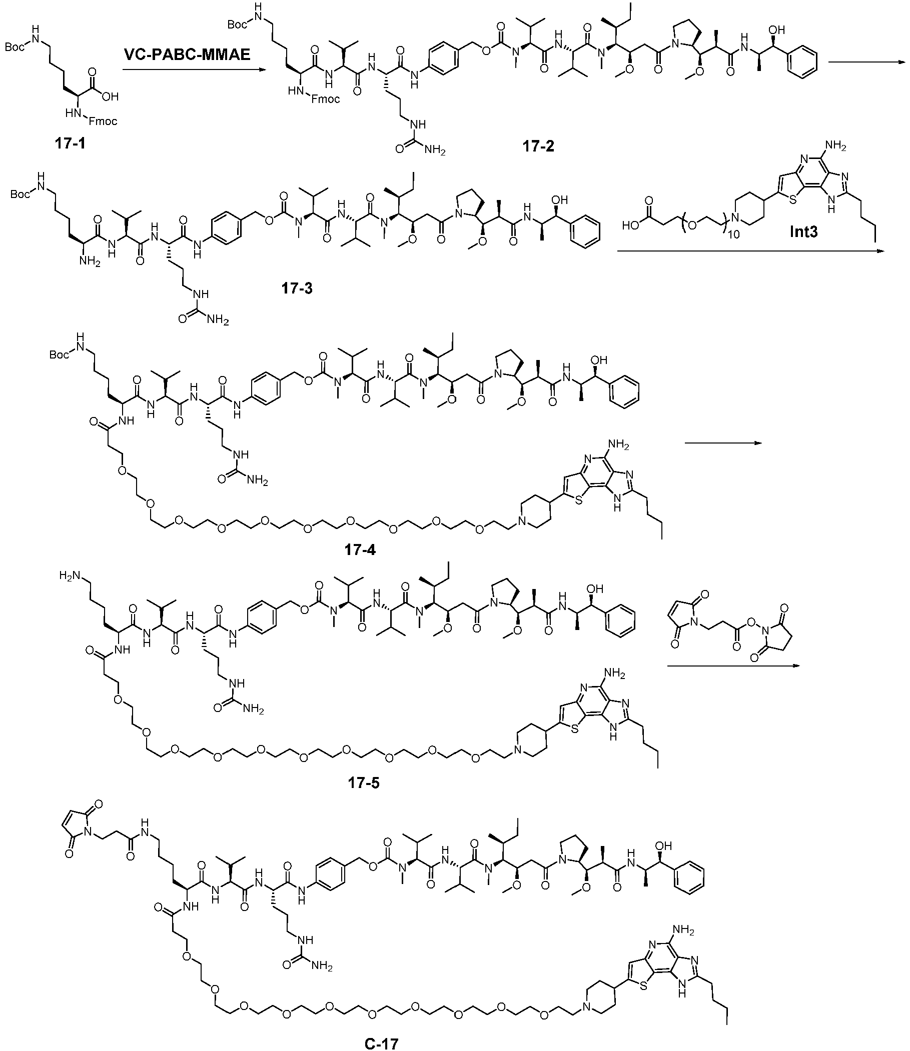

### Step 1: Preparation of Compound 17-2

VC-PABC-MMAE (60 mg, 53.41 µmol), *N²*-((((9*H*-fluoren-9-yl)methoxy)carbonyl)-*N⁶*-(tert-butoxycarbonyl)-*L*-lysine (25 mg, 53.41 µmol), *N,N-*diisopropylethylamine (17.2 mg, 133.52 µmol) and 2-(7-azobenzotriazole)-*N,N,N',N'*-tetramethylurea hexafluorophosphate (24.35 mg, 64.09 µmol) were added to *N,N*-dimethylformamide (1 mL) in sequence, and reacted at room temperature for 1 hour. The reaction system was poured into ice water (10 mL) and extracted with ethyl acetate (5 mL*3). The organic phases were combined and backwashed with saturated brine (3 mL*2), dried over anhydrous sodium sulfate, and concentrated to obtain the title compound of this step (82 mg, crude product).

MS m/z (ESI): 1574.0 [M+H]⁺.

### Step 2: Preparation of Compound 17-3

Compound **17-2** (82.00 mg, crude product) and piperidine (8.07 mg, 93.78 µmol) were added to *N,N*-dimethylformamide (1 mL) in sequence, and reacted at room temperature for 1 hour. The reaction solution was directly purified by reversed-phase column chromatography (preparation method B, elution gradient: A%=25% to 35%) to obtain the title compound of this step (41 mg, two-step total yield: 56.75%).

MS m/z (ESI): 1351.9 [M+H]⁺.

### Step 3: Preparation of Compound 17-4

Compound **17-3** (41.00 mg, 30.32 µmol), Compound **Int3** (25.52 mg, 30.32 µmol), *N,N*-diisopropylethylamine (9.78 mg, 75.81 µmol) and 2-(7-azobenzotriazole)-*N,N,N',N'*-tetramethylurea hexafluorophosphate (17.28 mg, 45.48 µmol) were added in sequence to *N,N*-dimethylformamide (1 mL), and reacted at room temperature for 2 hours. The reaction solution was directly purified by reversed-phase column chromatography (preparation method B, elution gradient: A%=45%-50%) to obtain the title compound of this step (35 mg, yield: 53.06%).

MS m/z (ESI): 1088.2 [M/2+H]⁺.

### Step 4: Preparation of Compound 17-5

Compound **17-4** (35 mg, 16.09 µmol) and anhydrous zinc bromide (14.28 mg, 64.36 µmol) were added to dichloromethane (2 mL) and reacted at room temperature for 3 hours. The reaction solution was directly purified by reversed-phase column chromatography (preparation method B, elution gradient: A%=25%-30%) to obtain the title compound of this step (25 mg, yield: 74.87%).

MS m/z (ESI): 1038.2 [M/2+H]⁺.

### Step 5: Preparation of Compound C-17

Compound **17-5** (25 mg, 12.05 µmol), 3-maleimidopropionic acid hydroxysuccinimide ester (4.37 mg, 16.26 µmol) and *N,N*-diisopropylethylamine (3.12 mg, 24.10 µmol) were added to *N,N*-dimethylformamide (1 mL) in sequence, and reacted at room temperature for 2 hours. The reaction solution was directly purified by reversed-phase column chromatography (preparation method B, elution gradient: A%=30%-45%) to obtain the title compound of this step (11.1 mg, yield: 41.38%).

MS m/z (ESI): 1113.7 [M/2+H]⁺.

### Example1-16: Preparation of Compound C-18

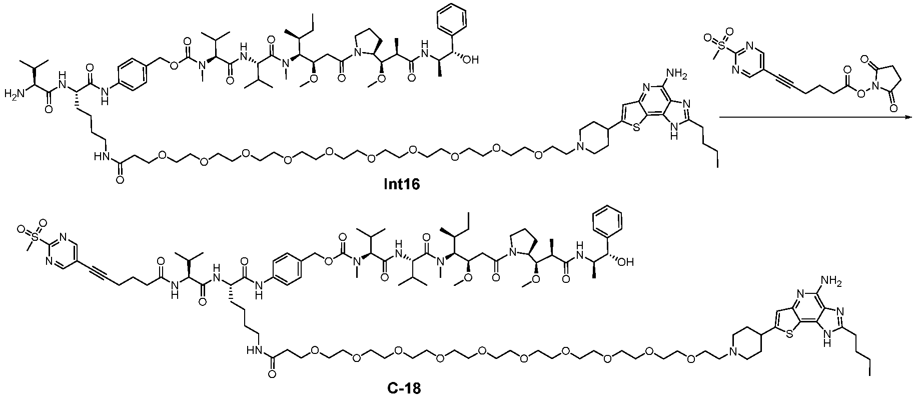

Compound **Int16** (16 mg, 8.34 µmol), 2,5-dioxopyrrolidin-1-yl-6-(2-(methylsulfonyl) pyrimidin-5-yl)hexa-5-ynoate (4.57 mg, 12.51 µmol) and *N,N*-diisopropylethylamine (2.15 mg, 16.68 µmol) were added to *N,N-*dimethylformamide (0.5 mL) in sequence, and reacted at room temperature for 1 hour. The reaction solution was directly purified by reversed-phase column chromatography (preparation method B, elution gradient: A%=25% to 40%) to obtain the title compound of this step (2.87 mg, yield: 15.87%).

MS m/z (ESI): 1084.6 [M/2+H]⁺.

### Example1-17: Preparation of Compound C-19

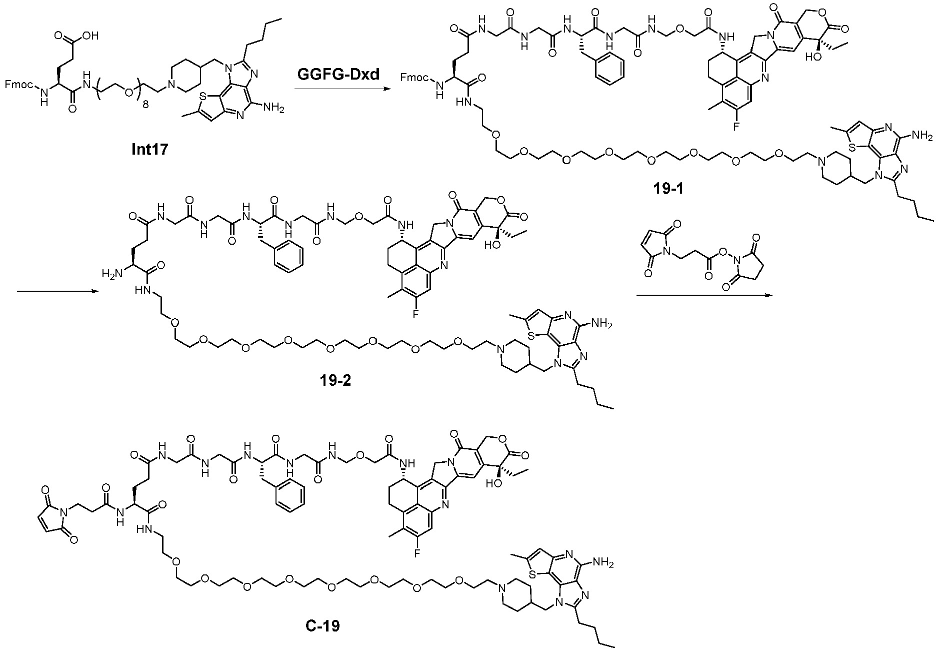

### Step 1: Preparation of Compound 19-1

Compound **Int17** (25 mg, 22.64 µmol),*N,N-*diisopropylethylamine (4.21 mg, 32.26 µmol), 2-(7-azobenzotriazole)-*N,N,N',N'*-tetramethylurea hexafluorophosphate (9.91 mg, 25.81 µmol) and GGFG-Dxd (20.09 mg, 22.64 µmol) were added to anhydrous *N,N*-dimethylformamide (3 mL) in sequence, and reacted at 0°C for 2 hours. The reaction solution was directly purified by reversed-phase column chromatography (preparation method C, elution gradient: A%=15% to 25%) to obtain the title compound of this step (38 mg, yield: 91.7%).

MS m/z (ESI): 964.0 [M/2+H]⁺.

### Step 2: Preparation of Compound 19-2

Compound **19-1** (31 mg, 16.08 µmol) and diethylamine (6.85 mg, 80.4 µmol) were added to *N,N-*dimethylformamide (2 mL) and reacted at 25°C for 2 hours. The reaction solution was directly purified by reversed-phase column chromatography (preparation method B, elution gradient: A%=50% to 60%) to obtain the title compound of this step (20 mg, yield: 72.9%).

MS m/z (ESI): 852.8 [M/2+H]⁺.

### Step 3: Preparation of Compound C-19

Compound 19-2 (20 mg, 11.73 µmol), 3-maleimidopropionic acid hydroxysuccinimide ester (6.25 mg, 23.46 µmol) and N,N-diisopropylethylamine (2.18 mg, 16.72 µmol) were dissolved in sequence in anhydrous *N,N-*dimethylformamide (2 mL) and reacted at 0°C for 2 hours. The reaction solution was purified by preparative high-performance liquid chromatography to obtain the title compound of this step (15 mg, yield: 72.5%).

MS m/z (ESI): 928.4 [M/2+H]⁺.

### Example1-18: Preparation of Compound C-20

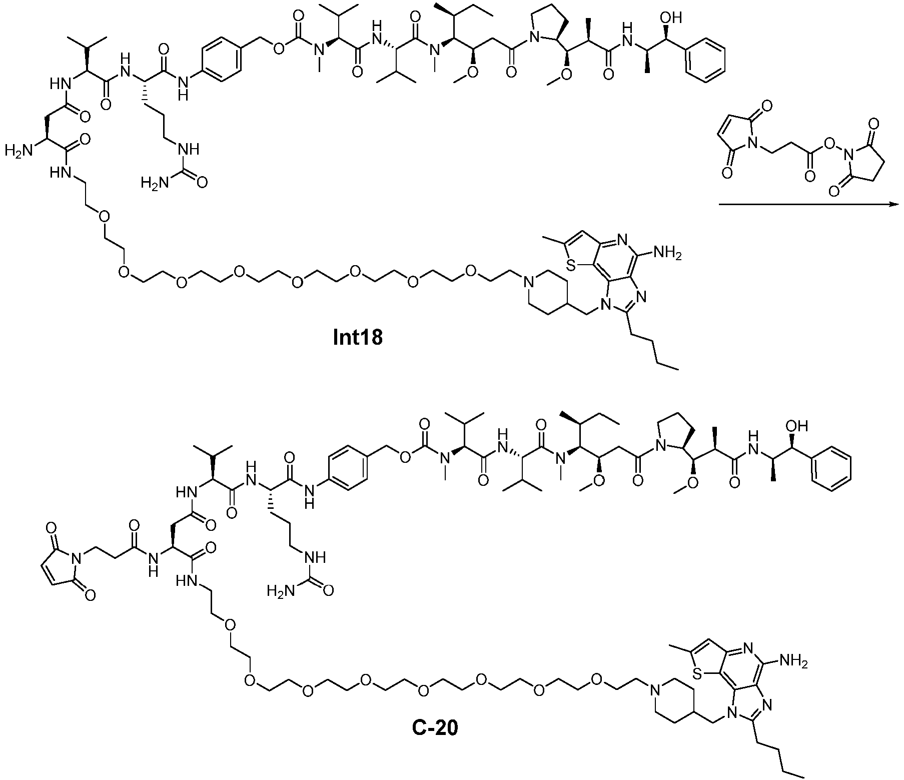

Compound **Int18** (28 mg, 14.19 µmol), 3-maleimidopropionic acid hydroxysuccinimide ester (5.66 mg, 21.28 µmol) and *N,N*-diisopropylethylamine (2.75 mg, 21.28 µmol) were added in sequence to *N,N-*dimethylformamide (0.5 mL) and reacted at room temperature for 2 hours. The reaction solution was directly purified by reversed-phase column chromatography (preparation method B, elution gradient: A%=20% to 35%) to obtain the title compound of this step (7.64 mg, yield: 25.34%).

MS m/z (ESI): 1062.6 [M/2+H]⁺.

### Example1-19: Preparation of Compound C-21

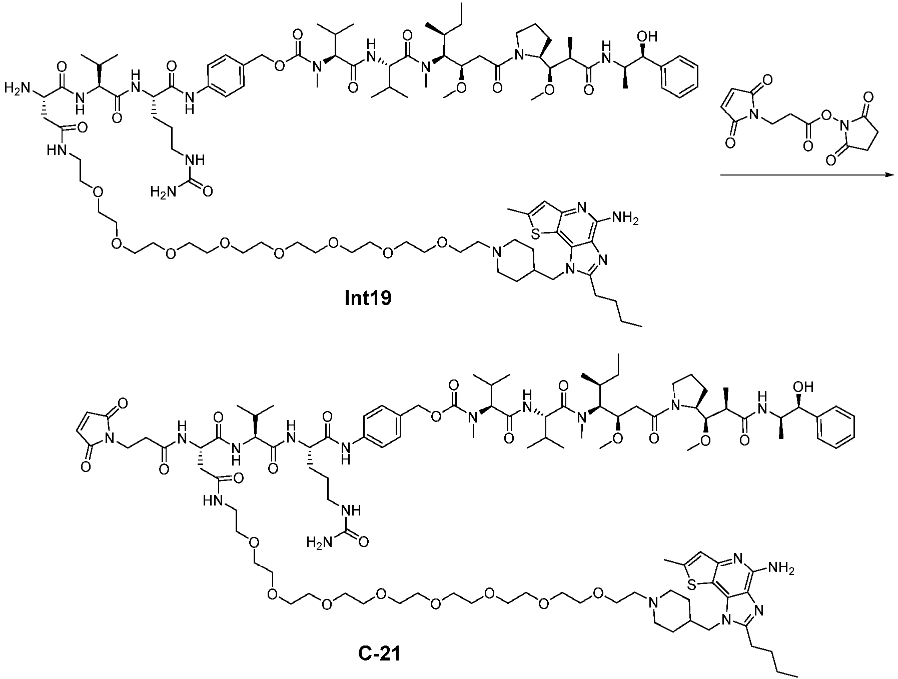

Compound **Int19** (23.15 mg, 11.73 µmol), 3-maleimidopropionic acid hydroxysuccinimide ester (6.25 mg, 23.46 µmol) and *N,N*-diisopropylethylamine (2.18 mg, 16.72 µmol) were dissolved in sequence in anhydrous *N,N*-dimethylformamide (2 mL), and reacted at 0°C for 2 hours. The reaction solution was purified by preparative high-performance liquid chromatography to obtain the title compound of this step (12 mg, yield: 50.7%).

MS m/z (ESI): 1062.7 [M/2+H]⁺.

### Example 1-20: Preparation of Compound C-22

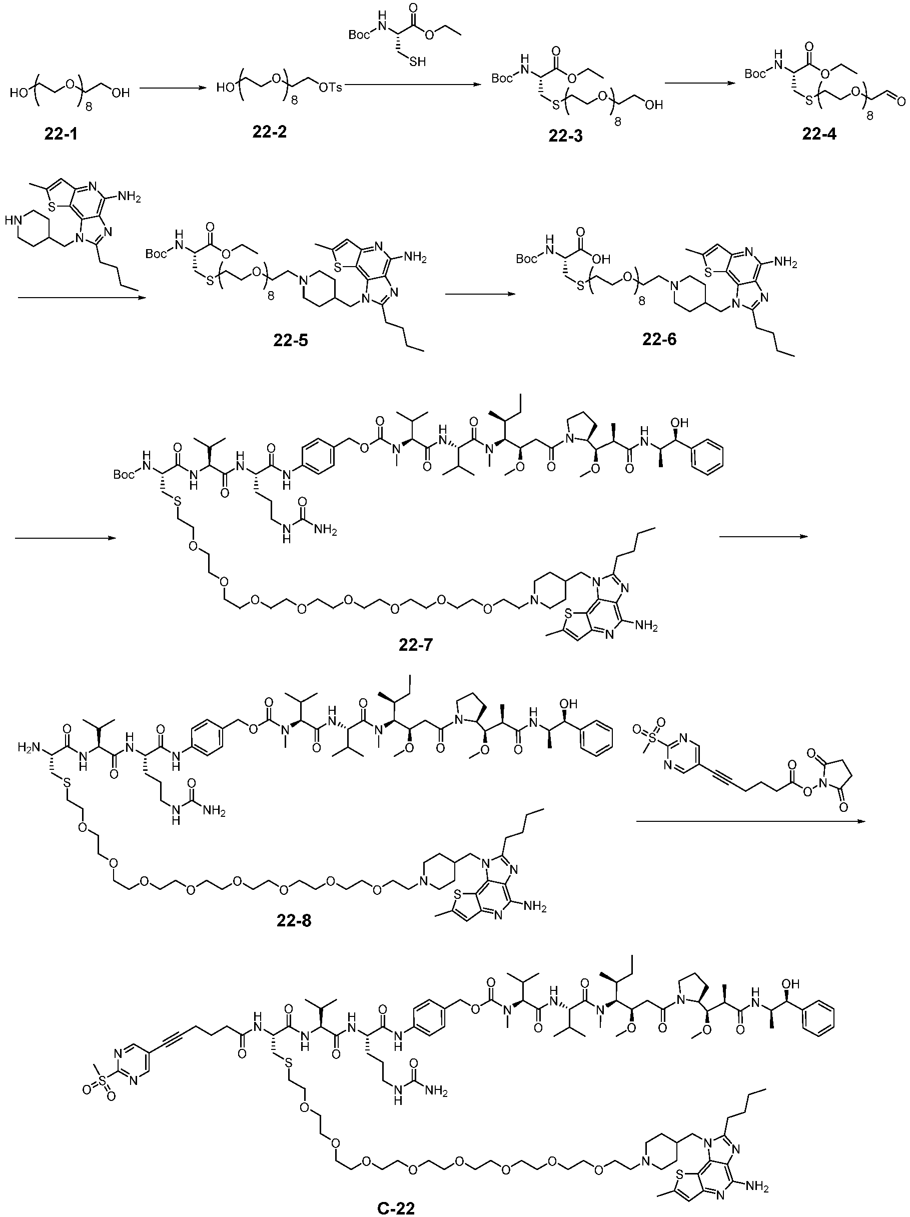

### Step 1: Preparation of Compound 22-2

Nona (ethylene glycol) (1.2 g, 2.9 mmol) and *p*-toluenesulfonyl chloride (250.52 mg, 2.9 mmol) were dissolved in anhydrous dichloromethane (20 mL), then added with potassium hydroxide (324.89 mg, 5.79 mmol), and reacted at 25°C for 10 hours. The reaction solution was concentrated, and the residue was purified by silica gel column chromatography (eluent: dichloromethane/methanol = 30/1 (v/v)) to obtain the title compound of this step (1.1 g, yield: 66.7%).

MS m/z (ESI): 586.4 [M+NH₄]⁺.

### Step 2: Preparation of Compound 22-3

Compound **22-2** (460 mg, 808.9 µmol) and *N*-tert-butoxycarbonyl-*L*-cysteine ethyl ester (242 mg, 970.67 µmol) were dissolved in *N,N-*dimethylformamide (6 mL), then added with potassium iodide (13.43 mg, 80.89 µmol) and *N,N*-diisopropylethylamine (150 mg, 1152.68 µmol), and reacted at 25°C for 10 hours. The reaction solution was concentrated, and the residue was purified by silica gel column chromatography (eluent: dichloromethane/methanol = 5/1 (v/v)) to obtain the title compound of this step (320 mg, yield: 64.5%).

MS m/z (ESI): 646.1 [M+H]⁺.

### Step 3: Preparation of Compound 22-4

Compound **22-3** (220 mg, 337.26 µmol) and Dess-Martin oxidant (216.66 mg, 505.89 µmol) were added to dichloromethane (15 mL) and reacted at 25°C for 10 hours. Saturated sodium thiosulfate solution (5 mL) and saturated sodium bicarbonate aqueous solution (10 mL) were added to the reaction solution, stirred until clear, extracted three times with dichloromethane, washed with saturated brine, dried over anhydrous sodium sulfate, and filtered, the filtrate was concentrated, and the residue was purified by silica gel column chromatography (eluent: dichloromethane/methanol = 20/1 (v/v)) to obtain the title compound of this step (185 mg, yield: 85.2%).

MS m/z (ESI): 644.3 [M+H]⁺.

### Step 4: Preparation of Compound 22-5

Compound **22-4** (146.31 mg, 227.26 µmol), Compound **Int13** (65 mg, 181.81 µmol), sodium cyanoborohydride (31.56 mg, 502.2 mmol) and glacial acetic acid (31.4 mg, 518.16 mmol) were added in sequence to a mixed solvent of 1,2-dichloroethane (4 mL) and methanol (2 mL) and reacted at 25°C for 4 hours. The crude product obtained by concentrating the reaction solution was dissolved in methanol (3 mL) and purified by reverse-phase column chromatography (preparation method B, elution gradient: A%=20% to 30%) to obtain the title compound of this step (109 mg, yield: 63.8%).

MS m/z (ESI): 985.5 [M+H]⁺.

### Step 5: Preparation of Compound 22-6

Compound **22-5** (100 mg, 101.49 µmol) was dissolved in methanol (4 mL), added with lithium hydroxide aqueous solution (21.31 mg, 507.46 µmol, 1 mL), and reacted at 25°C for 4 hours. The reaction solution was directly purified by reversed-phase column chromatography (preparation method B, elution gradient: A%=35% to 45%) to obtain the title compound of this step (85 mg, yield: 87.5%).

MS m/z (ESI): 957.5 [M+H]⁺.

### Step 6: Preparation of Compound 22-7

Compound **22-6** (65 mg, 67.91 µmol), *N,N*-diisopropylethylamine (12.63 mg, 96.76 µmol, 16.15 µL), 2-(7-azobenzotriazole)*-N,N,N',N'*-tetramethylurea hexafluorophosphate (34.69 mg, 90.31 µmol) and VC-PABC-MMAE (76.28 mg, 67.91 µmol) were added in sequence to anhydrous *N,N*-dimethylformamide (3 mL), and reacted at 0°C for 2 hours. The reaction solution was directly purified by reversed-phase column chromatography (preparation method B, elution gradient: A%=30%-45%) to obtain the title compound of this step (85 mg, yield: 63.9%).

MS m/z (ESI): 1031.6 [M/2+H]⁺.

### Step 7: Preparation of Compound 22-8

Compound **22-7** (38 mg, 18.42 µmol) and zinc bromide (31.85 mg, 140.01 µmol) were added to anhydrous dichloromethane (2 mL) and reacted at 25°C for 4 hours. The crude product obtained by concentrating the reaction solution was purified by reverse-phase column chromatography (preparation method B, elution gradient: A%=20% to 30%) to obtain the title compound of this step (30 mg, yield: 87.0%).

MS m/z (ESI): 981.5 [M/2+H]⁺.

### Step 8: Preparation of Compound C-22

Compound **22-8** (30 mg, 15.28 µmol), 2,5-dioxopyrrolidin-1-yl-6-(2-(methylsulfonyl) pyrimidin-5-yl)hexa-5-ynoate (8.94 mg, 24.46 µmol) and *N,N*-diisopropylethylamine (2.84 mg, 21.75 µmol, 3.64 µL) were dissolved in sequence in anhydrous *N,N-*dimethylformamide (2 mL), and reacted at 0°C for 2 hours. The reaction solution was purified by preparative high-performance liquid chromatography to obtain the title compound of this step (20 mg, yield: 62.2%).

MS m/z (ESI): 1106.6 [M/2+H]⁺.

### Example1-21: Preparation of Compound C-23

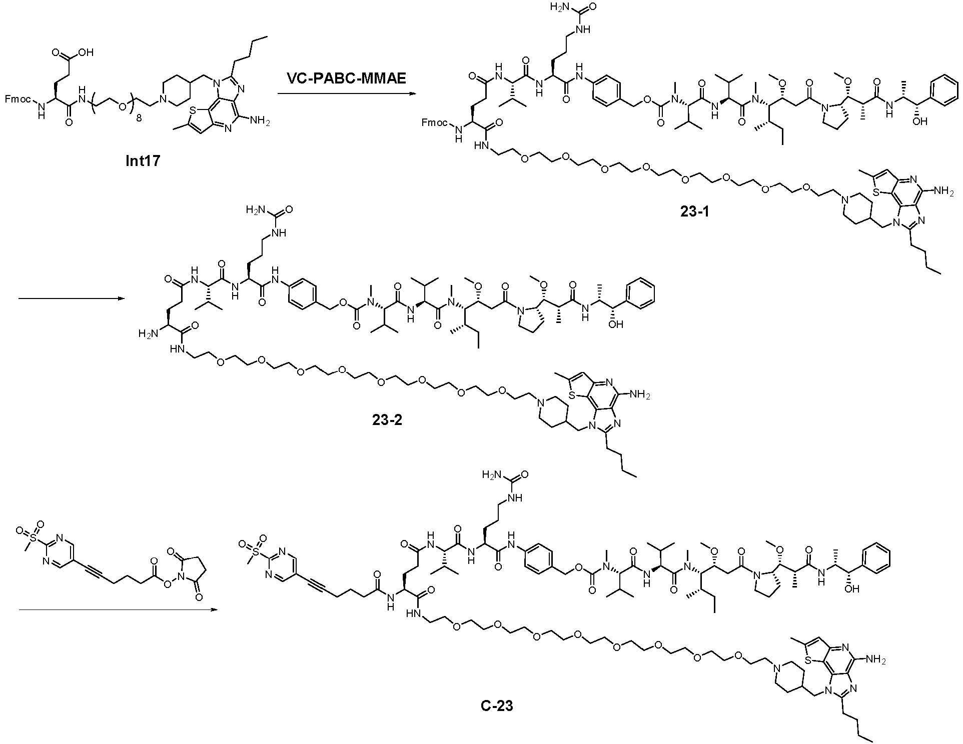

### Step 1: Preparation of Compound 23-1

Compound **Int17** (51 mg, 46.19 µmol), VC-PABC-MMAE (51.88 mg, 46.19 µmol), *N,N*-diisopropylethylamine (24.37 mg, 187.03 µmol) and 2-(7-azobenzotriazole)*-N,N,N',N'*-tetramethylurea hexafluorophosphate (19.14 mg, 49.88 µmol) were added in sequence to *N,N*-dimethylformamide (1 mL) and reacted at room temperature for 1.5 hours. The reaction system was poured into ice water (10 mL), extracted with ethyl acetate (5 mL*3). The organic phases were combined, backwashed with saturated brine (3 mL*2), dried over anhydrous sodium sulfate, and concentrated to obtain the title compound of this step (86 mg, yield: 84.27%).

MS m/z (ESI): 1105.2 [M/2+H]⁺.

### Step 2: Preparation of Compound 23-2

Compound **23-1** (86 mg, 38.93 µmol) and piperidine (5.36 mg, 62.27 µmol) were added to *N,N*-dimethylformamide (1 mL) and reacted at room temperature for 2 hours. The reaction solution was directly purified by reversed-phase column chromatography (preparation method B, elution gradient: A%=20% to 25%) to obtain the title compound of this step (47 mg, yield: 60.79%).

MS m/z (ESI): 1987.2 [M+H]⁺.

### Step 3: Preparation of Compound C-23

Compound **23-2** (47 mg, 23.66 µmol), 2,5-dioxopyrrolidin-1-yl-6-(2-(methylsulfonyl) pyrimidin-5-yl)hexa-5-ynoate (12.99 mg, 35.49 µmol) and *N,N*-diisopropylethylamine (5.55 mg, 42.57 µmol) were added in sequence to *N,N-*dimethylformamide (0.5 mL) and reacted at room temperature for 1.5 hours. The reaction solution was directly purified by reversed-phase column chromatography (preparation method B, elution gradient: A%=20% to 35%) to obtain the title compound of this step (37.38 mg, yield: 70.60%).

MS m/z (ESI): 1119.1 [M/2+H]⁺.

### Example 1-22: Preparation of Compound C-24

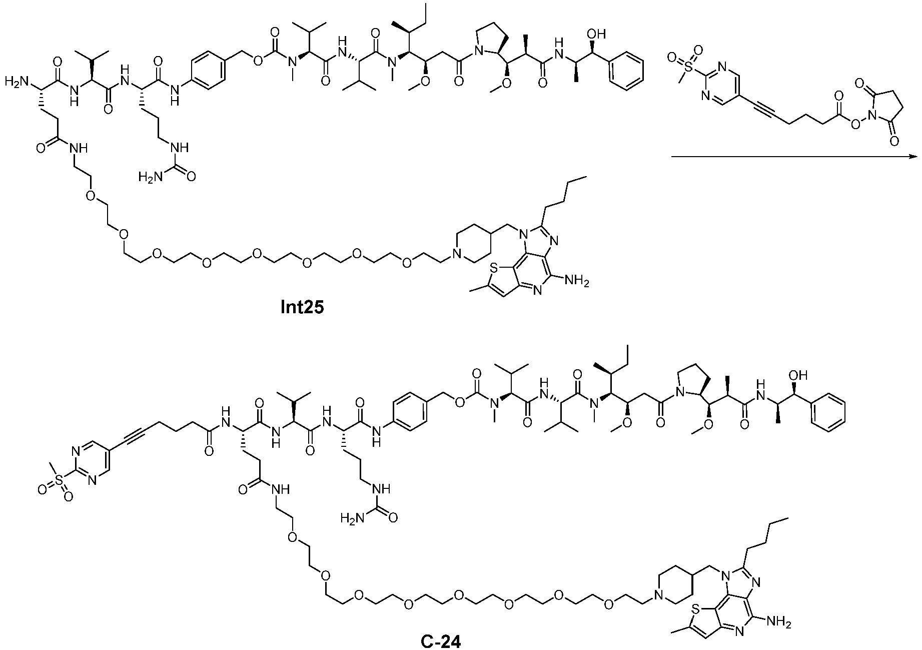

Compound **Int25** (28.80 mg, 14.49 µmol), 2,5-dioxopyrrolidin-1-yl-6-(2-(methylsulfonyl) pyrimidin-5-yl) hexa-5-ynoate (5.88 mg, 14.49 µmol) and *N,N-*diisopropylethylamine (3.78 mg, 28.98 µmol) were added in sequence to *N,N-*dimethylformamide (0.5 mL) and reacted at room temperature for 2 hours. The reaction solution was directly purified by reversed-phase column chromatography (preparation method B, elution gradient: A%=20% to 35%) to obtain the title compound of this step (13.57 mg, yield: 41.83%).

MS m/z (ESI): 1119.1 [M/2+H]⁺.

### Example 1-23: Preparation of Compound C-25

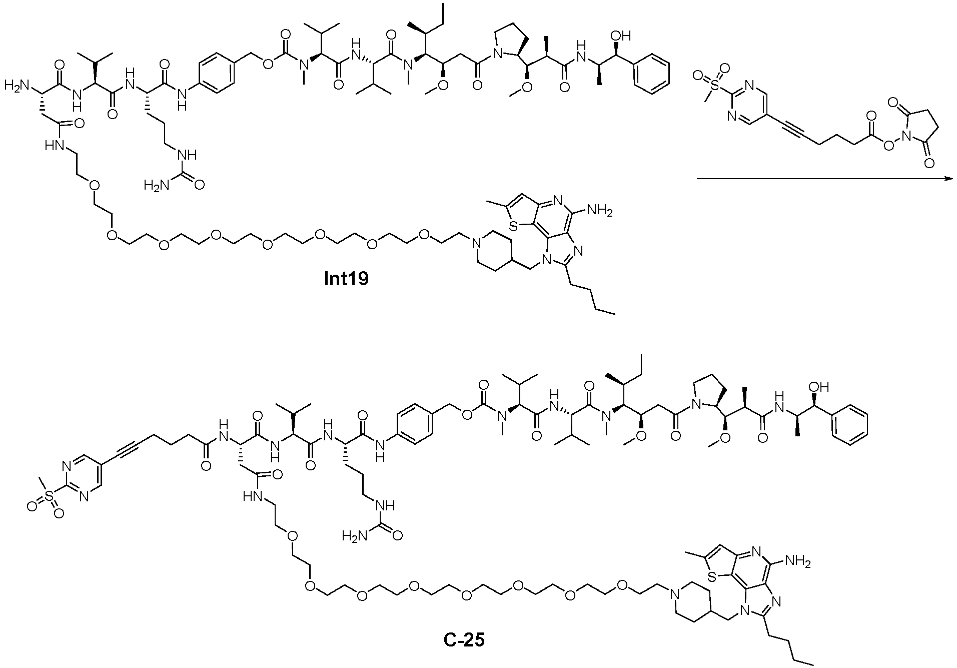

Compound **Int19** (24.7 mg, 12.52 µmol),2,5-dioxopyrrolidin-1-yl-6-(2-(methylsulfonyl) pyrimidin-5-yl)hexa-5-ynoate (5.08 mg, 12.52 µmol) and *N,N*-diisopropylethylamine (3.26 mg, 25.03 µmol) were added in sequence to *N,N*-dimethylformamide (1 mL) and reacted at room temperature for 2 hours. The reaction solution was directly purified by reversed-phase column chromatography (preparation method B, elution gradient: A%=20% to 35%) to obtain the title compound of this step (17.16 mg, yield: 61.66%).

MS m/z (ESI): 1112.1 [M/2+H]⁺.

### Example 1-24: Preparation of Compound C-26

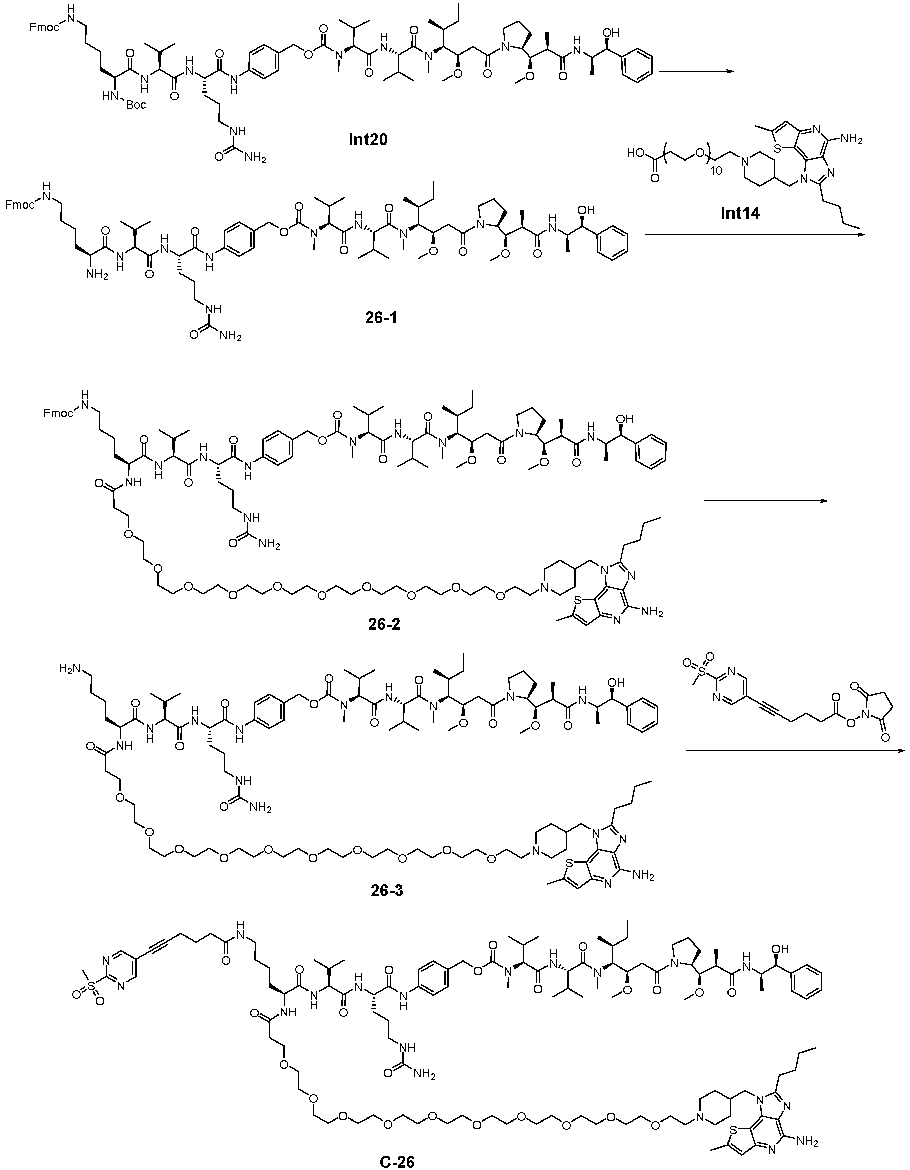

### Step 1: Preparation of Compound 26-1

Compound **Int20** (87 mg, 55.27 µmol) and zinc bromide (95.56 mg, 420.09 µmol) were added to anhydrous dichloromethane (3 mL) and reacted at 25°C for 3 hours. The reaction solution was directly purified by reversed-phase column chromatography (preparation method B, elution gradient: A%=35% to 45%) to obtain the title compound of this step (61 mg, yield: 78.8%).

MS m/z (ESI): 1473.8 [M+H]⁺.

### Step 2: Preparation of Compound 26-2

Compound **Int14** (21.25 mg, 24.42 µmol), *NN-*diisopropylethylamine (4.54 mg, 34.81 µmol), 2-(7-azobenzotriazole)-*N*,*N*,*N*'*,N'*-tetramethylurea hexafluorophosphate (12.48 mg, 32.49 µmol) and Compound **26-1** (36 mg, 24.42 µmol) were added in sequence to anhydrous N,N-dimethylformamide (3 mL), and reacted at 0°C for 1 hour. The reaction solution was directly purified by reversed-phase column chromatography (preparation method B, elution gradient: A%=20% to 35%) to obtain the title compound of this step (45 mg, yield: 83.4%).

MS m/z (ESI): 1163.2 [M/2+H]⁺.

### Step 3: Preparation of Compound 26-3

Compound **26-2** (40 mg, 17.03 µmol) and diethylamine (6.29 mg, 85.13 µmol) were added to *N,N*-dimethylformamide (4 mL) and reacted at 25°C for 2 hours. The reaction solution was directly purified by reversed-phase column chromatography (preparation method B, elution gradient: A%=25% to 35%) to obtain the title compound of this step (30 mg, yield: 83.8%).

MS m/z (ESI): 1052.3 [M/2+H]⁺.

### Step 4: Preparation of Compound C-26

Compound **26-3** (20 mg, 9.50 µmol), 2,5-dioxopyrrolidin-1-yl-6-(2-(methylsulfonyl) pyrimidin-5-yl)hexa-5-ynoate (5.56 mg, 15.21 µmol) and *N,N*-diisopropylethylamine (1.77 mg, 13.67 µmol) were added in sequence to anhydrous *N,N*-dimethylformamide (2 mL) and reacted at 0°C for 2 hours. The reaction solution was purified by preparative high-performance liquid chromatography to obtain the title compound of this step (8 mg, yield: 37.6%).

MS m/z (ESI): 1177.2 [M/2+H]⁺.

### Example 1-25: Preparation of Compound C-27

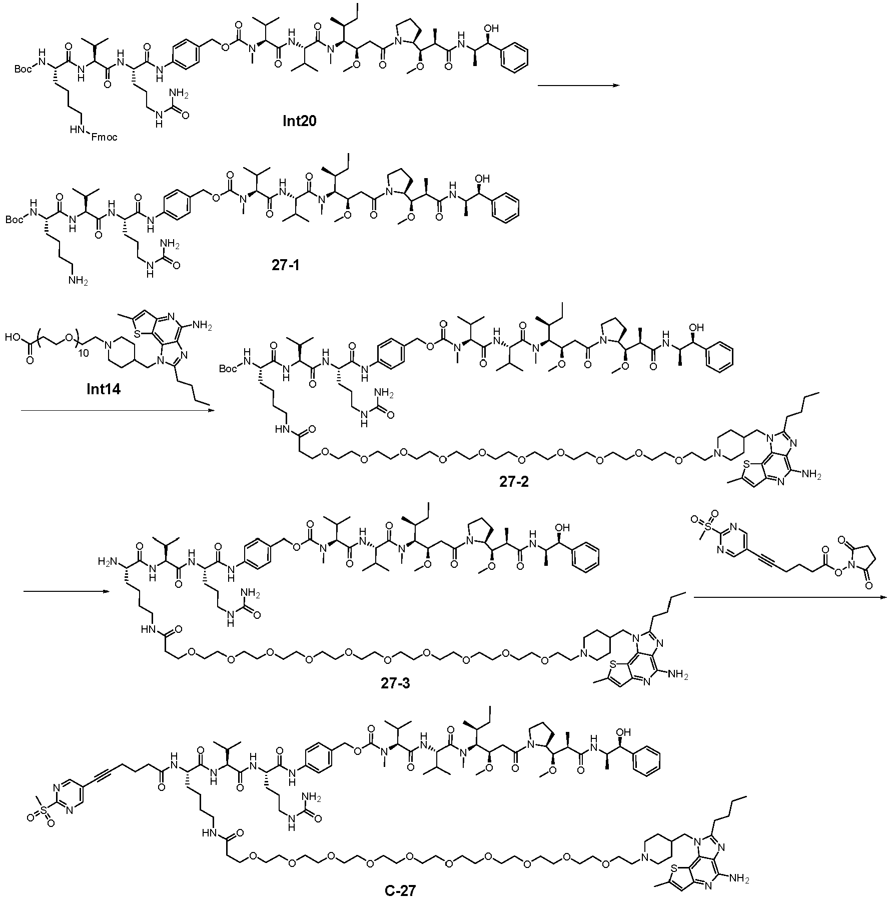

### Step 1: Preparation of Compound 27-1

Compound **Int20** (85 mg, 53.46 µmol) and diethylamine (19.75 mg, 267.32 µmol) were added to *N,N*-dimethylformamide (4 mL) and reacted at 25°C for 2 hours. The reaction solution was directly purified by reversed-phase column chromatography (preparation method B, elution gradient: A%=25% to 35%) to obtain the title compound of this step (73 mg, yield: 98.6%).

MS m/z (ESI): 1351.8 [M+H]⁺.

### Step 2: Preparation of Compound 27-2

Compound **Int14** (55.52 mg, 63.81 µmol), *N,N*-diisopropylethylamine (10.32 mg, 79.07 µmol), 2-(7-azobenzotriazole)-*N,N,N',N'*-tetramethylurea hexafluorophosphate (28.34 mg, 73.8 µmol) and Compound **27-1** (75 mg, 55.48 µmol) were added in sequence to anhydrous *N,N*-dimethylformamide (4 mL), and reacted at 0°C for 1 hour. The reaction solution was directly purified by reversed-phase column chromatography (preparation method B, elution gradient: A%=25% to 40%) to obtain the title compound of this step (38 mg, yield: 32.7%).

MS m/z (ESI): 1102.2 [M/2+H]⁺.

### Step 3: Preparation of Compound 27-3

Compound **27-2** (38 mg, 17.24 µmol) and zinc bromide (29.81 mg, 131.05 µmol) were added to anhydrous dichloromethane (3 mL) and reacted at 25°C for 3 hours. The crude product obtained by concentrating the reaction solution was purified by reverse-phase column chromatography (preparation method B, elution gradient: A%=20% to 30%) to obtain the title compound of this step (61 mg, yield: 78.8%).

MS m/z (ESI): 701.8 [M/3+H]⁺.

### Step 4: Preparation of Compound C-27

Compound **27-3** (20 mg, 9.50 µmol), 2,5-dioxopyrrolidin-1-yl-6-(2-(methylsulfonyl) pyrimidin-5-yl)hexa-5-ynoate (5.56 mg, 15.21 µmol) and *N,N*-diisopropylethylamine (1.77 mg, 13.67 µmol) were added in sequence to anhydrous *N,N*-dimethylformamide (2 mL) and reacted at 0°C for 2 hours. The reaction solution was purified by preparative high-performance liquid chromatography to obtain the title compound of this step (6 mg, yield: 28.2%).

MS m/z (ESI): 1177.2 [M/2+H]⁺.

### Example 1-26: Preparation of Compound C-28

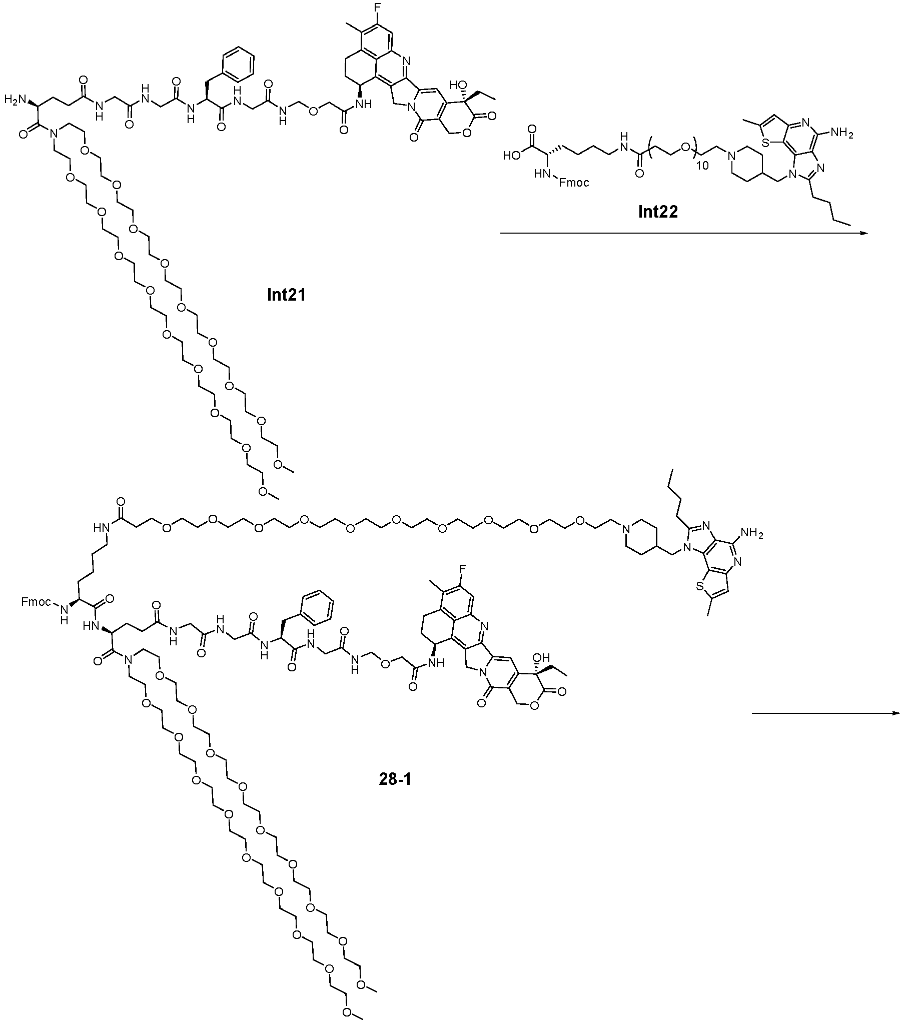

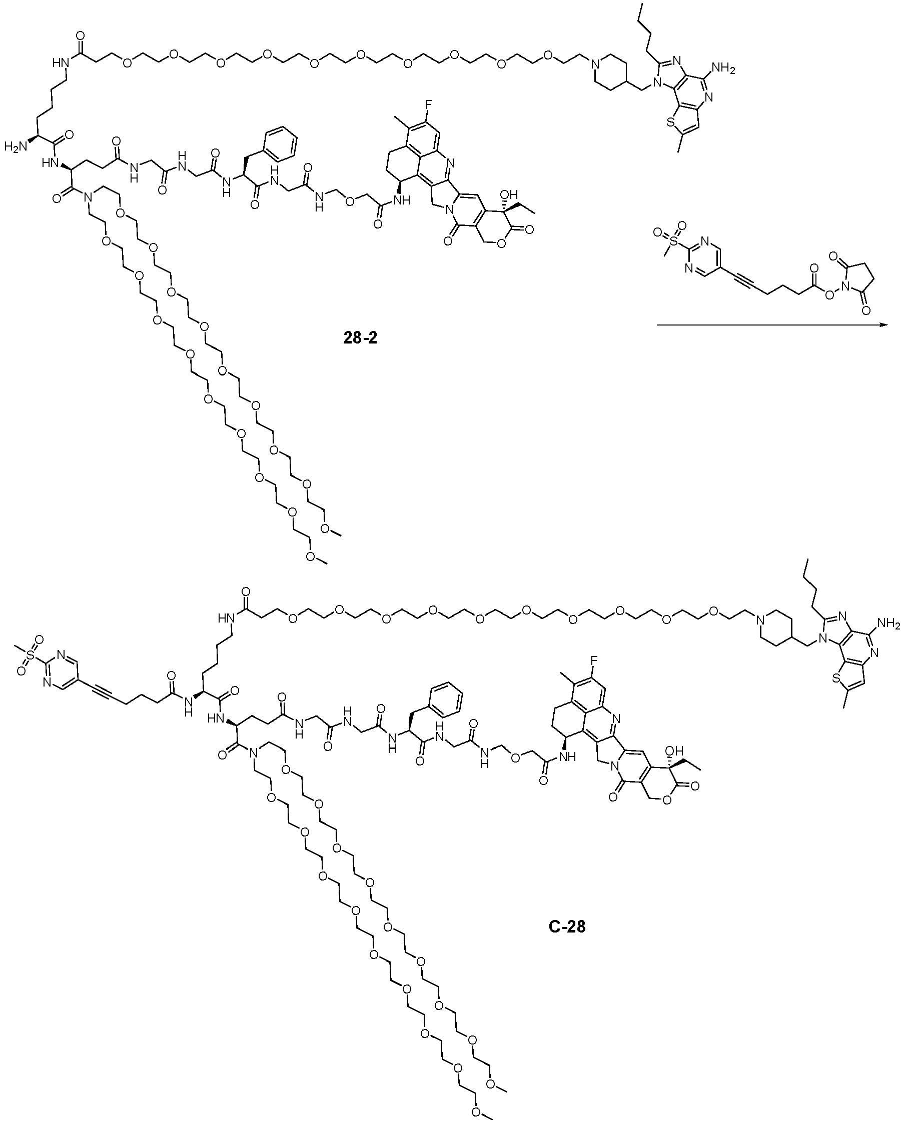

### Step 1: Preparation of Compound 28-1

Compound **Int22** (10.23 mg, 8.38 µmol), *N,N*-diisopropylethylamine (1.62 mg, 12.57 µmol), 2-(7-azobenzotriazole)-*N,N,N', N'* tetramethylurea hexafluorophosphate (3.82 mg, 10.04 µmol) and Compound **Int21** (15 mg, 8.38 µmol) were added to anhydrous *N,N-*dimethylformamide (3 mL), and reacted at 0°C for 2 hours. The reaction solution was purified by preparative high-performance liquid chromatography to obtain the title compound of this step (16 mg, yield: 67.2%).

MS m/z (ESI): 1496.3 [M/2+H]⁺.

### Step 2: Preparation of Compound 28-2

Compound **28-1** (16 mg, 5.35 µmol) and diethylamine (1.88 mg, 25.40 µmol) were added to *N,N*-dimethylformamide (2 mL) and reacted at 25°C for 2 hours. The reaction solution was directly purified by reversed-phase column chromatography (preparation method B, elution gradient: A%=20% to 35%) to obtain the title compound of this step (14 mg, yield: 99.5%).

MS m/z (ESI): 1385.2 [M/2+H]⁺.

### Step 3: Preparation of Compound C-28

Compound **28-2** (14 mg, 5.05 µmol), 2,5-dioxopyrrolidin-1-yl-6-(2-(methylsulfonyl) pyrimidin-5-yl)hexa-5-ynoate (2.95 mg, 8.08 µmol) and *N,N*-diisopropylethylamine (0.94 mg, 7.22 µmol) were added in sequence to anhydrous *N,N-*dimethylformamide (2 mL) and reacted at 0°C for 2 hours. The reaction solution was purified by preparative high-performance liquid chromatography to obtain the title compound of this step (1.61 mg, yield: 11.4%).

MS m/z (ESI): 1510.3 [M/2+H]⁺.

### Example 1-27: Preparation of Compound C-29

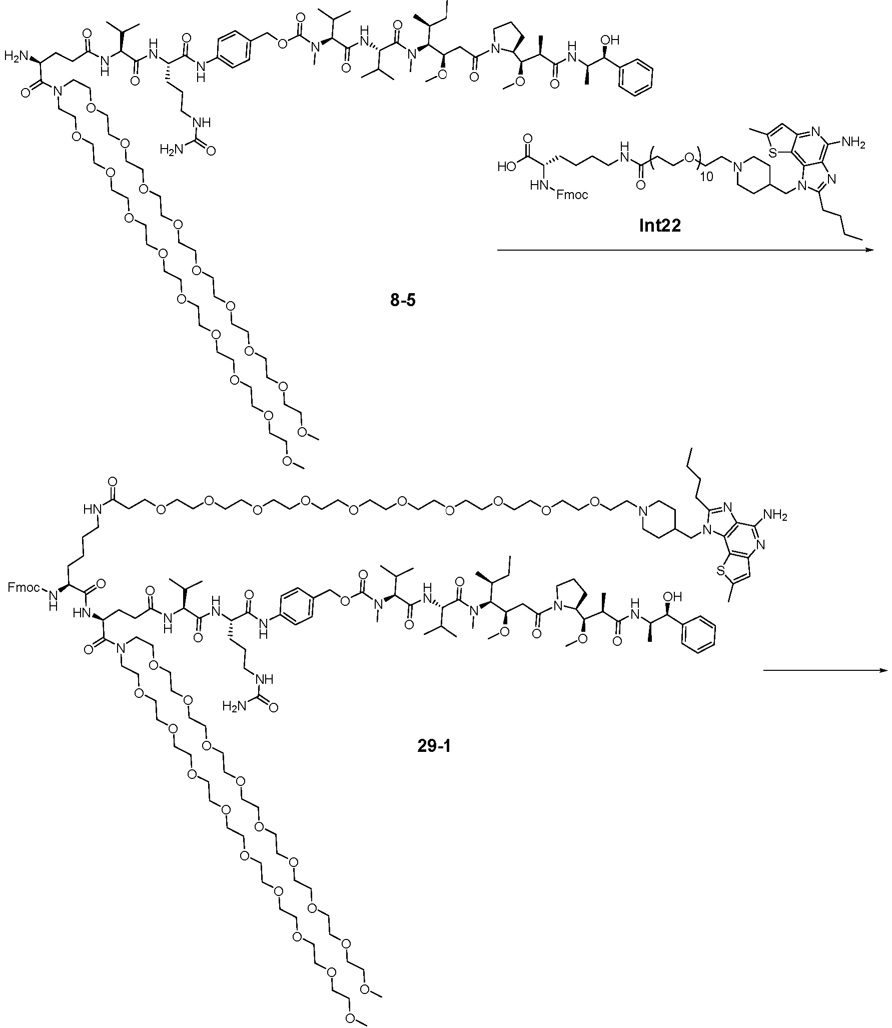

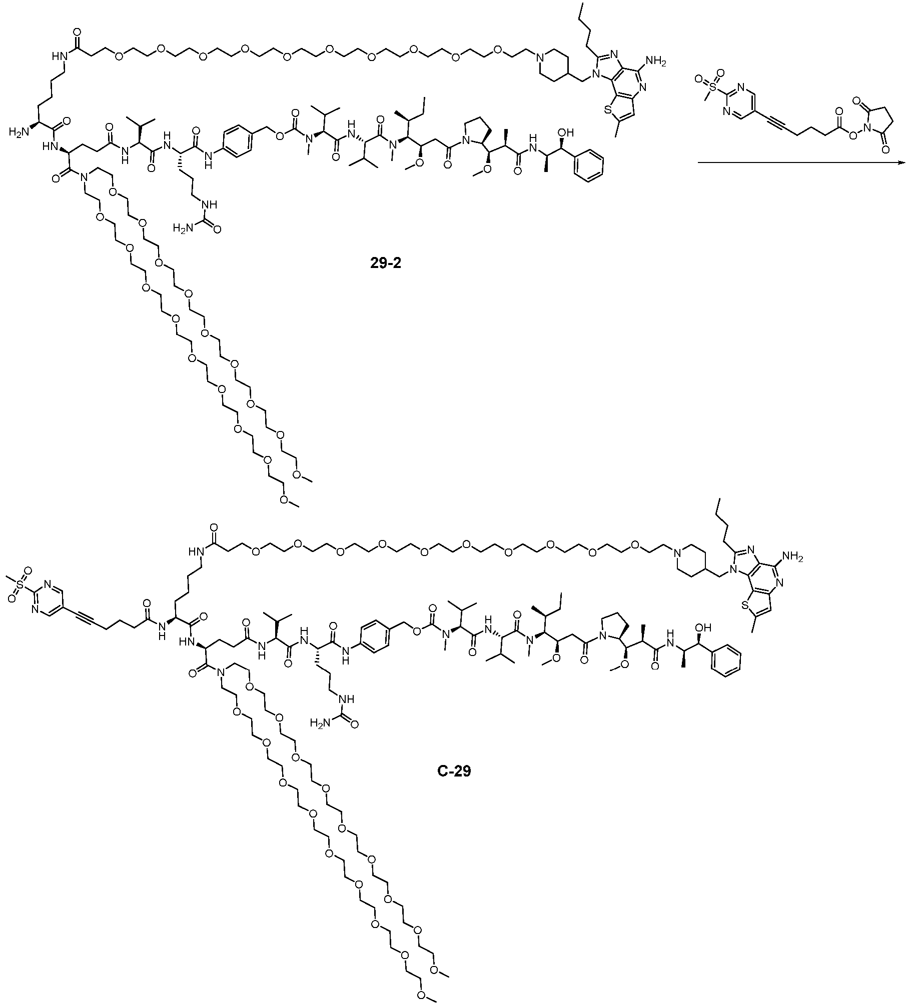

### Step 1: Preparation of Compound 29-1

Compound **Int22** (16.25 mg, 13.31 µmol), *N,N-*diisopropylethylamine (2.06 mg, 15.81 µmol, 2.64 µL), 2-(7-azobenzotriazole)*-N,N,N',N'*-tetramethylurea hexafluorophosphate (5.26 mg, 713.71 µmol) and Compound **8-5** (23 mg, 11.1 µmol) were added in sequence to anhydrous *N,N*-dimethylformamide (3 mL), and reacted at 0°C for 2 hours. The reaction solution was purified by preparative high-performance liquid chromatography to obtain the title compound of this step (16 mg, yield: 46.3%).

MS m/z (ESI): 1092.0 [M/3+H]⁺.

### Step 2: Preparation of Compound 29-2

Compound **29-1** (16 mg, 4.88 µmol) and diethylamine (1.71 mg, 23.21 µmol) were added to *N,N*-dimethylformamide (2 mL) and reacted at 25°C for 2 hours. The reaction solution was purified by preparative high-performance liquid chromatography to obtain the title compound of this step (9 mg, yield: 63.5%).

MS m/z (ESI): 1017.9 [M/3+H]⁺.

### Step 3: Preparation of Compound C-29

Compound **29-2** (9 mg, 2.95 µmol), 2,5-dioxopyrrolidin-1-yl-6-(2-(methylsulfonyl) pyrimidin-5-yl)hexa-5-ynoate (1.72 mg, 4.71 µmol) and *N,N*-diisopropylethylamine (0.55 mg, 4.2 µmol) were added in sequence to anhydrous *N,N-*dimethylformamide (2 mL) and reacted at 0°C for 2 hours. The reaction solution was purified by preparative high-performance liquid chromatography to obtain the title compound of this step (4.59 mg, yield: 48.9%).

MS m/z (ESI): 1101.3 [M/3+H]⁺.

### Example 1-28: Preparation of Compound C-30

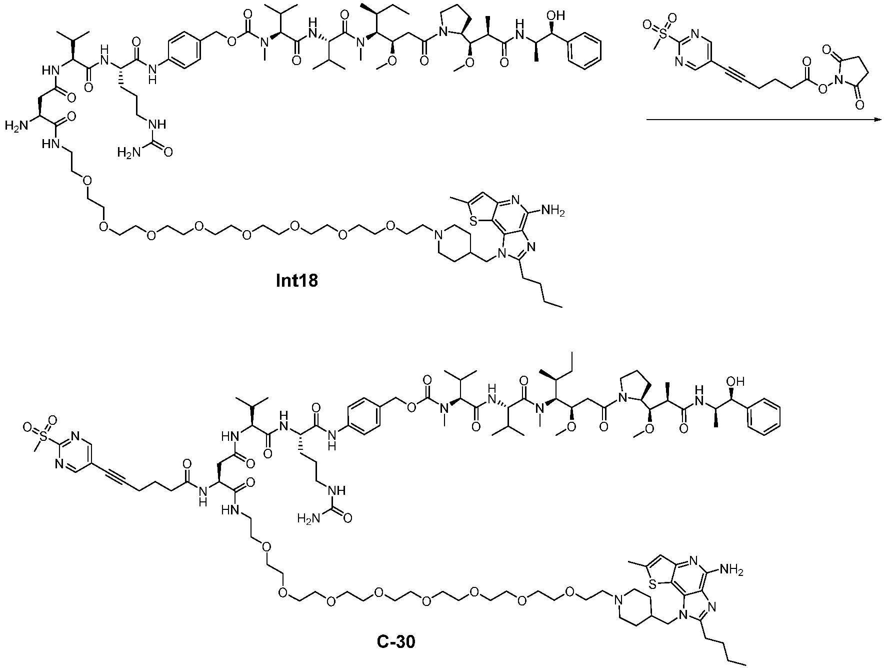

Compound **Int18** (27.90 mg, 14.14 µmol), 2,5-dioxopyrrolidin-1-yl-6-(2-(methylsulfonyl) pyrimidin-5-yl)hexa-5-ynoate (5.22 mg, 14.14 µmol) and *N,N*-diisopropylethylamine (3.68 mg, 28.27 µmol) were added in sequence to *N,N*-dimethylformamide (1 mL) and reacted at room temperature for 3 hours. The reaction solution was directly purified by reversed-phase column chromatography (preparation method B, elution gradient: A%=20% to 35%) to obtain the title compound of this step (7.88 mg, yield: 25.07%).

MS m/z (ESI): 1112.1 [M/2+H]⁺.

### Example 1-29: Preparation of Compound C-31

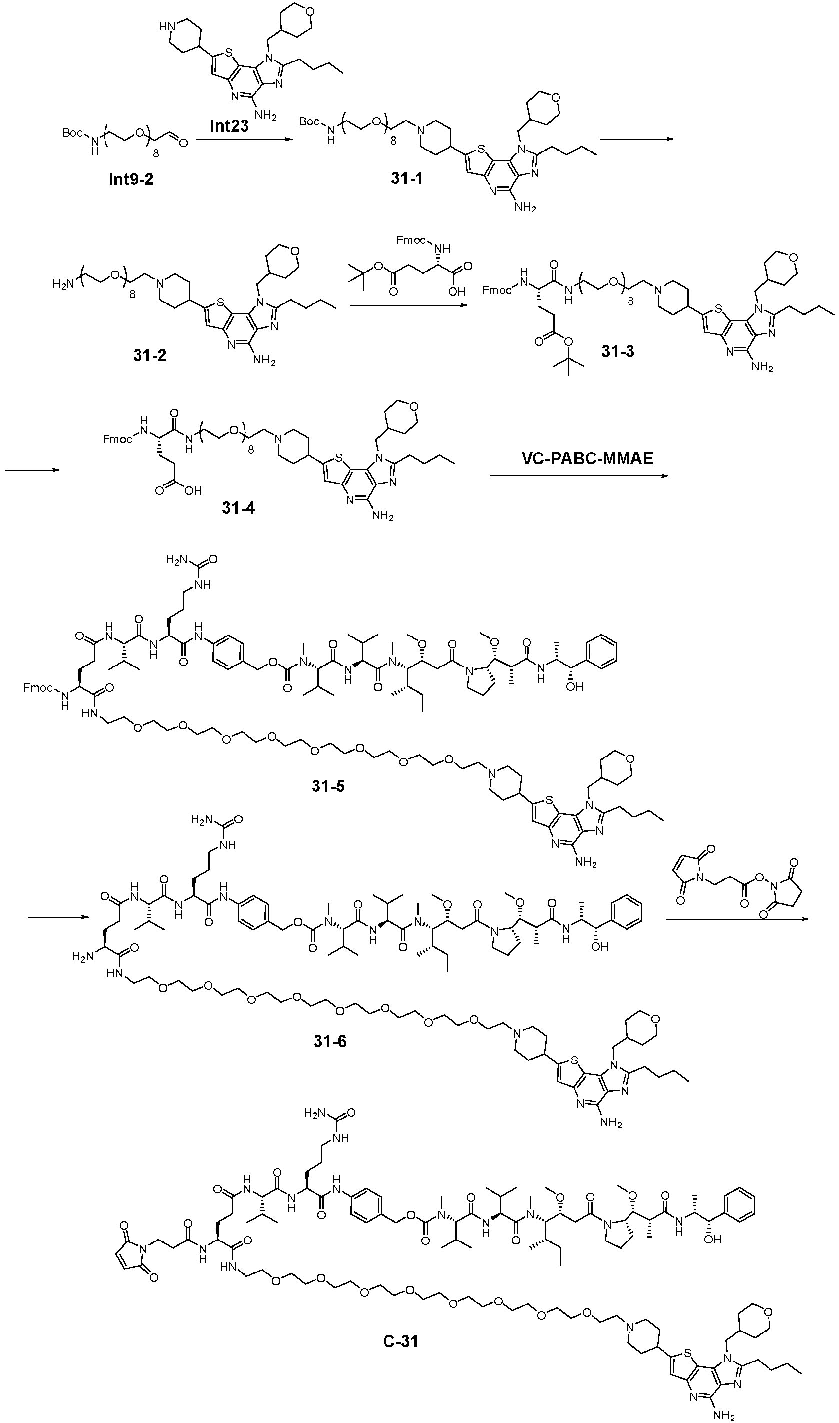

### Step 1: Preparation of Compound 31-1

Compound **Int9-2** (727.61 mg, 1347.36 µmol), Compound **Int23** (400 mg, 853.33 µmol), sodium cyanoborohydride (99.08 mg, 1576.7 µmol) and glacial acetic acid (51.72 mg, 853.33 µmol) were added in sequence to a mixed solvent of 1,2-dichloroethane (6 mL) and methanol (3 mL) and reacted at 25°C for 4 hours. The crude product obtained by concentrating the reaction solution was dissolved in methanol (3 mL) and purified by reverse-phase column chromatography (preparation method B, elution gradient: A%=20% to 35%) to obtain the title compound of this step (600 mg, yield: 76.2%).

MS m/z (ESI): 923.5 [M+H]⁺.

### Step 2: Preparation of Compound 31-2

Compound **31-1** (180 mg, 194.97 µmol) was added to dioxane hydrochloride solution (6 mL, 4M), and reacted at 25°C for 2 hours. The reaction solution was concentrated to obtain the title compound of this step (150 mg, yield: 93.3%).

MS m/z (ESI): 823.6 [M+H]⁺.

### Step 3: Preparation of Compound 31-3

(*S*)-2-((((9*H*-Fluoren-9-yl)methoxy)carbonyl)amino)-5-(tert-butoxy)-5-oxopentanoic acid (85.29 mg, 200.46 µmol), *N,N*-diisopropylethylamine (45.2 mg, 346.26 µmol), 2-(7-azobenzotriazole)-*N,N,N',N'*-tetramethylurea hexafluorophosphate (103.94 mg, 270.22 µmol) and Compound **31-2** (150 mg, 182.24 µmol) were added in sequence to anhydrous *N,N-*dimethylformamide (4 mL) and reacted at 0°C for 2 hours. The reaction solution was directly purified by reversed-phase column chromatography (preparation method B, elution gradient: A%=25% to 35%) to obtain the title compound of this step (170 mg, yield: 79.8%).

MS m/z (ESI): 1230.6 [M+H]⁺.

### Step 4: Preparation of Compound 31-4

Compound **31-3** (170 mg, 138.15 µmol) was dissolved in a mixed solvent of anhydrous dichloromethane (6 mL) and trifluoroacetic acid (3 mL) and reacted at 25°C for 2 hours. The reaction solution was concentrated to obtain the title compound of this step (133 mg, yield: 86.3%).

MS m/z (ESI): 1174.6 [M+H]⁺.

### Step 5: Preparation of Compound 31-5

Compound **31-4** (132.05 mg, 112.44 µmol), *NN-*diisopropylethylamine (23.24 mg, 178.03 µmol), 2-(7-azobenzotriazole)*-N,N,N',N'*-tetramethylurea hexafluorophosphate (51.28 mg, 133.52 µmol) and VC-PABC-MMAE (105.26 mg, 93.69 µmol) were added in sequence to anhydrous *N,N*-dimethylformamide (4 mL), and reacted at 0°C for 2 hours. The reaction solution was directly purified by reversed-phase column chromatography (preparation method B, elution gradient: A%=55% to 65%) to obtain the title compound of this step (110 mg, yield: 54.2%).

MS m/z (ESI): 1140.2 [M/2+H]⁺.

### Step 6: Preparation of Compound 31-6

Compound **31-5** (80 mg, 35.09 µmol) and diethylamine (12.19 mg, 166.68 µmol) were added to *N,N*-dimethylformamide (2 mL) and reacted at 25°C for 2 hours. The reaction solution was directly purified by reversed-phase column chromatography (preparation method B, elution gradient: A%=55% to 65%) to obtain the title compound of this step (50 mg, yield: 72.9%).

MS m/z (ESI): 686.4 [M/3+H]⁺.

### Step 7: Preparation of Compound C-31

Compound **31-6** (50 mg, 24.31 µmol), 3-maleimidopropionic acid hydroxysuccinimide ester (7.76 mg, 29.16 µmol) and *N,N*-diisopropylethylamine (4.52 mg, 34.63 µmol) were added in sequence to anhydrous *N,N*-dimethylformamide (2 mL) and reacted at 0°C for 2 hours. The reaction solution was purified by preparative high-performance liquid chromatography to obtain the title compound of this step (21.5 mg, yield: 41.3%).

MS m/z (ESI): 1104.6 [M/2+H]⁺.

### Example 1-30: Preparation of Compound C-32

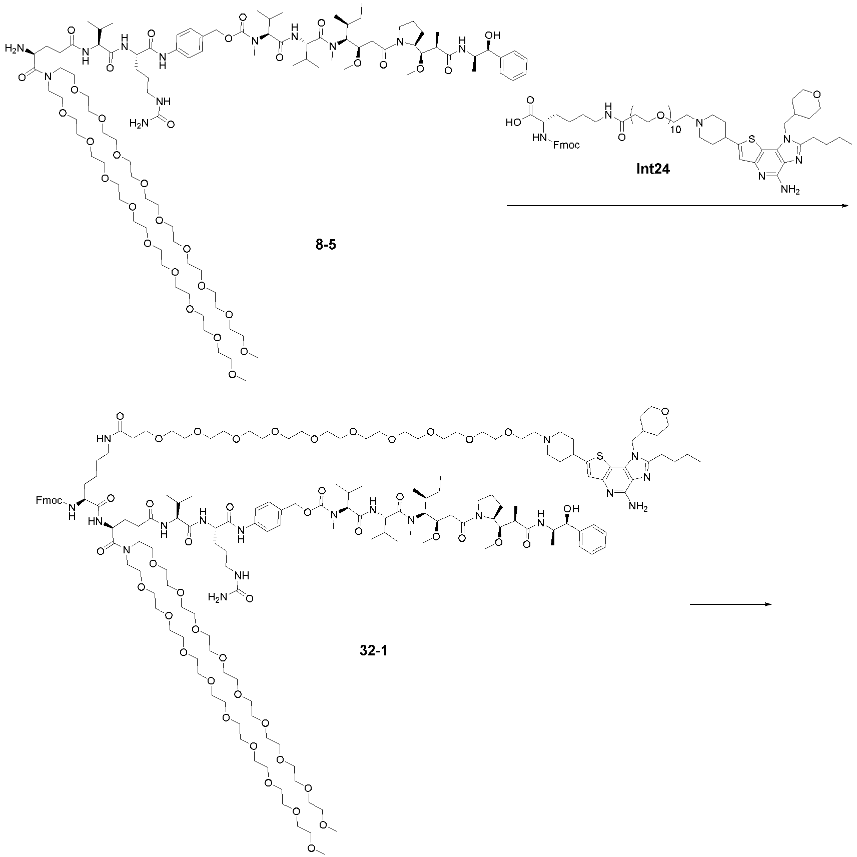

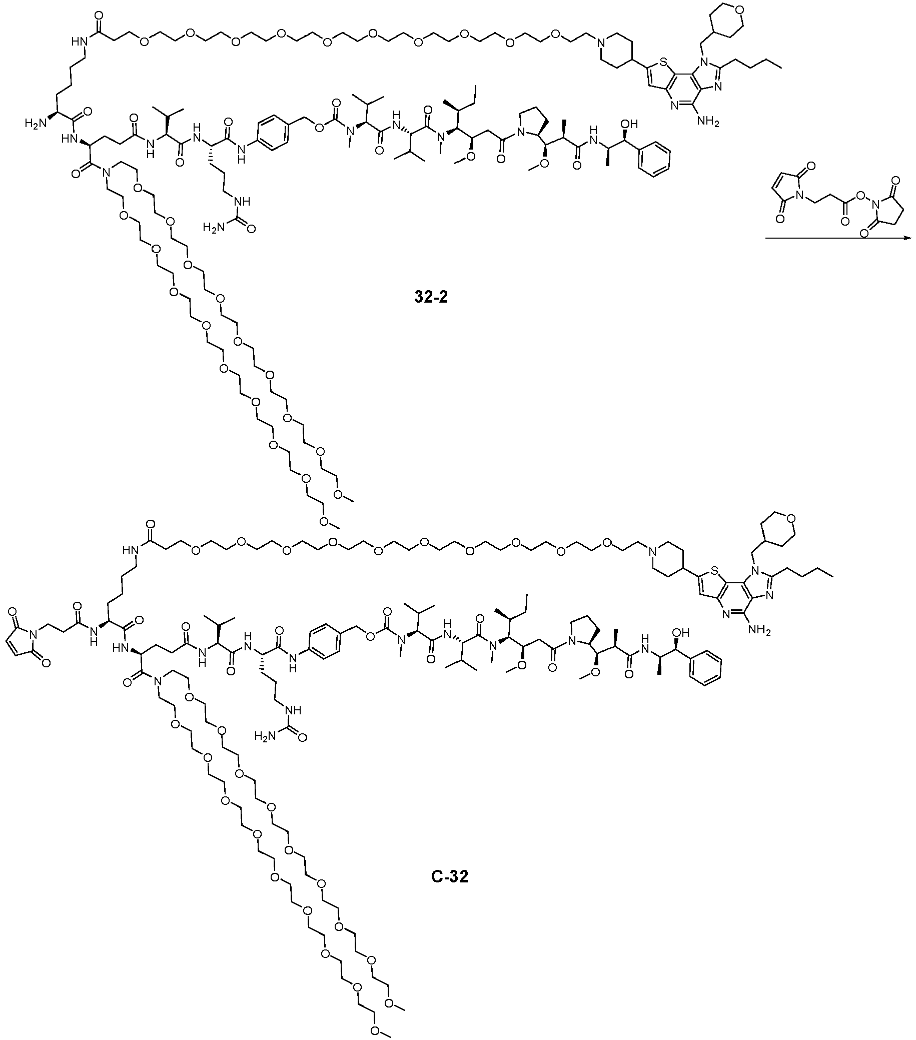

### Step 1: Preparation of Compound 32-1

Compound **Int24** (31.38 mg, 22.31 µmol), *NN-*diisopropylethylamine (3.77 mg, 28.88 µmol), 2-(7-azobenzotriazole)-*N,N,N',N'*-tetramethylurea hexafluorophosphate (9.61 mg, 25.03 µmol) and Compound **8-5** (42 mg, 20.26 µmol) were added in sequence to anhydrous *N,N-*dimethylformamide (4 mL), and reacted at 0°C for 2 hours. The reaction solution was directly purified by reversed-phase column chromatography (preparation method B, elution gradient: A%=35% to 45%) to obtain the title compound of this step (38 mg, yield: 59.0%).

MS m/z (ESI): 1672.5 [M/2+H]⁺.

### Step 2: Preparation of Compound 32-2

Compound **32-1** (38 mg, 11.36 µmol) and diethylamine (3.95 mg, 53.96 µmol) were added to *N,N*-dimethylformamide (2 mL) and reacted at 25°C for 2 hours. The reaction solution was purified by preparative high-performance liquid chromatography to obtain the title compound of this step (12 mg, yield: 61.2%).

MS m/z (ESI): 1041.3 [M/3+H]⁺.

### Step 3: Preparation of Compound C-32

Compound **32-2** (10 mg, 3.21 µmol), 3-maleimidopropionic acid hydroxysuccinimide ester (1.02 mg, 3.84 µmol) and *N,N*-diisopropylethylamine (0.6 mg, 4.56 µmol) were added in sequence to anhydrous *N,N*-dimethylformamide (2 mL) and reacted at 0°C for 2 hours. The reaction solution was purified by preparative high-performance liquid chromatography to obtain the title compound of this step (6.18 mg, yield: 35.6%).

MS m/z (ESI): 1637.0 [M/2+H]⁺.

### Example 1-31: Preparation of Compound C-33

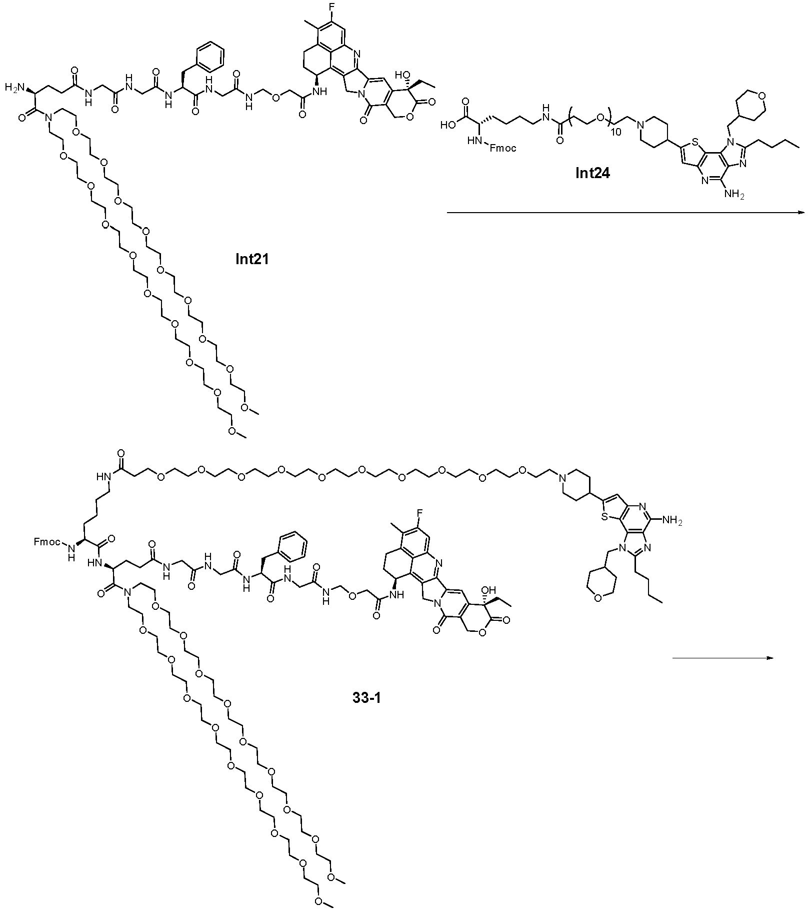

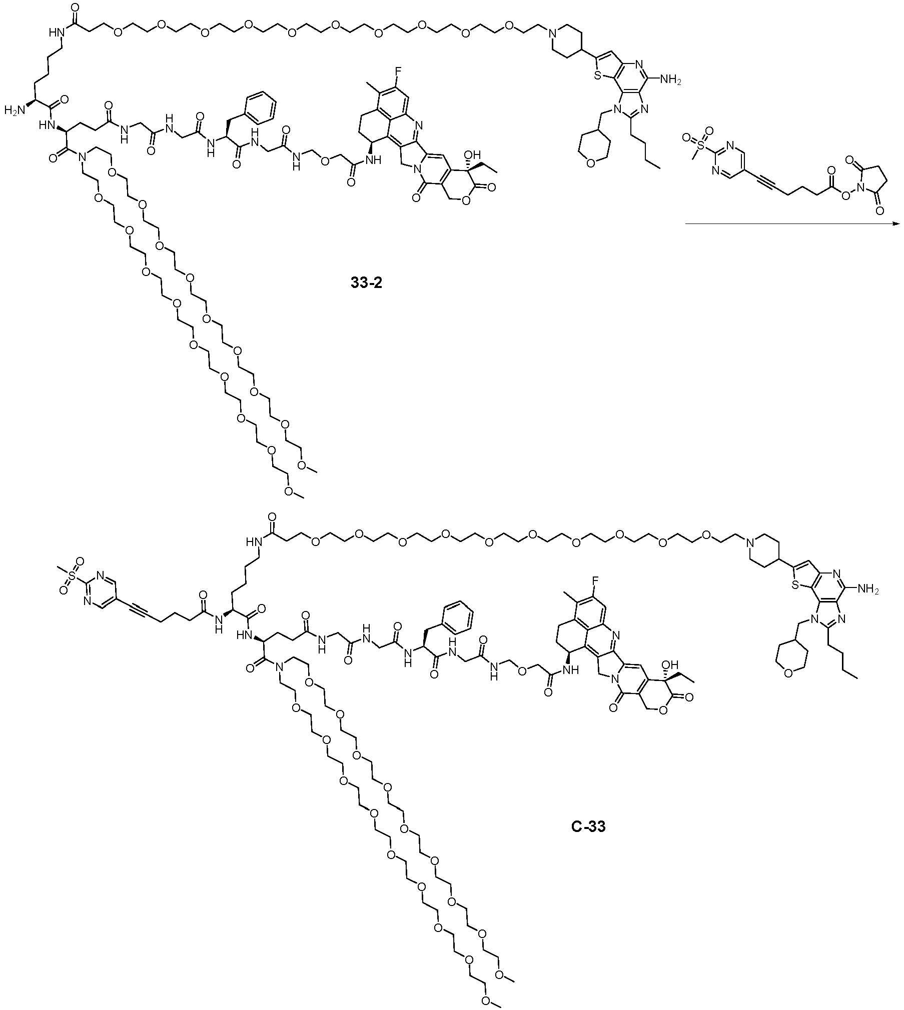

### Step 1: Preparation of Compound 33-1

Compound **Int24** (39.66 mg, 27.94 µmol), *NN-*diisopropylethylamine (6.93 mg, 53.07 µmol), 2-(7-azobenzotriazole)-*N,N,N',N'*-tetramethylurea hexafluorophosphate (15.93 mg, 33.48 µmol) and Compound **Int21** (50 mg, 27.94 µmol) were added in sequence to anhydrous *N,N-*dimethylformamide (4 mL), and reacted at 0°C for 2 hours. The reaction solution was directly purified by reversed-phase column chromatography (preparation method B, elution gradient: A%=20% to 30%) to obtain the title compound of this step (66 mg, yield: 81.2%).

MS m/z (ESI): 1531.3 [M/2+H]⁺.

### Step 2: Preparation of Compound 33-2

Compound **33-1** (66 mg, 21.55 µmol) and diethylamine (7.56 mg, 102.37 µmol) were added to *N,N*-dimethylformamide (2 mL) and reacted at 25°C for 2 hours. The reaction solution was purified by preparative high-performance liquid chromatography to obtain the title compound of this step (22 mg, yield: 37.8%).

MS m/z (ESI): 1420.3 [M/2+H]⁺.

### Step 3: Preparation of Compound C-33

Compound 33-2 (14 mg, 4.93 µmol), 2,5-dioxopyrrolidin-1-yl-6-(2-(methylsulfonyl) pyrimidin-5-yl)hexa-5-ynoate (2.88 mg, 7.88 µmol) and *N,N*-diisopropylethylamine (0.92 mg, 7.02 µmol) were added in sequence to anhydrous *N,N-*dimethylformamide (2 mL) and reacted at 0°C for 2 hours. The reaction solution was purified by preparative high-performance liquid chromatography to obtain the title compound of this step (6.28 mg, yield: 43.4%).

MS m/z (ESI): 1545.3 [M/2+H]⁺.

### Example 1-32: Preparation of Compound C-34

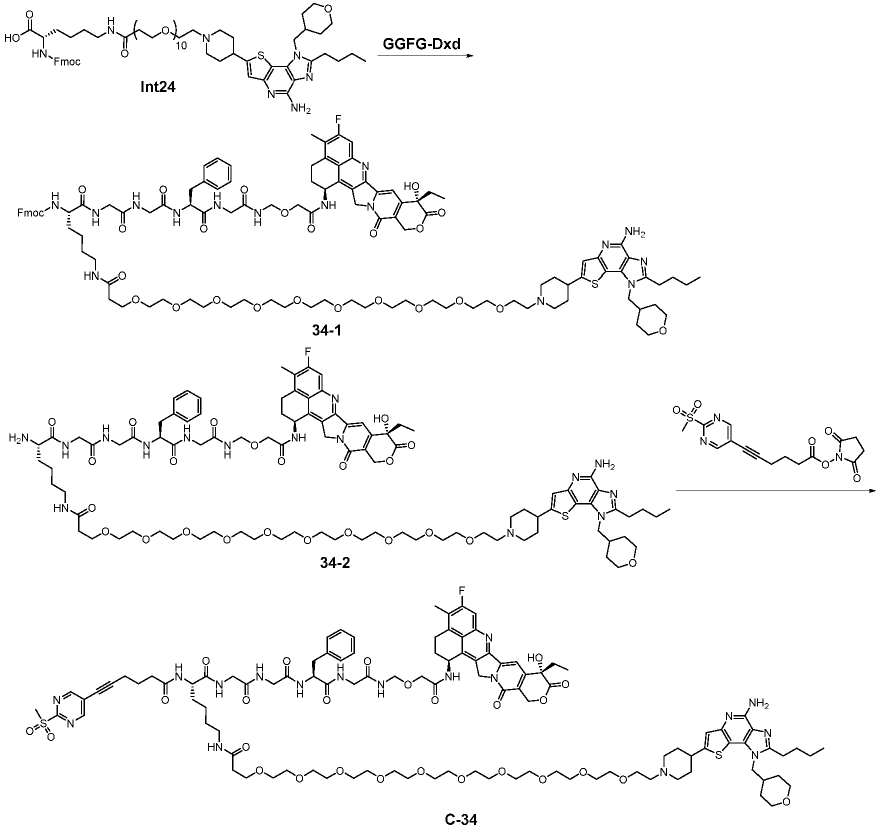

### Step 1: Preparation of Compound 34-1

Compound **Int24** (85 mg, 65.86 µmol), *NN-*diisopropylethylamine (14.85 mg, 113.76 µmol), 2-(7-azobenzotriazole)-*N,N,N',N'*-tetramethylurea hexafluorophosphate (29.6 mg, 77.83 µmol) and GGFG-Dxd (50.34 mg, 59.87 µmol) were added in sequence to anhydrous *N,N*-dimethylformamide (4 mL), and reacted at 0°C for 2 hours. The reaction solution was directly purified by reversed-phase column chromatography (preparation method B, elution gradient: A%=15% to 25%) to obtain the title compound of this step (66 mg, yield: 54.9%).

MS m/z (ESI): 1057.0 [M/2+H]⁺.

### Step 2: Preparation of Compound 34-2

Compound **34-1** (65 mg, 30.76 µmol) and diethylamine (4.5 mg, 61.51 µmol) were added to *N,N*-dimethylformamide (2 mL) and reacted at 25°C for 2 hours. The reaction solution was directly purified by reversed-phase column chromatography (preparation method B, elution gradient: A%=35% to 45%) to obtain the title compound of this step (36 mg, yield: 65.1 %).

MS m/z (ESI): 946.0 [M/2+H]⁺.

### Step 3: Preparation of Compound C-34

Compound **34-2** (36 mg, 19.03 µmol), 2,5-dioxopyrrolidin-1-yl-6-(2-(methylsulfonyl) pyrimidin-5-yl)hexa-5-ynoate (10.43 mg, 28.56 µmol) and *NN-*diisopropylethylamine (3.54 mg, 27.13 µmol) were added in sequence to anhydrous *N,N*-dimethylformamide (2 mL), and reacted at 0°C for 2 hours. The reaction solution was purified by preparative high-performance liquid chromatography to obtain the title compound of this step (8.47 mg, yield: 21.9%).

MS m/z (ESI): 1071.0 [M/2+H]⁺.

### Example1-33: Preparation of Compound C-35

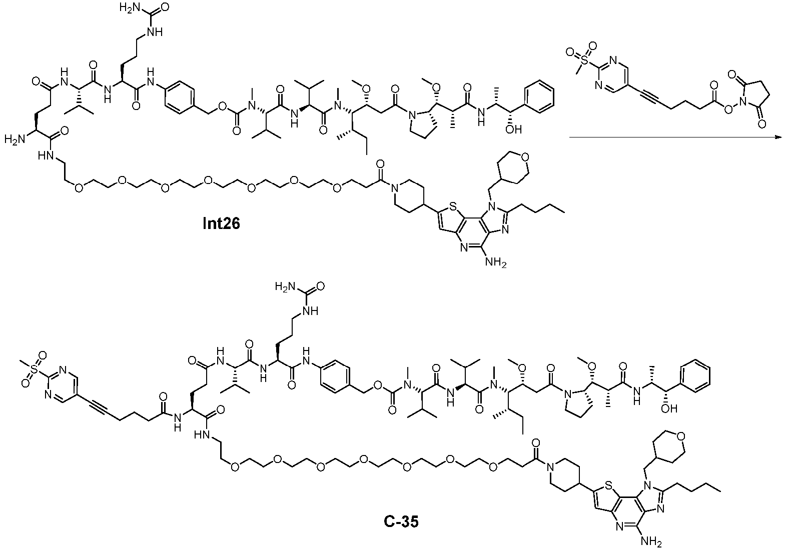

Compound **Int26** (113.67 mg, 55.68 µmol),2,5-dioxopyrrolidin-1-yl-6-(2-(methylsulfonyl) pyrimidin-5-yl)hexa-5-ynoate (22.27 mg, 60.95 µmol) and *N,N*-diisopropylethylamine (11.63 mg, 89.08 µmol) were added in sequence to anhydrous *N,N*-dimethylformamide (2 mL) and reacted at 0°C for 2 hours. The reaction solution was purified by preparative high-performance liquid chromatography to obtain the title compound of this step (55 mg, yield: 53.9%).

MS m/z (ESI): 1146.1 [M/2+H]⁺.

### Example 1-34: Preparation of Compound C-36

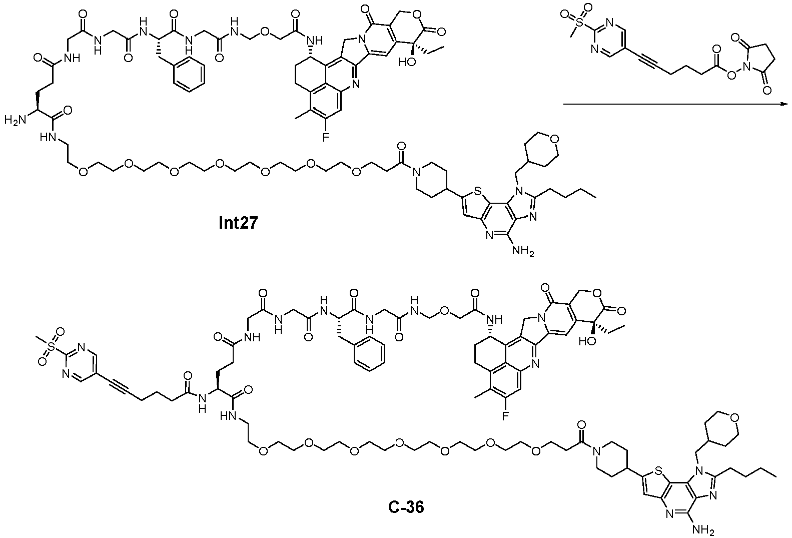

Compound **Int27** (90 mg, 51.17 µmol), 2,5-dioxopyrrolidin-1-yl-6-(2-(methylsulfonyl) pyrimidin-5-yl)hexa-5-ynoate (23.02 mg, 63.01 µmol) and *NN-*diisopropylethylamine (12.02 mg, 92.1 µmol) were added in sequence to anhydrous *N,N*-dimethylformamide (2 mL) and reacted at 0°C for 2 hours. The reaction solution was purified by preparative high-performance liquid chromatography to obtain the title compound of this step (27 mg, yield: 29.2%).

MS m/z (ESI): 1005.0 [M/2+H]⁺.

### Example1-35: Preparation of Compound C-37

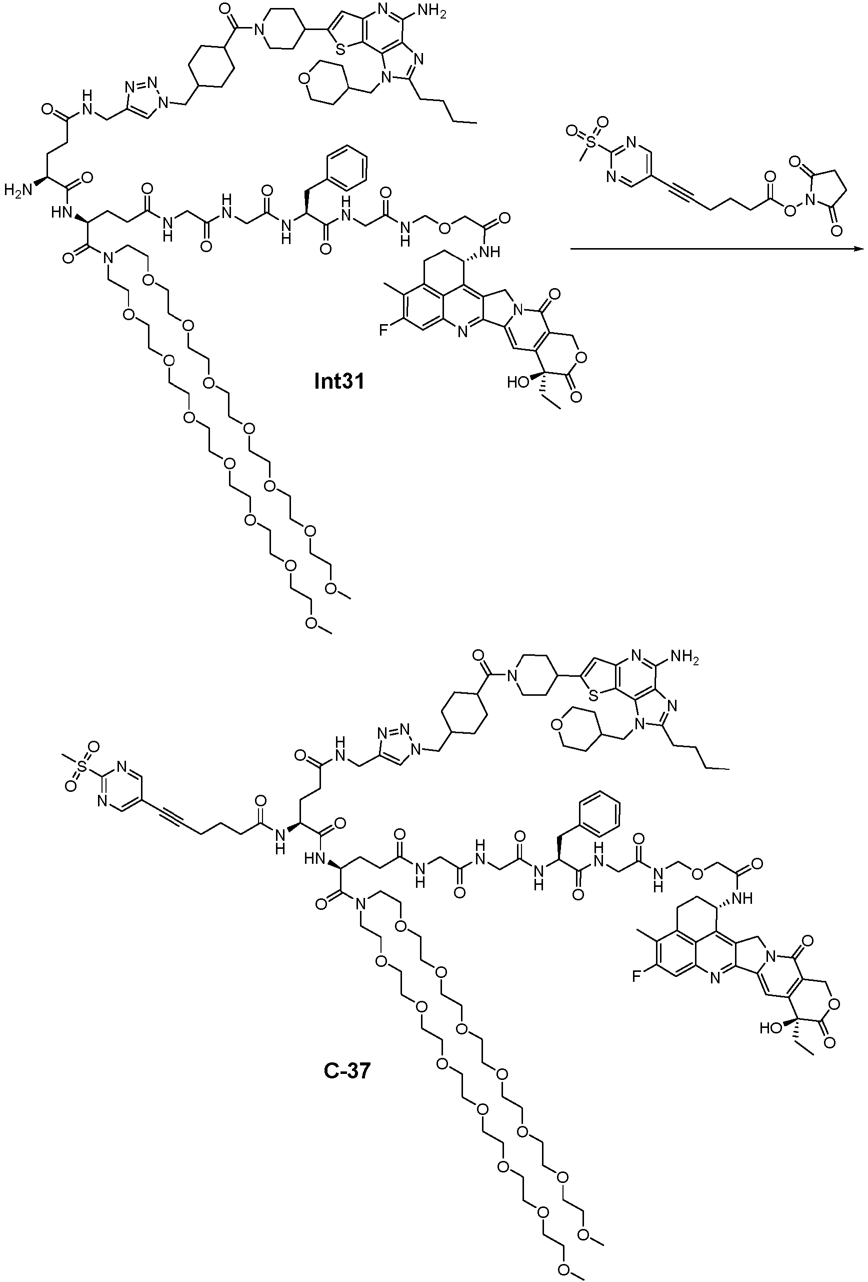

Compound **Int31** (12 mg, 5.05 µmol), 2,5-dioxopyrrolidin-1-yl-6-(2-(methylsulfonyl) pyrimidin-5-yl)hexa-5-ynoate (2.4 mg, 6.58 µmol) and N,N-diisopropylethylamine (1.25 mg, 9.61 µmol) were added in sequence to anhydrous *N,N-*dimethylformamide (2 mL) and reacted at 0°C for 2 hours. The reaction solution was purified by preparative high-performance liquid chromatography to obtain the title compound of this step (1.67 mg, yield: 13.3%).

MS m/z (ESI): 1311.6 [M/2+H]⁺.

### Example 1-36: Preparation of Compound C-38

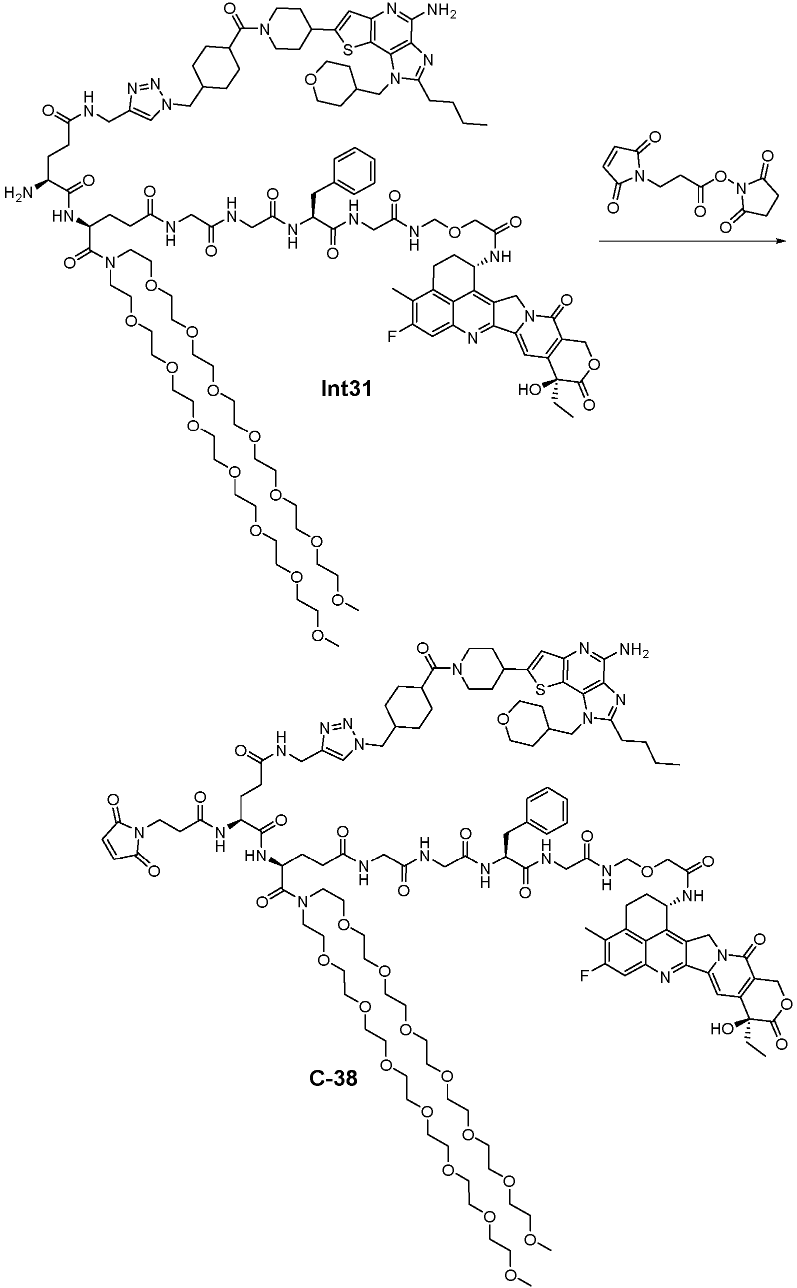

Compound **Int31** (12 mg, 5.05 µmol),3-maleimidopropionic acid hydroxysuccinimide ester (1.75 mg, 6.58 µmol) and *NN-*diisopropylethylamine (1.25 mg, 9.61 µmol) were added to anhydrous *N,N*-dimethylformamide (2 mL) and reacted at 0°C for 2 hours. The reaction solution was purified by preparative high-performance liquid chromatography to obtain the title compound of this step (1.92 mg, yield: 15.8%).

MS m/z (ESI): 1262.1 [M/2+H]⁺.

### Example 1-37: Preparation of Compound C-39

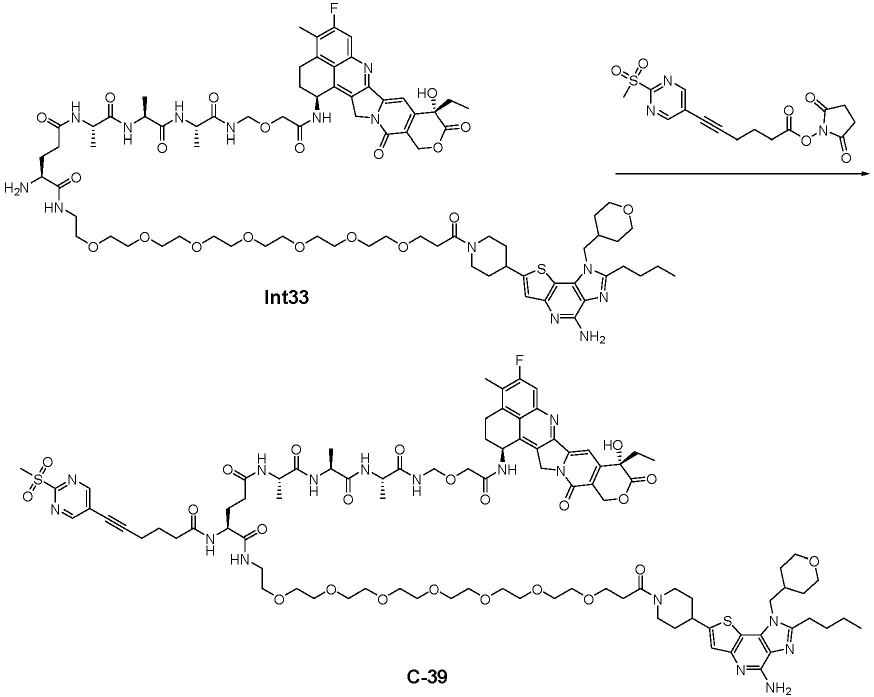

Compound **Int33** (70 mg, 42.33 µmol),2,5-dioxopyrrolidin-1-yl-6-(2-(methylsulfonyl) pyrimidin-5-yl)hexa-5-ynoate (20.1 mg, 55.02 µmol) and *N,N*-diisopropylethylamine (7.87 mg, 60.31 µmol) were added in sequence to anhydrous *N,N*-dimethylformamide (2 mL) and reacted at 0°C for 2 hours. The reaction solution was purified by preparative high-performance liquid chromatography to obtain the title compound of this step (2.53 mg, yield: 7.1%).

MS m/z (ESI): 1903.8 [M+H]⁺.

### Example1-38: Preparation of Compound C-40

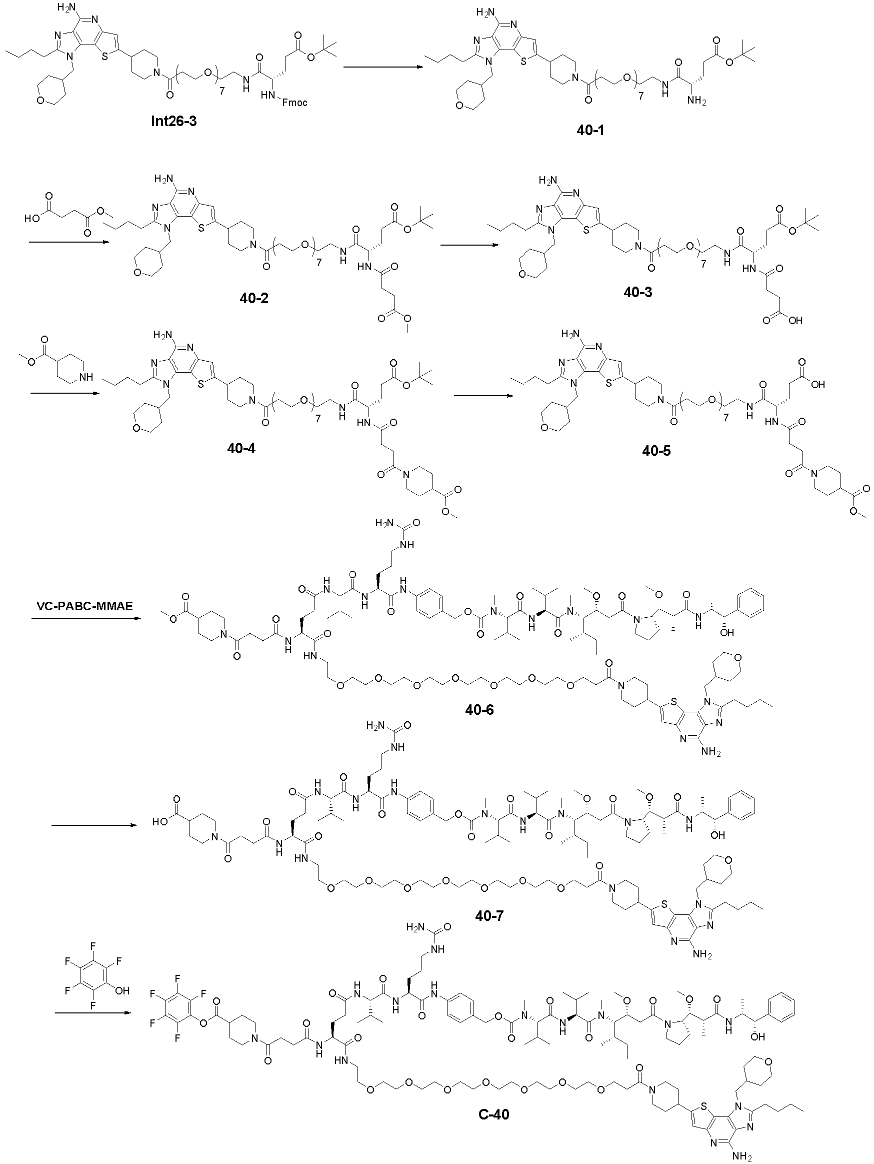

### Step 1: Preparation of Compound 40-1

Compound **Int26-3** (250 mg, 205.84 µmol) and diethylamine (73.14 mg, 1 mmol) were added to N,N-dimethylformamide (4 mL) and reacted at 25°C for 2 hours. The reaction solution was directly purified by reversed-phase column chromatography (preparation method B, elution gradient: A%=25% to 35%) to obtain the title compound of this step (175 mg, yield: 85.7%).

MS m/z (ESI): 992.6 [M+H]⁺.

### Step 2: Preparation of Compound 40-2

Monomethyl succinate (26.47 mg, 200.35 µmol), *N*,*N-*diisopropylethylamine (52.71 mg, 407.85 µmol), 2-(7-azobenzotriazole)-*N*,*N*,*N*',*N*'-tetramethylurea hexafluorophosphate (62.03 mg, 163.14 µmol) and Compound **40-1** (142 mg, 143.11 µmol) were added in sequence to anhydrous *N,N*-dimethylformamide (4 mL), and reacted at 0°C for 2 hours. The reaction solution was directly purified by reversed-phase column chromatography (preparation method B, elution gradient: A%=35% to 45%) to obtain the title compound of this step (120 mg, yield: 79.8%).

MS m/z (ESI): 1106.6 [M+H]⁺.

### Step 3: Preparation of Compound 40-3

Compound **40-2** (145 mg, 131.06 µmol) was dissolved in methanol (5 mL), added with lithium hydroxide aqueous solution (15.06 mg, 622.53 µmol, 2 mL), and reacted at 25°C for 4 hours. The reaction solution was directly purified by reversed-phase column chromatography (preparation method B, elution gradient: A%=35% to 45%) to obtain the title compound of this step (100 mg, yield: 73.5%).

MS m/z (ESI): 1092.6 [M+H]⁺.

### Step 4: Preparation of Compound 40-4

Compound **40-3** (50 mg, 47.16 µmol), N,N-diisopropylethylamine (12.63 mg, 96.76 µmol), 2-(7-azobenzotriazole)-*N,N,N',N'*-tetramethylurea hexafluorophosphate (119.84 mg, 52.18 µmol) and 4-piperidinecarboxylic acid methyl ester (6.55 mg, 47.16 µmol) were added in sequence to anhydrous N,N-dimethylformamide (3 mL), and reacted at 0°C for 2 hours. The reaction solution was directly purified by reversed-phase column chromatography (preparation method B, elution gradient: A%=20% to 30%) to obtain the title compound of this step (50 mg, yield: 94.4%).

MS m/z (ESI): 1217.7 [M+H]⁺.

### Step 5: Preparation of Compound 40-5

Compound **40-4** (50 mg, 41.06 µmol) was dissolved in a mixed solvent of anhydrous dichloromethane (3 mL) and trifluoroacetic acid (1 mL) and reacted at 25°C for 4 hours. The crude product obtained by concentrating the reaction solution was purified by reverse-phase column chromatography (preparation method B, elution gradient: A%=40% to 55%) to obtain the title compound of this step (40 mg, yield: 88.3%).

MS m/z (ESI): 1161.6 [M+H]⁺.

### Step 6: Preparation of Compound 40-6

Compound **40-5** (42 mg, 36.16 µmol), N,N-diisopropylethylamine (12.63 mg, 96.76 µmol), 2-(7-azobenzotriazole)-N,N,N',N'-tetramethylurea hexafluorophosphate (15.68 mg, 41.23 µmol) and VC-PABC-MMAE (40.63 mg, 36.16 µmol) were added in sequence to anhydrous N,N-dimethylformamide (3 mL), and reacted at 0°C for 2 hours. The crude product obtained by concentrating the reaction solution was purified by reverse-phase column chromatography (preparation method B, elution gradient: A%=20% to 30%) to obtain the title compound of this step (60 mg, yield: 77.1 %).

MS m/z (ESI): 1133.7 [M/2+H]⁺.

### Step 7: Preparation of Compound 40-7

Compound **40-6** (60 mg, 26.47 µmol) was dissolved in methanol (3 mL), added with lithium hydroxide aqueous solution (3.04 mg, 125.73 µmol, 1 mL), and reacted at 25°C for 4 hours. The reaction solution was directly purified by reversed-phase column chromatography (preparation method B, elution gradient: A%=35% to 45%) to obtain the title compound of this step (40 mg, yield: 70.6%).

MS m/z (ESI): 1126.7 [M/2+H]⁺.

### Step 8: Preparation of Compound C-40

Compound **40-7** (10 mg, 4.44 µmol), pentafluorophenol (1.62 mg, 8.8 µmol) and *N,N'*-dicyclohexylcarbodiimide (1.83 mg, 8.88 µmol) were added in sequence to anhydrous dichloromethane (2 mL), and reacted at 25°C for 3 hours. The crude product obtained by concentrating the reaction solution was purified by preparative high-performance liquid chromatography to obtain the title compound of this step (3 mg, yield: 29.4%).

MS m/z (ESI): 1209.7 [M/2+H]⁺.

### Example 1-39: Preparation of Compound C-41

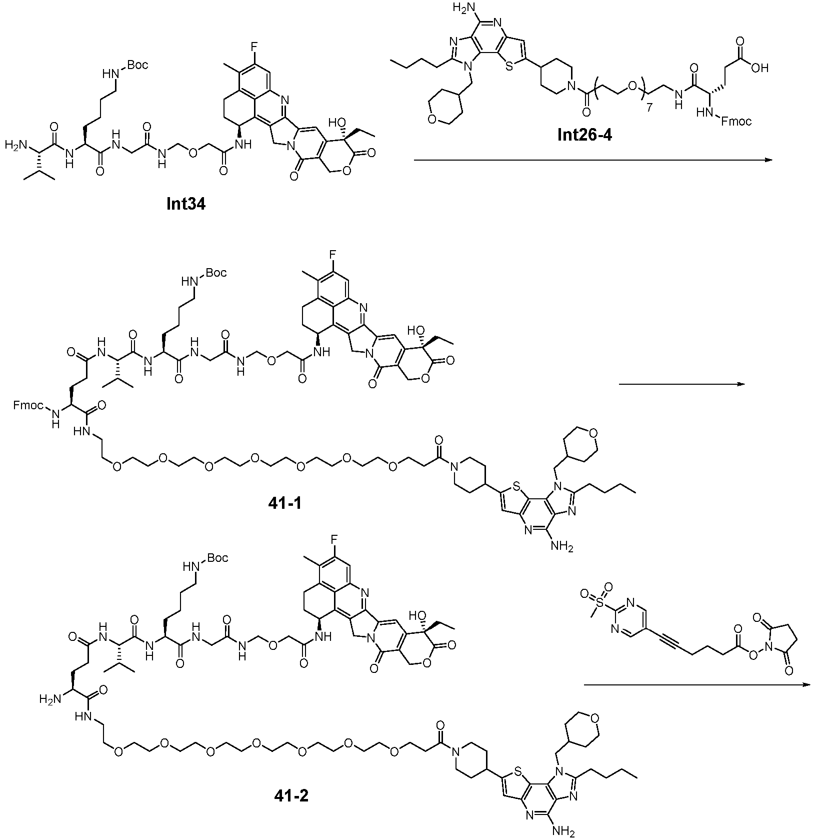

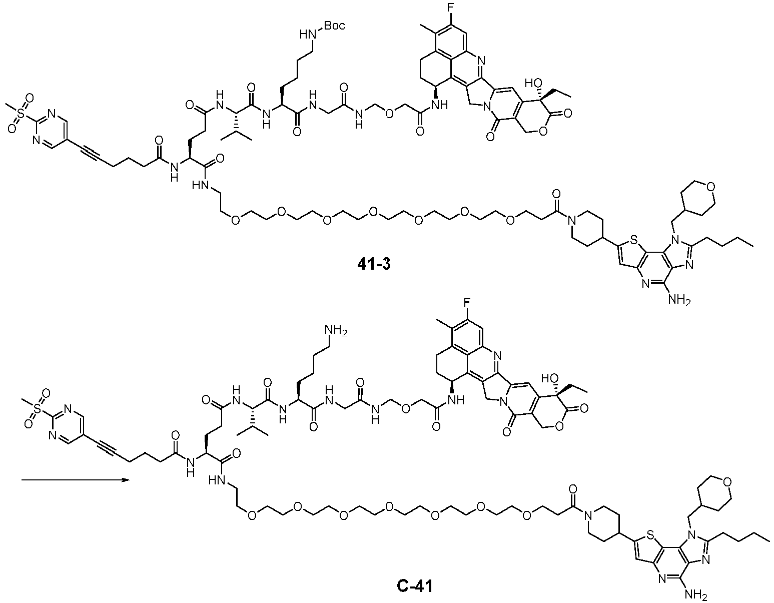

### Step 1: Preparation of Compound 41-1

Compound **Int26-4** (50 mg, 41.85 µmol), Compound Int34 (37.95 mg, 41.85 µmol), *N,N*-diisopropylethylamine (13.63 mg, 104.61 µmol) and 2-(7-azobenzotriazole)-*N,N,N',N'-*tetramethylurea hexafluorophosphate (19.27 mg, 50.21 µmol) were added in sequence to *N,N*-dimethylformamide (1 mL), and reacted at room temperature for 2 hours. The reaction solution was directly purified by reversed-phase column chromatography (preparation method A, elution gradient: A%=40%-48%) to obtain the title compound of this step (43 mg, yield: 50.19%).

MS m/z (ESI): 1024.0 [M/2+H]⁺.

### Step 2: Preparation of Compound 41-2

Compound **41-1** (40 mg, 19.54 µmol) and piperidine (3.33 mg, 39.07 µmol) were added to N,N-dimethylformamide (1 mL) and reacted at room temperature for 2 hours. After the reaction solution was concentrated, methyl tert-butyl ether (5 mL) was added, treated with ultrasonic for 5 minutes, allowed to stand, and filtered, and the solid was dried to obtain the title compound of this step (28 mg, yield: 70.67%).

MS m/z (ESI): 1824.9 [M+H]⁺.

### Step 3: Preparation of Compound 41-3

Compound **41-2** (28 mg, 15.34 µmol), 2,5-dioxopyrrolidin-1-yl-6-(2-(methylsulfonyl) pyrimidin-5-yl)hexa-5-ynoate (8.41 mg, 23.01 µmol) and N,N-diisopropylethylamine (4.00 mg, 30.68 µmol) were added in sequence to *N,N*-dimethylformamide (1 mL), and reacted at room temperature for 2 hours. The reaction solution was directly purified by reversed-phase column chromatography (preparation method A, elution gradient: A%=35%-40%) to obtain the title compound of this step (30 mg, yield: 94.22%).

MS m/z (ESI): 1038.0 [M/2+H]⁺.

### Step 4: Preparation of Compound C-41

Compound 41-3 (30 mg, 13.01 µmol) and anhydrous zinc bromide (11.72 mg, 52.04 µmol) were added to anhydrous dichloromethane (2 mL) and reacted at room temperature for 2 hours. The reaction solution was directly purified by reversed-phase column chromatography (preparation method A, elution gradient: A%=25% to 35%) to obtain the title compound of this step (4.65 mg, yield: 16.80%).

MS m/z (ESI): 1974.9 [M+H]⁺.

### Example 1-40: Preparation of Compound C-42

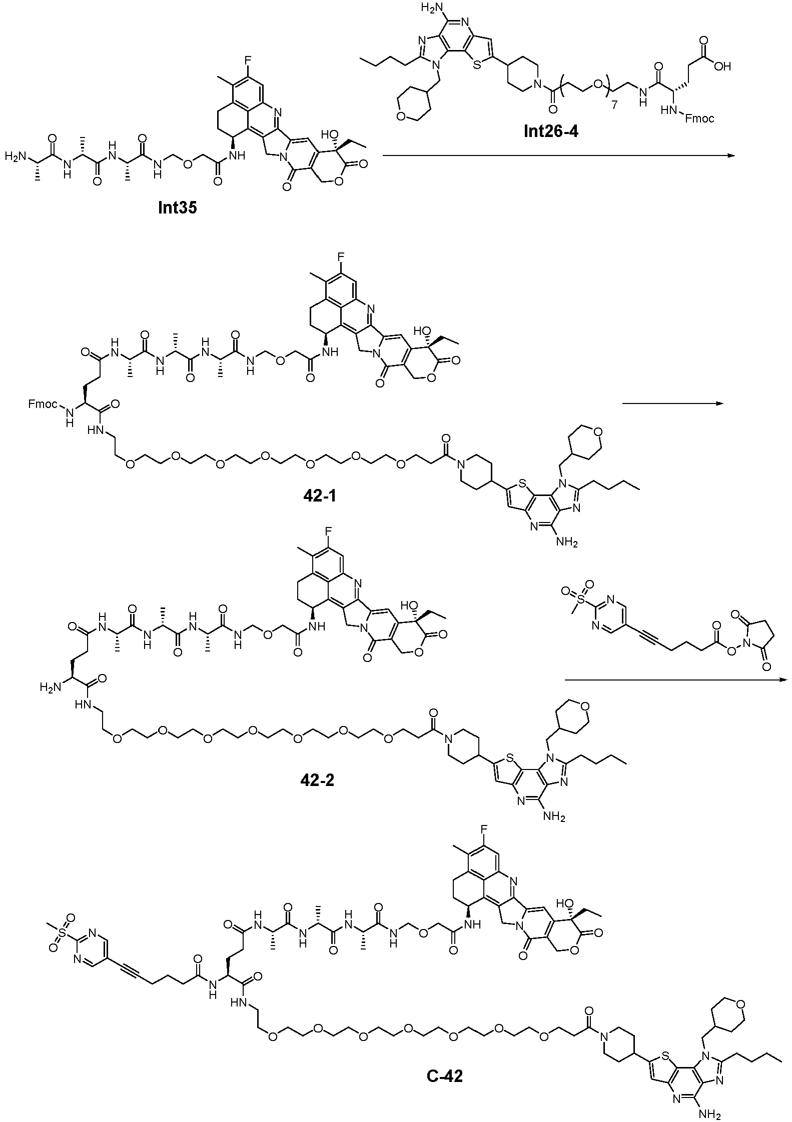

### Step 1: Preparation of Compound 42-1

Compound Int26-4 (51.95 mg, 44.85 µmol), N,N-diisopropylethylamine (11.09 mg, 85.84 µmol), 2-(7-azobenzotriazole)-*N*,*N*,*N*',*N*'-tetramethylurea hexafluorophosphate (22.85 mg, 60.08 µmol) and Compound Int35 (30 mg, 40.77 µmol) were added in sequence to anhydrous *N,N*-dimethylformamide (3 mL), and reacted at 0°C for 2 hours. The reaction solution was directly purified by reversed-phase column chromatography (preparation method B, elution gradient: A%=45%-50%) to obtain the title compound of this step (56 mg, yield: 73.2%).

MS m/z (ESI): 1875.8 [M+H]⁺.

### Step 2: Preparation of Compound 42-2

Compound **42-1** (57 mg, 30.38 µmol) and diethylamine (11.22 mg, 151.91 µmol) were added to N,N-dimethylformamide (2 mL) and reacted at 25°C for 2 hours. The reaction solution was directly purified by reversed-phase column chromatography (preparation method B, elution gradient: A%=45% to 55%) to obtain the title compound of this step (30 mg, yield: 59.7%).

MS m/z (ESI): 827.4 [M/2+H]⁺.

### Step 3: Preparation of Compound C-42

Compound **42-2** (30 mg, 18.14 µmol), 2,5-dioxopyrrolidin-1-yl-6-(2-(methylsulfonyl) pyrimidin-5-yl)hexa-5-ynoate (8.62 mg, 23.58 µmol) and N,N-diisopropylethylamine (3.55 mg, 27.21 µmol) were added in sequence to anhydrous N,N-dimethylformamide (2 mL) and reacted at 0°C for 2 hours. The reaction solution was purified by preparative high-performance liquid chromatography to obtain the title compound of this step (11 mg, yield: 31.8%).

MS m/z (ESI): 952.4 [M/2+H]⁺.

### 2. Preparation of immunomodulatory antibody-drug conjugate (iADC)

### Example 2-1: Trastuzumab-C-7

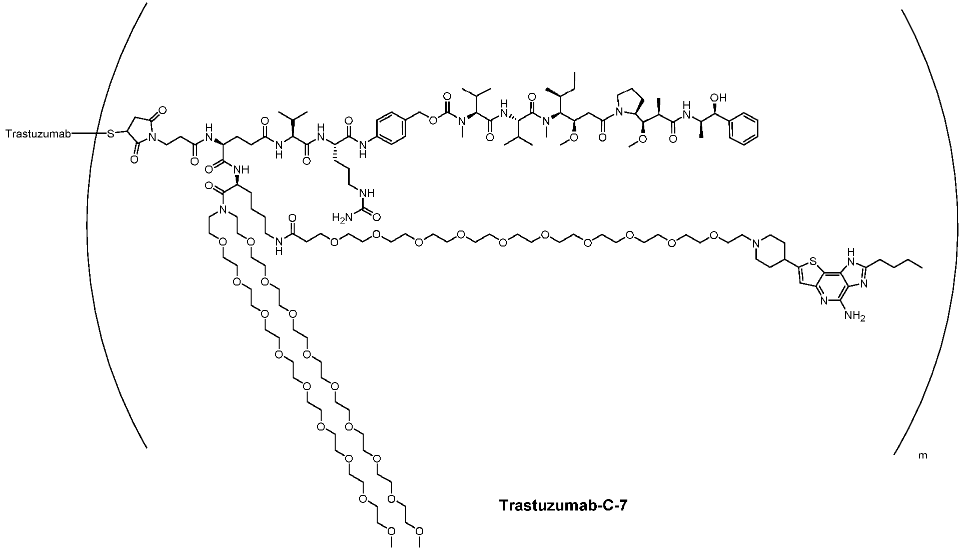

1 M phosphoric acid solution was used to adjust a Trastuzumab monoclonal antibody stock solution to pH = 6.59 to obtain a 9.5 mg/mL Trastuzumab monoclonal antibody buffer solution. To the resulting Trastuzumab monoclonal antibody buffer solution, 0.1M EDTA was added, and TCEP (2.5 eq, 10 mM) was added at the same time, shaken well, and reacted at 21°C for 4 hours, then Compound C-7 in DMSO solution (10 mM) in an amount of 5 times the molar equivalent of Trastuzumab monoclonal antibody was added thereto, shaken well, reacted at room temperature for 2 hours, allowed to stand at 4°C overnight, purified with NAP-5 gel column, and washed with histidine hydrochloride buffer solution (pH = 6.0-6.1), the filtrate was collected to obtain Trastuzumab-C-7 in histidine hydrochloride buffer (2.4 mg/mL, 1.5 mL), which was stored at -40°C.

### Example 2-2: Trastuzumab-C-8

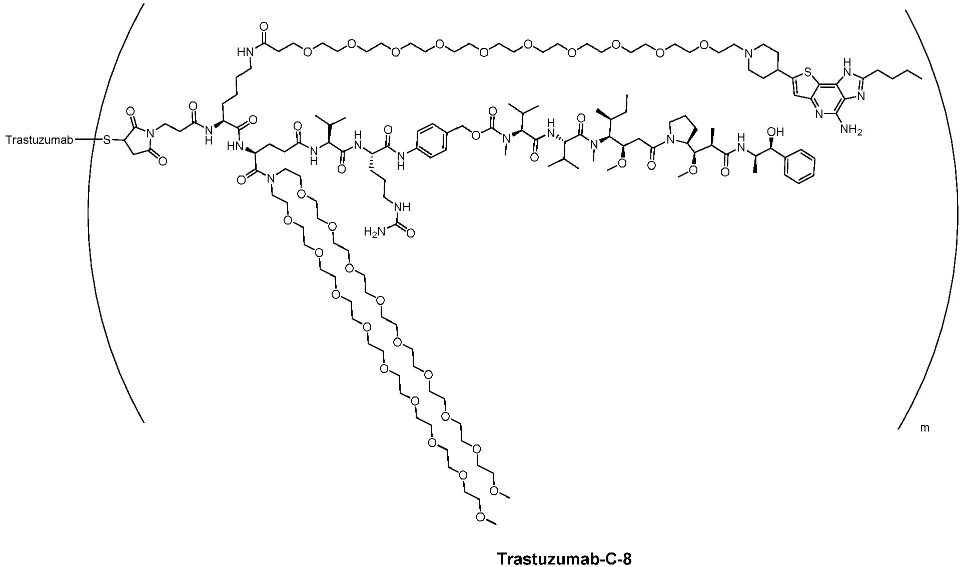

1 M phosphoric acid solution was used to adjust a Trastuzumab monoclonal antibody stock solution to pH = 6.55 to obtain a 9.5 mg/mL Trastuzumab monoclonal antibody buffer solution. To the resulting Trastuzumab monoclonal antibody buffer solution, 0.1M EDTA was added, and TCEP (2.5 eq, 10 mM) was added at the same time, shaken well, and reacted at 21°C for 4 hours, then Compound C-8 in DMSO solution (10 mM) in an amount of 5 times the molar equivalent of Trastuzumab monoclonal antibody was added thereto, shaken well, reacted at room temperature for 2 hours, allowed to stand at 4°C overnight, purified with NAP-5 gel column, and washed with histidine hydrochloride buffer solution (pH = 6.0-6.1), and the filtrate was collected to obtain **Trastuzumab-C-8** in histidine hydrochloride buffer (2.3 mg/mL, 1.5 mL), which was stored at -40°C.

### Example 2-3: Trastuzumab-C-9

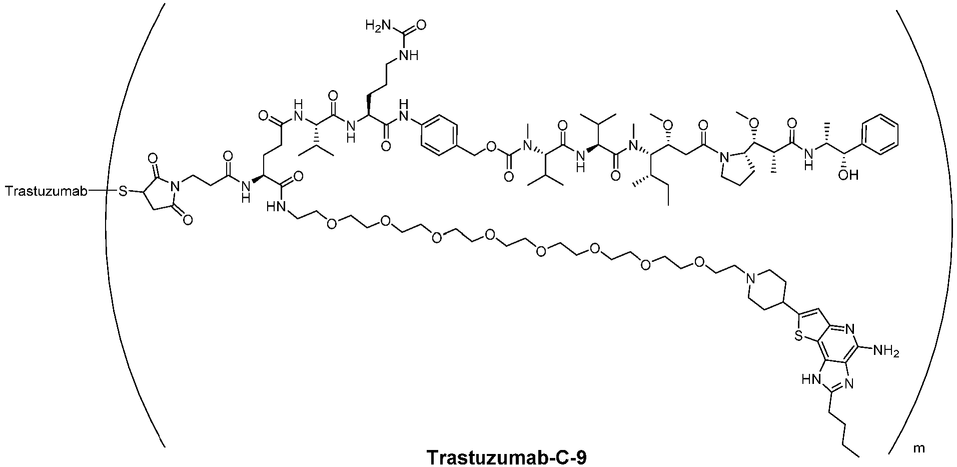

1 M phosphoric acid solution was used to adjust a Trastuzumab monoclonal antibody stock solution to pH = 6.55 to obtain a 9.5 mg/mL Trastuzumab monoclonal antibody buffer solution. To the resulting Trastuzumab monoclonal antibody buffer solution, 0.1M EDTA was added, and TCEP (2.5 eq, 10 mM) was added at the same time, shaken well, and reacted at 21°C for 4 hours, then Compound C-9 in DMSO solution (10 mM) in an amount of 5 times the molar equivalent of Trastuzumab monoclonal antibody was added thereto, shaken well, reacted at room temperature for 2 hours, allowed to stand at 4°C overnight, purified with NAP-5 gel column, and washed with histidine hydrochloride buffer solution (pH = 6.0-6.1), and the filtrate was collected to obtain **Trastuzumab-C-9** in histidine hydrochloride buffer (3.8 mg/mL, 2.5 mL), which was stored at -40°C.

### Example 2-4: Trastuzumab-C-10

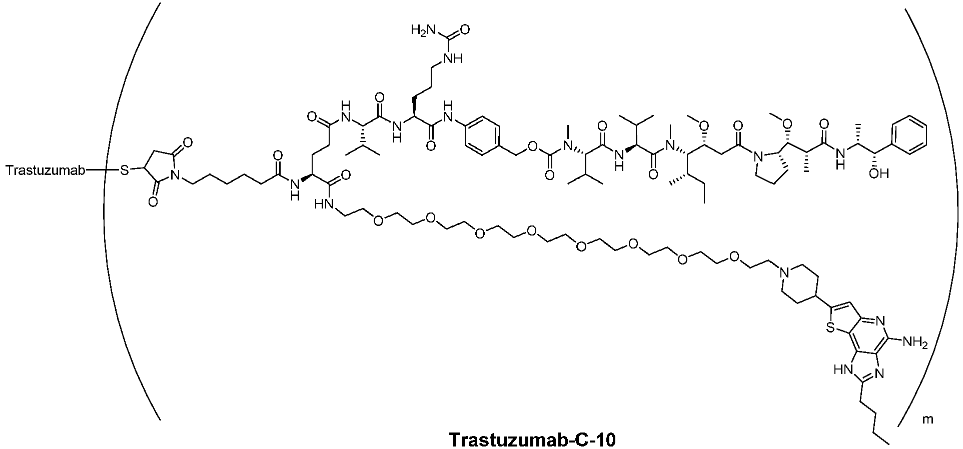

1 M phosphoric acid solution was used to adjust a Trastuzumab monoclonal antibody stock solution to pH = 6.53 to obtain a 9.5 mg/mL Trastuzumab monoclonal antibody buffer solution. To the resulting Trastuzumab monoclonal antibody buffer solution, 0.1M EDTA was added, and TCEP (2.5 eq, 10 mM) was added at the same time, shaken well, and reacted at 21°C for 4 hours, then Compound C-10 in DMSO solution (10 mM) in an amount of 5 times the molar equivalent of Trastuzumab monoclonal antibody was added thereto, shaken well, reacted at room temperature for 2 hours, allowed to stand at 4°C overnight, purified with NAP-5 gel column, and washed with histidine hydrochloride buffer solution (pH = 6.0-6.1), and the filtrate was collected to obtain **Trastuzumab-C-10** in histidine hydrochloride buffer (3.4 mg/mL, 2.5 mL), which was stored at -40°C.

### Example 2-5: Trastuzumab-C-11

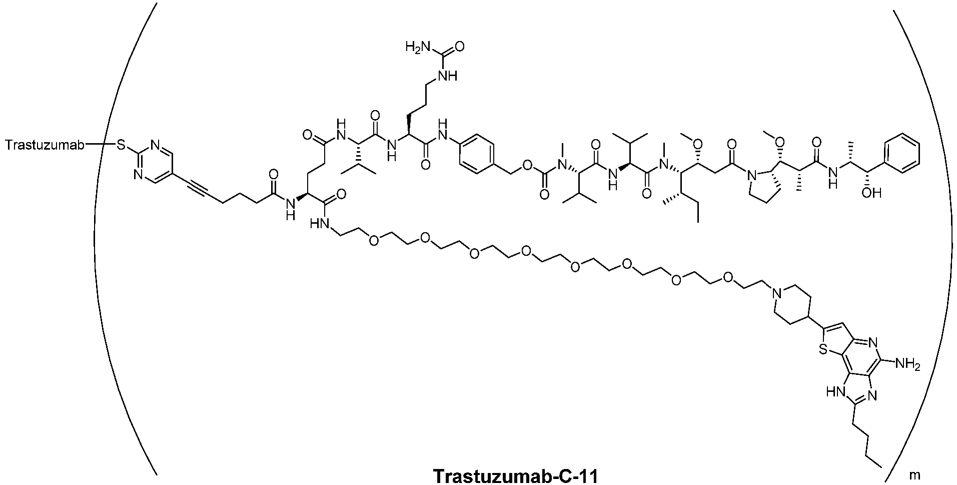

1 M phosphoric acid solution was used to adjust a Trastuzumab monoclonal antibody stock solution to pH = 6.59 to obtain a 9.5 mg/mL Trastuzumab monoclonal antibody buffer solution. To the resulting Trastuzumab monoclonal antibody buffer solution, 0.1M EDTA was added, and TCEP (2.5 eq, 10 mM) was added at the same time, shaken well, and reacted at 21°C for 4 hours, then Compound C-11 in DMSO solution (10 mM) in an amount of 5 times the molar equivalent of Trastuzumab monoclonal antibody was added thereto, shaken well, reacted at room temperature for 2 hours, allowed to stand at 4°C overnight, purified with NAP-5 gel column, and washed with histidine hydrochloride buffer solution (pH = 6.0-6.1), and the filtrate was collected to obtain **Trastuzumab-C-10** in histidine hydrochloride buffer (4.0 mg/mL, 2.5 mL), which was stored at -40°C.

### Example 2-6: Trastuzumab-C-15

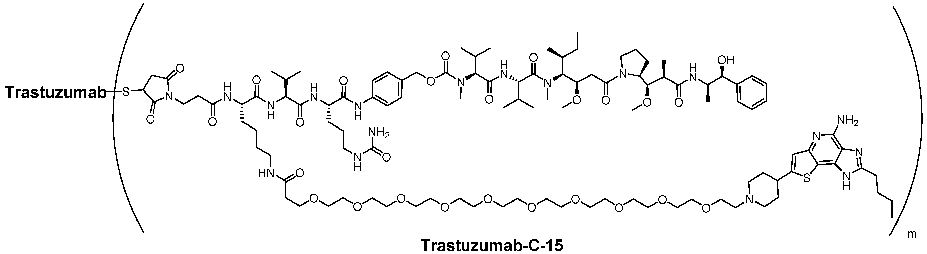

1 M disodium hydrogen phosphate solution was used to adjust a Trastuzumab monoclonal antibody stock solution to pH = 6.56, then phosphate buffered saline (PBS, pH = 6.5-6.6) was used to adjust the volume to 10 mg/mL to obtain a Trastuzumab monoclonal antibody buffer solution. To the resulting Trastuzumab monoclonal antibody buffer solution, 0.1M EDTA was added, and TCEP (2.5 eq, 10 mM) was added at the same time, shaken well, and reacted at room temperature for 2 hours, then Compound C-15 in DMSO solution (10 mM) in an amount of 5.5 times the molar equivalent of Trastuzumab monoclonal antibody was added thereto, shaken well, reacted at room temperature for 2 hours, purified with NAP-10, NAP-5 gel column, and washed with histidine hydrochloride buffer solution (pH = 6.0-6.1), and the filtrate was collected to obtain **Trastuzumab-C-15** in histidine hydrochloride buffer (3.4 mg/mL, 2.5 mL), which was stored at 4°C.

### Example 2-7: Trastuzumab-C-16

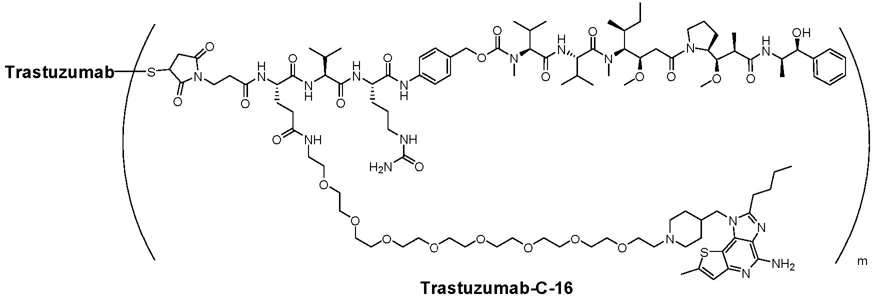

1 M disodium hydrogen phosphate solution was used to adjust a Trastuzumab monoclonal antibody stock solution to pH = 6.54, then phosphate buffered saline (PBS, pH = 6.5-6.6) was used to adjust the volume to 10 mg/mL to obtain a Trastuzumab monoclonal antibody buffer solution. To the resulting Trastuzumab monoclonal antibody buffer solution, 0.1M EDTA was added, and TCEP (2.5 eq, 10 mM) was added at the same time, shaken well, and reacted at room temperature for 2 hours, then Compound **C-16** in DMSO solution (10 mM) in an amount of 5 times the molar equivalent of Trastuzumab monoclonal antibody was added thereto, shaken well, reacted at room temperature for 2 hours, purified with NAP-10, NAP-5 gel column, and washed with histidine hydrochloride buffer solution (pH = 6.0-6.1), and the filtrate was collected to obtain **Trastuzumab-C-16** in histidine hydrochloride buffer (3.1 mg/mL, 2.5 mL), which was stored at 4°C.

### Example 2-8: Trastuzumab-C-17

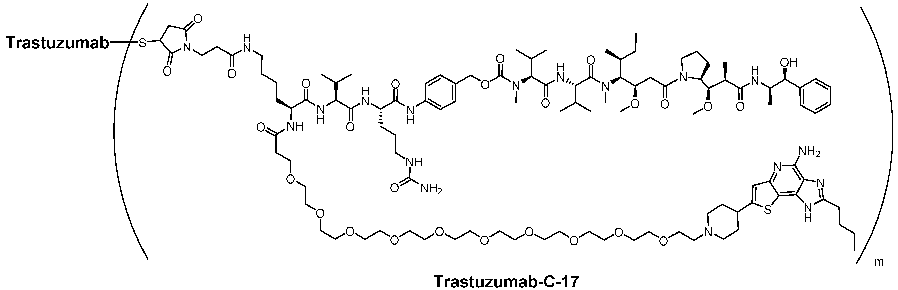

1 M disodium hydrogen phosphate solution was used to adjust a Trastuzumab monoclonal antibody stock solution to pH = 6.57, then phosphate buffered saline (PBS, pH = 6.5-6.6) was used to adjust the volume to 10 mg/mL to obtain a Trastuzumab monoclonal antibody buffer solution. To the resulting Trastuzumab monoclonal antibody buffer solution, 0.1M EDTA was added, and TCEP (2.5 eq, 10 mM) was added at the same time, shaken well, and reacted at room temperature for 2 hours, then Compound C-17 in DMSO solution (10 mM) in an amount of 5 times the molar equivalent of Trastuzumab monoclonal antibody was added thereto, shaken well, reacted at room temperature for 2 hours, purified with NAP-10, NAP-5 gel column, and washed with histidine hydrochloride buffer solution (pH = 6.0-6.1), and the filtrate was collected to obtain Trastuzumab-C-17 in histidine hydrochloride buffer (3.5 mg/mL, 2.5 mL), which was stored at 4°C.

### Example 2-9: Trastuzumab-C-20

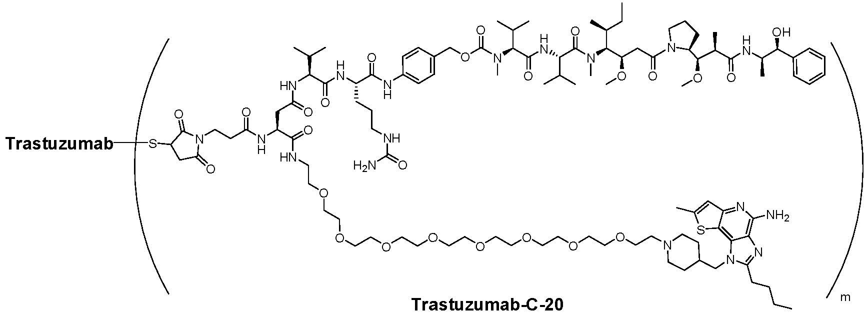

1 M disodium hydrogen phosphate solution was used to adjust a Trastuzumab monoclonal antibody stock solution to pH = 6.55, then phosphate buffered saline (PBS, pH = 6.5-6.6) was used to adjust the volume to 10 mg/mL to obtain a Trastuzumab monoclonal antibody buffer solution. To the resulting Trastuzumab monoclonal antibody buffer solution, 0.1M EDTA was added, and TCEP (2.5 eq, 10 mM) was added at the same time, shaken well, and reacted at room temperature for 2 hours, then Compound **C-20** in DMSO solution (10 mM) in an amount of 5.5 times the molar equivalent of Trastuzumab monoclonal antibody was added thereto, shaken well, reacted at room temperature for 2 hours, purified with NAP-10, NAP-5 gel column, and washed with histidine hydrochloride buffer solution (pH = 6.0-6.1), and the filtrate was collected to obtain **Trastuzumab-C-20** in histidine hydrochloride buffer (3.0 mg/mL, 2.5 mL), which was stored at 4°C.

### Example 2-10: Trastuzumab-C-21

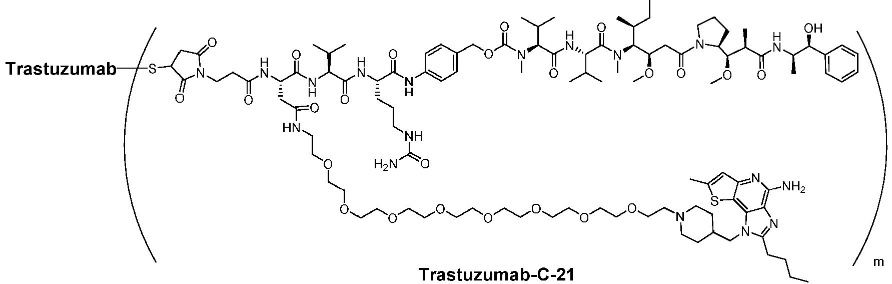

1 M disodium hydrogen phosphate solution was used to adjust a Trastuzumab monoclonal antibody stock solution to pH = 6.55, then phosphate buffered saline (PBS, pH = 6.5-6.6) was used to adjust the volume to 10 mg/mL to obtain a Trastuzumab monoclonal antibody buffer solution. To the resulting Trastuzumab monoclonal antibody buffer solution, 0.1M EDTA was added, and TCEP (2.5 eq, 10 mM) was added at the same time, shaken well, and reacted at room temperature for 2 hours, then Compound **C-21** in DMSO solution (10 mM) in an amount of 5.5 times the molar equivalent of Trastuzumab monoclonal antibody was added thereto, shaken well, reacted at room temperature for 2 hours, purified with NAP-10, NAP-5 gel column, and washed with histidine hydrochloride buffer solution (pH = 6.0-6.1), and the filtrate was collected to obtain **Trastuzumab-C-21** in histidine hydrochloride buffer (3.2 mg/mL, 2.5 mL), which was stored at 4°C.

### Example 2-11: Trastuzumab-C-22

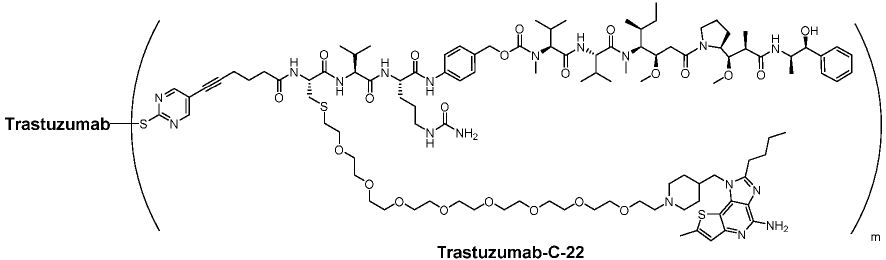

1 M disodium hydrogen phosphate solution was used to adjust a Trastuzumab monoclonal antibody stock solution to pH = 6.55, then phosphate buffered saline (PBS, pH = 6.5-6.6) was used to adjust the volume to 10 mg/mL to obtain a Trastuzumab monoclonal antibody buffer solution. To the resulting Trastuzumab monoclonal antibody buffer solution, 0.1M EDTA was added, and TCEP (2.5 eq, 10 mM) was added at the same time, shaken well, and reacted at room temperature for 2 hours, then Compound **C-22** in DMSO solution (10 mM) in an amount of 5.5 times the molar equivalent of Trastuzumab monoclonal antibody was added thereto, shaken well, reacted at room temperature for 2 hours, purified with NAP-10, NAP-5 gel column, and washed with histidine hydrochloride buffer solution (pH = 6.0-6.1), and the filtrate was collected to obtain **Trastuzumab-C-22** in histidine hydrochloride buffer (3.8 mg/mL, 2.5 mL), which was stored at 4°C.

### 3. Drug/antibody ratio of immunomodulatory antibody-drug conjugate (iADC): Determination of DAR value

LC-MS was used to determine the molecular weight of iADC and calculate the drug/antibody ratio DAR value.

Chromatography conditions:
Liquid chromatography column: Thermo MAbPac RP 3.0* 100mm;
Mobile phase A: 0.1% FA/H₂O; Mobile phase B: 0.1% FA/ACN;
Flow rate: 0.25 ml/min; sample chamber temperature: 8°C; column temperature: 60°C; injection volume: 1 µl;

| | | | | | | |
|---|---|---|---|---|---|---|
| Time (minutes) | 2 | 20 | 22 | 25 | 26 | 30 |
| Mobile phase A (volume %) | 75 | 60 | 5 | 5 | 75 | 75 |
| Mobile phase B (volume %) | 25 | 40 | 95 | 95 | 25 | 25 |

Mass spectrometry conditions:
Mass spectrometer model: AB Sciex Triple TOF 5600+;
GS 135; GS2 35; CUR 30; TEM 350; ISVF 5500; DP 250; CE 10; Accumulation time 0.5 s;
m/z 600-4000; Time bins to sum 40.

CE-SDS calculation showed that antibody-drug conjugate **Trastuzumab-C-7** had a DAR value of 4.42; **Trastuzumab-C-8** had a DAR value of 3.56; **Trastuzumab-C-9** had a DAR value of 3.53; **Trastuzumab-C-10** had a DAR value of 3.3; **Trastuzumab-C-11** had a DAR value of 2.3; **Trastuzumab-C-15** had a DAR value of 3.58; **Trastuzumab-C-16** had a DAR value of 2.84; **Trastuzumab-C-17** had a DAR value of 3.37; **Trastuzumab-C-20** had a DAR value of 3.53; **Trastuzumab-C-21** had a DAR value of 2.89; **Trastuzumab-C-22** had a DAR value of 3.38.

### Biological assays

Experimental Example 1: Inhibitory effect of immunomodulatory antibody-drug conjugate (iADC) on proliferation of HER2-positive tumor cells and HER2-negative tumor cells

### Experimental steps:

1. HER2-positive tumor cells HCC1954 (Jiangyin Concortis Biotechnology Co., Ltd.) and NCI-N87 (ATCC), as well as HER2-negative tumor cells NCI-H358 (Nanjing Co-Bioer) in the logarithmic growth phase were collected and counted.
2. The three types of tumor cells were inoculated into 96-well plates (Corning), respectively, with 2000 cells (HCC1954 or NCI-H358) or 3000 cells (NCI-N87) per well, with a volume of 80 µL, and cultured in a 37°C, 5% CO₂ incubator overnight to allow the cells to adhere (18 to 24 hours).
3. According to the experimental design, 5× drug working solutions with gradient concentrations were prepared. The 96-well cell plate was taken out, and 20 µL of working solution was transferred from the compound plate to the cell plate. The cell plate was cultured in a 37°C, 5% CO₂ incubator for 72 h.
4. Testing plate: The cell plate was taken out, and the cell status was observed under microscope. After the observation, according to the instructions of CellCounting-Lite^{®} 2.0 kit (Nuoweizan), a detection solution was added to the cell plate, and raw data were obtained through PHERA Star FS.
5. Inhibition rate was calculated: (Inhibition rate, IR%) = 1-(mean luminescence value of experimental wells/mean luminescence value of DMSO control wells)* 100%. Graphpad Prism 8.0 software was used for data analysis and graphing. The cell survival rate and the logarithm of drug concentration were used to fit a four-parameter curve to calculate IC₅₀ value.

### Experimental results:

The inhibitory effect of each iADC on the proliferation of three tumor cells was determined according to the above method, as shown in Table 1:

**Table 1. Inhibitory effect of iADC on proliferation of three types of tumor cells**

| Name of iADC | IC₅₀ (HCC1954, nM) | IC₅₀ (NCI-N87, nM) | IC₅₀ (NCI-H358, nM) |
|---|---|---|---|
| Trastuzumab-C-7 | 0.108 | 0.293 | 83.769 |
| Trastuzumab-C-8 | 0.112 | 0.250 | 374.766 |
| Trastuzumab-C-9 | 0.021 | 0.058 | 136.77 |
| Trastuzumab-C-10 | 0.011 | 0.023 | 164.13 |
| Trastuzumab-C-11 | 0.017 | 0.026 | 331.62 |
| Trastuzumab-C-15 | 0.017 | 0.17 | / |
| Trastuzumab-C-16 | 0.018 | 0.15 | / |
| Trastuzumab-C-17 | 0.014 | 0.32 | / |
| Trastuzumab-C-20 | 0.010 | 0.09 | / |
| Trastuzumab-C-21 | 0.018 | 0.12 | / |
| Trastuzumab-C-22 | 0.053 | 0.103 | / |

The results showed that each of the iADCs had strong inhibitory effect on the proliferation of HCC1954 and NCI-N87 cells (HER2-positive), and significantly weakened inhibitory effect on NCI-H358 cells (HER2-negative), indicating that each of the iADCs had selective killing effect on HER2-positive cells.

Experimental Example 2: Determination of ability of immunomodulatory antibody-drug conjugate (iADC) to stimulate peripheral blood mononuclear cells (PBMC) to secrete TNF-α in the presence of tumor cells

### Experimental steps:

1. HER2-expressing HCC1954 tumor cells (Jiangyin Concortis Biotechnology Co., Ltd.) in the logarithmic growth phase were collected, washed twice with PBS, and resuspended in RPMI1640+10% heat-inactivated FBS complete medium to adjust the cell density to 2×10⁵ cells/ mL, and the HCC1954 tumor cells were inoculated in 96-well plates (Corning), 1×10⁴ cells per well.
2. PBMCs (Sail Bio) were thawed, and the cells were resuspended in RPMI1640+10% heat-inactivated FBS complete medium, adjusted to have a density of 6×10⁵ cells/mL, and added to a 96-well plate inoculated with the tumor cells, 3×10⁴ PBMC cells per well.
3. 100 µL of the drug to be tested at different concentrations was added to the well plate so that the final concentrations were 500 nM, 166.7 nM, 55.6 nM, 18.52 nM, 6.17 nM, 2.06 nM, and 0.69 nM, and 100 µL of RPMI1640+10% heat-inactivated FBS complete medium was added to the blank wells.
4. The plate was incubated in a 5% CO₂, 37°C incubator for 24 hours, then 50 µL of supernatant was taken from each well for detection, and primary antibody and HRP-labeled second antibody were incubated according to the steps of the human TNF-α ELISA detection kit (Invitrogen). After color developing, absorbance values were read at 450 nm wavelength. Graphpad Prism software was used to calculate the concentration of TNF-α based on the standard curve.

### Experimental results:

The ability of each immunomodulatory antibody-drug conjugate (iADC) to stimulate human peripheral blood mononuclear cells (PBMC) to secrete TNF-α in the presence of HCC1954 tumor cells was determined according to the above method. The results were shown in Table 2:

**Table 2. Results of in vitro secretion of TNF-α stimulated by iADC in co-incubation system of HCC1954 tumor cells and PBMC**

| Drug | EC₅₀ (nM) | Emax (TNF-α, pg/mL) |
|---|---|---|
| Trastuzumab-C-7 | 11.25 | 1791.26 |
| Trastuzumab-C-8 | 17.72 | 1836.92 |
| Trastuzumab-C-9 | 24.41 | 470.23 |
| Trastuzumab-C-10 | 7.54 | 522.28 |
| Trastuzumab-C-11 | 32.59 | 427.95 |
| Trastuzumab-C-15 | 74.62 | 817.79 |
| Trastuzumab-C-16 | 4.61 | 411.5 |
| Trastuzumab-C-17 | 3.39 | 788.8 |
| Trastuzumab-C-20 | 2.18 | 791.6 |
| Trastuzumab-C-21 | 3.13 | 573.7 |
| Trastuzumab-C-22 | 14.05 | 1562.5 |

The results showed that each of the iADCs could effectively stimulate PBMC to secrete TNF-α in the co-incubation system of HCC1954 tumor cells and PBMC *in vitro.*

Various modifications of the present invention in addition to those described herein will become apparent to those skilled in the art from the foregoing description. Such modifications are also intended to fall within the scope of the appended claims.

## Claims

1. An antibody-drug conjugate of formula (I): 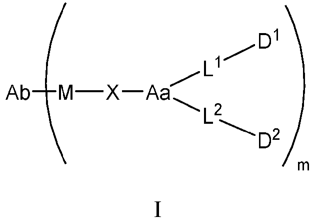 wherein,
Ab represents a targeting moiety;
M represents a linking site connected to the targeting moiety;
X represents a linker connecting M and Aa;
Aa represents an amino acid fragment, or a peptide fragment formed of two or more amino acids;
L¹ represents a covalent bond or a linker connecting Aa and D¹;
L² represents a linker connecting Aa and D²;
D¹ is a cytotoxic drug fragment;
D² is a TLR agonist fragment;
m is selected from 1 to 10, such as 1, 2, 3, 4, 5, 6, 7, 8, 9 or 10.

2. The antibody-drug conjugate according to claim 1, wherein Ab is a targeting moiety targeting a cell surface receptor or a tumor surface antigen.

3. The antibody-drug conjugate according to claim 1 or 2, wherein Ab is an antibody or antigen-binding fragment thereof, wherein the antibody or antigen-binding fragment comprises a monoclonal antibody, a polyclonal antibody, a linear antibody, a bispecific antibody, a multispecific antibody, a chimeric antibody, a murine antibody, a humanized antibody, a fully human antibody, or a fusion protein containing an antigen-binding region of an antibody.

4. The antibody-drug conjugate according to any one of claims 1 to 3, wherein Ab is an antibody or antigen-binding fragment thereof, wherein the antigen-binding fragment is selected from the group consisting of Fab, Fab', F(ab')₂, Fv, disulfide bond-linked Fv and scFv.

5. The antibody-drug conjugate according to any one of claims 1 to 4, wherein the antibody or antigen-binding fragment thereof is selected from the group consisting of anti-Her-2 antibody, anti-EGFR antibody, anti-VEGFR antibody, anti-PD-L1 antibody, anti-PD-1 antibody, anti-CTLA-4 antibody and anti-Trop-2 antibody.

6. The antibody-drug conjugate according to any one of claims 1 to 5, wherein M is a covalent bond or selected from the group consisting of the following structures: 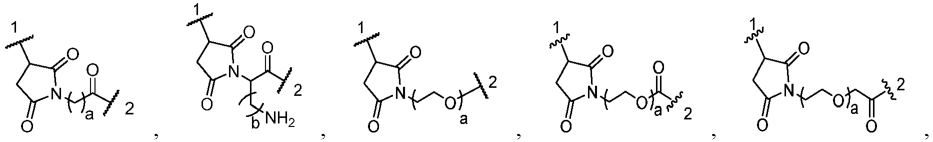 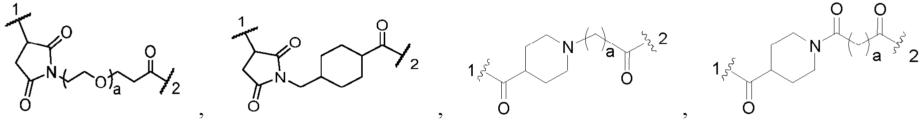 and 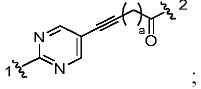 wherein, each a is independently an integer selected from 1 to 5, b is an integer selected from 1 to 3, Position 1 of M is connected to Ab, and Position 2 of M is connected to X.

7. The antibody-drug conjugate according to any one of claims 1 to 6, wherein M is a covalent bond or selected from the group consisting of the following structures: 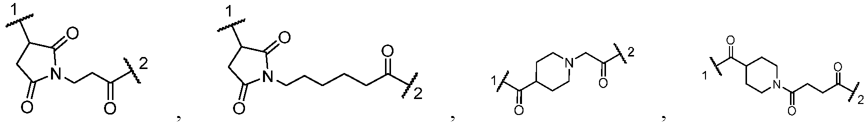 and

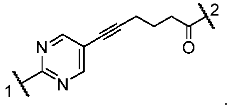

8. The antibody-drug conjugate according to any one of claims 1 to 7, wherein X is a covalent bond or selected from the group consisting of C₁₋₆ alkylene, -NH-(CH₂)_{c}-C(O)-, 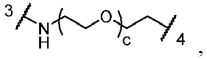 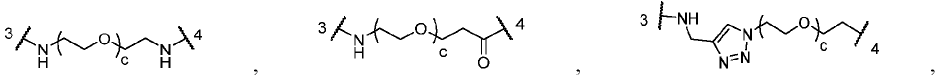 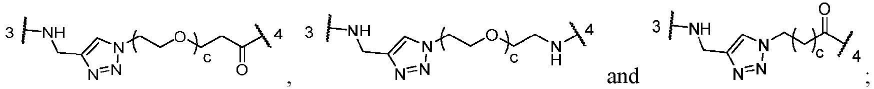 wherein, each c is independently an integer selected from 1 to 10; Position 3 of X is connected to M, and Position 4 of X is connected to Aa.

9. The antibody-drug conjugate according to any one of claims 1 to 8, wherein Aa is selected from the group consisting of an amino acid fragment, and a peptide fragment formed of two or more amino acids, wherein the amino acid is selected from the group consisting of Gly, Phe, Ala, Val, Ser, Thr, His, Trp, Cys, Asp, Glu, Lys, Tyr and Arg, optionally, each amino acid is independently modified by C₁₋₆ alkyl or a polyethylene glycol hydrophilic structural unit;
preferably, the polyethylene glycol hydrophilic structural unit is selected from the group consisting of 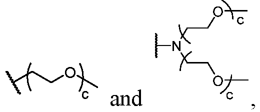 wherein each c is independently an integer selected from 1 to 10.

10. The antibody-drug conjugate according to any one of claims 1 to 9, wherein Aa is selected from the group consisting of a fragment of the following amino acids, and a peptide fragment formed of any combination of two or more of them: Gly, Phe, Ala, Val, Cys, Asp, Glu, Lys and Arg, and each amino acid is independently optionally modified by C₁₋₆ alkyl or a polyethylene glycol hydrophilic structural unit;
preferably, the polyethylene glycol hydrophilic structural unit is selected from the group consisting of 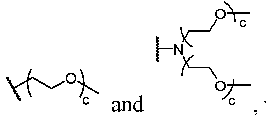 wherein each c is independently an integer selected from 1 to 10.

11. The antibody-drug conjugate according to any one of claims 1 to 10, wherein Aa is selected from the group consisting of the following structures: 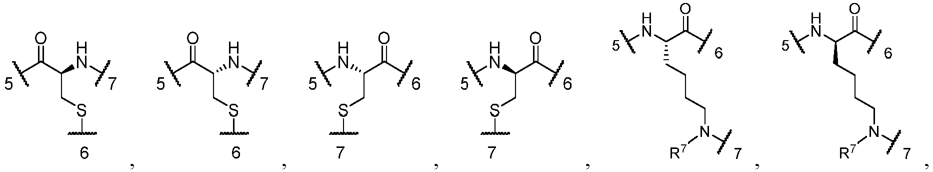 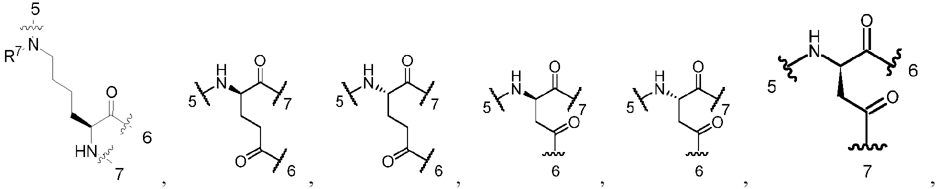 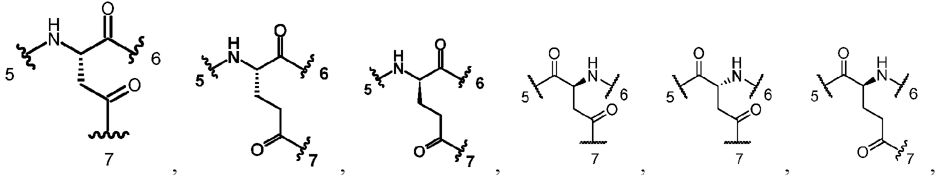 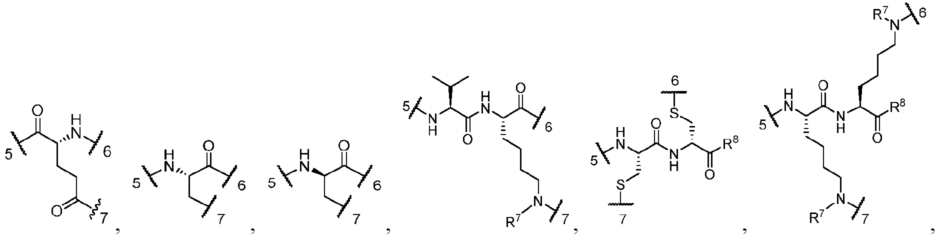 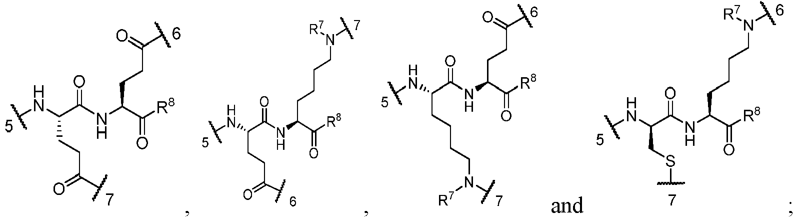
wherein, each R⁷ is independently selected from the group consisting of H, C₁₋₆ alkyl and a polyethylene glycol hydrophilic structural unit; each R⁸ is independently selected from the group consisting of hydroxyl and a polyethylene glycol hydrophilic structural unit; Position 5 is connected to X, Position 6 is connected to one of L¹ and L², and Position 7 is connected to the other of L¹ and L²; preferably, Position 5 is connected to X, Position 6 is connected to L¹, and Position 7 is connected to L²;
preferably, the polyethylene glycol hydrophilic structural unit is selected from the group consisting of 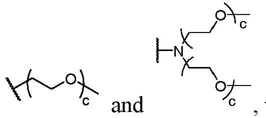 wherein each c is independently an integer selected from 1 to 10.

12. The antibody-drug conjugate according to any one of claims 1 to 11, wherein L¹ is a covalent bond or a non-cleavable linker or cleavable linker, wherein the cleavable linker is capable of being cleaved by an enzyme present in a pathological environment, and the enzyme is selected from the group consisting of protease, phosphatase, pyrophosphatase, β-glucuronidase, β-galactosidase and sulfatase.

13. The antibody-drug conjugate according to any one of claims 1 to 12, wherein L¹ is a covalent bond or -L^{a}-L^{b}-L^{c}-, wherein:
L^{a} is a covalent bond or selected from the group consisting of C₁₋₆ alkylene, -NH-(CH₂)_{c}-C(O)-, 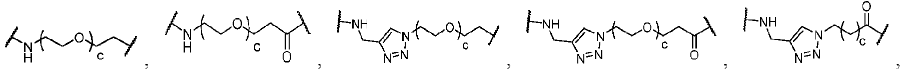 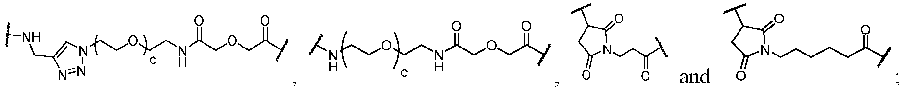
wherein, each c is independently an integer selected from 1 to 10;
L^{b} is a covalent bond or selected from the group consisting of an amino acid fragment, and a peptide fragment formed of two or more amino acids, wherein the amino acid is selected from the group consisting of Val, Cit, Glu, Lys, Arg, Phe, Leu, Gly, Ala and Asn;
preferably, L^{b} is a covalent bond or selected from the group consisting of the following structures: 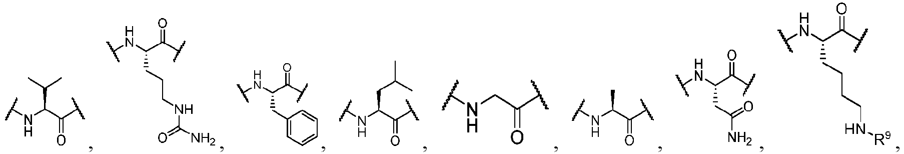 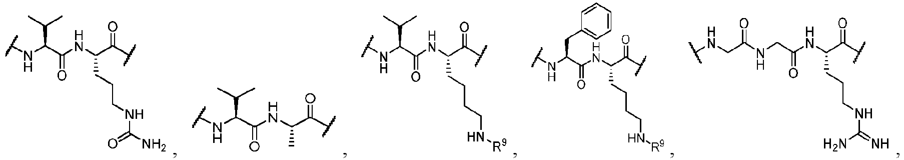 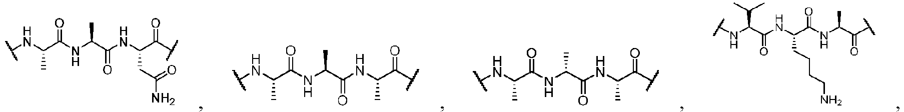 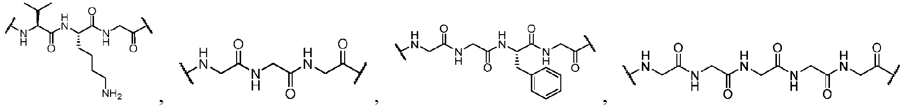 and 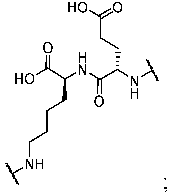 wherein, R⁹ is selected from the group consisting of H, acetyl, fluorenyloxycarbonyl, trityl and a polyethylene glycol hydrophilic structural unit; preferably, the polyethylene glycol hydrophilic 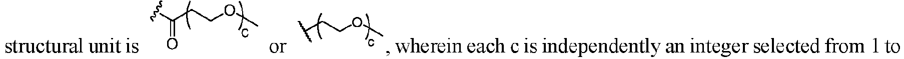 10;
L^{c} is a covalent bond, -NH-CH₂- or selected from the group consisting of the following structures:

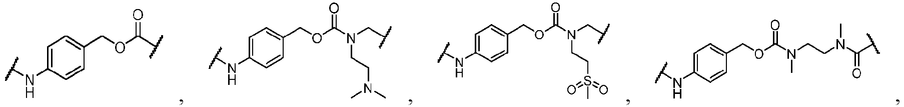

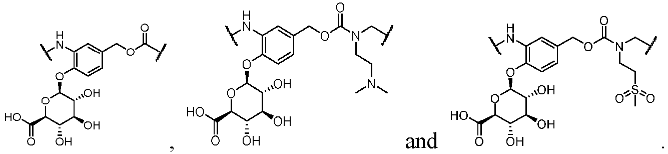

14. The antibody-drug conjugate according to any one of claims 1 to 13, wherein L¹ is a covalent bond or selected from the group consisting of the following structures: 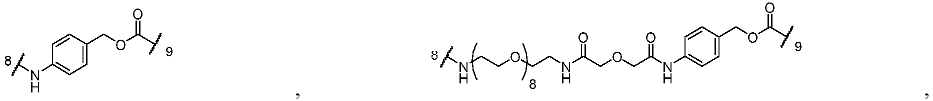 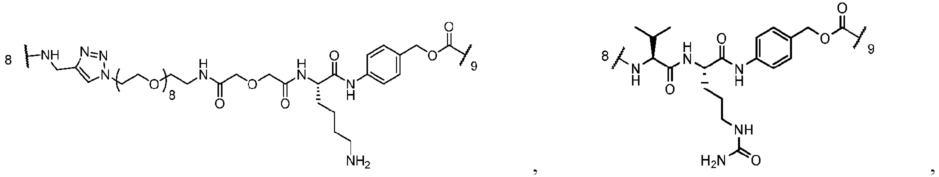 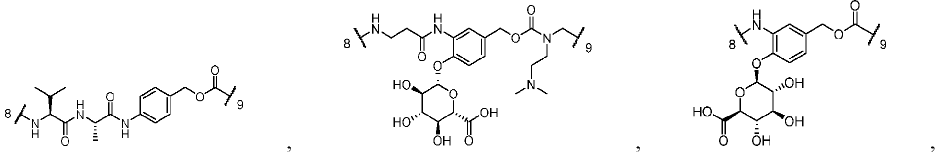 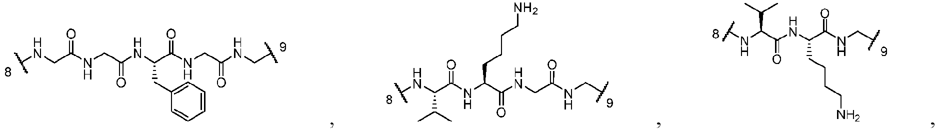 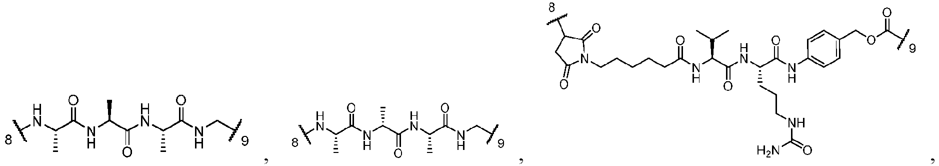 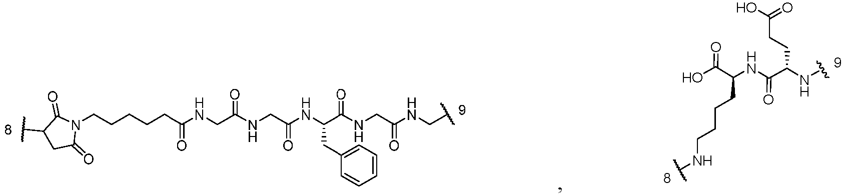 and 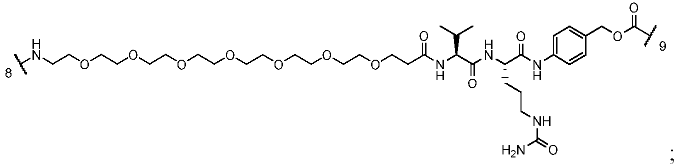
wherein, Position 8 is connected to Aa, and Position 9 is connected to D¹;
preferably, L¹ is: 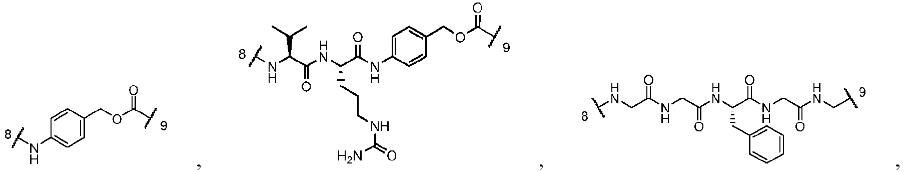 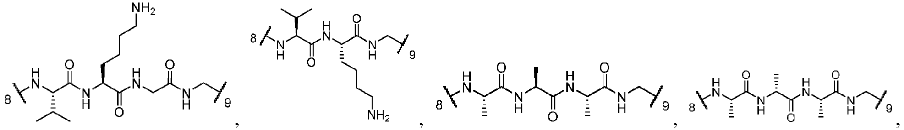 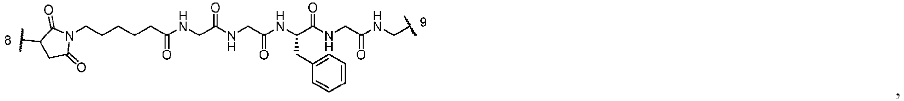 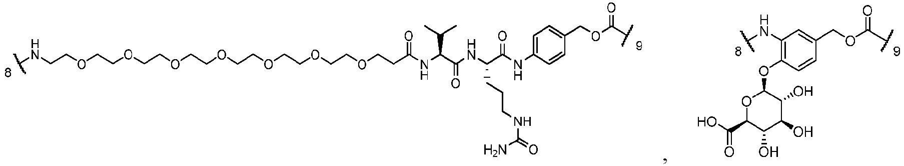 or

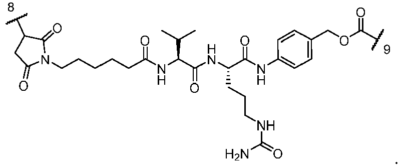

15. The antibody-drug conjugate according to any one of claims 1 to 14, wherein L² is a non-cleavable linker or a cleavable linker, wherein the cleavable linker is capable of being cleaved by an enzyme present in a pathological environment, and the enzyme is selected from the group consisting of protease, phosphatase, pyrophosphatase, *β*-glucuronidase, β-galactosidase and sulfatase.

16. The antibody-drug conjugate according to any one of claims 1 to 15, wherein L² is -L^{d}-L^{e}-L^{f}-, wherein:
L^{d} is a covalent bond or a divalent structure consisting of one or more selected from the following: C₁₋₆ alkylene, -C(O)-(CH₂)_{d}-NH-, 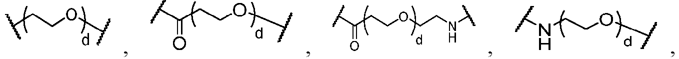 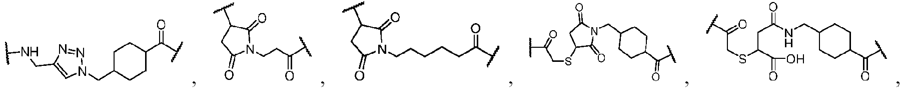 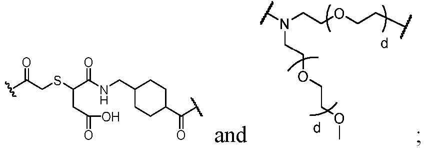 wherein, each d is independently an integer selected from 1 to 12;
L^{e} is a covalent bond or selected from the group consisting of an amino acid fragment, and a peptide fragment formed of two or more amino acids, wherein the amino acid is selected from the group consisting of Val, Cit, Glu, Lys, Arg, Phe, Leu, Gly, Ala and Asn;
preferably, L^{e} is a covalent bond or selected from the group consisting of the following structures: 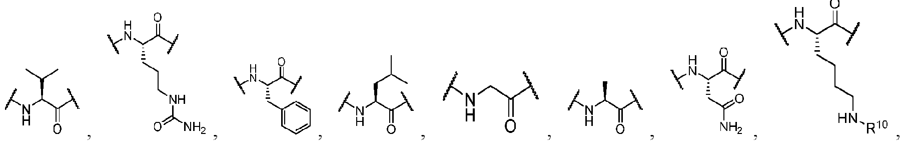 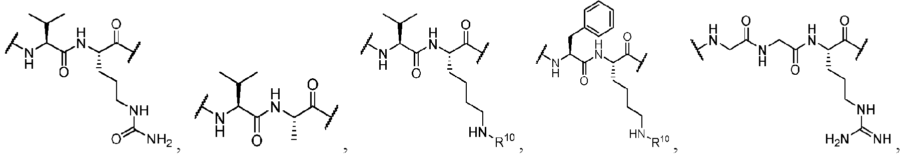 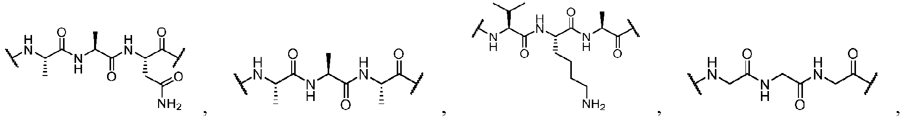 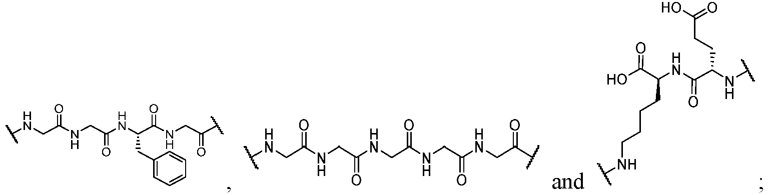
wherein, each R¹⁰ is independently selected from the group consisting of H, acetyl, fluorenyloxycarbonyl, trityl and a polyethylene glycol hydrophilic structural unit; preferably, the polyethylene glycol hydrophilic structural unit is 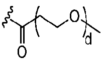 or 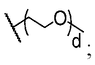 each d is independently an integer selected from 1 to 10;
L^{f} is a covalent bond, -NH-CH₂- or selected from the group consisting of the following structures:

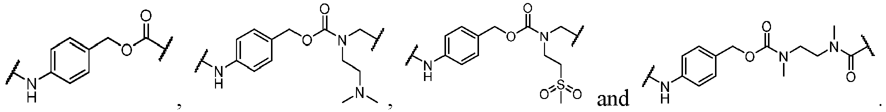

17. The antibody-drug conjugate according to any one of claims 1 to 16, wherein L² is selected from the group consisting of the following structures: 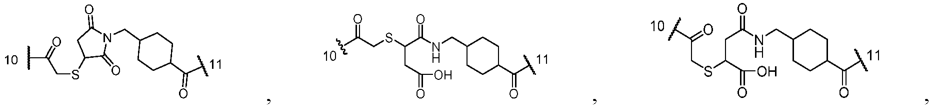 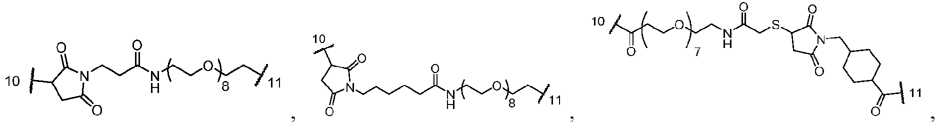 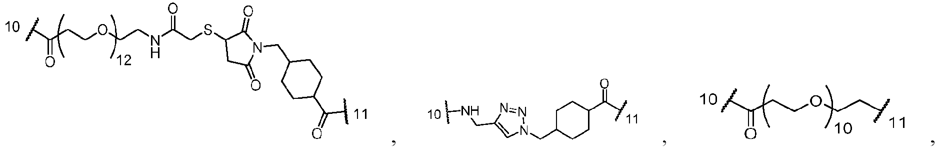 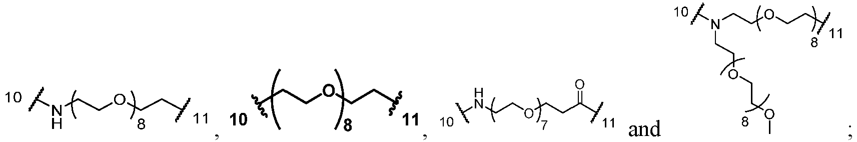
wherein, Position 10 is connected to Aa, and Position 11 is connected to D²;
preferably, L² is selected from the group consisting of the following structures: 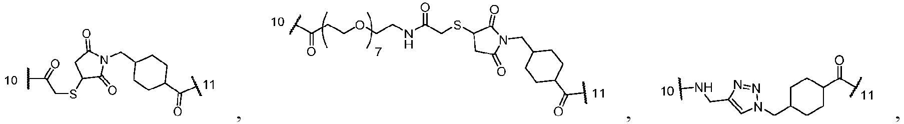 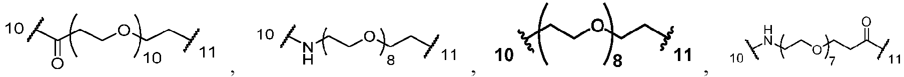 and

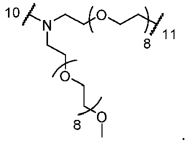

18. The antibody-drug conjugate according to any one of claims 1 to 17, wherein D¹ is selected from the group consisting of cytotoxic drug fragments, and the cytotoxic drug is selected from the group consisting of tubulin inhibitors, DNA damaging agents and topoisomerase inhibitors, the tubulin inhibitor comprises dolastatin, auristatins, maytansines, tubulysins and cryptomycins, the DNA damaging agent comprises PBDs, duocarmycins and calicheamicins, and the topoisomerase inhibitor comprises camptothecin and derivatives thereof.

19. The antibody-drug conjugate according to any one of claims 1 to 18, wherein the tubulin inhibitor is selected from the group consisting of dolastatin 10, MMAE, MMAF, maytansine, DM1, DM3 and DM4, and the topoisomerase inhibitor is selected from the group consisting of camptothecin, SN-38, exatecan, topotecan, belotecan, 10-hydroxy-camptothecin, 9-amino-camptothecin, doxorubicin, epirubicin and PNU-159682.

20. The antibody-drug conjugate according to any one of claims 1 to 19, wherein D¹ is selected from the group consisting of the following structures: 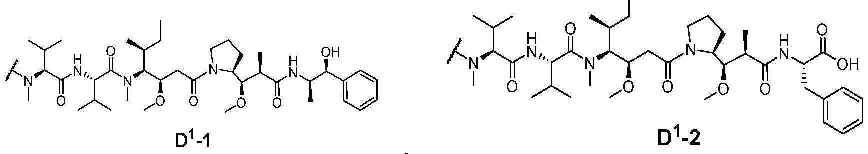 and

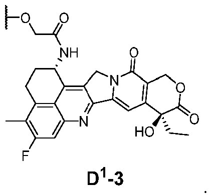

21. The antibody-drug conjugate according to any one of claims 1 to 20, wherein D² is a TLR agonist fragment, wherein the TLR agonist is selected from the group consisting of TLR2 agonists, TLR4 agonists, TLR6 agonists, TLR7 agonists, TLR8 agonists, TLR7/8 agonists, and TLR9 agonists.

22. The antibody-drug conjugate according to any one of claims 1 to 21, wherein D² is a TLR agonist fragment, and the TLR agonist is selected from the group consisting of TLR7 agonists, TLR8 agonists and TLR7/8 agonists.

23. The antibody-drug conjugate according to any one of claims 1 to 22, wherein D² is a TLR agonist fragment, and the TLR agonist is a compound represented by Formula (II): 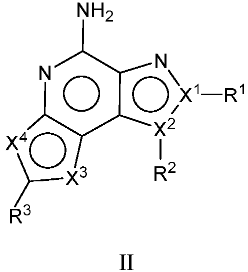 wherein,
X¹ is N or C;
X² is N or C;
and at least one of X¹ and X² is N;
X³ is selected from the group consisting of O, S and N;
X⁴ is selected from the group consisting of O, S, N and CR⁴;
R¹ is selected from the group consisting of C₁₋₆ alkyl and -C₁₋₆ alkylene-O-C₁₋₆ alkyl;
R² is hydrogen or the formula -L³-L⁴-L⁵-L⁶;
L³ is a covalent bond or C₁₋₆ alkylene;
L⁴ is selected from the group consisting of a covalent bond, C₃₋₁₀ cycloalkyl, 3- to 12-membered heterocyclyl, C₆₋₁₀ aryl, and 5- to 10-membered heteroaryl, and the cycloalkyl, heterocyclyl, aryl and heteroaryl is optionally substituted with one or more groups selected from the group consisting of hydrogen, halogen, cyano, C₁₋₆ alkyl, C₁₋₆ alkoxy and -C₁₋₆ alkylene-NH₂;
L⁵ is selected from the group consisting of a covalent bond, -O-, -S-, -C(O)-, -O-C(O)-, -C(O)-O-, -O-C(O)-O-, -NR⁵-, -C(O)-NR⁵-, -NR⁵-C(O)-, -O-C(O)-NR⁵-, -NR⁵-C(O)-O-, -NR⁵-C(O)-NR⁵-, -S(O)ᵣ-NR⁵-, -NR⁵-S(O)ᵣ- and -NR⁵-S(O)ᵣ-NR⁵-;
L⁶ is selected from the group consisting of hydrogen, C₁₋₆ alkyl, -(O-CH₂CH₂)ₙ O-C₁₋₆ alkyl and -C₁₋₆ alkylene-(O-CH₂CH₂)ₙO-C₁₋₆ alkyl, and the alkyl is optionally substituted with one or more groups selected from the group consisting of hydrogen, halogen, hydroxyl, -NH₂, -NH-C(O)-O-C₁₋₆ alkyl, C₁₋₆ alkoxy, carboxyl and -C(O)-O-C₁₋₆ alkyl;
R³ is hydrogen or the formula -L⁷-L⁸-L⁹-L¹⁰;
L⁷ is selected from the group consisting of a covalent bond and C₁₋₆ alkylene;
L⁸ is selected from the group consisting of a covalent bond, C₃₋₁₀ cycloalkyl, 3- to 12-membered heterocyclyl, C₆₋₁₀ aryl, and 5- to 10-membered heteroaryl, and the cycloalkyl, heterocyclyl, aryl and heteroaryl is optionally substituted with one or more groups selected from the group consisting of hydrogen, halogen, cyano, C₁₋₆ alkyl, C₁₋₆ alkoxy and -C₁₋₆ alkylene-NH₂;
L⁹ is selected from the group consisting of a covalent bond, -O-, -S-, -C(O)-, -O-C(O)-, -C(O)-O-, -O-C(O)-O-, -NR⁶-, -C(O)-NR⁶-, -NR⁶-C(O)-, -O-C(O)-NR⁶-, -NR⁶-C(O)-O-, -NR⁶-C(O)-NR⁶-, -S(O)ₚ-NR⁶-, -NR⁶-S(O)ₚ- and -NR⁶-S(O)ₚ-NR⁶-;
L¹⁰ is selected from the group consisting of hydrogen, C₁₋₆ alkyl, -(O-CH₂CH₂)_{q}-O-C₁₋₆ alkyl, and -C₁₋₆ alkylene-(O-CH₂CH₂)_{q}-O-C₁₋₆ alkyl, and the alkyl is optionally substituted with one or more groups selected from the group consisting of hydrogen, halogen, hydroxyl, -NH₂, -NH-C(O)-O-C₁₋₆ alkyl, C₁₋₆ alkoxy, carboxyl and -C(O)-O-C₁₋₆ alkyl;
R⁴ is selected from the group consisting of hydrogen, halogen, cyano, hydroxyl, C₁₋₆ alkyl, C₁₋₆ haloalkyl, C₁₋₆ alkoxy and C₃₋₁₀ cycloalkyl;
each R⁵ is independently hydrogen or C₁₋₆ alkyl;
each R⁶ is independently hydrogen or C₁₋₆ alkyl;
each r is independently 1 or 2;
each n is independently an integer selected from 1 to 25;
each p is independently 1 or 2;
each q is independently an integer selected from 1 to 25;
D² is connected to L² through R² or R³ in Formula (II).

24. The antibody-drug conjugate according to any one of claims 1 to 23, wherein D² is selected from the group consisting of the following structures:

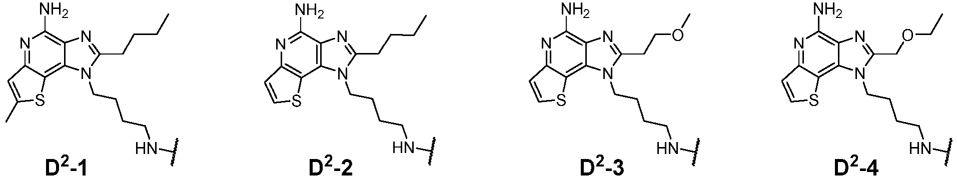

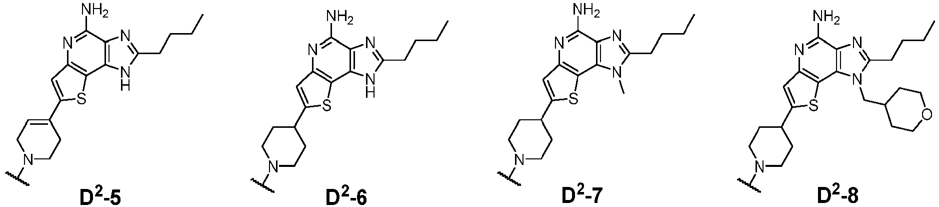

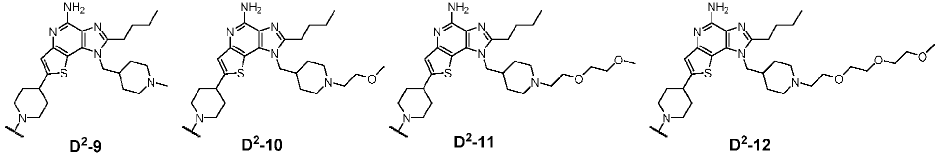

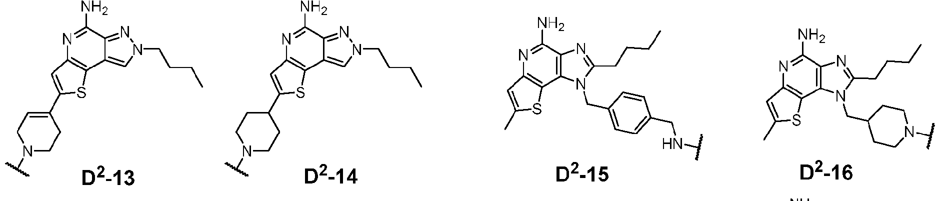

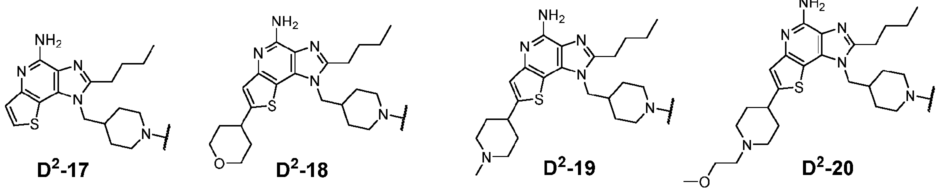

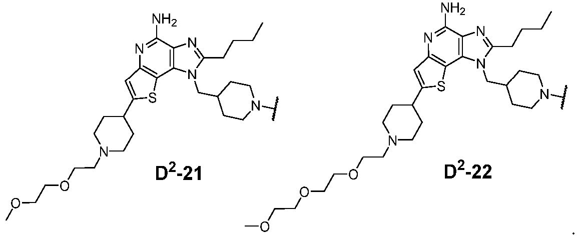

25. The antibody-drug conjugate according to any one of claims 1 to 24, which is selected from the group consisting of the following structures: 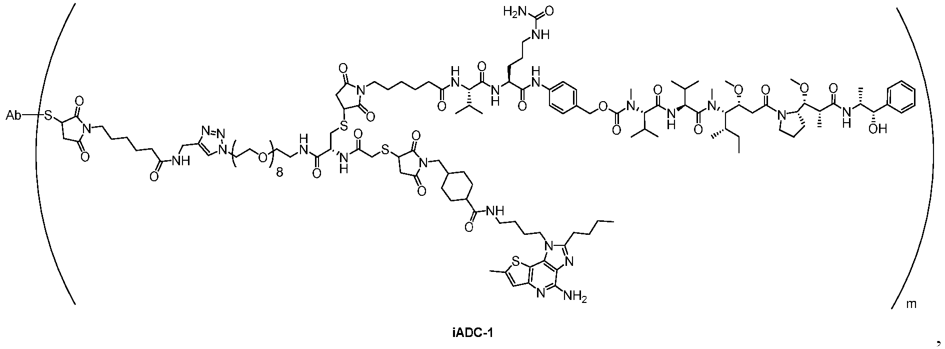 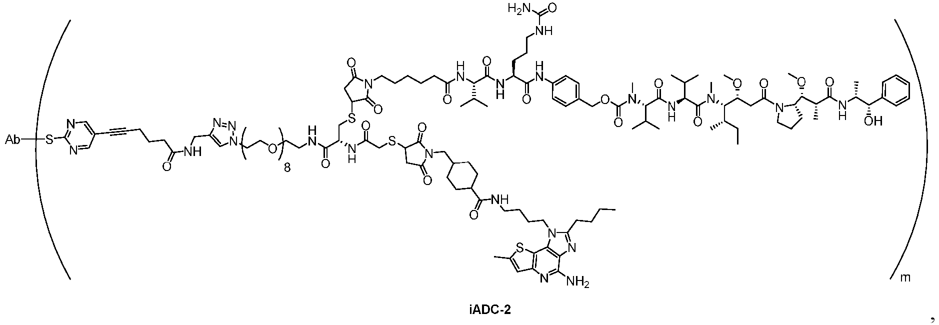 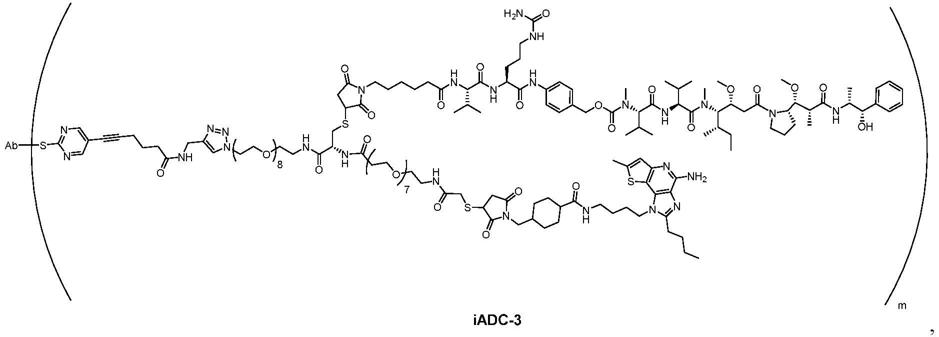 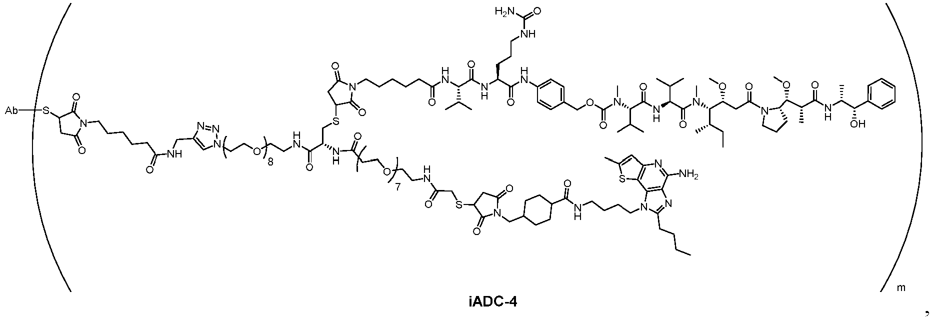 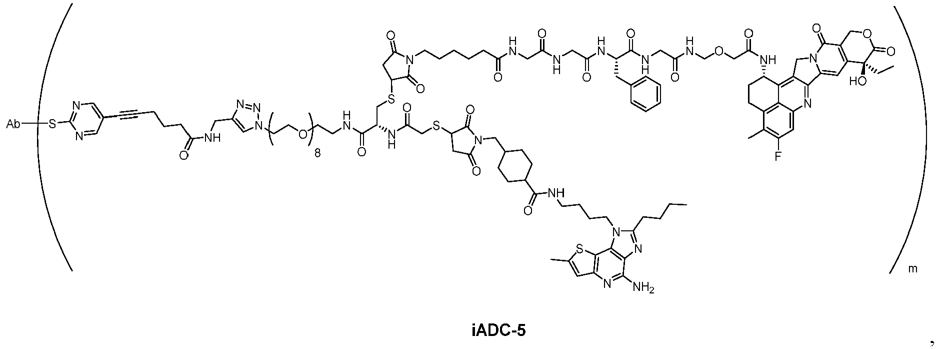 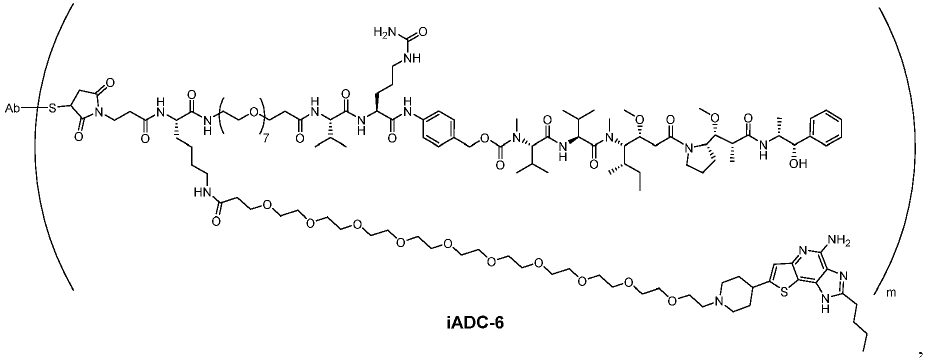 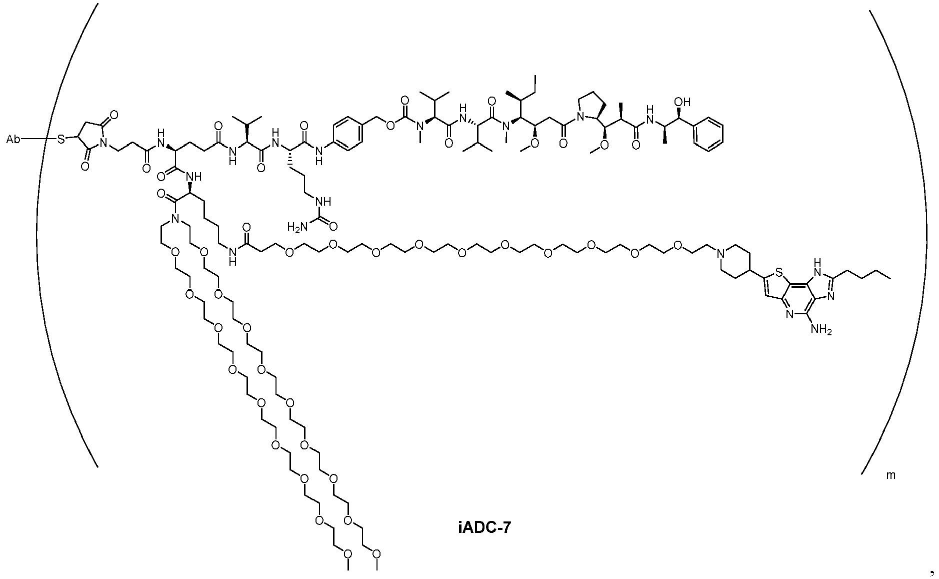 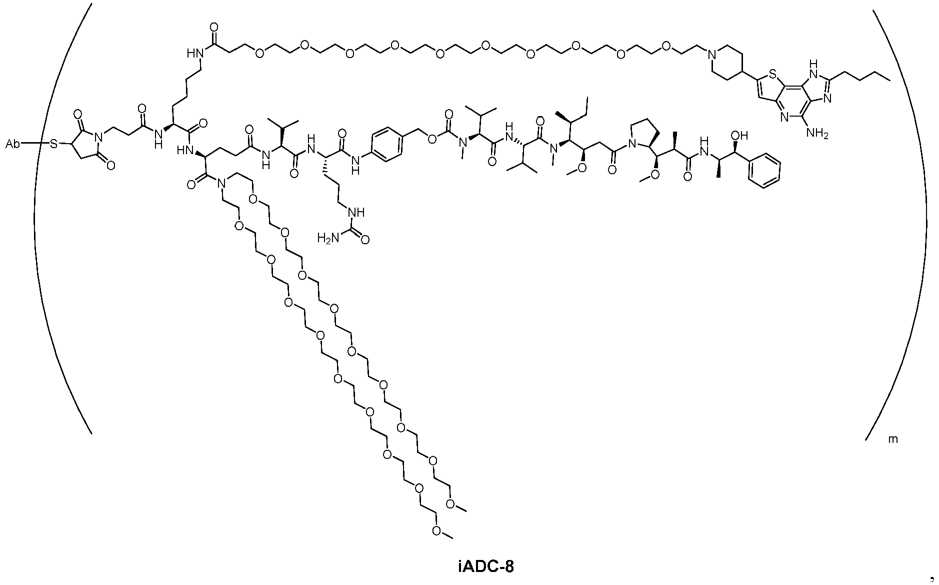 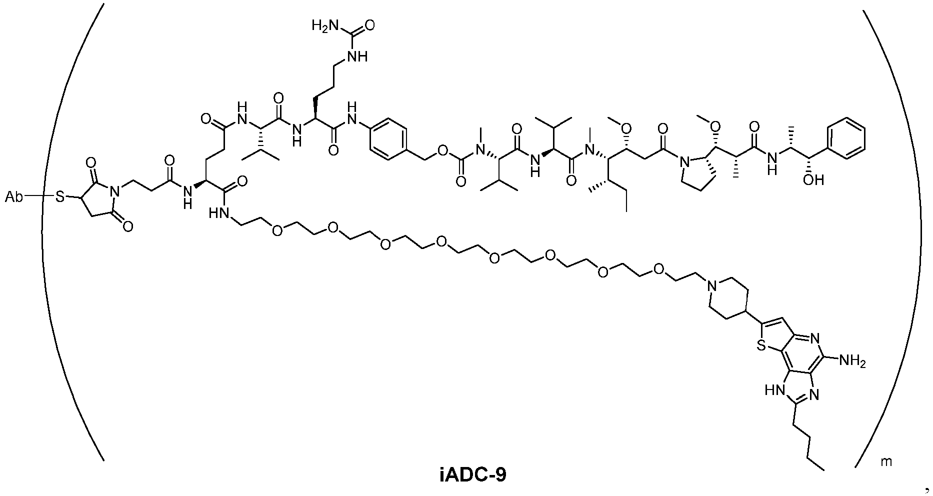 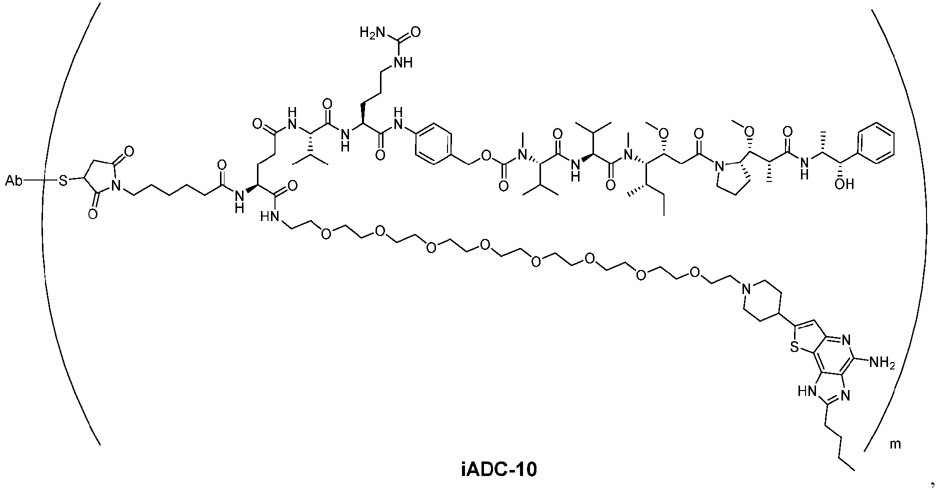 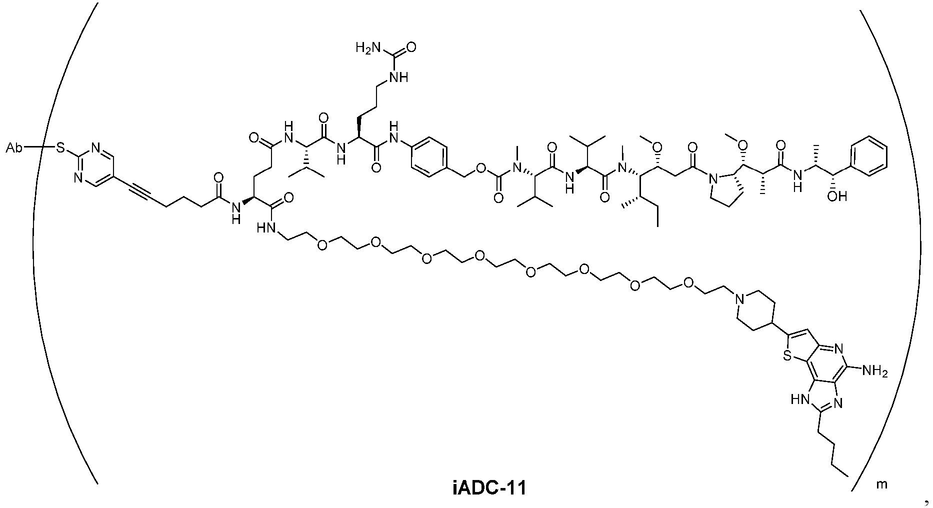 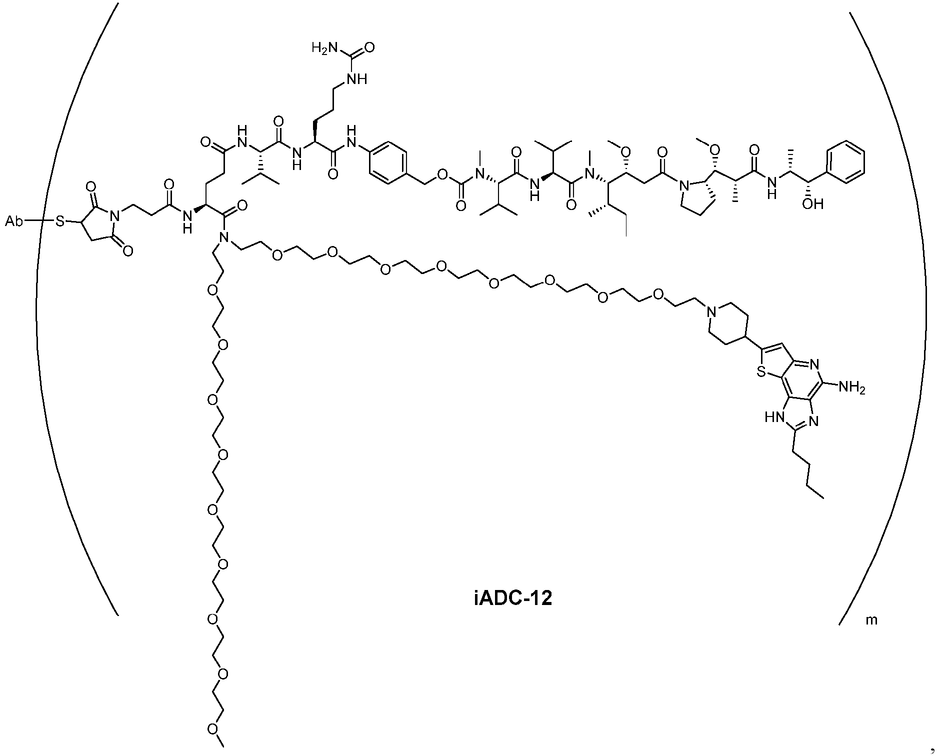 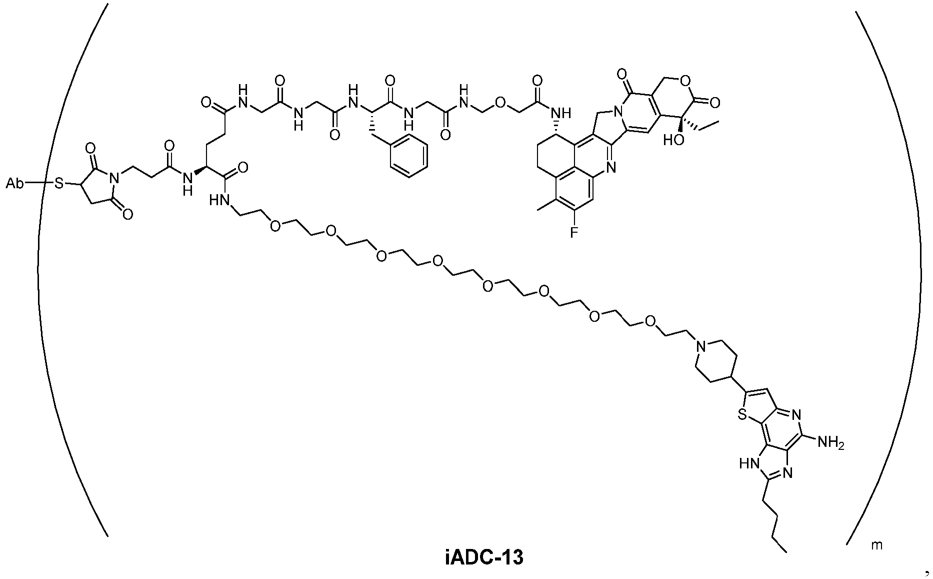 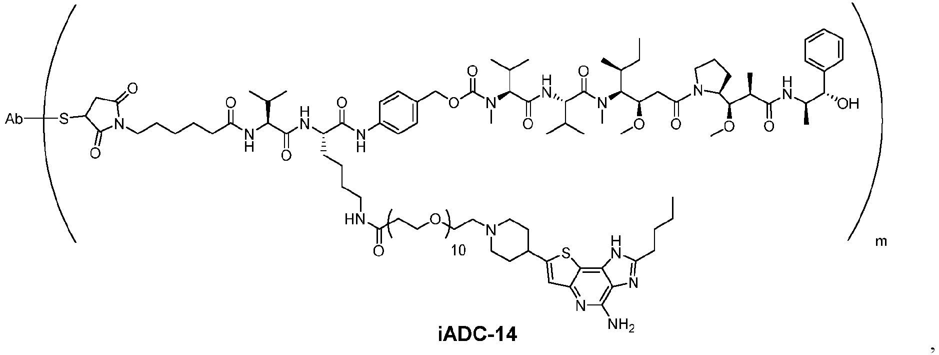 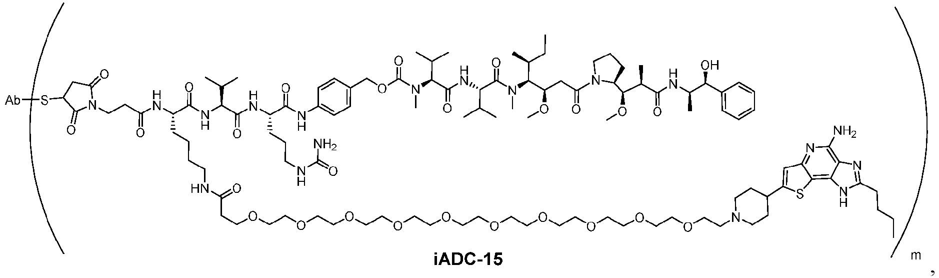 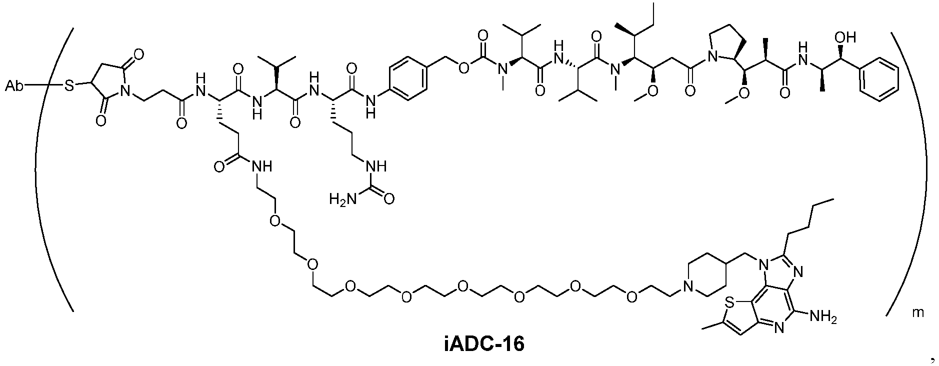 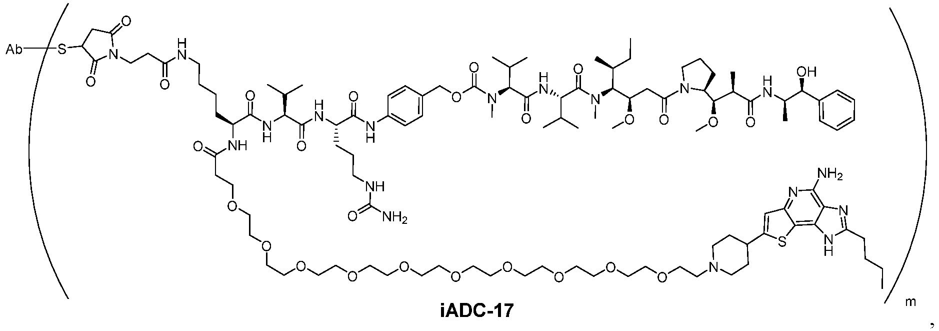 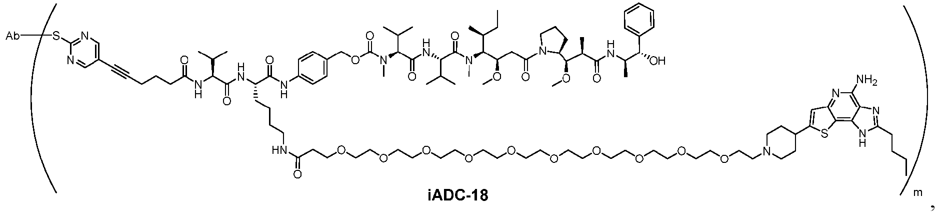 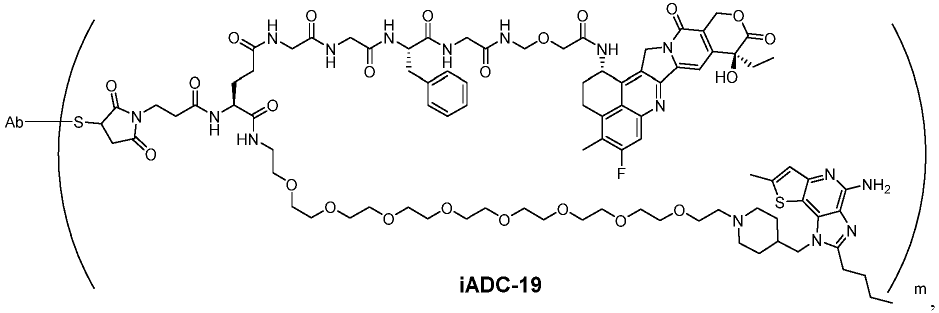 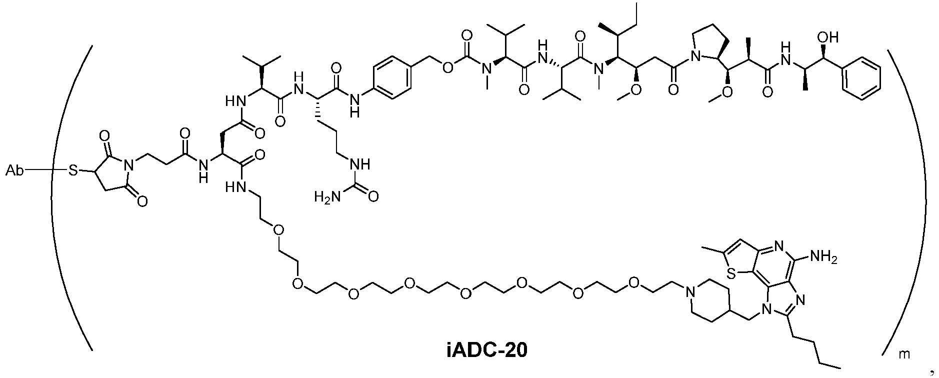 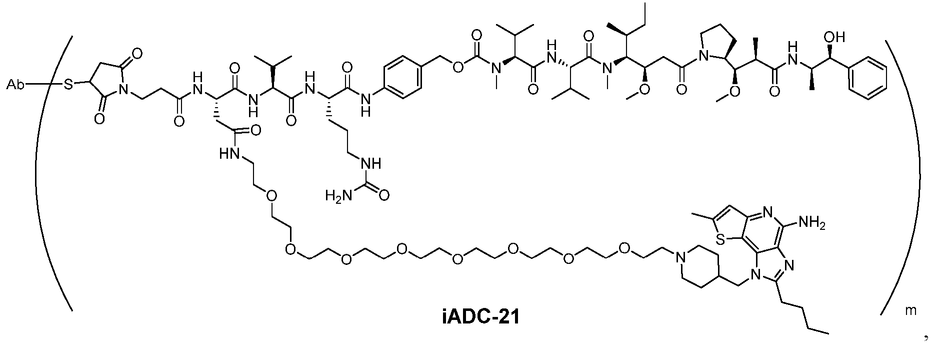 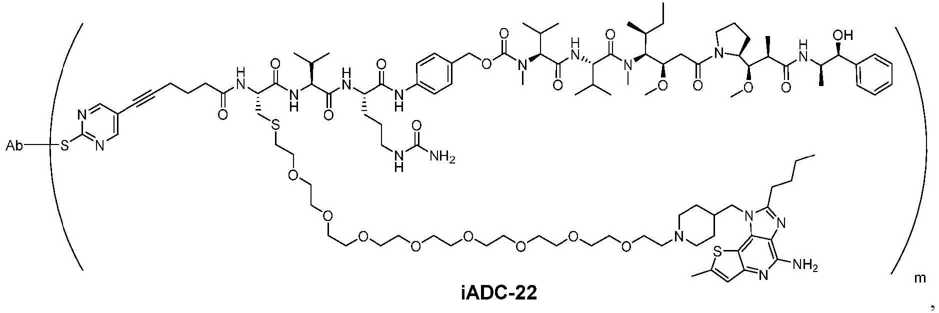 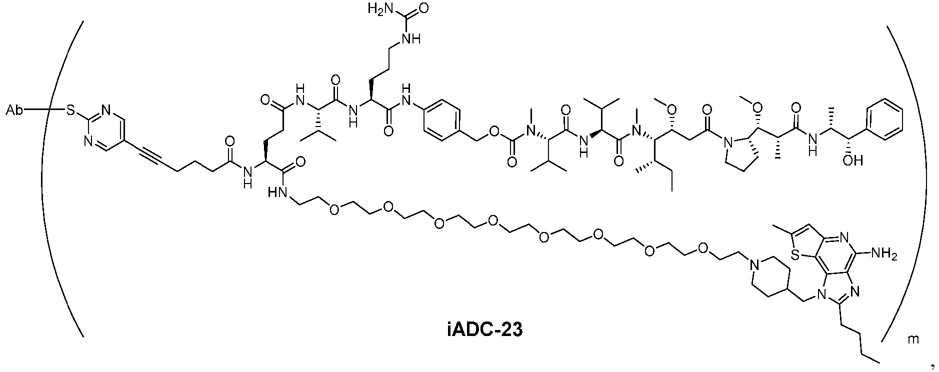 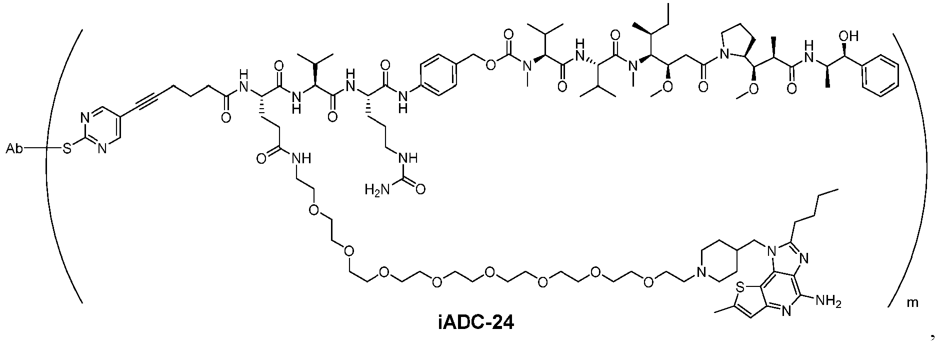 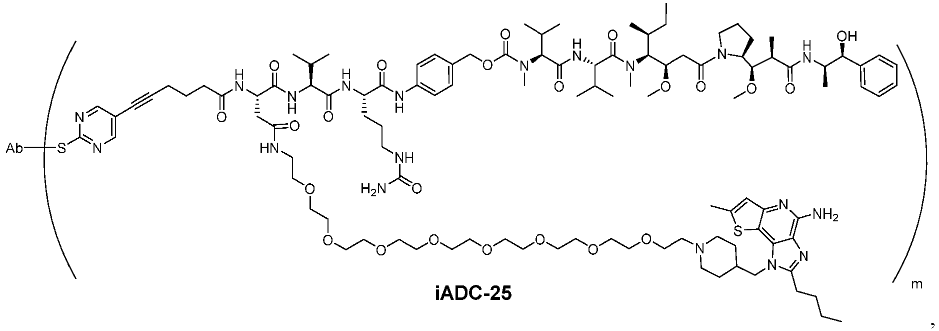 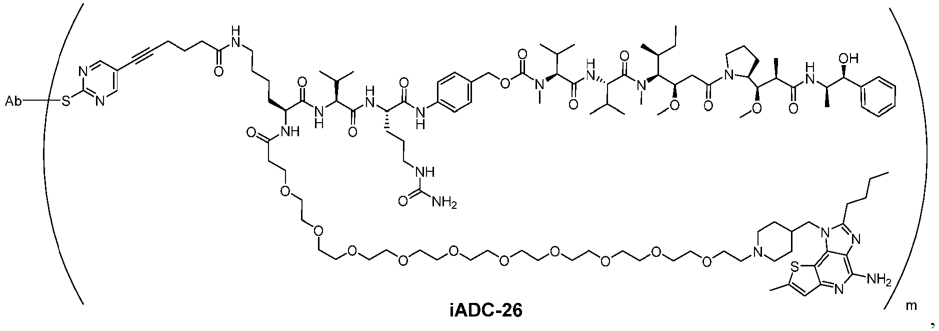 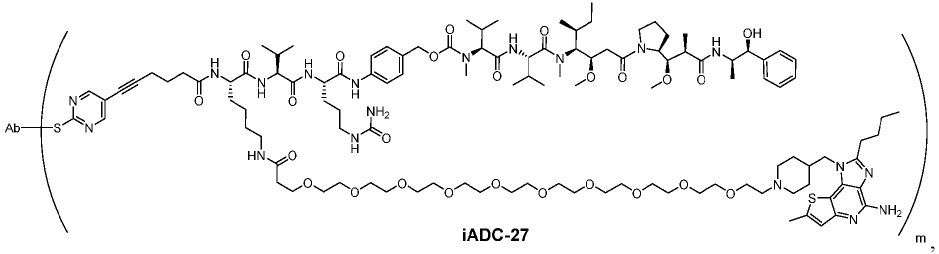 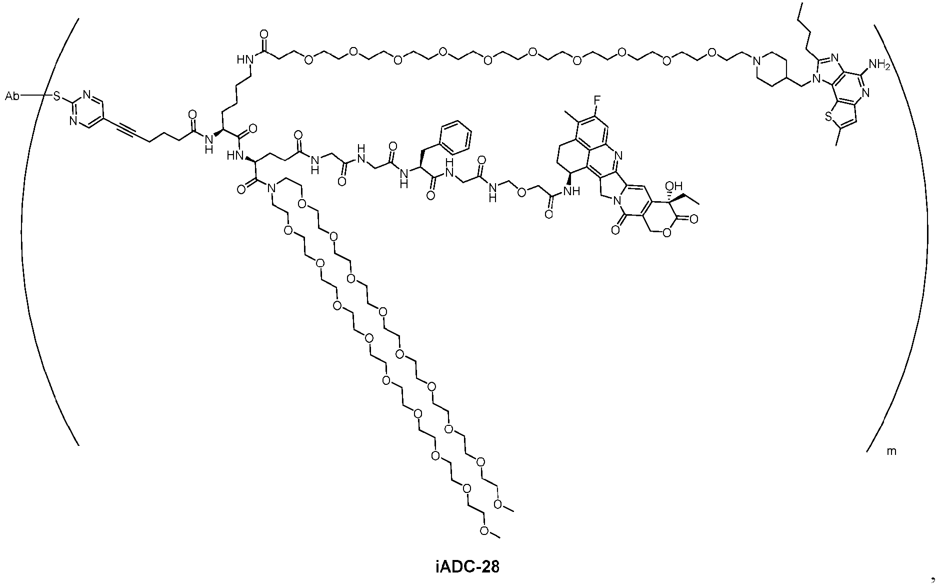 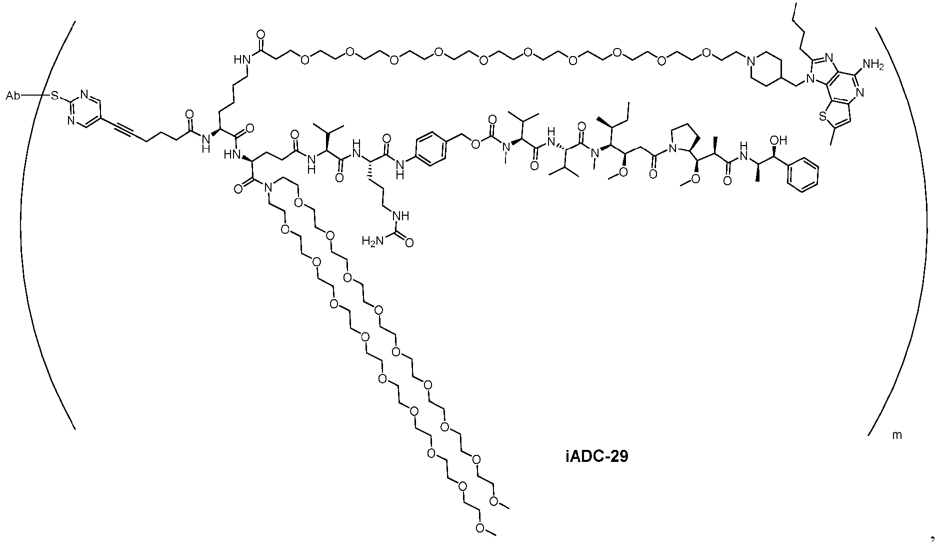 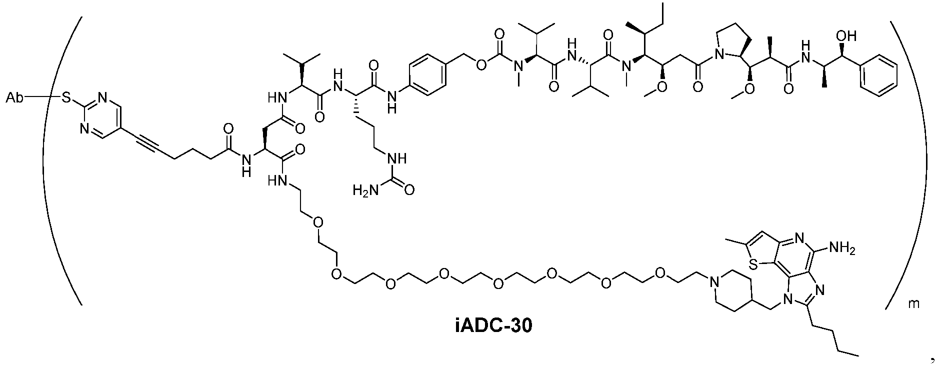 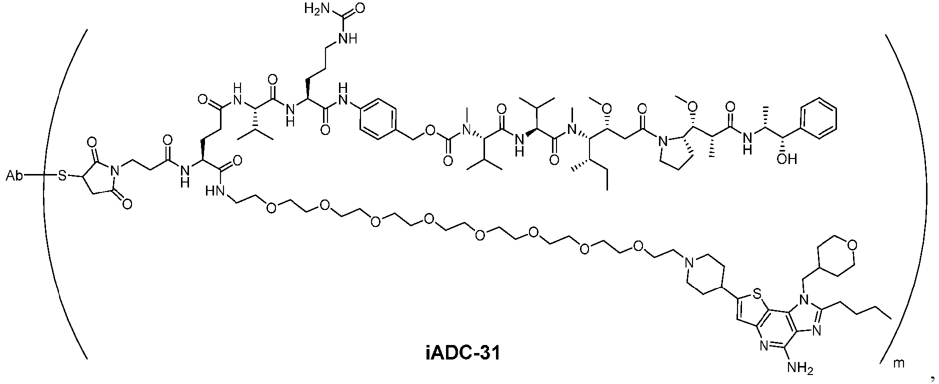 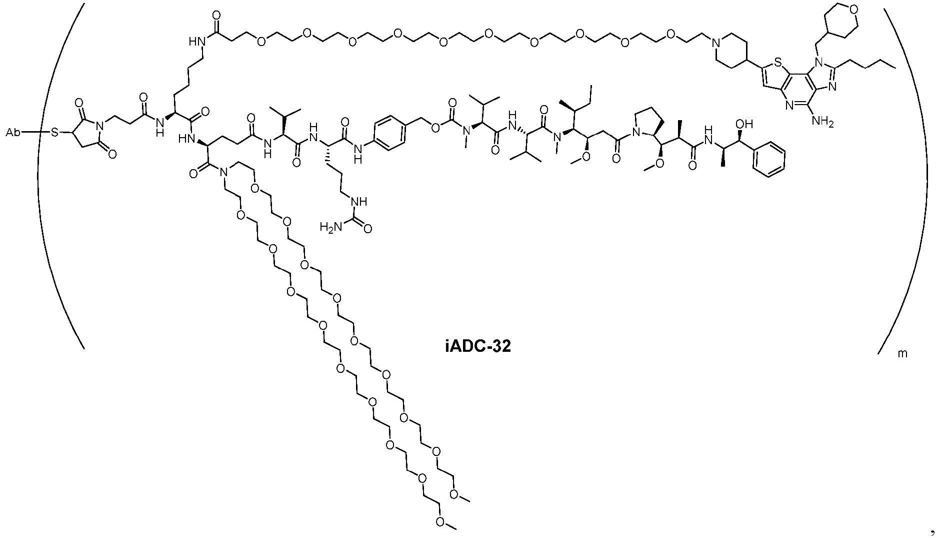 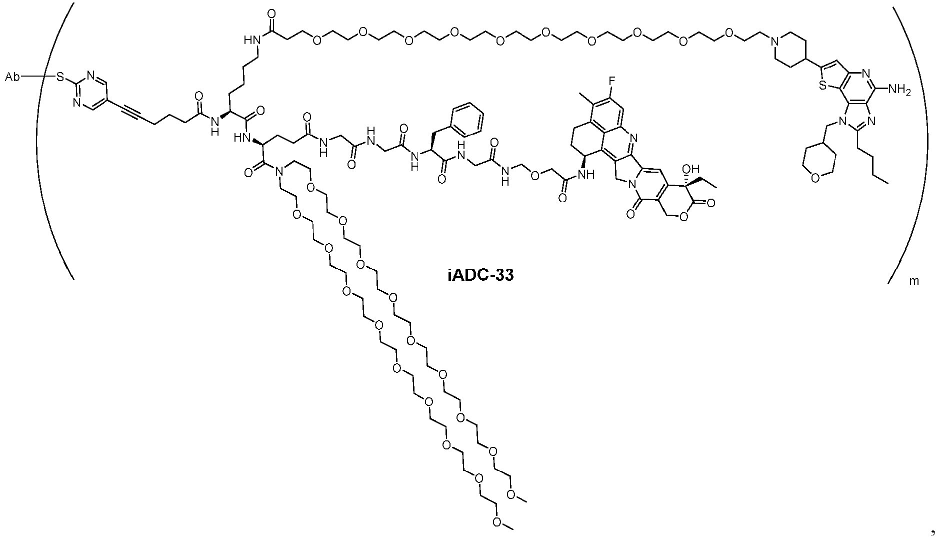 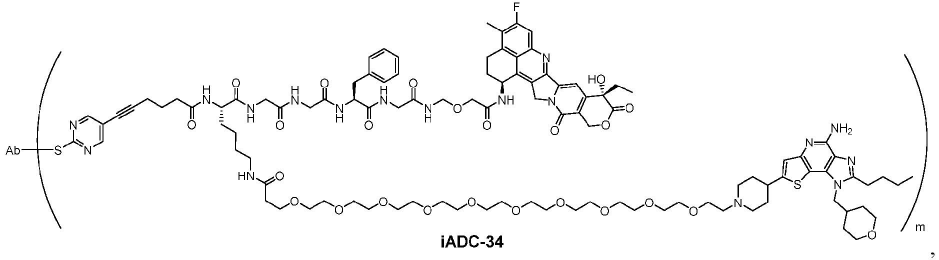 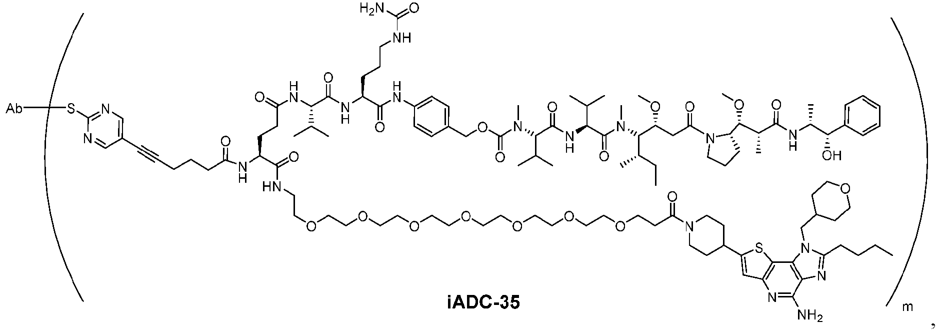 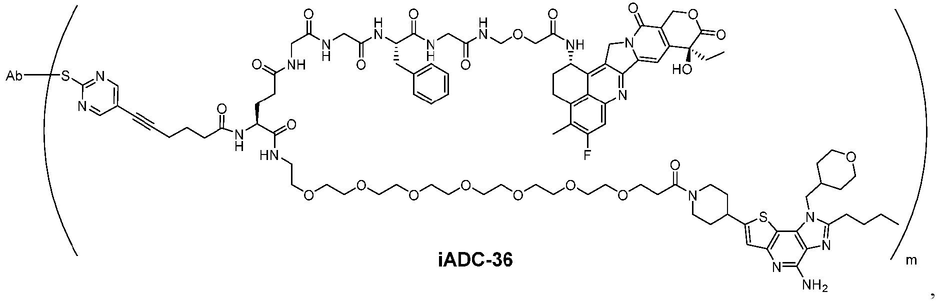 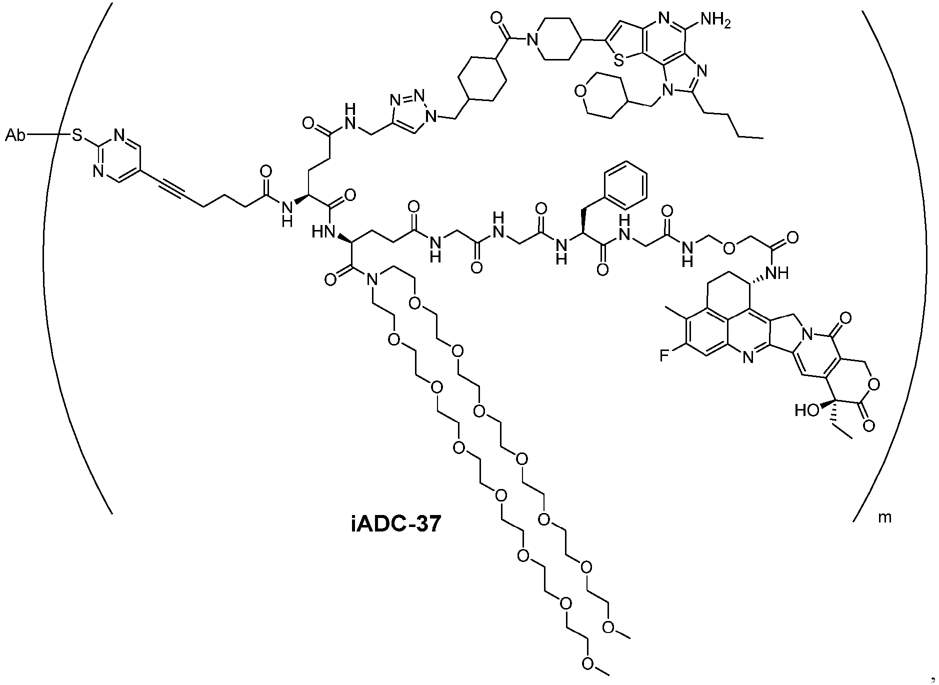 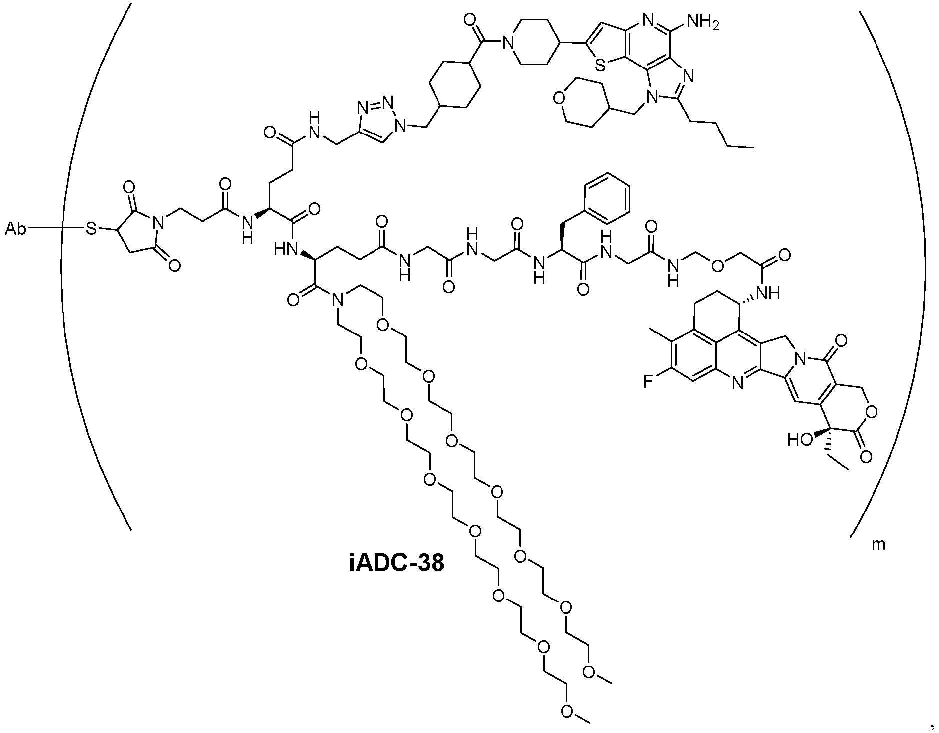 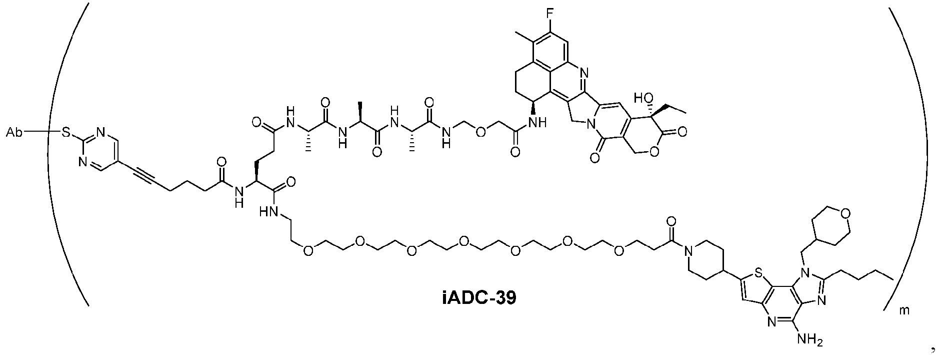 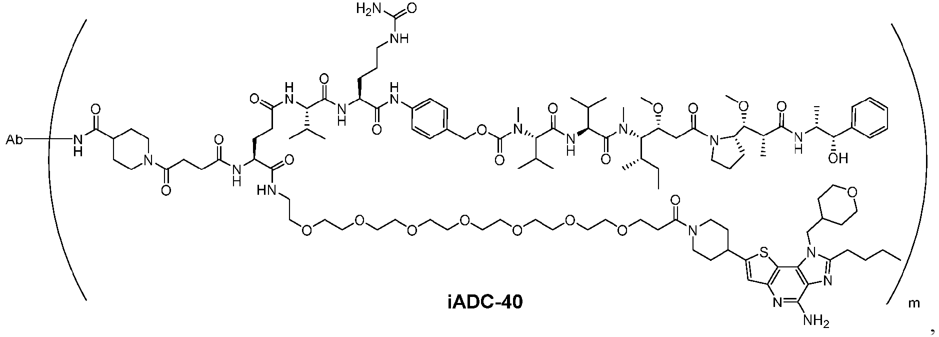 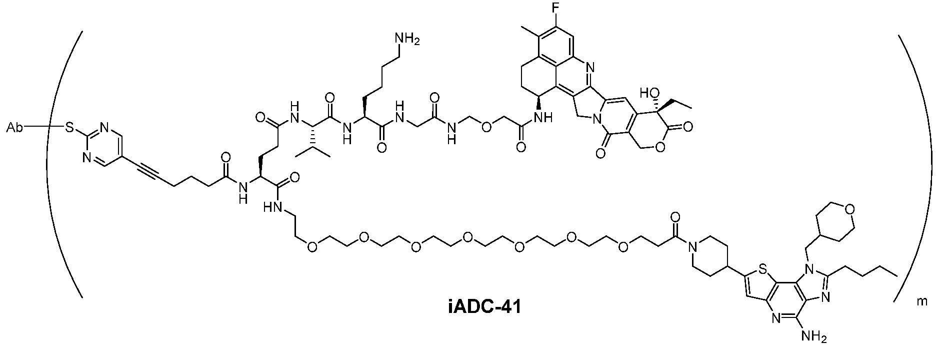 and 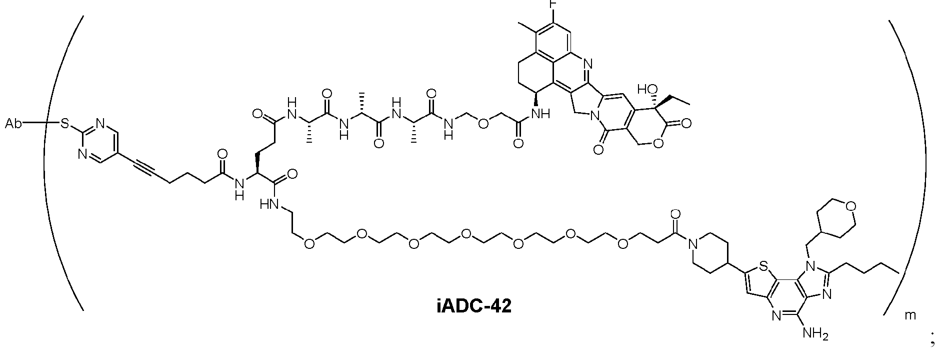
each m is independently selected from 1 to 10, such as 1, 2, 3, 4, 5, 6, 7, 8, 9 or 10;
preferably, Ab is Trastuzumab;
preferably, the antibody-drug conjugate has a DAR value of 2.0 to 5.0, such as 2.0 to 2.5, 2.0 to 3.0, 2.0 to 3.5, 2.0 to 4.0, 2.0 to 4.5, 2.0 to 5.0, 2.5 to 3.0, 2.5 to 3.5, 2.5 to 4.0, 2.5 to 4.5, 2.5 to 5.0, 3.0 to 3.5, 3.0 to 4.0, 3.0 to 4.5, 3.0 to 5.0, 3.5 to 4.0, 3.5 to 4.5, 3.5 to 5.0, 4.0 to 4.5, 4.0 to 5.0, or 4.0 to 5.0.

26. A compound of Formula (III) or pharmaceutically acceptable salt, ester, stereoisomer, tautomer, polymorph, solvate, N-oxide, isotopically labeled compound, metabolite or prodrug thereof: 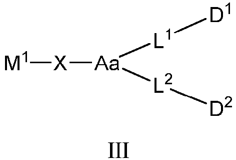 wherein,
M¹ is a precursor of a linking site connected to a targeting moiety, wherein the targeting moiety is as defined in any one of claims 1 to 25;
X is a linker connecting M and Aa;
Aa is an amino acid fragment, or a peptide fragments formed of two or more amino acids;
L¹ is a covalent bond or a linker connecting Aa and D¹;
L² is a linker connecting Aa and D²;
D¹ is a cytotoxic drug fragment;
D² is a TLR agonist fragment;
preferably, X, Aa, L¹, L², D¹ and D² are as defined in any one of claims 1 to 25.

27. The compound according to claim 26, wherein M¹ is selected from the group consisting of the following structures: 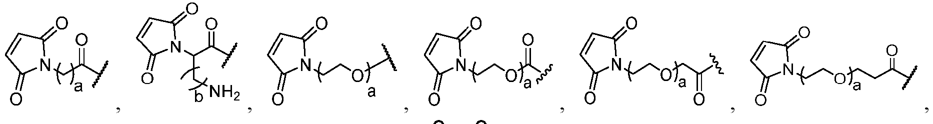 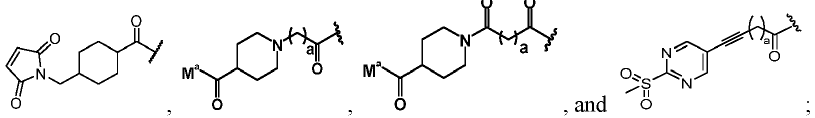
wherein, each a is independently an integer selected from 1 to 5, b is an integer selected from 1 to 3, and M^{a} is a carboxyl-activating group (e.g., pentafluorophenoxy);
preferably, M¹ is a covalent bond or selected from the group consisting of the following structures:

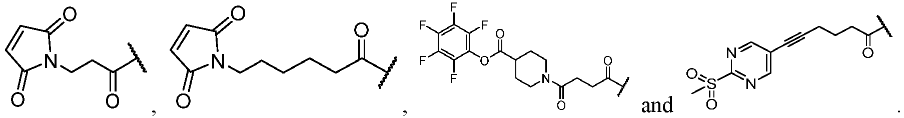

28. The compound or pharmaceutically acceptable salt, ester, stereoisomer, tautomer, polymorph, solvate, N-oxide, isotopically labeled compound, metabolite or prodrug thereof according to claim 26 or 27, which is selected from the group consisting of the following structures: 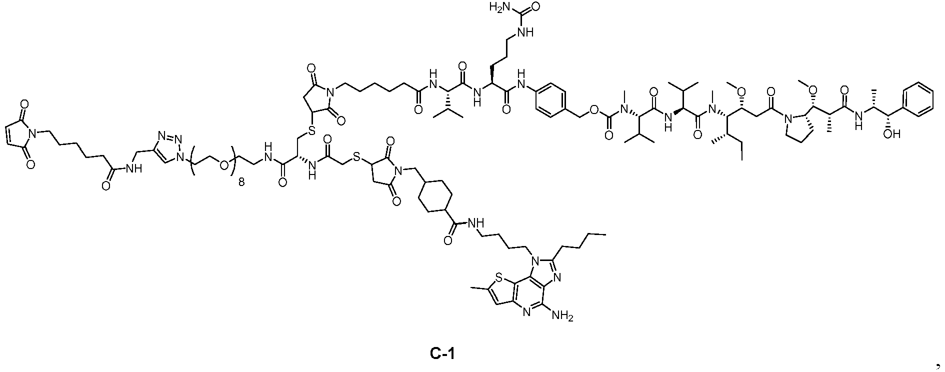 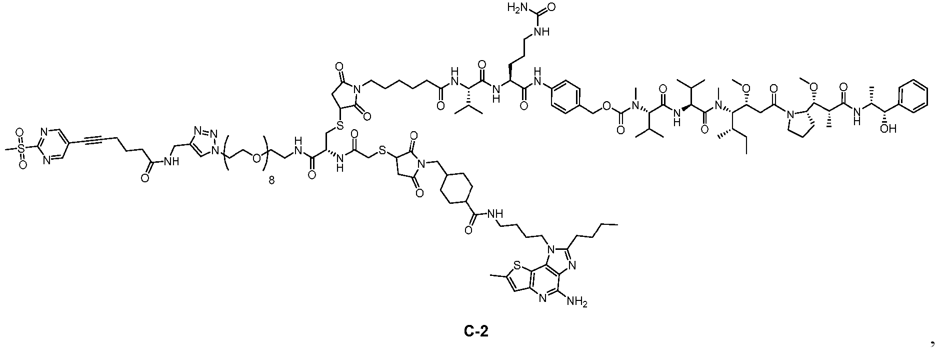 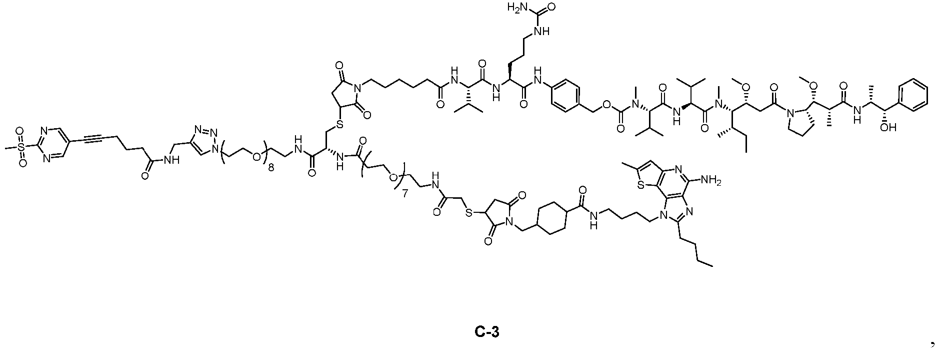 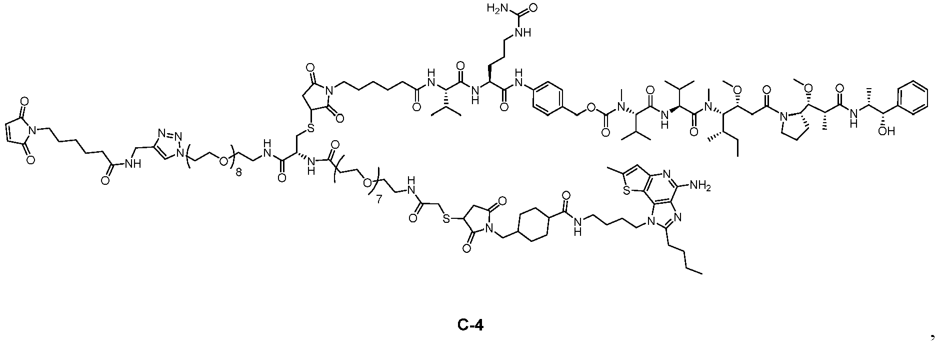 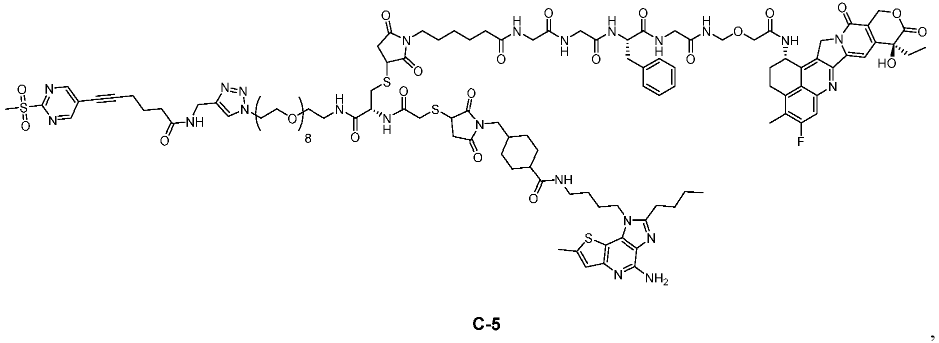 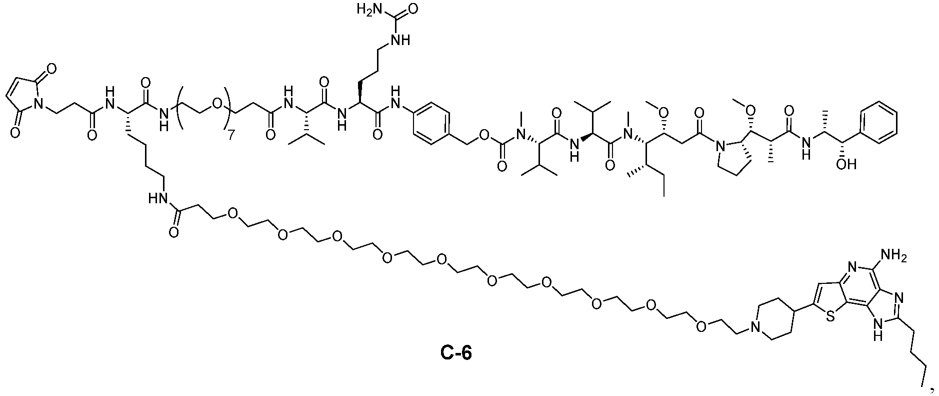 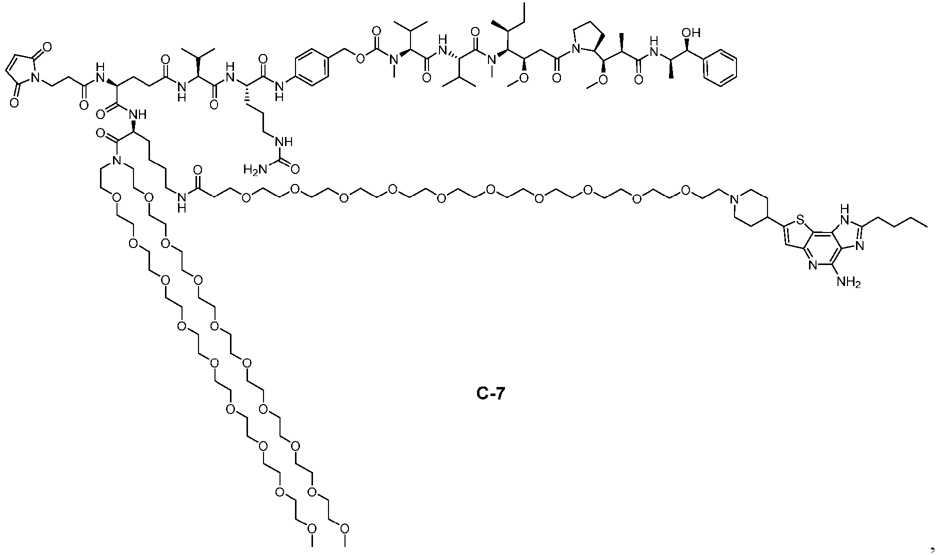 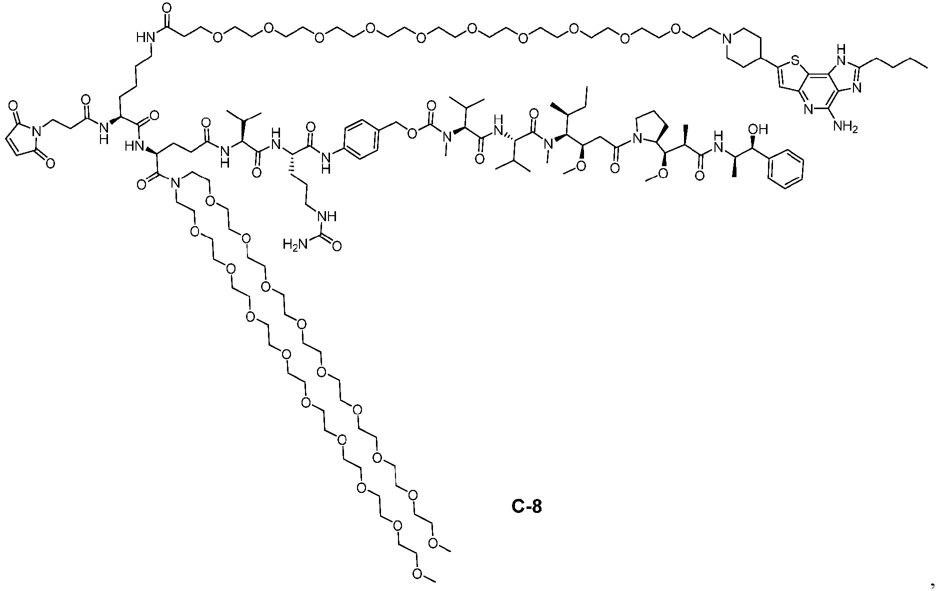 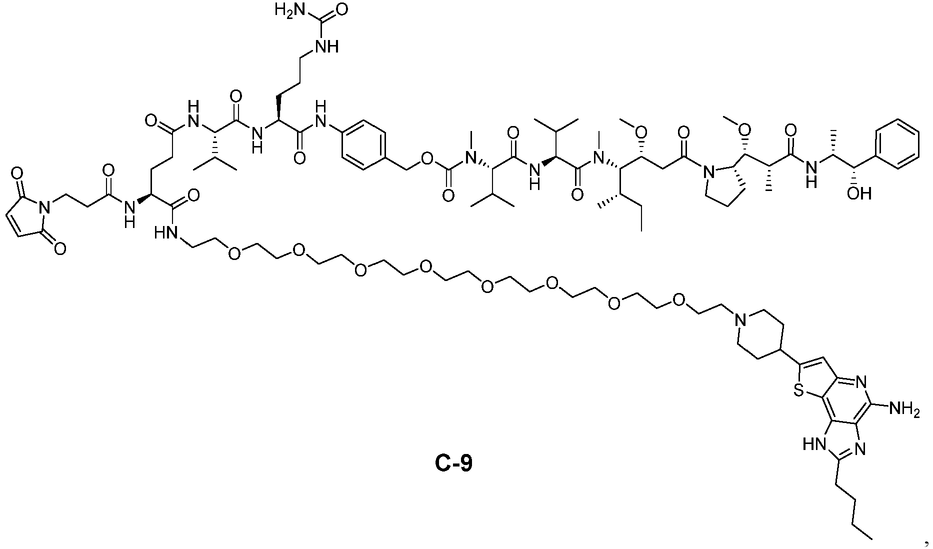 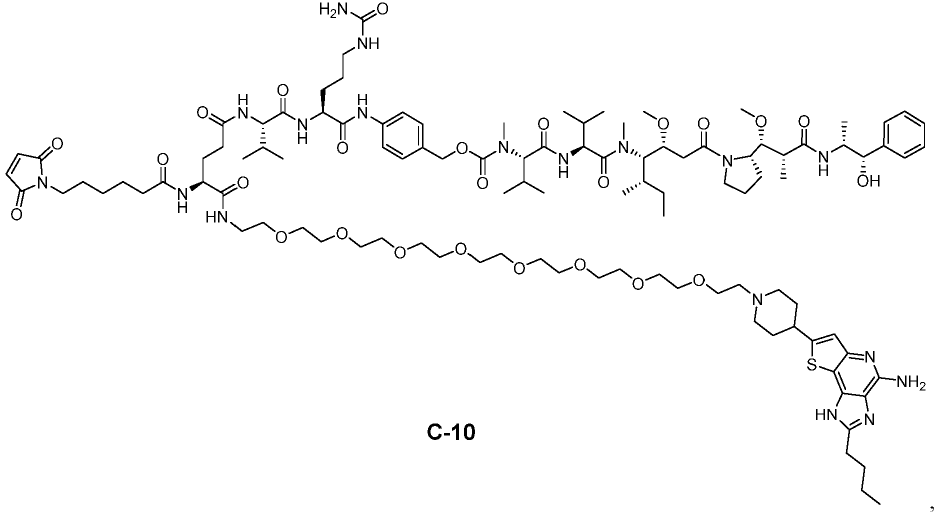 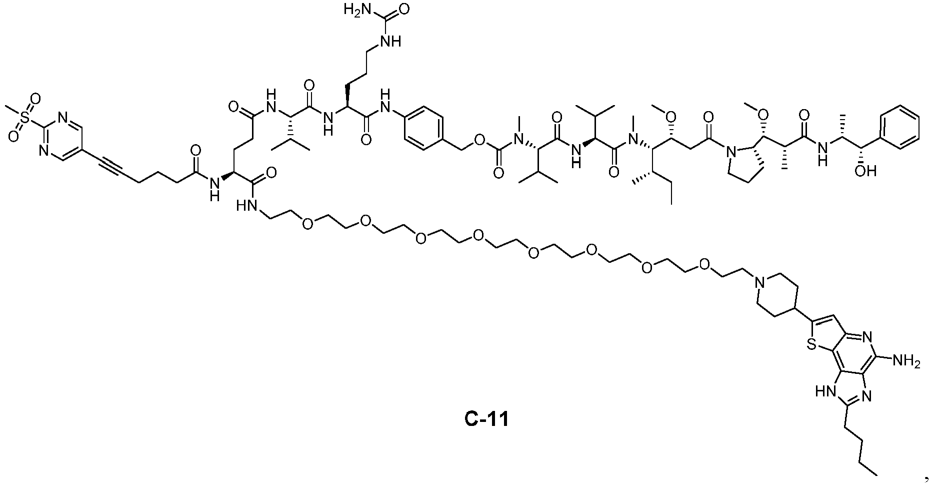 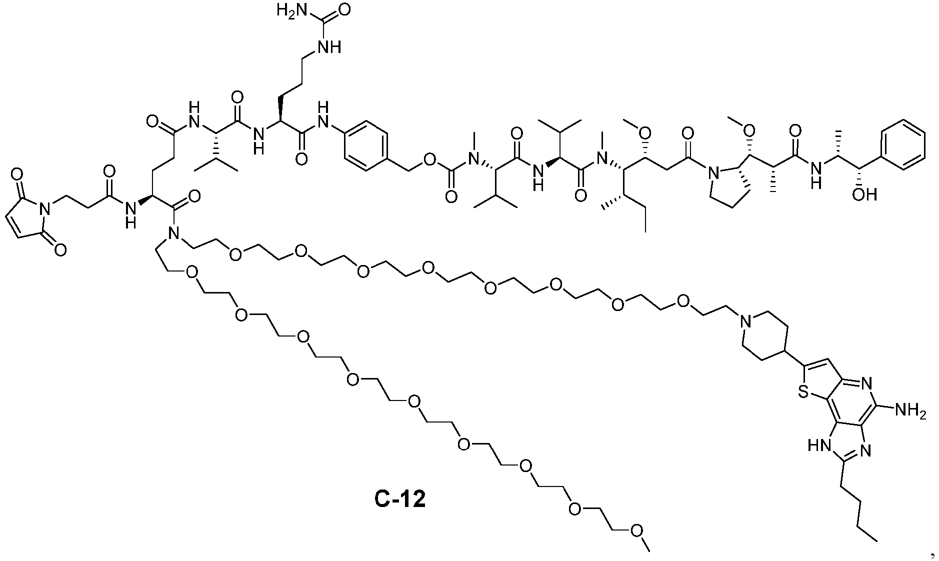 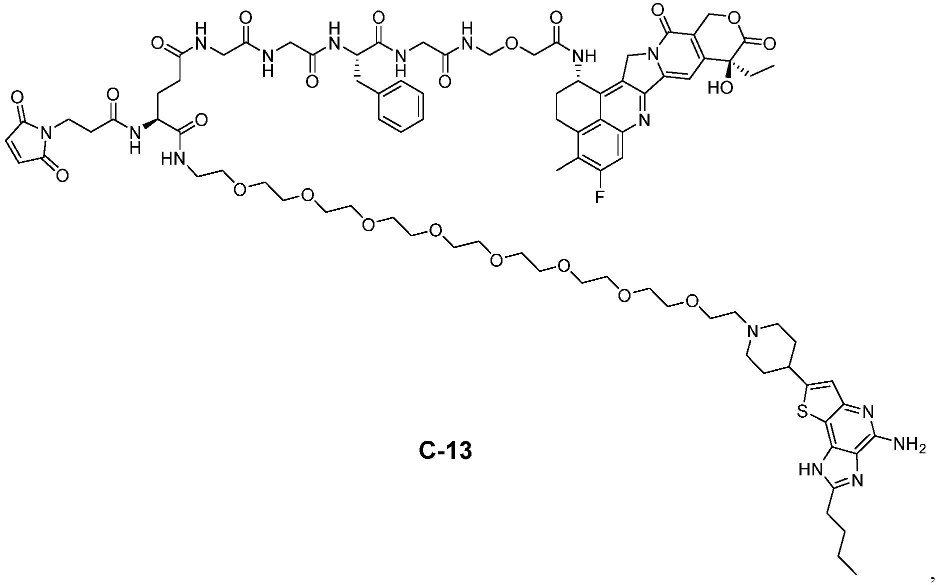 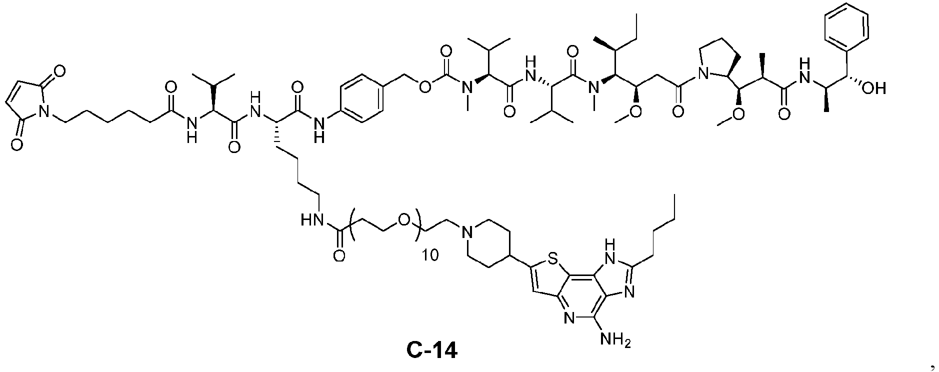 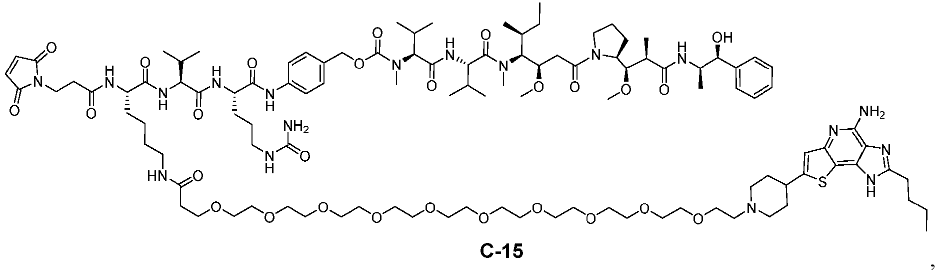 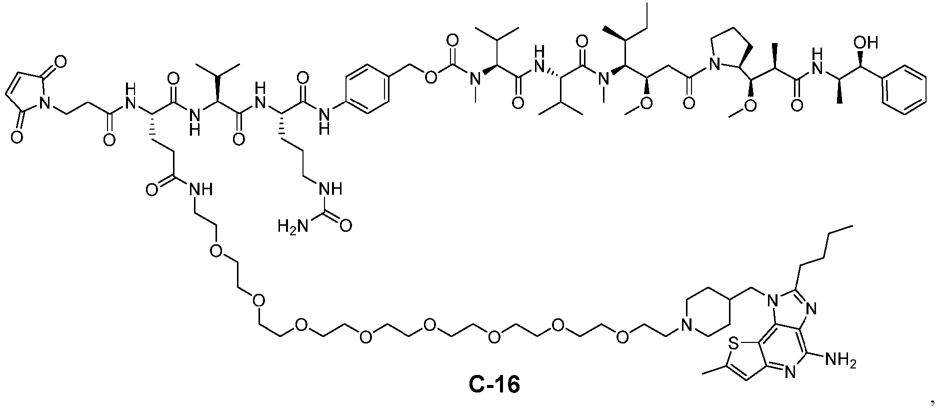 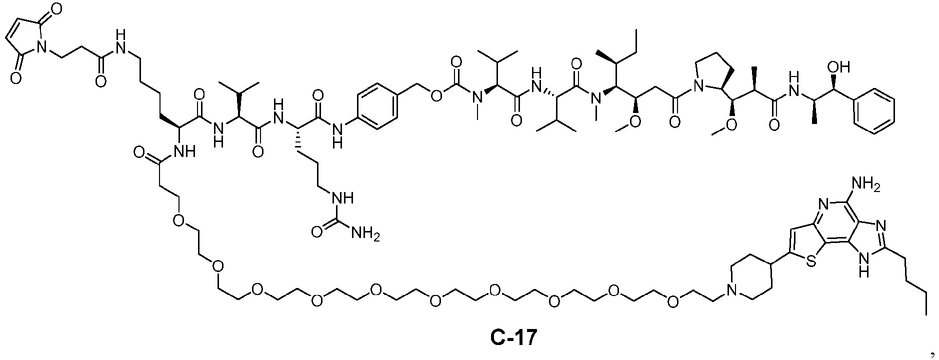 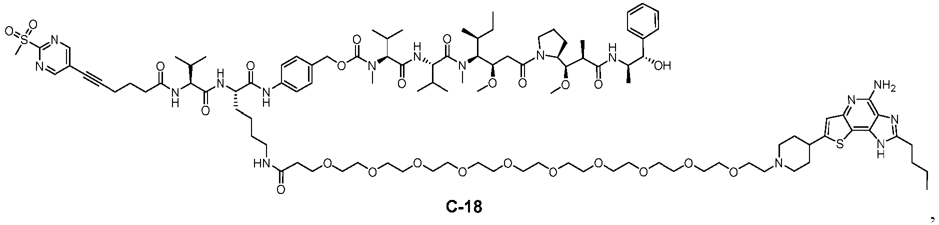 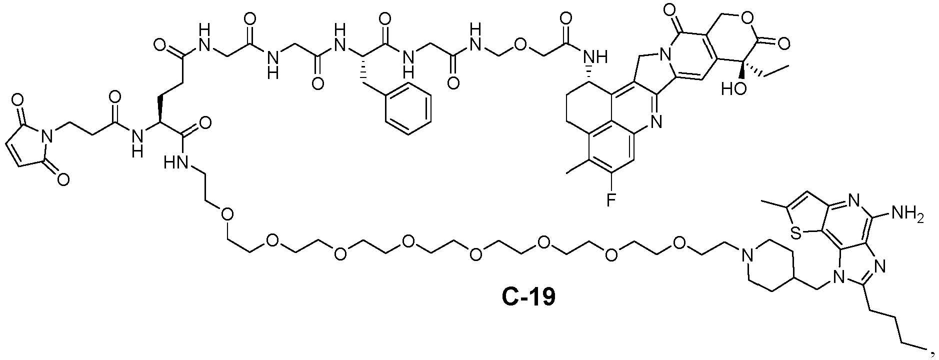 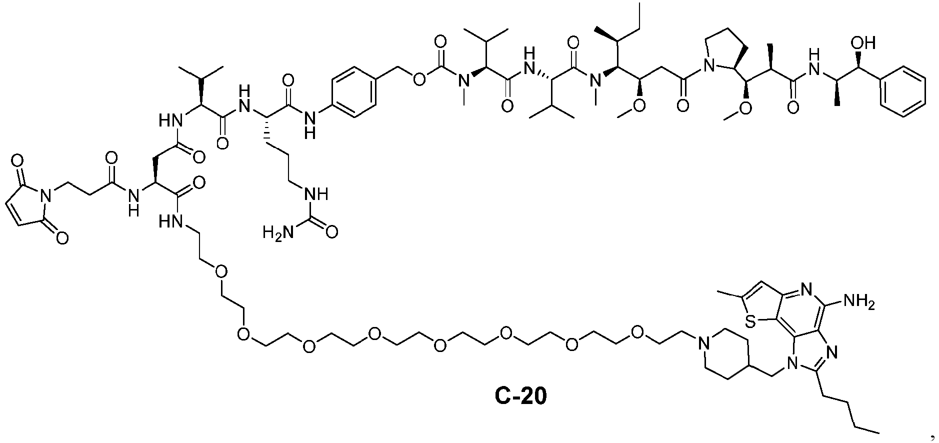 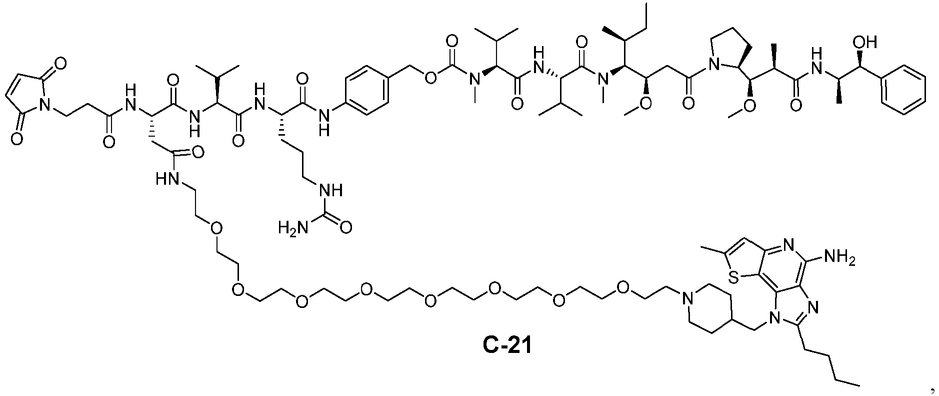 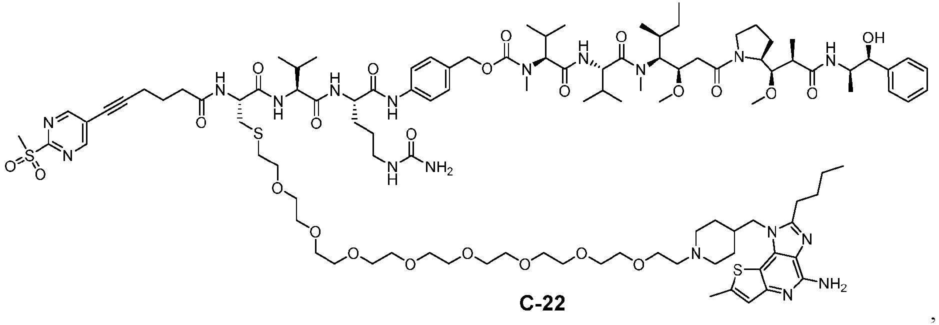 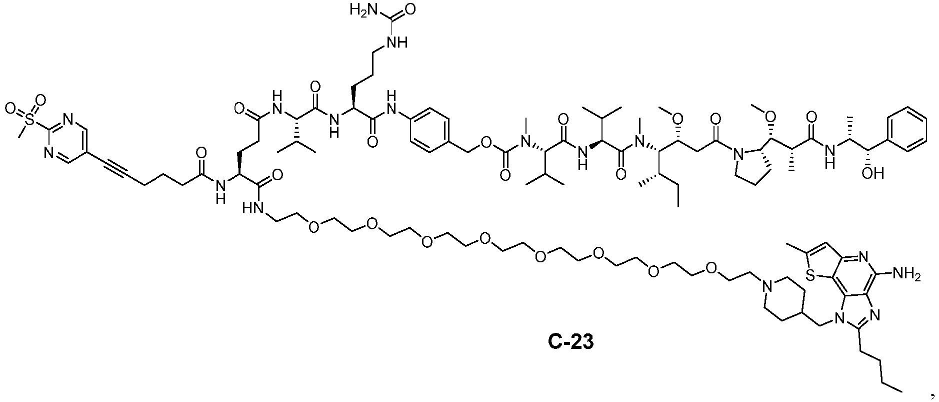 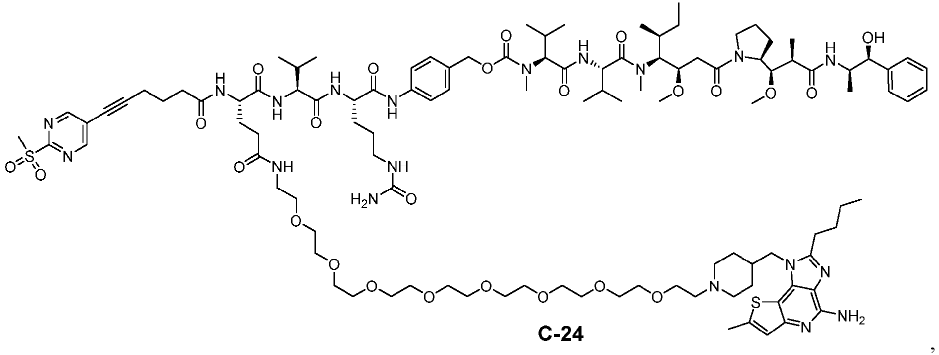 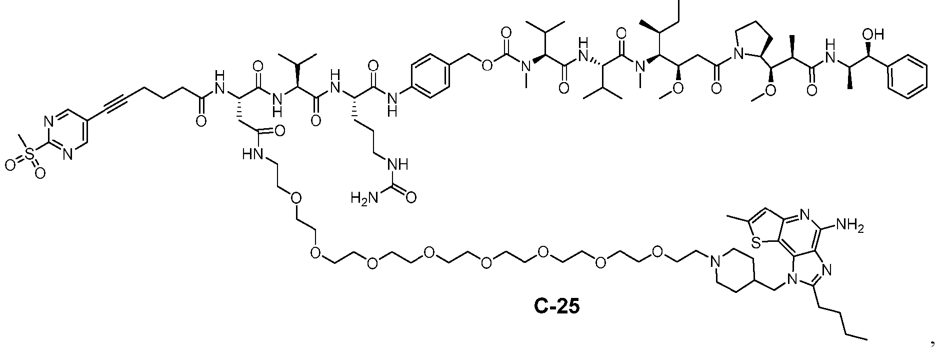 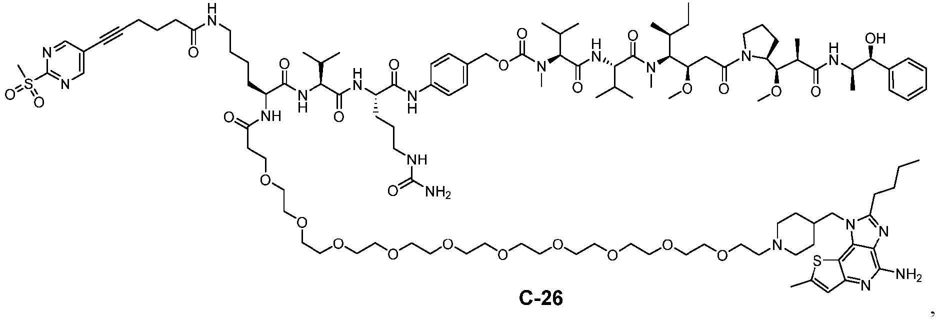 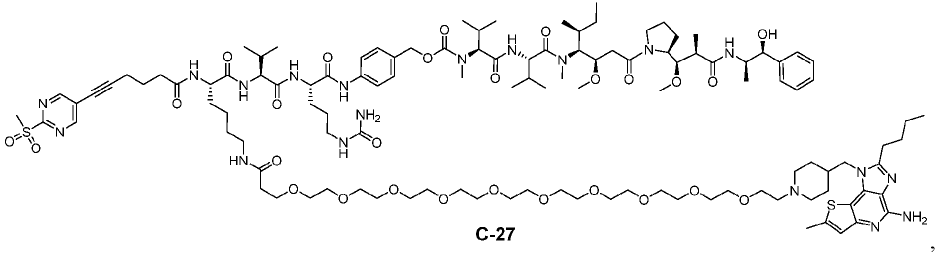 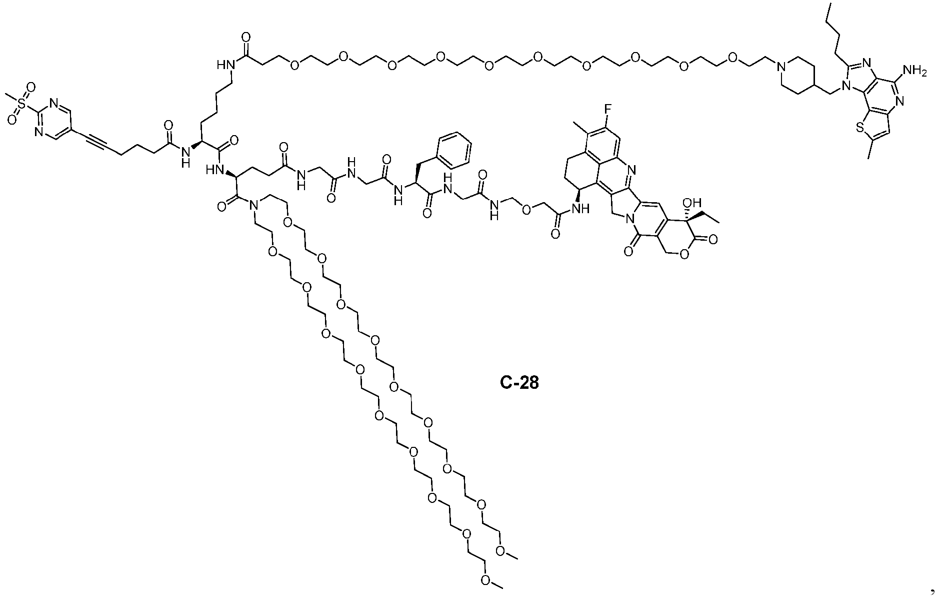 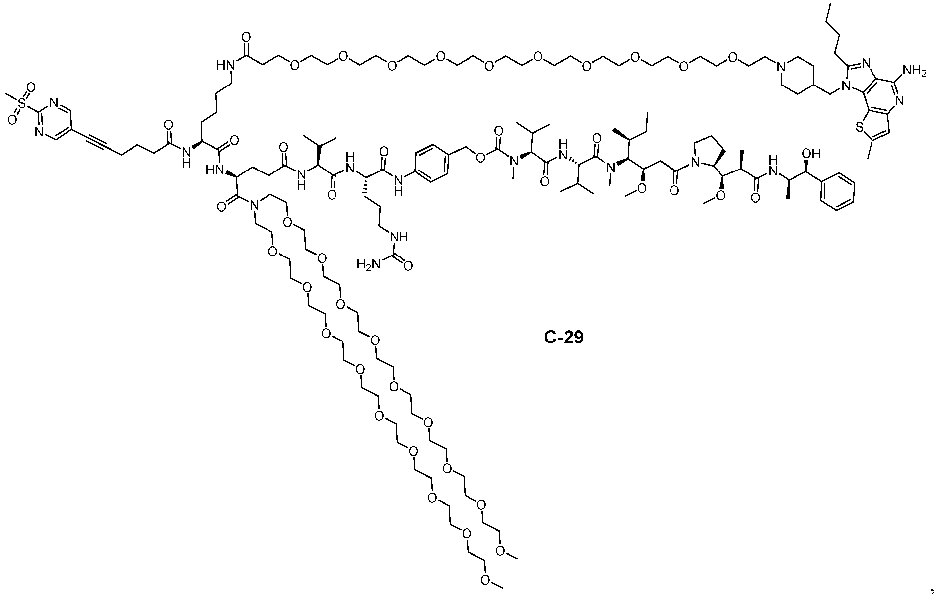 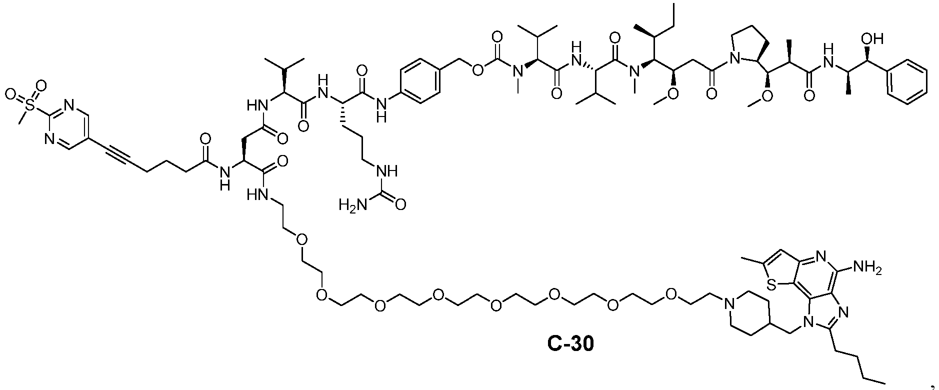 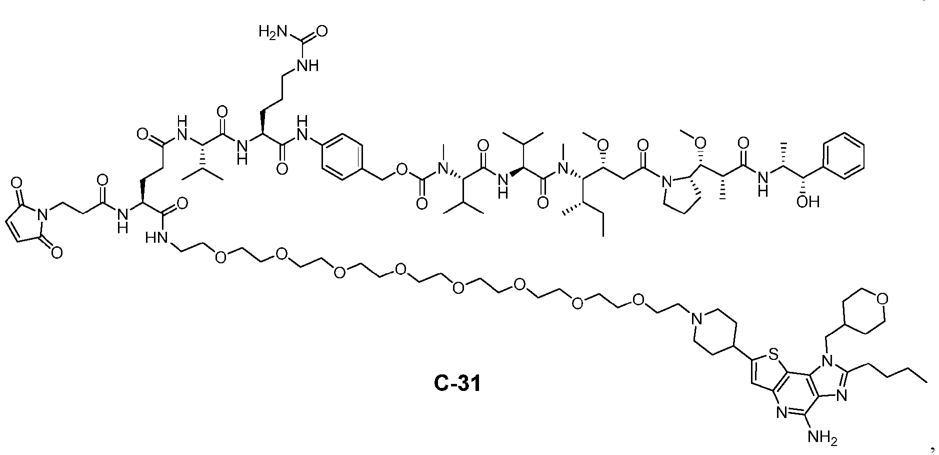 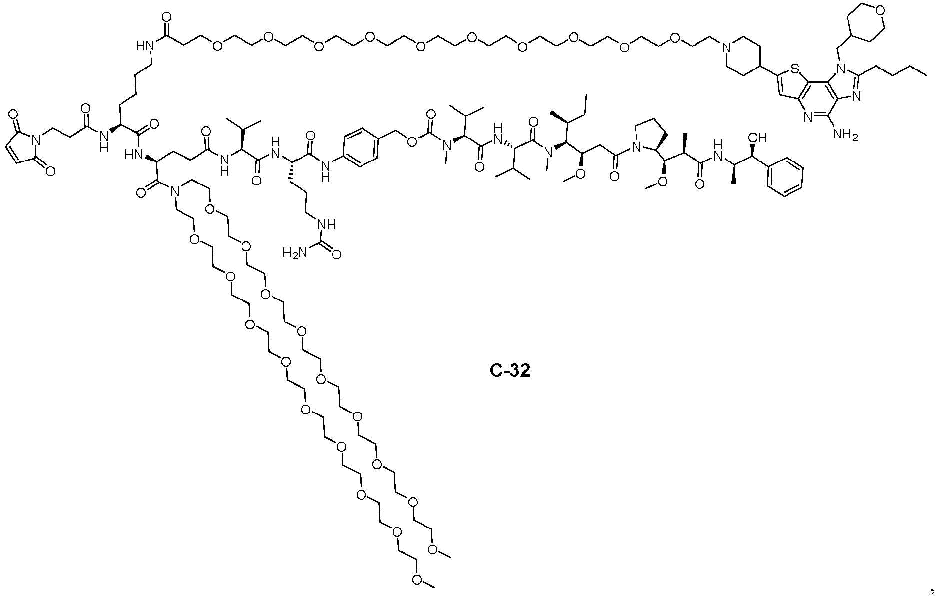 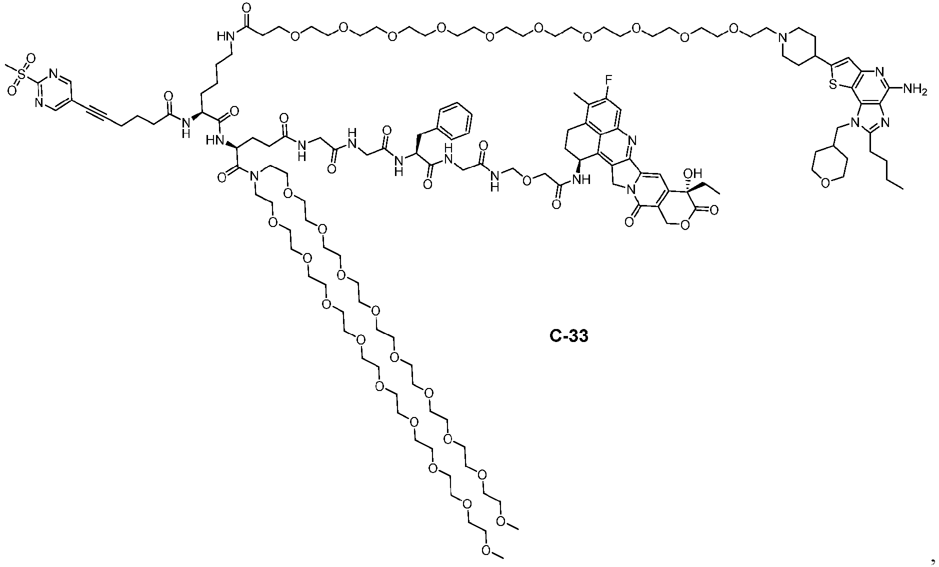 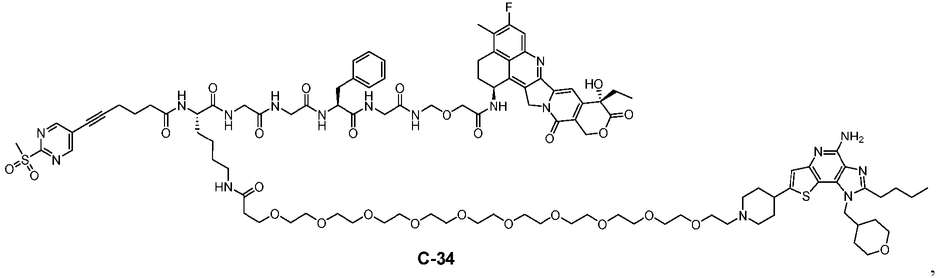 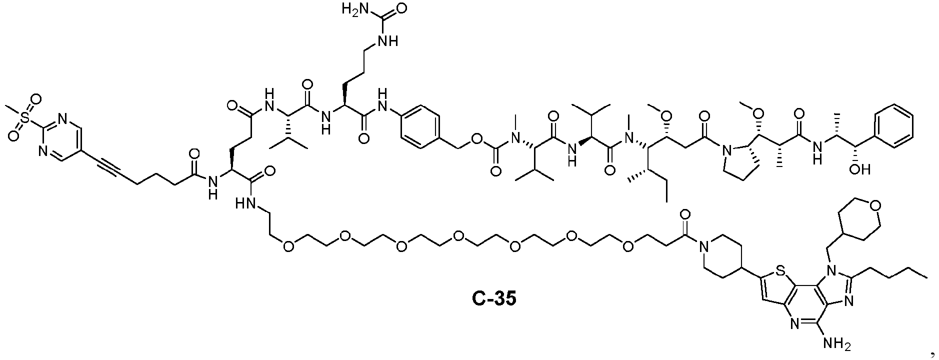 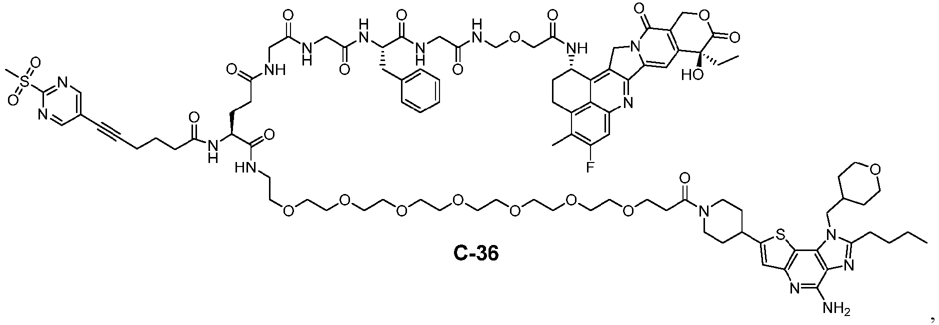 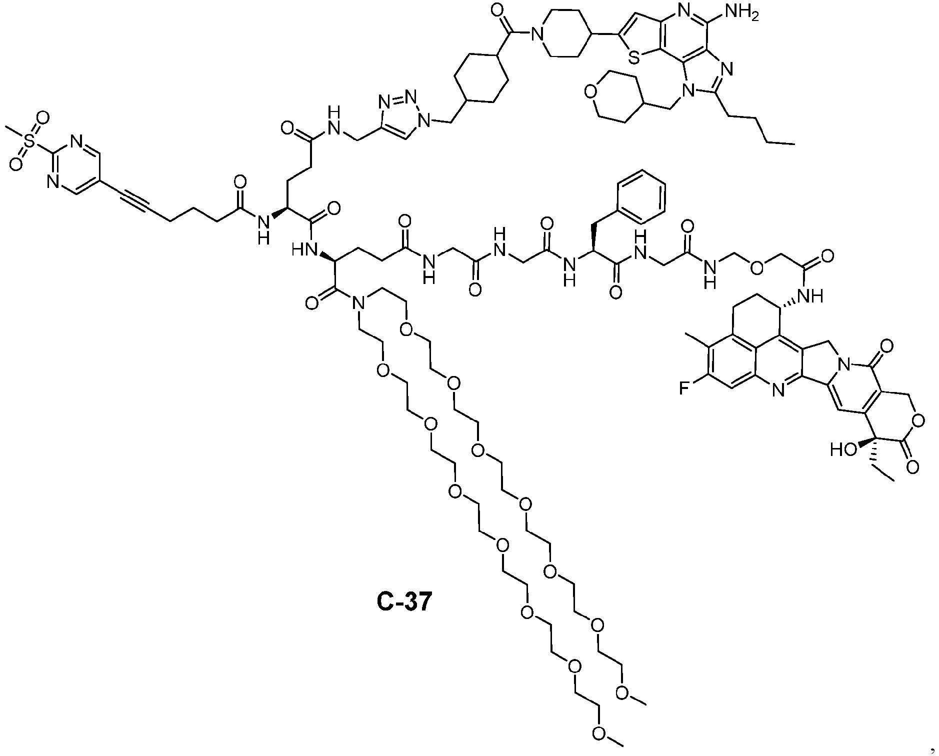 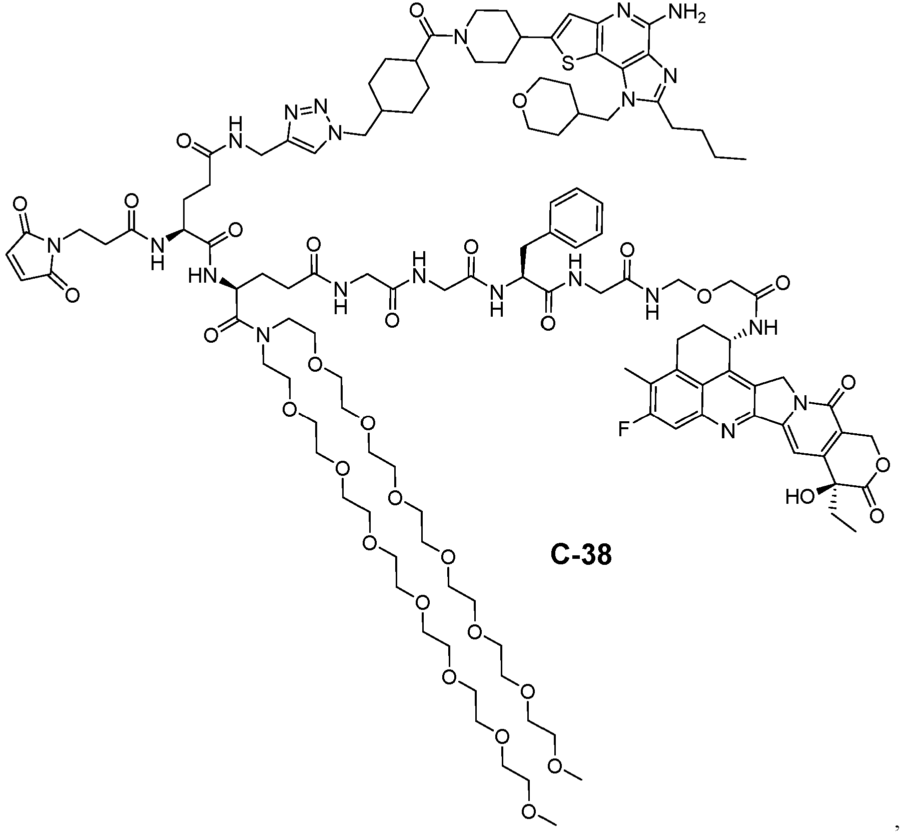 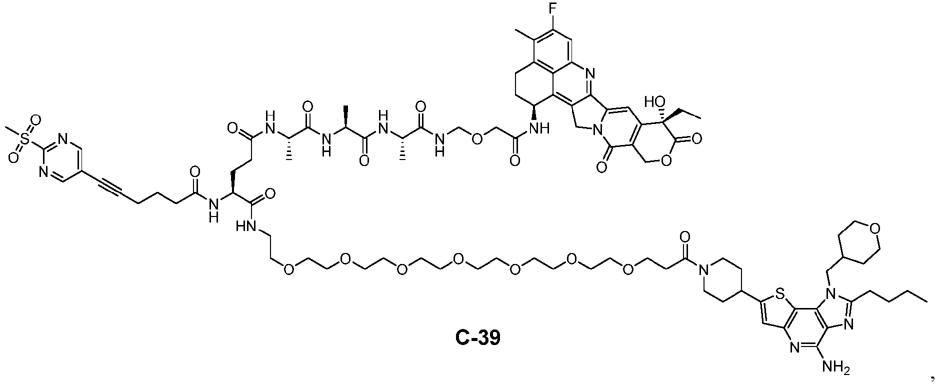 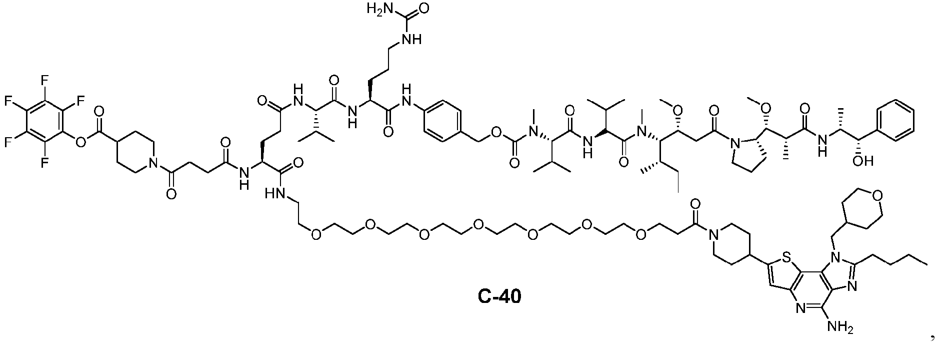 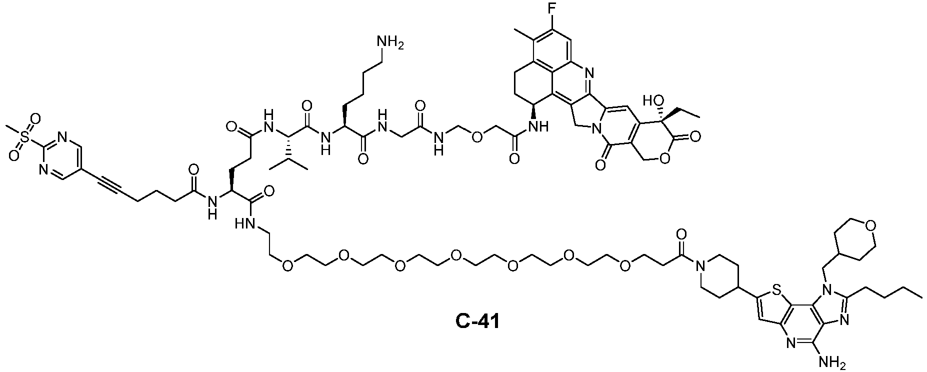 and

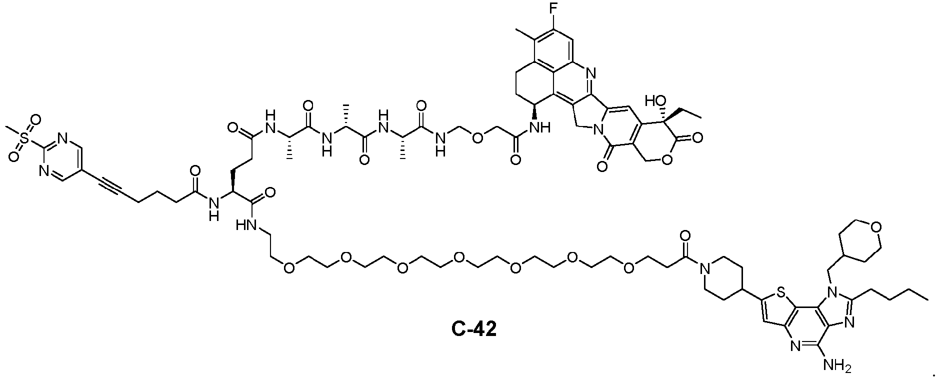

29. A pharmaceutical composition, which comprises the antibody-drug conjugate according to any one of claims 1 to 25, and optionally the compound or pharmaceutically acceptable salt, ester, stereoisomer, tautomer, polymorph, solvate, N-oxide, isotopically labeled compound, metabolite or prodrug thereof according to any one of claims 26 to 28, and one or more pharmaceutical excipients.

30. Use of the compound or pharmaceutically acceptable salt, ester, stereoisomer, tautomer, polymorph, solvate, N-oxide, isotopically labeled compound, metabolite or prodrug thereof according to any one of claims 26 to 28 in the manufacture of an antibody-drug conjugate (e.g., the antibody-drug conjugate of any one of claims 1 to 25).

31. Use of the antibody-drug conjugate according to any one of claims 1 to 25, the compound or pharmaceutically acceptable salt, ester, stereoisomer, tautomer, polymorph, solvate, N-oxide, isotopically labeled compound, metabolite or prodrug thereof according to any one of claims 26 to 28, or the pharmaceutical composition according to claim 29, in the manufacture of a medicament for treating and/or preventing a cancer (e.g., a HER2-positive cancer, such as HER2-positive gastric cancer, breast cancer, or non-small cell lung cancer).

32. The antibody-drug conjugate according to any one of claims 1 to 25, the compound or pharmaceutically acceptable salt, ester, stereoisomer, tautomer, polymorph, solvate, N-oxide, isotopically labeled compound, metabolite or prodrug thereof according to any one of claims 26 to 28, or the pharmaceutical composition according to claim 29, for use in treating and/or preventing a cancer (e.g., a HER2-positive cancer, such as HER2-positive gastric cancer, breast cancer, or non-small cell lung cancer).

33. A method of treating and/or preventing a cancer (e.g., a HER2-positive cancer, such as a HER2-positive gastric cancer, breast cancer, or non-small cell lung cancer), comprising administering to a subject in need thereof an therapeutically and/or prophylactically effective amount of the antibody-drug conjugate according to any one of claims 1 to 25, the compound or pharmaceutically acceptable salt, ester, stereoisomer, tautomer, polymorph, solvate, N-oxide, isotopically labeled compound, metabolite or prodrug thereof according to any one of claims 26 to 28, or the pharmaceutical composition according to claim 29.
